(19) Europäisches Patentamt
European Patent Office
Office européen des brevets

(11) EP 1 392 287 B1

(12) **EUROPEAN PATENT SPECIFICATION**

(45) Date of publication and mention of the grant of the patent:
**22.11.2006 Bulletin 2006/47**

(21) Application number: **02774113.1**

(22) Date of filing: **22.05.2002**

(51) Int Cl.:
***A61K 31/397*** *(2006.01)* ***A61P 25/28*** *(2006.01)*

(86) International application number:
**PCT/US2002/016306**

(87) International publication number:
**WO 2002/096415 (05.12.2002 Gazette 2002/49)**

(54) **USE OF AZETIDINONE SUBSTITUTED DERIVATIVES IN THE TREATMENT OF ALZHEIMER'S DISEASE**

VERWENDUNG VON MIT AZETIDINON SUBSTITUIERTEN DERIVATEN BEI DER BEHANDLUNG DER ALZHEIMER-KRANKHEIT

Usage de dérivés de l'azétidinone pour le traitement de la maladie d' Alzheimer.

(84) Designated Contracting States:
**AT BE CH CY DE DK ES FI FR GB GR IE IT LI LU MC NL PT SE TR**
Designated Extension States:
**AL LT LV MK RO SI**

(30) Priority: **25.05.2001 US 293651 P**
**21.09.2001 US 323911 P**

(43) Date of publication of application:
**03.03.2004 Bulletin 2004/10**

(73) Proprietor: **SCHERING CORPORATION**
**Kenilworth, New Jersey 07033-0530 (US)**

(72) Inventors:
- **DAVIS, Harry, R.**
  **Berkeley Heights, NJ 07922 (US)**
- **PARKER, Eric, McFee**
  **Scotch Plains, NJ 07076 (US)**
- **VAN HEEK, Margaret**
  **Scotch Plains, NJ 07076 (US)**
- **WONG, Gwendolyn, Tse**
  **Lexington, MA 02421 (US)**
- **MERKEL, Laura, B.**
  **Princeton, NJ 08540 (US)**

(74) Representative: **HOFFMANN EITLE**
**Patent- und Rechtsanwälte**
**Arabellastrasse 4**
**81925 München (DE)**

(56) References cited:
**WO-A-95/08532** **WO-A-97/21676**
**WO-A-97/41098** **WO-A-99/38498**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

**Description**

FIELD OF THE INVENTION

The present invention relates to uses of azetidinones for the manufacture of a medicament for treating Alzheimer's Disease, regulating levels of amyloid β (Aβ) peptides and/or regulating the amount of ApoE isoform 4 in the bloodstream and/or brain of a subject by administering a composition comprising an effective amount of at least one of the compounds of Formulae I-X below.

BACKGROUND OF THE INVENTION

Alzheimer's Disease ("AD") is a neurodegenerative brain disease that is a major cause of dementia among the elderly. Symptoms of AD can include progressive loss of learning and memory functions, personality changes, neuromuscular changes, seizures and occasionally psychotic behavior. AD is characterized pathologically by the extracellular accumulation of senile plaques in various brain regions and vascular walls, as well as by the intraneuronal accumulation of neurofibrillary tangles in various brain regions. The main constituents of senile plaques are amyloid β (Aβ) peptides, such as for example 40-42 amino acid Aβ peptides, which are formed by proteolytic processing of the β-amyloid precursor protein (β-APP) by two enzymes known as β-secretase and γ-secretase.

The apolipoprotein E isoform 4 (ApoE isoform 4) is a major genetic risk factor for AD. PCT Patent Application No. WO 95/06470 discloses administration of an HMG-CoA reductase inhibitor (statin) to regulate levels of (ApoE isoform 4) in humans to prevent and treat Alzheimer's Disease.

A normal cellular function of ApoE is uptake and delivery of lipids. The ApoE isoform correlates with an increased risk for atherosclerosis, increased amyloid plaque deposition and increased risk of AD. K. Fassbender et al., "Simvastatin Strongly Reduces Levels of Alzheimer's Disease β-amyloid peptides Aβ42 and Aβ40 *in vitro* and *in vivo",* PNAS 98: 5856-5861 (2001).

PCT Patent Application WO 00/28981 discloses at page 3 that patients possessing the ApoE isoform 4 have an increased risk for AD, as well as elevated levels of cholesterol and increased risk for heart disease.

PCT WO 00/28981 also discloses methods for treating AD using HMG-CoA reductase inhibitors, but notes at page 6 that "it was unexpectedly discovered that while some HGM (sic) CoA reductase inhibitors exhibit a dramatic reduction in degree and prevalence of AD, patients taking other types of drugs used to treat cardiovascular disorders, such as beta blockers, furosemide, and captopril, did not show any significant reduction in the prevalence or degree of AD."

U.S. Patent No. 6,080,778 (col. 1, lines 39-41) points out that known genetic causes of AD can account for only a small proportion of the total number of cases and that most cases of AD are sporadic (i.e., without a known genetic cause) and occur in the elderly. This patent discloses methods for decreasing the production of Aβ peptides by administering a composition which decreases blood cholesterol levels to a person with elevated cholesterol levels who is at risk of, or exhibits symptoms of, Alzheimer's Disease. The disclosed methods include administration of compounds which increase uptake of cholesterol by the liver (e.g., HMG CoA reductase inhibitors), compounds which block endogenous cholesterol production, compounds which prevent uptake of dietary cholesterol (e.g., bile acid binding resins and fibrates), and combinations of any of these which are effective in lowering blood cholesterol levels (see col. 1, line 58 - col. 2, line 5). Fassbender et al. disclose that use of simvastatin and lovastatin, alone or in combination with methyl-β-cyclodextrin, can reduce intracellular and secreted Aβ levels *in vitro* and that treatment of animals with simvastatin reduces brain and cerebrospinal fluid levels of Aβ *in vivo.*

U.S. Patent No. 6,071,899 discloses compounds of Formula (1):

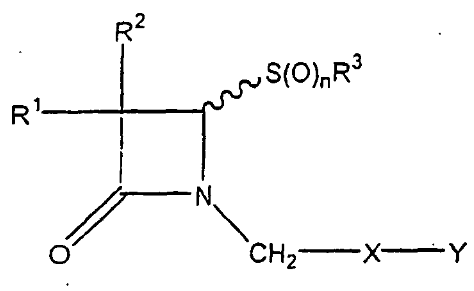

having substituents as defined therein, which may have a general application in any disorder that involves endothelial dysfunction, such as atherosclerosis, or may have a general application in any disorder that involves lipid peroxidation

in conjunction with enzyme activity, including inflammatory conditions of the brain such as Alzheimer's Disease (see col. 5, lines 16-29). Compounds of a similar structure having the same general application are disclosed in WO 97/41098 and WO 97/21676.

PCT Patent Application WO 99/38498 discloses methods for preventing or treating AD by administering a plasma-triglyceride level-lowering agent (e.g., fibrates), optionally in combination with a cholesterol level-lowering agent such as statins, bile acid sequestrants or agents that block intestinal cholesterol absorption (e.g., β-sitosterol, SCH 48461 ((3R,4S)-1,4-bis-(4-methoxyphenyl)-3-(3-phenylpropyl)-2-azetidinone), CP-148,623, saponins, neomycin and ACAT inhibitors).

U.S. Patents Nos. 5,767,115, 5,624,920, 5,688,990, 5,656,624 and 5,688,787, respectively, disclose hydroxy-substituted azetidinone compounds and substituted β-lactam compounds useful for lowering cholesterol and/or in inhibiting the formation of cholesterol-containing lesions in mammalian arterial walls, but does not disclose treatment of Alzheimer's Disease.

Despite recent improvements in the treatment of Alzheimer's disease, there remains a need in the art for improved methods of treatment which are effective and avoid undesirable side effects.

## SUMMARY OF THE INVENTION

In one embodiment, the present invention provides the use of a composition comprising at least one compound represented by Formula (I):

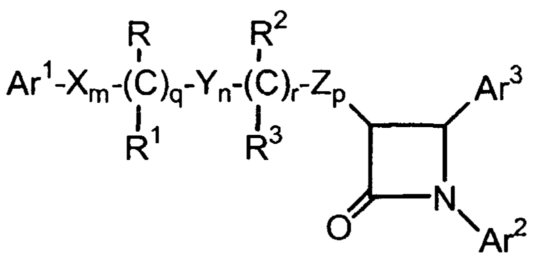

(I)

or a pharmaceutically acceptable salt thereof or solvate thereof,
wherein:

$Ar^1$ and $Ar^2$ are independently selected from the group consisting of aryl and $R^4$-substituted aryl;
$Ar^3$ is aryl or $R^5$-substituted aryl;
X, Y and Z are independently selected from the group consisting of $-CH_2-$, -CH(lower alkyl)- and -C(dilower alkyl)-;
R and $R^2$ are independently selected from the group consisting of $-OR^6$, $-O(CO)R^6$, $-O(CO)OR^9$ and $-O(CO)NR^6R^7$;
$R^1$ and $R^3$ are independently selected from the group consisting of hydrogen, lower alkyl and aryl;
q is 0 or 1;
r is 0 or 1;
m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$, $-CH=CH-COOR^6$, $-CF_3$, -CN, $-NO_2$ and halogen;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^B$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$ and $-CH=CH-COOR^6$;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl for the manufacture of a medicament for treating preventing, or ameliorating symptoms of Alzheimer's Disease in the subject.

In another embodiment, the use of a composition comprising a compound represented by Formula (II) below:

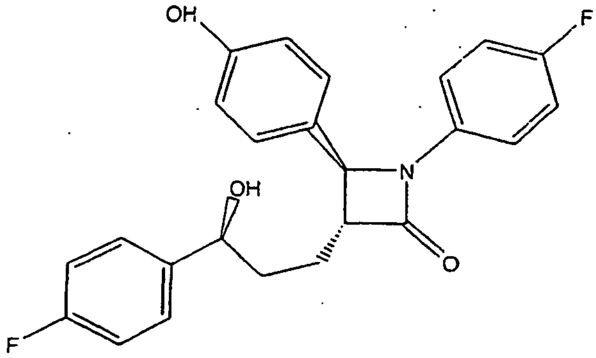

(II)

or a pharmaceutically acceptable salt or solvate of the compound of Formula (II) for the manufacture of a medicament for treating preventing, or ameliorating symptoms of Alzheimer's Disease in the subject is provided

In yet another embodiment, the present invention provides a method of preventing, treating, or ameliorating symptoms of Alzheimer's Disease comprising the step of administering to a subject in need of such treatment an effective amount of a composition comprising at least one compound represented by Formula (III):

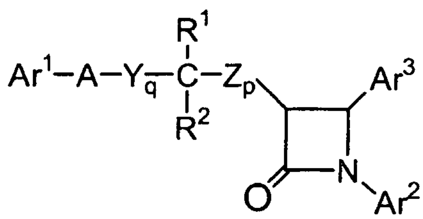

(III)

or a pharmaceutically acceptable salt thereof or solvate thereof, wherein, in Formula (III) above:

$Ar^1$ is $R^3$-substituted aryl;
$Ar^2$ is $R^4$-substituted aryl;
$Ar^3$ is $R^5$-substituted aryl;
Y and Z are independently selected from the group consisting of $-CH_2-$, -CH(lower alkyl)- and -C(dilower alkyl)-;
A is selected from -O-, -S-, -S(O)- or $-S(O)_2-$;
$R^1$ is selected from the group consisting of $-OR^6$, $-O(CO)R^6$, $-O(CO)OR^9$ and $-O(CO)NR^6R^7$; $R^2$ is selected from the group consisting of hydrogen, lower alkyl and aryl; or $R^1$ and $R^2$ together are =O;
q is 1, 2 or 3;
p is 0, 1, 2, 3 or 4;
$R^5$ is 1-3 substituents independently selected from the group consisting of $-OR^6$, $-O(CO)R^6$, $-O(CO)OR^9$, $-O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-lower alkyl, $-NR^6SO_2$-aryl, $-CONR^6R^7$, $-COR^6$, $-SO_2NR^6R^7$, $S(O)_{0-2}$-alkyl, $S(O)_{0-2}$-aryl, $-O(CH_2)_{1-10}-COOR^6$, $-O(CH_2)_{1-10}CONR^6R^7$, o-halogeno, m-halogeno, o-lower alkyl, m-lower alkyl, -(lower alkylene)-$COOR^6$, and $-CH=CH-COOR^6$;
$R^3$ and $R^4$ are independently 1-3 substituents independently selected from the group consisting of $R^5$, hydrogen, p-lower alkyl, aryl, $-NO_2$, $-CF_3$ and p-halogeno;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl to prevent, treat, or ameliorate symptoms of Alzheimer's Disease in the subject.

In yet another embodiment, the present invention provides the use of a composition comprising at least one compound represented by Formula (IV):

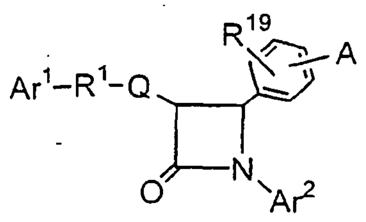

(IV)

or a pharmaceutically acceptable salt thereof or solvate thereof, wherein, in Formula (IV) above:

A is selected from the group consisting of $R^2$-substituted heterocycloalkyl, $R^2$-substituted heteroaryl, $R^2$-substituted benzofused heterocycloalkyl, and $R^2$-substituted benzofused heteroaryl;
$Ar^1$ is aryl or $R^3$-substituted aryl;
$Ar^2$ is aryl or $R^4$-substituted aryl;
Q is a bond or, with the 3-position ring carbon of the azetidinone, forms the spiro group

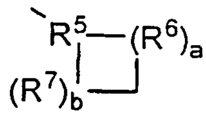 ;

and
$R^1$ is selected from the group consisting of:

- $(CH_2)_q$-, wherein q is 2-6, provided that when Q forms a spiro ring, q can also be zero or 1;
- $(CH_2)_e$-G-$(CH_2)_r$-, wherein G is -O-, -C(O)-, phenylene, $-NR^8-$ or $-S(O)_{0-2}-$, e is 0-5 and r is 0-5, provided that the sum of e and r is 1-6;
- ($C_2$-$C_6$ alkenylene)-; and
- $(CH_2)_f$-V-$(CH_2)_g$-, wherein V is $C_3$-$C_6$ cycloalkylene, f is 1-5 and g is 0-5, provided that the sum of f and g is 1-6;

$R^5$ is selected from:

-CH-, -C($C_1$-$C_6$ alkyl)-, -CF-, -C(OH)-, -C($C_6H_4$-$R^9$)-, -N-, or $-^{+}NO^{-}$ ;

$R^6$ and $R^7$ are independently selected from the group consisting of $-CH_2-$, -CH($C_1$-$C_6$ alkyl)-, -C(di-($C_1$-$C_6$) alkyl), -CH=CH- and -C($C_1$-$C_6$ alkyl)=CH-; or $R^5$ together with an adjacent $R^6$, or $R^5$ together with an adjacent $R^7$, form a -CH=CH- or a -CH=C($C_1$-$C_6$ alkyl)- group;
a and b are independently 0, 1, 2 or 3, provided both are not zero; provided that when $R^6$ is -CH=CH- or -C($C_1$-$C_6$ alkyl)=CH-, a is 1; provided that when $R^7$ is -CH=CH- or -C($C_1$-$C_6$ alkyl)=CH-, b is 1; provided that when a is 2 or 3, the $R^6$'s can be the same or different; and provided that when b is 2 or 3, the $R^7$'s can be the same or different; and when Q is a bond, $R^1$ also can be selected from:

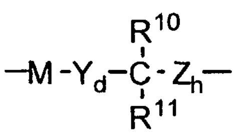 , 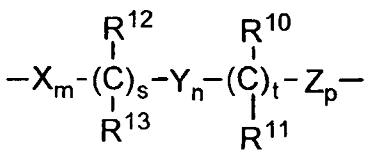 or 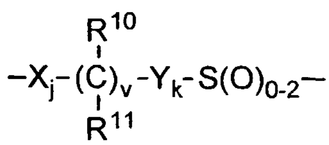 ;

where M is -O-, -S-, -S(O)- or $-S(O)_2-$;
X, Y and Z are independently selected from the group consisting of $-CH_2-$, $-CH(C_1-C_6$ alkyl)- and $-C(di-(C_1-C_6)$ alkyl);
$R^{10}$ and $R^{12}$ are independently selected from the group consisting of $-OR^{14}$, $-O(CO)R^{14}$, $-O(CO)OR^{16}$ and $-O(CO)NR^{14}R^{15}$;
$R^{11}$ and $R^{13}$ are independently selected from the group consisting of hydrogen, $(C_1-C_6)$alkyl and aryl; or $R^{10}$ and $R^{11}$ together are =O, or $R^{12}$ and $R^{13}$ together are =O;
d is 1, 2 or 3;
h is 0, 1, 2, 3 or 4;
s is 0 or 1; t is 0 or 1; m, n and p are independently 0-4; provided that at least one of s and t is 1, and the sum of m, n, p, s and t is 1-6; provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
$R^2$ is 1-3 substituents on the ring carbon atoms selected from the group consisting of hydrogen, $(C_1-C_{10})$alkyl, $(C_2-C_{10})$alkenyl, $(C_2-C_{10})$alkynyl, $(C_3-C_6)$cycloalkyl, $(C_3-C_6)$cycloalkenyl, $R^{17}$-substituted aryl, $R^{17}$-substituted benzyl, R17-substituted benzyloxy, $R^{17}$-substituted aryloxy, halogeno, $-NR^{14}R^{15}$, $NR^{14}R^{15}(C_1-C_6$ alkylene)-, $NR^{14}R^{15}C(O)(C_1-C_6$ alkylene)-,$-NHC(O)R^{16}$, OH, $C_1-C_6$ alkoxy, $-OC(O)R^{16}$, $-COR^{14}$, hydroxy$(C_1-C_6)$alkyl, $(C_1-C_6)$alkoxy$(C_1-C_6)$alkyl, $NO_2$, $-S(O)_{0-2}R^{16}$, $-SO_2NR^{14}R^{15}$ and $-(C_1-C_6$ alkylene)$COOR^{14}$; when $R^2$ is a substituent on a heterocycloalkyl ring, $R^2$ is as defined, or is =O or

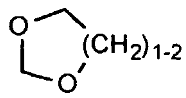
;

and, where $R^2$ is a substituent on a substitutable ring nitrogen, it is hydrogen, $(C_1-C_6)$alkyl, aryl, $(C_1-C_6)$alkoxy, aryloxy, $(C_1-C_6)$alkylcarbonyl, arylcarbonyl, hydroxy, $-(CH_2)_{1-6}CONR^{18}R^{18}$,

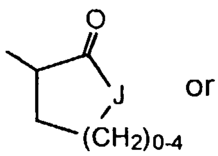
or
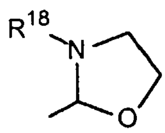
;

wherein J is -O-, -NH-, $-NR^{18}-$ or $-CH_2-$;
$R^3$ and $R^4$ are independently selected from the group consisting of 1-3 substituents independently selected from the group consisting of $(C_1-C_6)$alkyl, $-OR^{14}$, $-O(CO)R^{14}$, $-O(CO)OR^{16}$, $-O(CH_2)_{1-5}OR^{14}$, $-O(CO)NR^{14}R^{15}$, $-NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $S(O)_{0-2}R^{16}$, $-O(CH_2)_{1-10}-COOR^{14}$, $-O(CH_2)_{1-10}CONR^{14}R^{15}$, $-(C_1-C_6$ alkylene)-$COOR^{14}$, $-CH=CH-COOR^{14}$, $-CF_3$, -CN, $-NO_2$ and halogen;
$R^8$ is hydrogen, $(C_1-C_6)$alkyl, aryl $(C_1-C_6)$alkyl, $-C(O)R^{14}$ or $-COOR^{14}$;
$R^9$ and $R^{17}$ are independently 1-3 groups independently selected from the group consisting of hydrogen, $(C_1-C_6)$alkyl, $(C_1-C_6)$alkoxy, -COOH, $NO_2$, $-NR^{14}R^{15}$, OH and halogeno;
$R^{14}$ and $R^{15}$ are independently selected from the group consisting of hydrogen, $(C_1-C_6)$alkyl, aryl and aryl-substituted $(C_1-C_6)$alkyl;
$R^{16}$ is $(C_1-C_6)$alkyl, aryl or $R^{17}$-substituted aryl;
$R^{18}$ is hydrogen or $(C_1-C_6)$alkyl; and
$R^{19}$ is hydrogen, hydroxy or $(C_1-C_6)$alkoxy,

for the manufacture of a medicament for treating preventing, or ameliorating symptoms of Alzheimer's Disease in the subject.

In another embodiment, the present invention provides a method of preventing, treating, or ameliorating symptoms of Alzheimer's Disease comprising the step of administering to a subject in need of such treatment an effective amount of a composition comprising at least one compound represented by Formula (V):

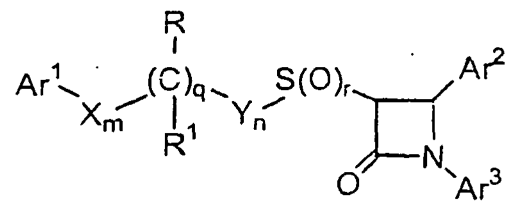

(V)

or a pharmaceutically acceptable salt thereof or solvate thereof, wherein, in Formula (V) above:

$Ar^1$ is aryl, $R^{10}$-substituted aryl or heteroaryl;
$Ar^2$ is aryl or $R^4$-substituted aryl;
$Ar^3$ is aryl or $R^5$-substituted aryl;
X and Y are independently selected from the group consisting of $-CH_2-$, -CH(lower alkyl)- and -C(dilower alkyl)-;
R is $-OR^6$, $-O(CO)R^6$, $-O(CO)OR^9$ or $-O(CO)NR^6R^7$; $R^1$ is hydrogen, lower alkyl or aryl; or R and $R^1$ together are =O;
q is 0 or 1;
r is 0, 1 or 2;
m and n are independently 0, 1, 2. 3, 4 or 5; provided that the sum of m, n and q is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$. $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$ and $-CH=CH-COOR^6$;
$R^5$ is 1-5 substituents independently selected from the group consisting of $-OR^6$, $-O(CO)R^6$, $-O(CO)OR^9$, $=O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, $-CF_3$, -CN, $-NO_2$, halogen, -(lower alkylene)$COOR^6$ and $-CH=CH-COOR^6$;
$R^6$, $R^7$ and $R^8$ are independently selected from the group consisting of hydrogen, lower alkyl, aryl and aryl-substituted lower alkyl;
$R^9$ is lower alkyl, aryl or aryl-substituted lower alkyl; and
$R^{10}$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}-COOR^6$, $-O(CH_2)_{1-10}CONR^6R^7$, $-CF_3$, -CN, $-NO_2$ and halogen to prevent, treat, or ameliorate symptoms of Alzheimer's Disease in the subject.

In yet another embodiment, the present invention provides the use of a composition comprising at least one compound represented by Formula (VI):

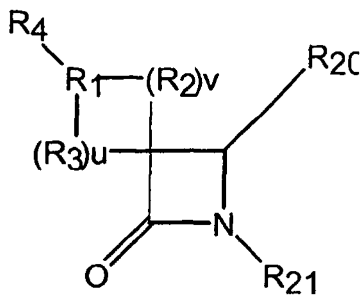

(VI)

or a pharmaceutically acceptable salt thereof or solvate thereof, wherein:

$R_1$ is

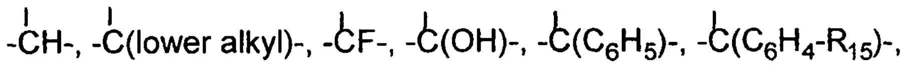

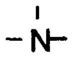 or 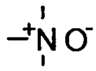 ;

$R_2$ and $R_3$ are independently selected from the group consisting of: $-CH_2-$, -CH(lower alkyl)-, -C(di-lower alkyl)-, -CH=CH- and -C(lower alkyl)=CH-; or $R_1$ together with an adjacent $R_2$, or $R_1$ together with an adjacent $R_3$, form a -CH=CH- or a -CH=C(lower alkyl)- group;

u and v are independently 0, 1, 2 or 3, provided both are not zero; provided that when $R_2$ is -CH=CH- or -C(lower alkyl)=CH-, v is 1; provided that when $R_3$ is -CH=CH- or -C(lower alkyl)=CH-, u is 1; provided that when v is 2 or 3, the $R_2$'s can be the same or different; and provided that when u is 2 or 3, the $R_3$'s can be the same or different;

$R_4$ is selected from $B-(CH_2)_mC(O)-$, wherein m is 0, 1, 2, 3, 4 or 5; $B-(CH_2)q-$, wherein q is 0, 1, 2, 3, 4, 5 or 6;

$B-(CH_2)_e-Z-(CH_2)_r-$, wherein Z is -O-, -C(O)-, phenylene, $-N(R_8)-$ or $-S(O)_{0-2}-$, e is 0, 1, 2, 3, 4 or 5 and r is 0, 1, 2, 3, 4 or 5, provided that the sum of e and r is 0, 1, 2, 3, 4, 5 or 6;

$B-(C_2-C_6$ alkenylene)-;

$B-(C_4-C_6$ alkadienylene)-;

$B-(CH_2)_t-Z-(C_2-C_6$ alkenylene)-, wherein Z is as defined above, and wherein t is 0, 1, 2 or 3, provided that the sum of t and the number of carbon atoms in the alkenylene chain is 2, 3, 4, 5 or 6;

$B-(CH_2)_f-V-(CH_2)_g-$, wherein V is $C_3-C_6$ cycloalkylene, f is 1, 2, 3, 4 or 5 and g is 0, 1, 2, 3, 4 or 5, provided that the sum of f and g is 1, 2, 3, 4, 5 or 6;

$B-(CH_2)_t-V-(C_2-C_6$ alkenylene)- or $B-(C_2-C_6$ alkenylene)-$V-(CH_2)t-$, wherein V and t are as defined above, provided that the sum of t and the number of carbon atoms in the alkenylene chain is 2, 3, 4, 5 or 6; $B-(CH_2)_a-Z-(CH_2)_b-V-(CH_2)_d-$, wherein Z and V are as defined above and a, b and d are independently 0, 1, 2, 3, 4, 5 or 6, provided that the sum of a, b and d is 0, 1, 2, 3, 4, 5 or 6; or

$T-(CH_2)_s-$, wherein T is cycloalkyl of 3-6 carbon atoms and s is 0, 1, 2, 3, 4, 5 or 6; or

$R_1$ and $R_4$ together form the group

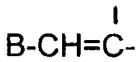 ;

B is selected from indanyl, indenyl, naphthyl, tetrahydronaphthyl, heteroaryl or W-substituted heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, or

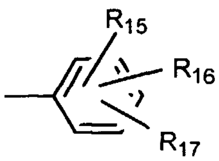

W is 1 to 3 substituents independently selected from the group consisting of lower alkyl, hydroxy lower alkyl, lower alkoxy, alkoxyalkyl, alkoxyalkoxy, alkoxycarbonylalkoxy, (lower alkoxyimino)-lower alkyl, lower alkanedioyl, lower alkyl lower alkanedioyl, allyloxy, $-CF_3$, $-OCF_3$, benzyl, $R_7$-benzyl, benzyloxy, $R_7$-benzyloxy, phenoxy, $R_7$-phenoxy, dioxolanyl, $NO_2$, $-N(R_8)(R_9)$, $N(R_8)(R_9)$-lower alkylene-, $N(R_8)(R_9)$-lower alkylenyloxy-, OH, halogeno, -CN, $-N_3$, $-NHC(O)OR_{10}$, $-NHC(O)R_{10}$, $R_{11}O_2SNH-$, $(R_{11}O_2S)_2N-$, $-S(O)_2NH_2$, $-S(O)_{0-2}R_8$, tert-butyldimethyl-silyloxymethyl, $-C(O)R_{12}$, $-COOR_{19}$, $-CON(R_8)(R_9)$, $-CH=CHC(O)R_{12}$, -lower alkylene-$C(O)R_{12}$, $R_{10}C(O)$(lower alkylenyloxy)-, N $(R_8)(R_9)C(O)$(lower alkylenyloxy)- and

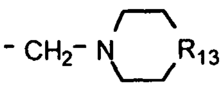

for substitution on ring carbon atoms, and the substituents on the substituted heteroaryl ring nitrogen atoms, when present, are selected from the group consisting of lower alkyl, lower alkoxy, $-C(O)OR_{10}$, $-C(O)R_{10}$, OH, $N(R_8)(R_9)$-lower alkylene-, $N(R_8)(R_9)$-lower alkylenyloxy-, $-S(O)_2NH_2$ and 2-(trimethylsilyl)-ethoxymethyl;

$R_7$ is 1-3 groups independently selected from the group consisting of lower alkyl, lower alkoxy, -COOH, $NO_2$, $-N(R_8)(R_9)$, OH, and halogeno;

$R_8$ and Rg are independently selected from H or lower alkyl;

$R_{10}$ is selected from lower alkyl, phenyl, $R_7$-phenyl, benzyl or $R_7$-benzyl;

$R_{11}$ is selected from OH, lower alkyl, phenyl, benzyl, $R_7$-phenyl or $R_7$-benzyl;

$R_{12}$ is selected from H, OH, alkoxy, phenoxy, benzyloxy,

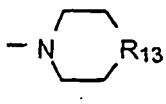

,

$-N(R_8)(R_9)$, lower alkyl, phenyl or $R_7$-phenyl;

$R_{13}$ is selected from -O-, $-CH_2-$, -NH-, -N(lower alkyl)- or $-NC(O)R_{19}$;

$R_{15}$, $R_{16}$ and $R_{17}$ are independently selected from the group consisting of H and the groups defined for W; or $R_{15}$ is hydrogen and $R_{16}$ and $R_{17}$, together with adjacent carbon atoms to which they are attached, form a dioxoiany! ring;

$R_{19}$ is H, lower alkyl, phenyl or phenyl lower alkyl; and

$R_{20}$ and $R_{21}$ are independently selected from the group consisting of phenyl, W-substituted phenyl, naphthyl, W-substituted naphthyl, indanyl, indenyl, tetrahydronaphthyl, benzodioxolyl, heteroaryl, W-substituted heteroaryl, benzofused heteroaryl, W-substituted benzofused heteroaryl and cyclopropyl, wherein heteroaryl is as defined above,

for the manufacture of a medicament for treating preventing, or ameliorating symptoms of Alzheimer's Disease in the subject.

In yet another embodiment, the present invention provides the use of a composition comprising at least one compound represented by Formula (VII):

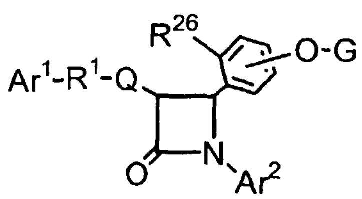

(VII)

or a pharmaceutically acceptable salt thereof or solvate thereof, wherein, in Formula (VII) above,

$R^{26}$ is H or $OG^1$;

G and $G^1$ are independently selected from the group consisting of H,

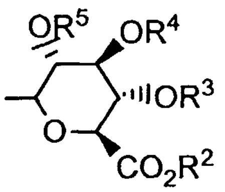

,

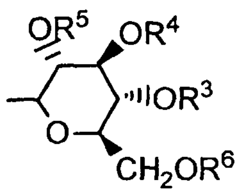

,

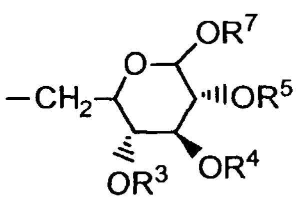

and

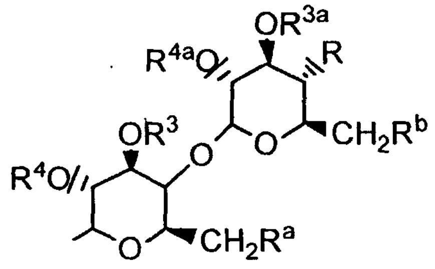 ;

provided that when $R^{26}$ is H or OH, G is not H;

R, $R^a$ and $R^b$ are independently selected from the group consisting of H, -OH, halogeno, $-NH_2$, azido, $(C_1-C_6)$alkoxy $(C_1-C_6)$-alkoxy or $-W-R^{30}$;

W is independently selected from the group consisting of -NH-C(O)-, -O-C(O)-, $-O-C(O)-N(R^{31})-$, $-NH-C(O)-N(R^{31})-$ and $-O-C(S)-N(R^{31})-$;

$R^2$ and $R^6$ are independently selected from the group consisting of H, $(C_{1-}C_6)$alkyl, aryl and aryl$(C_1-C_6)$alkyl;

$R^3$, $R^4$, $R^5$, $R^7$, $R^{3a}$ and $R^{4a}$ are independently selected from the group consisting of H, $(C_1-C_6)$alkyl, aryl$(C_1-C_6)$alkyl, $-C(O)(C_1-C_6)$alkyl and -C(O)aryl;

$R^{30}$ is selected from the group consisting of $R^{32}$-substituted T, $R^{32}$-substituted-T-$(C_1-C_6)$alkyl, $R^{32}$-substituted-$(C_2-C_4)$alkenyl, $R^{32}$-substituted-$(C_1-C_6)$alkyl, $R^{32}$-substituted-$(C_3$-C7)cycloalkyl and $R^{32}$-substituted-$(C_3$-C7)cycloalkyl$(C_1-C_6)$alkyl;

$R^{31}$ is selected from the group consisting of H and $(C_1-C_4)$alkyl;

T is selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;

$R^{32}$ is independently selected from 1-3 substituents independently selected from the group consisting of halogeno, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsulfinyl, $(C_1-C_4)$alkylsulfonyl, $-N(CH_3)_2$, $-C(O)-NH(C_1-C_4)$alkyl, $-C(O)-N((C_1-C_4)alkyl)_2$, $-C(O)-(C_1-C_4)$alkyl, $-C(O)-(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$alkokycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^{11}$-substituted aryl;

Q is a bond or, with the 3-position ring carbon of the azetidinone, forms the spiro group

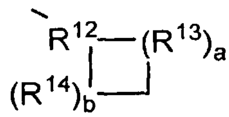 ;

and

$R^1$ is selected from the group consisting of

$-(CH_2)q-$, wherein q is 2-6, provided that when Q forms a spiro ring, q can also be zero or 1;

$-(CH_2)e-E-(CH_2)_r-$, wherein E is -O-, -C(O)-, phenylene, $-NR^{22}-$ or $-S(O)_{0-2}-$, e is 0-5 and r is 0-5, provided that the sum of e and r is 1-6;

$-(C_2-C_6)$alkenylene-; and

$-(CH_2)_f-V-(CH_2)_g-$, wherein V is $C_3-C_6$ cycloalkylene, f is 1-5 and g is 0-5, provided that the sum of f and g is 1-6;

$R^{12}$ is

$$-\overset{|}{C}H-,\ -\overset{|}{C}(C_1-C_6\ alkyl)-,\ -\overset{|}{C}F-,\ -\overset{|}{C}(OH)-,\ -\overset{|}{C}(C_6H_4-R^{23})-,\ -\overset{|}{N}-,\ \text{or}\ -{}^{+}\underset{|}{\overset{|}{N}}O^{-}\ ;$$

$R^{13}$ and $R^{14}$ are independently selected from the group consisting of $-CH_2-$, $-CH(C_1-C_6$ alkyl$)-$, $-C(di-(C_1-C_6)$ alkyl), -CH=CH- and $-C(C_1-C_6$ alkyl)=CH-; or $R^{12}$ together with an adjacent $R^{13}$, or $R^{12}$ together with an adjacent $R^{14}$, form

a $-CH=CH-$ or a $-CH=C(C_1-C_6$ alkyl)- group;
a and b are independently 0, 1, 2 or 3, provided both are not zero;
provided that when $R^{13}$ is $-CH=CH-$ or $-C(C_1-C_6$ alkyl)=CH-, a is 1;
provided that when $R^{14}$ is $-CH=CH-$ or $-C(C_1-C_6$ alkyl)=CH-, b is 1;
provided that when a is 2 or 3, the $R^{13}$'s can be the same or different; and
provided that when b is 2 or 3, the $R^{14}$'s can be the same or different;
and when Q is a bond, $R^1$ also can be:

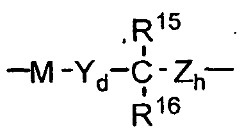
,
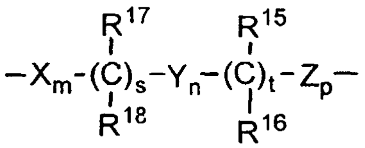
or
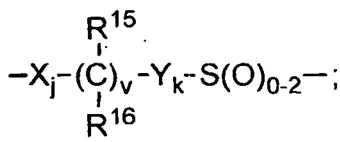
;

M is -O-, -S-, -S(O)- or $-S(O)_2-$;
X, Y and Z are independently selected from the group consisting of $-CH_2-$, $-CH(C_1-C_6)$alkyl- and $-C(di-(C_1-C_6)alkyl)$;
$R^{10}$ and $R^{11}$ are independently selected from the group consisting of 1-3 substituents independently selected from the group consisting of $(C_1-C_6)$alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$ alkylene)-$COOR^{19}$, $-CH=CH-COOR^{19}$, $-CF_3$, -CN, $-NO_2$ and halogen;
$R^{15}$ and $R^{17}$ are independently selected from the group consisting of $-OR^{19}$, $-O(CO)R^{19}$, $-O(CO)OR^{21}$ and $-O(CO)NR^{19}R^{20}$;
$R^{16}$ and $R^{18}$ are independently selected from the group consisting of H, $(C_1-C_6)$alkyl and aryl; or $R^{15}$ and $R^{16}$ together are =O, or $R^{17}$ and $R^{18}$ together are =O;
d is 1, 2 or 3;
h is 0, 1, 2, 3 or 4;
s is 0 or 1; t is 0 or 1; m, n and p are independently 0-4;
provided that at least one of s and t is 1, and the sum of m, n, p, s and t is 1-6;
provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
and when Q is a bond and $R^1$ is

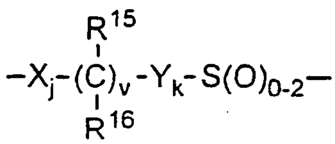
,

$Ar^1$ can also be pyridyl, isoxazolyl, furanyl, pyrrolyl, thienyl, imidazolyl, pyrazolyl, thiazolyl, pyrazinyl, pyrimidinyl or pyridazinyl;
$R^{19}$ and $R^{20}$ are independently selected from the group consisting of H, $(C_1-C_6)$alkyl, aryl and aryl-substituted $(C_1-C_6)$ alkyl;
$R^{21}$ is $(C_1-C_6)$alkyl, aryl or $R^{24}$-substituted aryl;
$R^{22}$ is H, $(C_1-C_6)$alkyl, aryl $(C_1-C_6)$alkyl, $-C(O)R^{19}$ or $-COOR^{19}$;
$R^{23}$ and $R^{24}$ are independently 1-3 groups independently selected from the group consisting of H, $(C_1-C_6)$alkyl, $(C_1-C_6)$alkoxy, -COOH, $NO_2$, $-NR^{19}R^{20}$, -OH and halogeno; and
$R^{25}$ is H, -OH or $(C_1-C_6)$alkoxy,

for the manufacture of a medicament for treating preventing, or ameliorating symptoms of Alzheimer's Disease in the subject.

In yet another embodiment, the present invention provides the use of a composition comprising at least one compound represented by Formula (VIII):

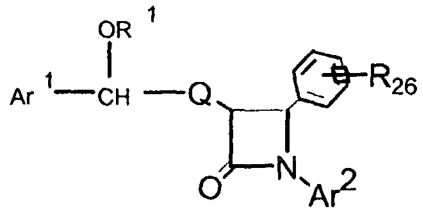

(VIII)

or a pharmaceutically acceptable salt or solvate thereof, wherein

$R^{26}$ is selected from the group consisting of:

a) OH;
b) $OCH_3$;
c) fluorine and
d) chlorine.

$R^1$ is selected from the group consisting of H,

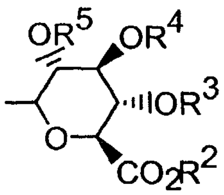 , 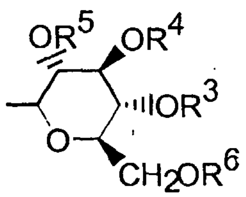 , 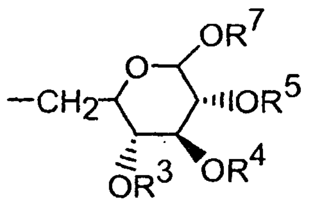 ,

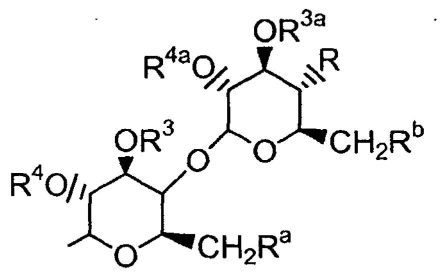 ,

$-SO_3H$; natural and unnatural amino acids.

R, $R^a$ and $R^b$ are independently selected from the group consisting of H, -OH, halogeno, $-NH_2$, azido, $(C_1-C_6)$alkoxy $(C_1-C_6)$-alkoxy and $-W-R^{30}$;

W is independently selected from the group consisting of -NH-C(O)-, -O-C(O)-, $-O-C(O)-N(R^{31})-$, $-NH-C(O)-N(R^{31})-$ and $-O-C(S)-N(R^{31})-$;

$R^2$ and $R^6$ are independently selected from the group consisting of H, $(C_1-C_6)$alkyl, aryl and aryl$(C_1-C_6)$alkyl;

$R^3$, $R^4$, $R^5$, $R^7$, $R^{3a}$ and $R^{4a}$ are independently selected from the group consisting of H, $(C_1-C_6)$alkyl, aryl$(C_1-C_6)$ alkyl, $-C(O)(C_1-C_6)$alkyl and -C(O)aryl;

$R^{30}$ is independently selected form the group consisting of $R^{32}$-substituted T, $R^{32}$-substituted-T-$(C_1-C_6)$alkyl, $R^{32}$-substituted-$(C_2-C_4)$alkenyl, $R^{32}$-substituted-$(C_1-C_6)$alkyl, $R^{32}$-substituted-$(C_3-C_7)$cycloalkyl and $R^{32}$-substituted-$(C_3-C_7)$cycloalkyl$(C_1-C_6)$alkyl;

$R^{31}$ is independently selected from the group consisting of H and $(C_1-C_4)$alkyl;

T is independently selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;

$R^{32}$ is independently selected from 1-3 substituents independently selected from the group consisting of H, halogeno, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkyl-

sulfinyl, ($C_1$-$C_4$)alkylsulfonyl, -N($CH_3$)$_2$, -C(O)-NH($C_1$-$C_4$)alkyl, -C(O)-N(($C_1$-$C_4$)alkyl)$_2$, -C(O)-($C_1$-$C_4$)alkyl, -C (O)-($C_1$-$C_4$)alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a ($C_1$-$C_4$)alkoxycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^{11}$-substituted aryl;

Q is -($CH_2$)q-, wherein q is 2-6, or, with the 3-position ring carbon of the azetidinone, forms the spiro group

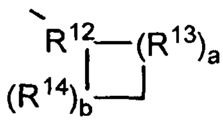 ;

$R^{12}$ is

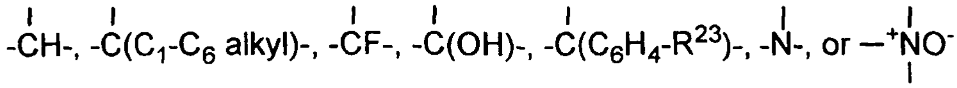 ;

$R^{13}$ and $R^{14}$ are independently selected from the group consisting of -$CH_2$-, -CH($C_1$-$C_6$ alkyl)-, -C(di-($C_1$-$C_6$) alkyl), -CH=CH- and -C($C_1$-$C_6$ alkyl)=CH-; or $R^{12}$ together with an adjacent $R^{13}$, or $R^{12}$ together with an adjacent $R^{14}$, form a -CH=CH- or a -CH=C($C_1$-$C_6$ alkyl)- group;

a and b are independently 0, 1, 2 or 3, provided both are not zero; provided that when $R^{13}$ is -CH=CH- or -C($C_1$-$C_6$ alkyl)=CH-, a is 1; provided that when $R^{14}$ is -CH=CH- or -C($C_1$-$C_6$ alkyl)=CH-, b is 1; provided that when a is 2 or 3, the $R^{13}$'s can be the same or different; and provided that when b is 2 or 3, the $R^{14}$'s can be the same or different;

$R^{10}$ and $R^{11}$ are independently selected from the group consisting of 1-3 substituents independently selected from the group consisting of ($C_1$-$C_6$)alkyl, -$OR^{19}$, -O(CO)$R^{19}$, -O(CO)$OR^{21}$, -O($CH_2$)$_{1-5}$$OR^{19}$, -O(CO)$NR^{19}R^{20}$, -$NR^{19}R^{20}$, -$NR^{19}$(CO)$R^{20}$, -$NR^{19}$(CO)$OR^{21}$, -$NR^{19}$(CO)$NR^{20}R^{25}$, -$NR^{19}SO_2R^{21}$, -$COOR^{19}$, -$CONR^{19}R^{20}$, -$COR^{19}$, -$SO_2NR^{19}R^{20}$, S(O)$_{0-2}$$R^{21}$, -O($CH_2$)$_{1-10}$-$COOR^{19}$, -O($CH_2$)$_{1-10}$$CONR^{19}R^{20}$, -($C_1$-$C_6$ alkylene)-$COOR^{19}$, -CH=CH-$COOR^{19}$, -$CF_3$, -CN, -$NO_2$ and halogen;

$R^{19}$ and $R^{20}$ are independently selected from the group consisting of H, ($C_1$-$C_6$)alkyl, aryl and aryl-substituted ($C_1$-$C_6$) alkyl;

$R^{21}$ is ($C_1$-$C_6$)alkyl, aryl or $R^{24}$-substituted aryl;

$R^{22}$ is H, ($C_1$-$C_6$)alkyl, aryl ($C_1$-$C_6$)alkyl, -C(O)$R^{19}$ or -$COOR^{19}$;

$R^{23}$ and $R^{24}$ are independently 1-3 groups independently selected from the group consisting of H, ($C_1$-$C_6$)alkyl, ($C_1$-$C_6$)alkoxy, -COOH, $NO_2$, -$NR^{19}R^{20}$, -OH and halogeno; and

$R^{25}$ is H, -OH or ($C_1$-$C_6$)alkoxy,

for the manufacture of a medicament for treating preventing, or ameliorating symptoms of Alzheimer's Disease in the subject.

In yet another embodiment, the present invention provides the use of a composition comprising at least one compound represented by Formulae (I-X), or a pharmaceutically acceptable salt or solvate thereof, to regulate the production of amyloid β peptide in the subject.

In yet another embodiment, the present invention provides the use of a composition comprising at least one compound represented by Formulae (I-X), or a pharmaceutically acceptable salt or solvate thereof, for the manufacture of a medicament for regulating a level of one or more amyloid β peptides in the bloodstream and/or the brain of the subject.

In yet another embodiment, the present invention provides the use of a composition comprising at least one compound represented by Formulae (I-X), for the manufacture of a medicament for regulating the amount of ApoE. isoform 4 in the bloodstream and/or brain of the subject.

Other than in the operating examples, or where otherwise indicated, all numbers expressing quantities of ingredients, reaction conditions, and so forth used in the specification and claims are to be understood as being modified in all instances by the term "about."

DETAILED DESCRIPTION

In one embodiment, the present invention provides uses of a composition comprising at least one compound represented by Formulae (I-X) described below for the manufacture of a medicament for treating preventing, or ameliorating, the symptoms of AD in the subject.

In another embodiment, the present invention provides uses of a composition comprising at least one compound represented by Formulae (I-X) described below for the manufacture of a medicament for regulating levels of one or more amyloid β peptides in the bloodstream and/or the brain of the subject.

In another embodiment, present invention provides uses of a composition comprising at least one compound represented by Formulae (I-X) described below for the manufacture of a medicament for regulating levels of ApoE isoform 4 in the bloodstream and/or the brain of the subject.

As discussed above, AD is the most common cause of dementia in the elderly and can be pathologically characterized by the accumulation of senile plaques comprising amyloid β peptides in the extracellular space of various brain regions and in vascular walls. As used herein, "amyloid β peptide" means Aβ peptides which are derived from the larger β-amyloid precursor protein (βAPP) through the endopeptidase action of β and γ secretases. Non-limiting examples of such Aβ peptides include those that contain 40 or 42 amino acids.

In familial forms of AD, the pathological appearance of Aβ peptides in the brain Is driven by the presence of mutations in the βAPP gene or in the genes coding for the proteins presenilin 1 and 2. The Apolipoprotein E type 4 allele (which encodes the protein ApoE isoform 4) is genetically associated with common late onset sporadic forms of AD.

The term "effective amount" means that amount or dosage of a compound or a combination of compounds represented by Formulae (I-X) described below, that will elicit a biological or medical response of a tissue, system or subject that is being sought by the administrator (such as a researcher or doctor) which includes amelioration or alleviation of one or more of the symptoms of the condition or disease (such as Alzheimer's Disease, regulating production of or regulating or reducing levels of one or more amyloid β peptides and/or regulating or reducing levels of ApoE isoform 4 in the bloodstream and/or the brain) being treated and/or the prevention, slowing or halting of progression of the condition. As used herein, the phrase "ameliorating the symptoms of AD" means alleviating, reducing or eliminating one or more of the symptoms experienced by a subject afflicted with AD, including one or more of the following symptoms: progressive loss of learning and memory functions, personality changes, neuromuscular changes, seizures and psychotic behavior.

Examples of suitable dosages are discussed in detail below.

Examples of suitable subjects that can be treated according to the methods of the present invention include mammals, such as humans or dogs, and other animals.

As used herein, "combination therapy" or "therapeutic combination" means the administration of two or more compounds represented by Formulae (I-X) or administration of one or more compounds represented by Formulae (I-X) with cholesterol biosynthesis inhibitors and/or lipid-lowering agents and/or other Alzheimer's disease treatments different from compounds represented by Formulae (I-X) discussed below, to prevent or treat Alzheimer's Disease, reduce levels of one or more amyloid β peptides, regulate production of amyloid β peptides and/or regulate levels of ApoE isoform 4 in the bloodstream and/or the brain. Such administration includes coadministration of these therapeutic agents in a substantially simultaneous manner, such as in a single tablet or capsule having a fixed ratio of active ingredients or in multiple, separate capsules for each therapeutic agent. Also, such administration includes use of each type of therapeutic agent in a sequential manner. In either case, the treatment using the combination therapy will provide beneficial effects in treating the condition.

A potential advantage of the combination therapy disclosed herein may be a reduction in the required amount of an individual therapeutic compound or the overall total amount of therapeutic compounds that are effective in treating the condition. By using a combination of therapeutic agents, the side effects of the individual compounds can be reduced as compared to a monotherapy, which can improve subject compliance. Also, therapeutic agents can be selected to provide a broader range of complimentary effects or complimentary modes of action.

In one embodiment, the present invention provides uses, of a composition comprising one or more compounds represented by Formula (I) below:

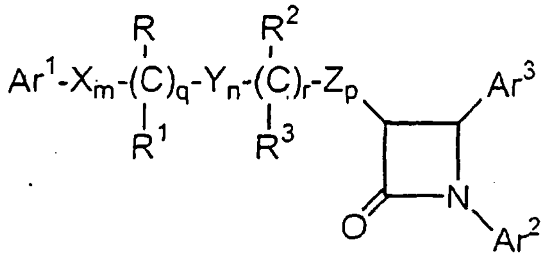

(I)

or a pharmaceutically acceptable salt thereof or solvate thereof for the manufacture of a medicament. In Formula (I) above:

$Ar^1$ and $Ar^2$ are independently selected from the group consisting of aryl and $R^4$ -substituted aryl;
$Ar^3$ is aryl or $R^5$-substituted-aryl;
X, Y and Z are independently selected from the group consisting of $-CH_2-$, -CH(lower alkyl)- and -C(dilower alkyl)-;
R and $R^2$ are independently selected from the group consisting of $-OR^6$, $-O(CO)R^6$, $-O(CO)OR^9$ and $-O(CO)NR^6R^7$;
$R^1$ and $R^3$ are independently selected from the group consisting of hydrogen, lower alkyl and aryl;
q is 0 or 1; r is 0 or 1; m. n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$, $-CH=CH-COOR^6$, $-CF_3$, -CN, $-NO_2$ and halogen;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$,- (lower alkylene)$COOR^6$ and $-CH=CH-COOR^6$;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl.

Preferably, $R^4$ is 1-3 independently selected substituents, and $R^5$ is preferably 1-3 independently selected substituents.

As used herein, the term "alkyl" or "lower alkyl" means straight or branched alkyl chains having from 1 to 6 carbon atoms and "alkoxy" means alkoxy groups having 1 to 6 carbon atoms. Non-limiting examples of lower alkyl groups include, for example methyl, ethyl, propyl, and butyl groups.

"Alkenyl" means straight or branched carbon chains having one or more double bonds in the chain, conjugated or unconjugated. Similarly, "alkynyl" means straight or branched carbon chains having one or more triple bonds in the chain. Where an alkyl, alkenyl or alkynyl chain joins two other variables and is therefore bivalent, the terms alkylene, alkenylene and alkynylene are used.

"Cycloalkyl" means a saturated carbon ring of 3 to 6 carbon atoms, while "cycloalkylene" refers to a corresponding bivalent ring, wherein the points of attachment to other groups include all positional isomers.

"Halogeno" refers to fluorine, chlorine, bromine or iodine radicals.

"Aryl" means phenyl, naphthyl, indenyl, tetrahydronaphthyl or indanyl.

"Phenylene" means a bivalent phenyl group, including ortho, meta and para-substitution.

The statements wherein, for example, R, $R^1$, $R^2$ and $R^3$, are said to be independently selected from a group of substituents, mean that R, $R^1$, $R^2$ and $R^3$ are independently selected, but also that where an R, $R^1$, $R^2$ and $R^3$ variable occurs more than once in a molecule, each occurrence is independently selected (e.g., if R is $-OR^6$, wherein $R^6$ is hydrogen, $R^2$ can be $-OR^6$ wherein $R^6$ is lower alkyl). Those skilled in the art will recognize that the size and nature of the substituent(s) will affect the number of substituents that can be present.

Compounds used in the invention have at least one asymmetrical carbon atom and therefore all isomers, including enantiomers, stereoisomers, rotamers, tautomers and racemates of the compounds of Formula (I-X) (where they exist) are contemplated as being part of this invention. The invention includes d and l isomers in both pure form and in admixture, including racemic mixtures. Isomers can be prepared using conventional techniques, either by reacting optically pure or optically enriched starting materials or by separating isomers of a compound of the Formulae I-X.

Isomers may also include geometric isomers, e.g., when a double bond is present.

Those skilled in the art will appreciate that for some of the compounds of the Formulas I-X, one isomer will show greater pharmacological activity than other isomers.

Compounds used in the invention with an amino group can form pharmaceutically acceptable salts with organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salt is prepared by contacting the free base form with a sufficient amount of the desired acid to produce a salt. The free base form may be regenerated by treating the salt with a suitable dilute aqueous base solution such as dilute aqueous sodium bicarbonate. The free base form differs from its respective salt form somewhat in certain physical properties, such as solubility in polar solvents, but the salt is otherwise equivalent to its respective free base forms for purposes of the invention.

Certain compounds used in the invention are acidic (e.g., those compounds which possess a carboxyl group). These compounds form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, N-methylglucamine and the like.

As used herein, "solvate" means a molecular or ionic complex of molecules or ions of solvent with those of solute (for example, one or more compounds of Formulae I-X, isomers of the compounds of Formulae I-X, or prodrugs of the compounds of Formulae I-X). Non-limiting examples of useful solvents include polar, protic solvents such as water and/or alcohols (for example methanol).

Prodrugs of the compounds of Formulae I-X are contemplated as being part of this invention. As used herein, "prodrug" means compounds that are drug precursors which, following administration to a subject, release the drug *in vivo* via some chemical or physiological process (e.g., a prodrug on being brought to the physiological pH or through enzyme action is converted to the desired drug form).

Preferred compounds of Formula (I) are those in which $Ar^1$ is phenyl or $R^4$-substituted phenyl, more preferably (4-$R^4$)-substituted phenyl. $Ar^2$ is preferably phenyl or $R^4$-substituted phenyl, more preferably (4-$R^4$)-substituted phenyl. $Ar^3$ is preferably $R^5$-substituted phenyl, more preferably (4-$R^5$)-substituted phenyl. When $Ar^1$ is (4-$R^4$)-substituted phenyl, $R^4$ is preferably a halogen. When $Ar^2$ and $Ar^3$ are $R^4$-and $R^5$-substituted phenyl, respectively, $R^4$ is preferably halogen or -$OR^6$ and $R^5$ is preferably -$OR^6$, wherein $R^6$ is lower alkyl or hydrogen. Especially preferred are compounds wherein each of $Ar^1$ and $Ar^2$ is 4-fluorophenyl and $Ar^3$ is 4-hydroxyphenyl or 4-methoxyphenyl.

X, Y and Z are each preferably -$CH_2$-. $R^1$ and $R^3$ are each preferably hydrogen. R and $R^2$ are preferably -$OR^6$ wherein $R^6$ is hydrogen, or a group readily metabolizable to a hydroxyl (such as -O(CO)$R^6$, -O(CO)$OR^9$ and -O(CO)$NR^6R^7$, defined above).

The sum of m, n, p, q and r is preferably 2, 3 or 4, more preferably 3. Preferred are compounds wherein m, n and r are each zero, q is 1 and p is 2.

Also preferred are compounds of Formula (I) in which p, q and n are each zero, r is 1 and m is 2 or 3. More preferred are compounds wherein m, n and r are each zero, q is 1, p is 2, Z is -$CH_2$ and R is -$OR^6$, especially when $R^6$ is hydrogen.

Also more preferred are compounds of Formula (I) wherein p, q and n are each zero, r is 1, m is 2, X is -$CH_2$- and $R^2$ is -$OR^6$, especially when $R^6$ is hydrogen.

Another group of preferred compounds of Formula (I) is that in which $Ar^1$ is phenyl or $R^4$-substituted phenyl, $Ar^2$ is phenyl or $R^4$-substituted phenyl and $Ar^3$ is $R^5$-substituted phenyl. Also preferred are compounds in which $Ar^1$ is phenyl or $R^4$-substituted phenyl, $Ar^2$ is phenyl or $R^4$-substituted phenyl, $Ar^3$ is $R^5$-substituted phenyl, and the sum of m, n, p, q and r is 2, 3 or 4, more preferably 3. More preferred are compounds wherein $Ar^1$ is phenyl or $R^4$-substituted phenyl, $Ar^2$ is phenyl or $R^4$-substituted phenyl, $Ar^3$ is $R^5$-substituted phenyl, and wherein m, n and r are each zero, q is 1 and p is 2, or wherein p, q and n are each zero, r is 1 and m is 2 or 3.

In a preferred embodiment, a compound of Formula (I) used in the present invention is represented by Formula (II) (ezetimibe) below:

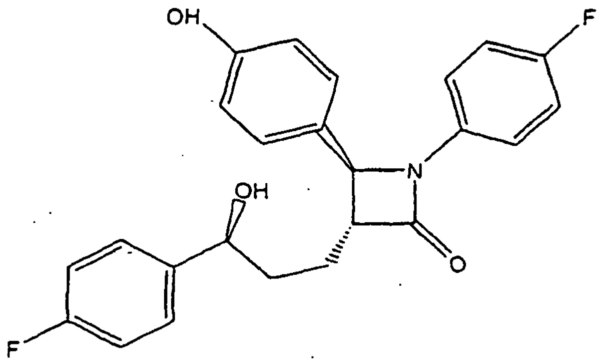

(II)

or a pharmaceutically acceptable salt or solvate thereof. The compound of Formula (II) can be in anhydrous or hydrated form.

Compounds of Formula I can be prepared by a variety of methods well known to those skilled in the art, for example such as are disclosed in U.S. Patents Nos. 5,631,365, 5,767,115, 5,846,966, 6,207,822, U.S. Patent Application No. 10/105,710 filed March 25, 2002 and PCT Patent Application WO 93/02048, each of which is incorporated herein by reference, and in the Example below. For example, suitable compounds of Formula I can be prepared by a method comprising the steps of:

(a) treating with a strong base a lactone of the Formula A or B:

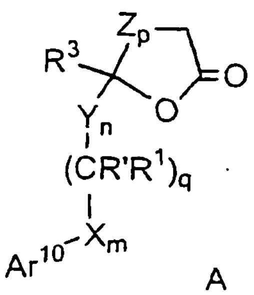

A

or

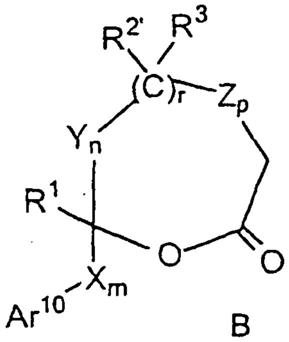

B

wherein R' and $R^{2'}$ are R and $R^2$, respectively, or are suitably protected hydroxy groups; $Ar^{10}$ is $Ar^1$, a suitably protected hydroxy-substituted aryl or a suitably protected amino-substituted aryl; and the remaining variables are as defined above for Formula I, provided that in lactone of formula B, when n and r are each zero, p is 1-4;

(b) reacting the product of step (a) with an imine of the formula

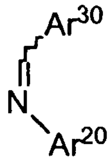

wherein $Ar^{20}$ is $Ar^2$, a suitably protected hydroxy-substituted aryl or a suitably protected amino-substituted aryl; and $Ar^{30}$ is $Ar^3$, a suitably protected hydroxy-substituted aryl or a suitably protected amino-substituted aryl;

c) quenching the reaction with an acid;

d) optionally removing the protecting groups from R', $R^{2'}$, $Ar^{10}$, $Ar^{20}$ and $Ar^{30}$, when present; and

e) optionally functionalizing hydroxy or amino substituents at R, $R^2$, $Ar^1$, $Ar^2$ and $Ar^3$.

Using the lactones shown above, compounds of Formula IA and IB are obtained as follows:

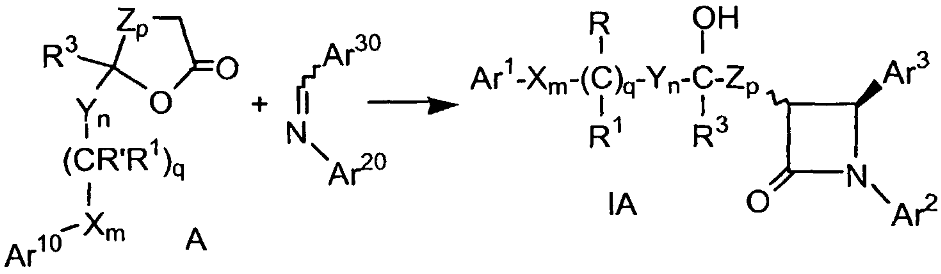

A IA

wherein the variables are as defined above; and

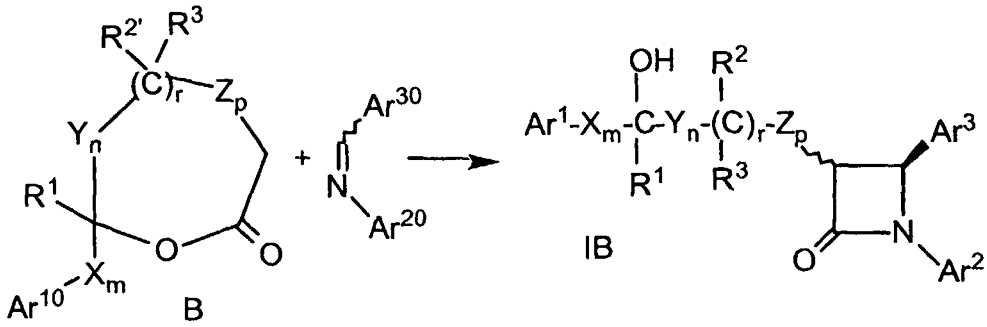

B IB

wherein the variables are as defined above.

Alternative compounds used in the present invention are represented by Formula (III) below:

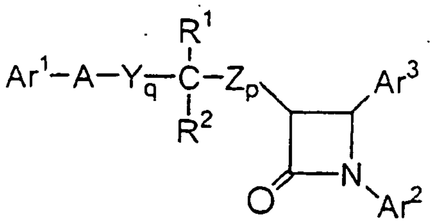

(III)

or a pharmaceutically acceptable salt thereof or solvate thereof, wherein, in Formula (III) above:

$Ar^1$ is $R^3$-substituted aryl;
$Ar^2$ is $R^4$-substituted aryl;
$Ar^3$ is $R^5$-substituted aryl;
Y and Z are independently selected from the group consisting of $-CH_2-$, -CH(lower alkyl)- and -C(dilower alkyl)-;
A is selected from -O-, -S-, -S(O)- or $-S(O)_2-$;
$R^1$ is selected from the group consisting of $-OR^6$, $-O(CO)R^6$, $-O(CO)OR^9$ and $-O(CO)NR^6R^7$; $R^2$ is selected from the group consisting of hydrogen, lower alkyl and aryl; or $R^1$ and $R^2$ together are =O;
q is 1, 2 or 3;
p is 0, 1, 2, 3 or 4;
$R^5$ is 1-3 substituents independently selected from the group consisting of $-OR^6$, $-O(CO)R^6$, $-O(CO)OR^9$, $O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-lower alkyl, $-NR^6SO_2$-aryl, $-CONR^6R^7$, $-COR^6$, $-SO_2NR^6R^7$, $S(O)_{0-2}$-alkyl, $S(O)_{0-2}$-aryl, $-O(CH_2)_{1-10}-COOR^6$, $-O(CH_2)_{1-10}CONR^6R^7$, o-halogeno, m-halogeno, o-lower alkyl, m-lower alkyl, -(lower alkylene)-$COOR^6$, and $-CH=CH-COOR^6$;

$R^3$ and $R^4$ are independently 1-3 substituents independently selected from the group consisting of $R^5$, hydrogen, p-lower alkyl, aryl, $-NO_2$, $-CF_3$ and p-halogeno;

$R^6$, $R^7$ and $R^8$ are independently selected from the group consisting of hydrogen, lower alkyl, aryl and aryl-substituted lower alkyl; and $R^9$ is lower alkyl, aryl or aryl-substituted lower alkyl.

Preferred compounds of Formula III include those in which $Ar^1$ is $R^3$-substituted phenyl, especially (4-$R^3$)-substituted phenyl. $Ar^2$ is preferably $R^4$-substituted phenyl, especially (4-$R^4$)-substituted phenyl. $Ar^3$ is preferably $R^5$-substituted phenyl, especially (4-$R^5$)-substituted phenyl. Mono-substitution of each of $Ar^1$, $Ar^2$ and $Ar^3$ is preferred.

Y and Z are each preferably $-CH_2-$. $R^2$ is preferably hydrogen. $R^1$ is preferably $-OR^6$ wherein $R^6$ is hydrogen, or a group readily metabolizable to a hydroxyl (such as $-O(CO)R^6$, $-O(CO)OR^9$ and $-O(CO)NR^6R^7$, defined above). Also preferred are compounds wherein $R^1$ and $R^2$ together are =O.

The sum of q and p is preferably 1 or 2, more preferably 1. Preferred are compounds wherein p is zero and q is 1. More preferred are compounds wherein p is zero, q is 1, Y is $-CH_2-$ and $R^1$ is $-OR^6$, especially when $R^6$ is hydrogen.

Another group of preferred compounds is that in which $Ar^1$ is $R^3$-substituted phenyl, $Ar^2$ is $R^4$-substituted phenyl and $Ar^3$ is $R^5$-substituted phenyl.

Also preferred are compounds wherein $Ar^1$ is $R^3$-substituted phenyl, $Ar^2$ is $R^4$-substituted phenyl, $Ar^3$ is $R^5$-substituted phenyl, and the sum of p and q is 1 or 2, especially 1. More preferred are compounds wherein $Ar^1$ is $R^3$-substituted phenyl, $Ar^2$ is $R^4$-substituted phenyl, $Ar^3$ is $R^5$-substituted phenyl, p is zero and q is 1.

A is preferably -O-.

$R^3$ is preferably $-COOR^6$, $-CONR^6R^7$, $-COR^6$, $-SO_2NR^6R^7$, $S(O)_{0-2}$-alkyl, $S(O)_{0-2}$-aryl, $NO_2$ or halogeno. A more preferred definition for $R^3$ is halogeno, especially fluoro or chloro.

R is preferably hydrogen, lower alkyl, $-OR^6$, ,$-O(CO)R^6$, $-O(CO)OR^9$, $-O(CO)NR^6R^7$, $-NR^6R^7$, $COR^6$ or halogeno, wherein $R^6$ and $R^7$ are preferably independently hydrogen or lower alkyl, and $R^9$ is preferably lower alkyl. A more preferred definition for $R^4$ is hydrogen or halogeno, especially fluoro or chloro.

$R^5$ is preferably $-OR^6$, $-O(CO)R^6$, $-O(CO)OR^9$, $-O(CO)NR^6R^7$, $-NR^6R^7$, -(lower alkylene)-$COOR^6$ or $-CH=CH-COOR^6$, wherein $R^6$ and $R^7$ are preferably independently hydrogen or lower alkyl, and $R^9$ is preferably lower alkyl. A more preferred definition for $R^5$ is $-OR^6$, -(lower alkylene)-$COOR^6$ or $-CH=CH-COOR^6$, wherein $R^6$ is preferably hydrogen or lower alkyl.

Methods for making compounds of Formula III are well known to those skilled in the art. Non-limiting examples of suitable methods are disclosed in U.S. Patent No. 5,688,990, which is incorporated herein by reference.

In another embodiment, compounds used in the present invention are represented by Formula (IV) below:

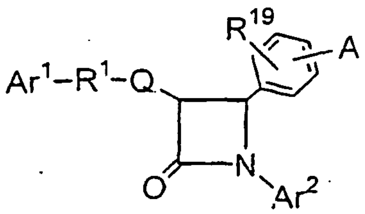

(IV)

or a pharmaceutically acceptable salt thereof or solvate thereof, wherein, in Formula (IV) above:

A is selected from the group consisting of $R^2$-substituted heterocycloalkyl, $R^2$-substituted heteroaryl, $R^2$-substituted benzofused heterocycloalkyl, and $R^2$-substituted benzofused heteroaryl;

$Ar^1$ is aryl or $R^3$-substituted aryl;

$Ar^2$ is aryl or $R^4$-substituted aryl;

Q is a bond or, with the 3-position ring carbon of the azetidinone, forms the spiro group

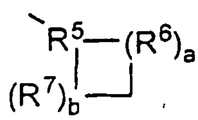

;

and

$R^1$ is selected from the group consisting of:

- $(CH_2)_q$-, wherein q is 2-6, provided that when Q forms a spiro ring, q can also be zero or 1;
- $(CH_2)_e$-G-$(CH_2)_r$-, wherein G is -O-, -C(O)-, phenylene, -$NR^8$- or -$S(O)_{0-2}$-, e is 0-5 and r is 0-5, provided that the sum of e and r is 1-6;
- ($C_2$-$C_6$ alkenylene)-; and
- $(CH_2)_f$-V-$(CH_2)_g$-, wherein V is $C_3$-$C_6$ cycloalkylene, f is 1-5 and g is 0-5, provided that the sum of f and g is 1-6;

$R^5$ is selected from:

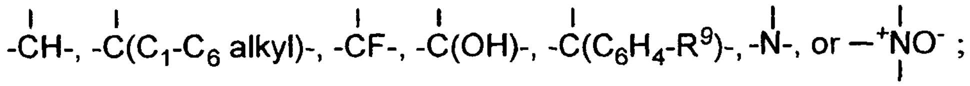

$R^6$ and $R^7$ are independently selected from the group consisting of -$CH_2$-, -CH($C_1$-$C_6$ alkyl)-, -C(di-($C_1$-$C_6$) alkyl), -CH=CH- and -C($C_1$-$C_6$ alkyl)=CH-; or $R^5$ together with an adjacent $R^6$, or $R^5$ together with an adjacent $R^7$, form a -CH=CH- or a -CH=C($C_1$-$C_6$ alkyl)- group;
a and b are independently 0, 1, 2 or 3, provided both are not zero; provided that when $R^6$ is -CH=CH- or -C($C_1$-$C_6$ alkyl)=CH-, a is 1; provided that when $R^7$ is -CH=CH- or -C($C_1$-$C_6$ alkyl)=CH-, b is 1; provided that when a is 2 or 3, the $R^6$'s can be the same or different; and provided that when b is 2 or 3, the $R^7$'s can be the same or different; and when Q is a bond, $R^1$ also can be selected from:

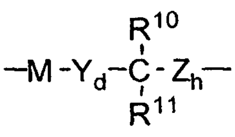 , 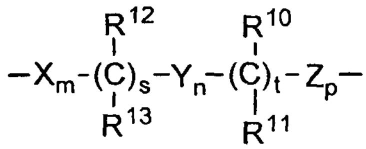 or 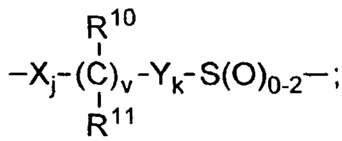

where M is -O-, -S-, -S(O)- or -$S(O)_2$-;
X, Y and Z are independently selected from the group consisting of -$CH_2$-, -CH($C_1$-$C_6$ alkyl)- and -C(di-($C_1$-$C_6$) alkyl);
$R^{10}$ and $R^{12}$ are independently selected from the group consisting of -$OR^{14}$, -O(CO)$R^{14}$, -O(CO)$OR^{16}$ and -O(CO)$NR^{14}R^{15}$;
$R^{11}$ and $R^{13}$ are independently selected from the group consisting of hydrogen, ($C_1$-$C_6$)alkyl and aryl; or $R^{10}$ and $R^{11}$ together are =O, or $R^{12}$ and $R^{13}$ together are =O;
d is 1, 2 or 3;
h is 0, 1, 2, 3 or 4;
s is 0 or 1; t is 0 or 1; m, n and p are independently 0-4; provided that at least one of s and t is 1, and the sum of m, n, p, s and t is 1-6; provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
$R^2$ is 1-3 substituents on the ring carbon atoms selected from the group consisting of hydrogen, ($C_1$-$C_{10}$)alkyl, ($C_2$-$C_{10}$)alkenyl, ($C_2$-$C_{10}$)alkynyl, ($C_3$-$C_6$)cycloalkyl, ($C_3$-$C_6$)cycloalkenyl, $R^{17}$-substituted aryl, $R^{17}$-substituted benzyl, $R^{17}$-substituted benzyloxy, $R^{17}$-substituted aryloxy, halogeno, -$NR^{14}R^{15}$, $NR^{14}R^{15}$($C_1$-$C_6$ alkylene)-, $NR^{14}R^{15}$C(O)($C_1$-$C_6$ alkylene)-,-NHC(O)$R^{16}$, OH, $C_1$-$C_6$ alkoxy, -OC(O)$R^{16}$, -$COR^{14}$, hydroxy($C_1$-$C_6$)alkyl, ($C_1$-$C_6$)alkoxy($C_1$-$C_6$)alkyl, $NO_2$, -$S(O)_{0-2}R^{16}$, -$SO_2NR^{14}R^{15}$ and -($C_1$-$C_6$ alkylene)$COOR^{14}$; when $R^2$ is a substituent on a heterocycloalkyl ring, $R^2$ is as defined, or is =O or

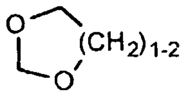 ;

and, where $R^2$ is a substituent on a substitutable ring nitrogen, it is hydrogen, $(C_1\text{-}C_6)$alkyl, aryl, $(C_1\text{-}C_6)$alkoxy, aryloxy, $(C_1\text{-}C_6)$alkylcarbonyl, arylcarbonyl, hydroxy, $-(CH_2)_{1\text{-}6}CONR^{18}R^{18}$,

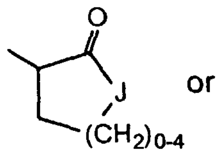 or 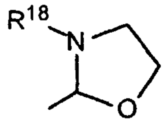 ;

wherein J is -O-, -NH-, $-NR^{18}-$ or $-CH_2-$;

$R^3$ and $R^4$ are independently selected from the group consisting of 1-3 substituents independently selected from the group consisting of $(C_1\text{-}C_6)$alkyl, $-OR^{14}$, $-O(CO)R^{14}$, $-O(CO)OR^{16}$, $-O(CH_2)_{1\text{-}5}OR^{14}$, $-O(CO)NR^{14}R^{15}$, $-NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $S(O)_{0\text{-}2}R^{16}$, $-O(CH_2)_{1\text{-}10}\text{-}COOR^{14}$,- $O(CH_2)_{1\text{-}10}CONR^{14}R^{15}$, $-(C_1\text{-}C_6$ alkylene$)\text{-}COOR^{14}$, $-CH{=}CH\text{-}COOR^{14}$, $-CF_3$, -CN, $-NO_2$ and halogen;

$R^8$ is hydrogen, $(C_1\text{-}C_6)$alkyl, aryl $(C_1\text{-}C_6)$alkyl, $-C(O)R^{14}$ or $-COOR^{14}$;

$R^9$ and $R^{17}$ are independently 1-3 groups independently selected from the group consisting of hydrogen, $(C_1\text{-}C_6)$ alkyl, $(C_1\text{-}C_6)$alkoxy, -COOH, $NO_2$, $-NR^{14}R^{15}$, OH and halogeno;

$R^{14}$ and $R^{15}$ are independently selected from the group consisting of hydrogen, $(C_1\text{-}C_6)$alkyl, aryl and aryl-substituted $(C_1\text{-}C_6)$alkyl;

$R^{16}$ is $(C_1\text{-}C_6)$alkyl, aryl or $R^{17}$-substituted aryl;

$R^{18}$ is hydrogen or $(C_1\text{-}C_6)$alkyl; and

$R^{19}$ is hydrogen, hydroxy or $(C_1\text{-}C_6)$alkoxy.

As used in Formula (IV) above, "A" is preferably an $R^2$-substituted, 6-membered heterocycloalkyl ring containing 1 or 2 nitrogen atoms. Preferred heterocycloalkyl rings are piperidinyl, piperazinyl and morpholinyl groups. The ring "A" is preferably joined to the phenyl ring through a ring nitrogen. Preferred $R^2$ substituents are hydrogen and lower alkyl. $R^{19}$ is preferably hydrogen.

$Ar^2$ is preferably phenyl or $R^4$-phenyl, especially $(4\text{-}R^4)$-substituted phenyl. Preferred definitions of $R^4$ are lower alkoxy, especially methoxy, and halogeno, especially fluoro.

$Ar^1$ is preferably phenyl or $R^3$-substituted phenyl, especially $(4\text{-}R^3)$-substituted phenyl.

There are several preferred definitions for the $-R^1\text{-}Q\text{-}$ combination of variables:

Q is a bond and $R^1$ is lower alkylene, preferably propylene;

Q is a spiro group as defined above, wherein preferably $R^6$ and $R^7$ are each ethylene and $R^5$ is

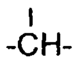 or 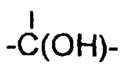 ;

Q is a bond and $R^1$ is

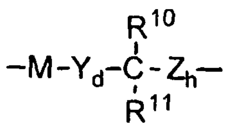

wherein the variables are chosen such that $R^1$ is $-O\text{-}CH_2\text{-}CH(OH)-$;

Q is a bond and $R^1$ is

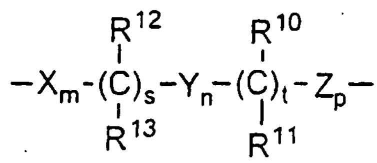

wherein the variables are chosen such that $R^1$ is $-CH(OH)-(CH_2)_2-$; and

Q is a bond and $R^1$ is

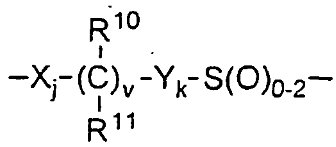

wherein the variables are chosen such that $R^1$ is $-CH(OH)-CH_2-S(O)_{0-2}-$.

Methods for making compounds of Formula IV are well known to those skilled in the art. Non-limiting examples of suitable methods are disclosed in U.S. Patent No. 5,656,624, which is incorporated herein by reference.

In another embodiment, compounds used in the present invention are represented by Formula (V) below:

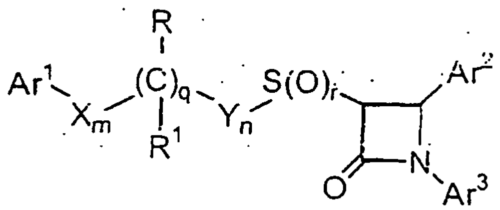

(V).

or a pharmaceutically acceptable salt thereof or solvate thereof, wherein, in Formula (V) above:

$Ar^1$ is aryl, $R^{10}$-substituted aryl or heteroaryl;

$Ar^2$ is aryl or $R^4$-substituted aryl;

$Ar^3$ is aryl or $R^5$-substituted aryl;

X and Y are independently selected from the group consisting of $-CH_2-$, -CH(lower alkyl)- and -C(dilower alkyl)-;

R is $-OR^6$, $-O(CO)R^6$, $-O(CO)OR^9$ or $-O(CO)NR^6R^7$; $R^1$ is hydrogen, lower alkyl or aryl; or R and $R^1$ together are =O;

q is 0 or 1;

r is 0, 1 or 2;

m and n are independently 0, 1, 2, 3, 4 or 5; provided that the sum of m, n and q is 1, 2, 3, 4 or 5;

$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$ and $-CH=CH-COOR^6$;

$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, $-CF_3$, -CN, $-NO_2$, halogen, -(lower alkylene)$COOR^6$ and $-CH=CH-COOR^6$;

$R^6$, $R^7$ and $R^8$ are independently selected from the group consisting of hydrogen, lower alkyl, aryl and aryl-substituted lower alkyl;

$R^9$ is lower alkyl, aryl or aryl-substituted lower alkyl; and

$R^{10}$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$,

$-COOR^6$, $-CONR^6R^7$, $-COR^6$, $-SO_2NR^6R^7$, $-S(O)_{0-2}R^9$, $-O(CH_2)_{1-10}-COOR^6$, $-O(CH_2)_{1-10}CONR^6R^7$, $-CF_3$, -CN, $-NO_2$ and halogen.

Within the scope of Formula V, there are included two preferred structures. In Formula VA, q is zero and the remaining variables are as defined above, and in Formula VB, q is 1 and the remaining variables are as defined above:

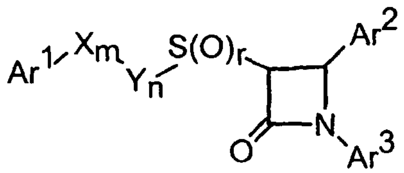

VA

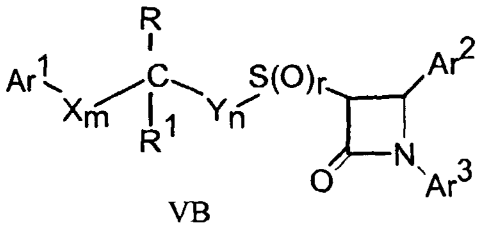

VB

$R^4$, $R^5$ and $R^{10}$ are each preferably 1-3 independently selected substituents as set forth above. Preferred are compounds of Formula (V) wherein $Ar^1$ is phenyl, $R^{10}$-substituted phenyl or thienyl, especially (4-$R^{10}$)-substituted phenyl or thienyl. $Ar^2$ is preferably $R^4$-substituted phenyl, especially (4-$R^4$)-substituted phenyl. $Ar^3$ is preferably phenyl or $R^5$-substituted phenyl, especially (4-$R^5$)-substituted phenyl. When $Ar^1$ is $R^{10}$-substituted phenyl, $R^{10}$ is preferably halogeno, especially fluoro. When $Ar^2$ is $R^4$-substituted phenyl, $R^4$ is preferably $-OR^6$, especially wherein $R^6$ is hydrogen or lower alkyl. When $Ar^3$ is $R^5$-substituted phenyl, $R^5$ is preferably halogeno, especially fluoro. Especially preferred are compounds of Formula (V) wherein $Ar^1$ is phenyl, 4-fluorophenyl or thienyl, $Ar^2$ is 4-(alkoxy or hydroxy)phenyl, and $Ar^3$ is phenyl or 4-fluorophenyl.

X and Y are each preferably $-CH_2-$. The sum of m, n and q is preferably 2, 3 or 4, more preferably 2. When q is 1, n is preferably 1 to 5.

Preferences for X, Y, $Ar^1$, $Ar^2$ and $Ar^3$ are the same in each of Formulae (VA) and (VB).

In compounds of Formula (VA), the sum of m and n is preferably 2, 3 or 4, more preferably 2. Also preferred are compounds wherein the sum of m and n is 2, and r is 0 or 1.

In compounds of Formula (VB), the sum of m and n is preferably 1, 2 or 3, more preferably 1. Especially preferred are compounds wherein m is zero and n is 1. $R^1$ is preferably hydrogen and R is preferably $-OR^6$ wherein $R^6$ is hydrogen, or a group readily metabolizable to a hydroxyl (such as $-O(CO)R^6$, $-O(CO)OR^9$ and $-O(CO)NR^6R^7$, defined above), or R and $R^1$ together form a =O group.

Methods for making compounds of Formula V are well known to those skilled in the art. Non-limiting examples of suitable methods are disclosed in U.S. Patent No. 5,624,920, which is incorporated herein by reference.

In another embodiment, compounds used in the present invention are represented by Formula (VI) below:

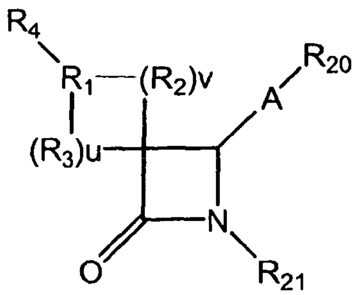

(VI)

or a pharmaceutically acceptable salt thereof or solvate thereof, wherein in Formula (VI) below:

$R_1$ is

$-\overset{|}{C}H-$, $-\overset{|}{C}(\text{lower alkyl})-$, $-\overset{|}{C}F-$, $-\overset{|}{C}(OH)-$, $-\overset{|}{C}(C_6H_5)-$, $-\overset{|}{C}(C_6H_4-R_{15})-$,

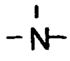 or 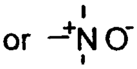 ;

$R_2$ and $R_3$ are independently selected from the group consisting of: $-CH_2-$, -CH(lower alkyl)-, -C(di-lower alkyl)-, -CH=CH- and -C(lower alkyl)=CH-; or $R_1$ together with an adjacent $R_2$, or $R_1$ together with an adjacent $R_3$, form a -CH=CH- or a -CH=C(lower alkyl)- group;

u and v are independently 0, 1, 2 or 3, provided both are not zero; provided that when $R_2$ is -CH=CH- or -C(lower alkyl)=CH-, v is 1; provided that when $R_3$ is -CH=CH- or -C(lower alkyl)=CH-, u is 1; provided that when v is 2 or 3, the $R_2$'s can be the same or different; and provided that when u is 2 or 3, the $R_3$'s can be the same or different;

$R_4$ is selected from $B-(CH_2)_mC(O)-$, wherein m is 0, 1, 2, 3, 4 or 5; $B-(CH_2)_q-$, wherein q is 0, 1, 2, 3, 4, 5 or 6; $B-(CH_2)_e-Z-(CH_2)_r-$, wherein Z is -O-, -C(O)-, phenylene, $-N(R_8)-$ or $-S(O)_{0-2}-$, e is 0, 1, 2, 3, 4 or 5 and r is 0, 1, 2, 3, 4 or 5, provided that the sum of e and r is 0, 1, 2, 3, 4, 5 or 6;

B-($C_2$-$C_6$ alkenylene)-;

B-($C_4$-$C_6$ alkadienylene)-;

$B-(CH_2)_t-Z-$($C_2$-$C_6$ alkenylene)-, wherein Z is as defined above, and wherein t is 0, 1, 2 or 3, provided that the sum of t and the number of carbon atoms in the alkenylene chain is 2, 3, 4, 5 or 6;

$B-(CH_2)_f-V-(CH_2)_g-$, wherein V is $C_3$-$C_6$ cycloalkylene, f is 1, 2, 3, 4 or 5 and g is 0, 1, 2, 3, 4 or 5, provided that the sum of f and g is 1, 2, 3, 4, 5 or 6; $B-(CH_2)_t-V-$($C_2$-$C_6$ alkenylene)- or

B-($C_2$-$C_6$ alkenylene)-$V-(CH_2)_t-$, wherein V and t are as defined above, provided that the sum of t and the number of carbon atoms in the alkenylene chain is 2, 3, 4, 5 or 6;

$B-(CH_2)_a-Z-(CH_2)_b-V-(CH_2)_d-$, wherein Z and V are as defined above and a, b and d are independently 0, 1, 2, 3, 4, 5 or 6, provided that the sun of a, b and d is 0, 1, 2, 3, 4, 5 or 6; or

$T-(CH_2)_s-$, wherein T is cycloalkyl of 3-6 carbon atoms and s is 0, 1, 2, 3, 4, 5 or 6; or

$R_1$ and $R_4$ together form the group

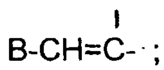

B is selected from indanyl, indenyl, naphthyl, tetrahydronaphthyl, heteroaryl or W-substituted heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, or

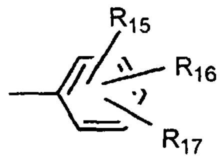

W is 1 to 3 substituents independently selected from the group consisting of lower alkyl, hydroxy lower alkyl, lower alkoxy, alkoxyalkyl, alkoxyalkoxy, alkoxycarbonylalkoxy, (lower alkoxyimino)-lower alkyl, lower alkanedioyl, lower alkyl lower alkanedioyl, allyloxy, $-CF_3$, $-OCF_3$, benzyl,

$R_7$-benzyl, benzyloxy, $R_7$-benzyloxy, phenoxy, $R_7$-phenoxy, dioxolanyl, $NO_2$, $-N(R_8)(R_9)$, $N(R_8)(R_9)$-lower alkylene-, $N(R_8)(R_9)$-lower alkylenyloxy-, OH, halogeno, -CN, $-N_3$, $-NHC(O)OR_{10}$, $-NHC(O)R_{10}$, $R_{11}O_2SNH-$, $(R_{11}O_2S)_2N-$, $-S(O)_2NH2$, $-S(O)_{0-2}R_8$, tert-butyldimethyl-silyloxymethyl, $-C(O)R_{12}$,- $COOR_{19}$, $-CON(R_8)(R_9)$, $-CH=CHC(O)R_{12}$, -lower alkylene-$C(O)R_{12}$, $R_{10}C(O)$(lower alkylenyloxy)-, $N(R_8)(R_9)C(O)$(lower alkylenyloxy)- and

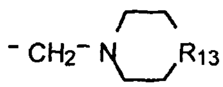

for substitution on ring carbon atoms,

and the substituents on the substituted heteroaryl ring nitrogen atoms, when present, are selected from the group consisting of lower alkyl, lower alkoxy, $-C(O)OR_{10}$, $-C(O)R_{10}$, OH, $N(R_8)(R_9)$-lower alkylene-,

$N(R_8)(R_9)$-lower alkylenyloxy-, $-S(O)_2NH_2$ and 2-(trimethylsilyl)-ethoxymethyl;

$R_7$ is 1-3 groups independently selected from the group consisting of lower alkyl, lower alkoxy, -COOH, $NO_2$, -N ($R_8$)($R_9$), OH, and halogeno;
$R_8$ and Rg are independently selected from H or lower alkyl;
$R_{10}$ is selected from lower alkyl, phenyl, $R_7$-phenyl, benzyl or $R_7$-benzyl;
$R_{11}$ is selected from OH, lower alkyl, phenyl, benzyl, $R_7$-phenyl or $R_7$-benzyl;
$R_{12}$ is selected from H, OH, alkoxy, phenoxy, benzyloxy,

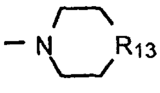,

-N($R_8$)($R_9$), lower alkyl, phenyl or $R_7$-phenyl;
$R_{13}$ is selected from -O-, -$CH_2$-, -NH-, -N(lower alkyl)- or -NC(O)$R_{19}$;
$R_{15}$, $R_{16}$ and $R_{17}$ are independently selected from the group consisting of H and the groups defined for W; or $R_{15}$ is hydrogen and $R_{16}$ and $R_{17}$, together with adjacent carbon atoms to which they are attached, form a dioxolanyl ring;
$R_{19}$ is H, lower alkyl, phenyl or phenyl lower alkyl; and
$R_{20}$ and $R_{21}$ are independently selected from the group consisting of phenyl, W-substituted phenyl, naphthyl, W-substituted naphthyl, indanyl, indenyl, tetrahydronaphthyl, benzodioxolyl, heteroaryl, W-substituted heteroaryl, benzofused heteroaryl, W-substituted benzofused heteroaryl and cyclopropyl, wherein heteroaryl is as defined above.

One group of preferred compounds of Formula VI is that in which $R_{21}$ is selected from phenyl, W-substituted phenyl, indanyl, benzofuranyl, benzodioxolyl, tetrahydronaphthyl, pyridyl, pyrazinyl, pyrimidinyl, quinolyl or cyclopropyl, wherein W is lower alkyl, lower alkoxy, OH, halogeno, -N($R_8$)($R_9$), -NHC(O)O$R_{10}$, -NHC(O)$R_{10}$, $NO_2$, -CN, -$N_3$, -SH, -S (O)$_{0-2}$-(lower alkyl), -COO$R_{19}$, -CON($R_8$)($R_9$), -CO$R_{12}$, phenoxy, benzyloxy, -O$CF_3$, -CH=C(O)$R_{12}$ or tert-butyldimethylsilyloxy, wherein $R_8$, $R_9$, $R_{10}$, $R_{12}$ and $R_{19}$ are as defined for Formula IV. When W is 2 or 3 substituents, the substituents can be the same or different.

Another group of preferred compounds of Formula VI is that in which $R_{20}$ is phenyl or W-substituted phenyl, wherein preferred meanings of W are as defined above for preferred definitions of $R_{21}$.

More preferred are compounds of Formula VI wherein $R_{20}$ is phenyl or W-substituted phenyl and $R_{21}$ is phenyl, W-substituted phenyl, indanyl, benzofuranyl, benzodioxolyl, tetrahydronaphthyl, pyridyl, pyrazinyl, pyrimidinyl, quinolyl or cyclopropyl; W is lower alkyl, lower alkoxy, OH, halogeno, -N($R_8$)($R_9$), -NHC(O)O$R_{10}$, -NHC(O)$R_{10}$, $NO_2$, -CN, -$N_3$, -SH, -S(O)$_{0-2}$-(lower alkyl), -COO$R_{19}$, -CON($R_8$)($R_9$), -CO$R_{12}$, phenoxy, benzyloxy, -CH=CHC(O)$R_{12}$, -O$CF_3$ or tert-butyl-dimethyl-silyloxy, wherein when W is 2 or 3 substituents, the substituents can be the same or different, and wherein $R_8$, $R_9$, $R_{10}$, $R_{12}$ and $R_{19}$ are as defined in Formula VI.

Also preferred are compounds of Formula VI wherein $R_1$ is

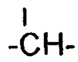

or

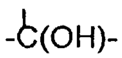.

Another group of preferred compounds of Formula VI is in which $R_2$ and $R_3$ are each -$CH_2$- and the sum of u and v is 2, 3 or 4, with u=v=2 being more preferred.

$R_4$ is preferably B-($CH_2$)$_q$- or B-($CH_2$)$_e$-Z-($CH_2$)$_r$-, wherein B, Z, q, e and r are as defined above. B is preferably

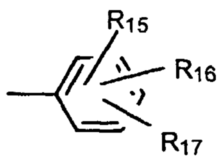,

wherein $R_{16}$ and $R_{17}$ are each hydrogen and wherein $R_{15}$ is preferably H, OH, lower alkoxy, especially methoxy, or halogeno, especially chloro.

Preferably Z is -O-, e is 0, and r is 0.

Preferably q is 0-2.

$R_{20}$ is preferably phenyl or W-substituted phenyl.

Preferred W substituents for $R_{20}$ are lower alkoxy, especially methoxy and ethoxy, OH, and $-C(O)R_{12}$, wherein $R_{12}$ is preferably lower alkoxy.

Preferably $R_{21}$ is selected from phenyl, lower alkoxy-substituted phenyl and F-phenyl.

Especially preferred are compounds of Formula VI wherein $R_1$ is

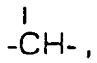

or

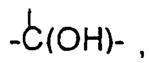

$R_2$ and $R_3$ are each $-CH_2-$, u=v=2, $R_4$ is $B-(CH_2)_q-$, wherein B is phenyl or phenyl substituted by lower alkoxy or chloro, q is 0-2, $R_{20}$ is phenyl, OH-phenyl, lower alkoxy-substituted phenyl or lower alkoxycarbonyl-substituted phenyl, and $R_{21}$ is phenyl, lower alkoxy-substituted phenyl or F-phenyl.

An example of another useful compound of Formula VI is shown below in Formula VIa:

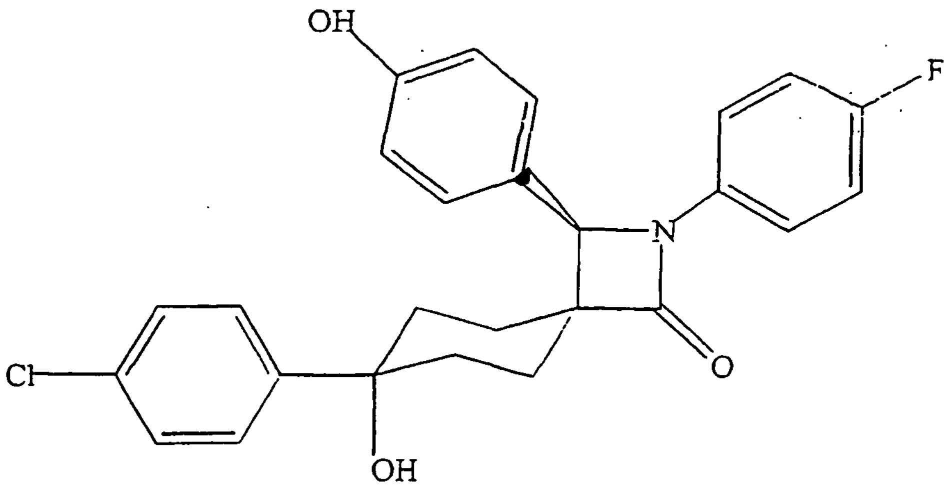

(VIa)

or a pharmaceutically acceptable salt or solvate thereof.

Methods for making compounds of Formula VI are well known to those skilled in the art. Non-limiting examples of suitable methods are disclosed in U.S. Patent No. 5,698,548, which is incorporated herein by reference.

In another embodiment, compounds used in the present invention are represented by Formula (VII):

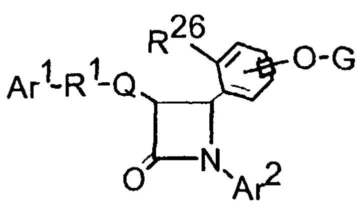

(VII)

or a pharmaceutically acceptable salt thereof or solvate thereof, wherein, in Formula (VII) above,

$R^{26}$ is H or $OG^1$;
G and $G^1$ are independently selected from the group consisting of H,

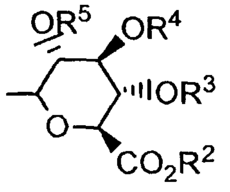 , 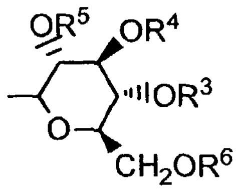 , 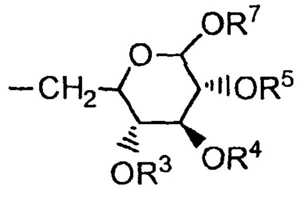

and

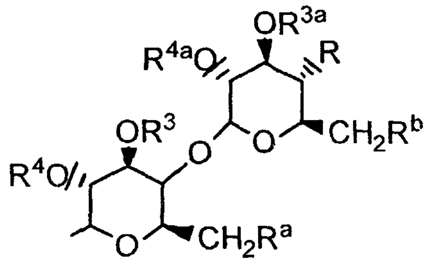 ;

provided that when $R^{26}$ is H or OH, G is not H;
R, $R^a$ and $R^b$ are independently selected from the group consisting of H, -OH, halogeno, $-NH_2$, azido, $(C_1-C_6)$alkoxy $(C_1-C_6)$-alkoxy or $-W-R^{30}$;
W is independently selected from the group consisting of -NH-C(O)-, -O-C(O)-, $-O-C(O)-N(R^{31})-$, $-NH-C(O)-N(R^{31})-$ and $-O-C(S)-N(R^{31})-$;
$R^2$ and $R^6$ are independently selected from the group consisting of H, $(C_{1-}C_6)$alkyl, aryl and aryl$(C_1-C_6)$alkyl;
$R^3$, $R^4$, $R^5$, $R^7$, $R^{3a}$ and $R^{4a}$ are independently selected from the group consisting of H, $(C_1-C_6)$alkyl, aryl$(C_1-C_6)$alkyl, $-C(O)(C_1-C_6)$alkyl and -C(O)aryl;
$R^{30}$ is selected from the group consisting of $R^{32}$-substituted T,
$R^{32}$-substituted-T-$(C_1-C_6)$alkyl, $R^{32}$-substituted-$(C_2-C_4)$alkenyl,
$R^{32}$-substituted-$(C_1-C_6)$alkyl, $R^{32}$-substituted-$(C_3-C_7)$cycloalkyl and
$R^{32}$-substituted-$(C_3-C_7)$cycloalkyl$(C_1-C_6)$alkyl;
$R^{31}$ is selected from the group consisting of H and $(C_1-C_4)$alkyl;
T is selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;
$R^{32}$ is independently selected from 1-3 substituents independently selected from the group consisting of halogeno, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsulfinyl, $(C_1-C_4)$alkylsulfonyl, $-N(CH_3)_2$, $-C(O)-NH(C_1-C_4)$alkyl, $-C(O)-N((C_1-C_4)alkyl)_2$, $-C(O)-(C_1-C_4)$alkyl, $-C(O)-(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$alkoxycarbonyl-substituted pyrrolidinyl, piperidinyl, N-methylpiperazinyl, indolinyl or morpholinyl group;
$Ar^1$ is aryl or $R^{10}$-substituted aryl;
$Ar^2$ is aryl or $R^{11}$-substituted aryl;
Q is a bond or, with the 3-position ring carbon of the azetidinone, forms the spiro group

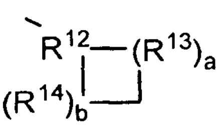 ;

and

$R^1$ is selected from the group consisting of

$-(CH_2)_q-$, wherein q is 2-6, provided that when Q forms a spiro ring, q can also be zero or 1;

$-(CH_2)_e-E-(CH2)_r-$, wherein E is -O-, -C(O)-, phenylene, $-NR^{22}-$ or $-S(O)_{0-2}-$, e is 0-5 and r is 0-5, provided that the sum of e and r is 1-6;

$-(C_2-C_6)$alkenylene-; and

$-(CH_2)_f-V-(CH_2)_g-$, wherein V is $C_3-C_6$ cycloalkylene, f is 1-5 and g is 0-5, provided that the sum of f and g is 1-6;

$R^{12}$ is

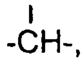, 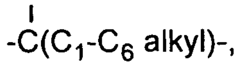, 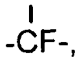, 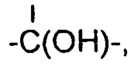, 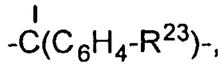, 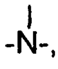, or 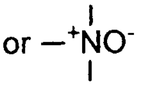 ;

$R^{13}$ and $R^{14}$ are independently selected from the group consisting of $-CH_2-$, $-CH(C_1-C_6$ alkyl)-, $-C(di-(C_1-C_6)$ alkyl), -CH=CH- and $-C(C_1-C_6$ alkyl)=CH-; or $R^{12}$ together with an adjacent $R^{13}$, or $R^{12}$ together with an adjacent $R^{14}$, form a -CH=CH- or a $-CH=C(C_1-C_6$ alkyl)- group;

a and b are independently 0, 1, 2 or 3, provided both are not zero;

provided that when $R^{13}$ is -CH=CH- or-$C(C_1-C_6$ alkyl)=CH-, a is 1;

provided that when $R^{14}$ is -CH=CH- or $-C(C_1-C_6$ alkyl)=CH-, b is 1;

provided that when a is 2 or 3, the $R^{13}$'s can be the same or different; and

provided that when b is 2 or 3, the $R^{14}$'s can be the same or different;

and when Q is a bond, $R^1$ also can be:

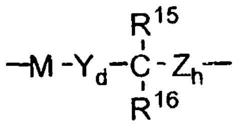 , 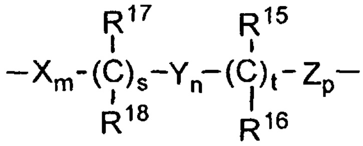 or 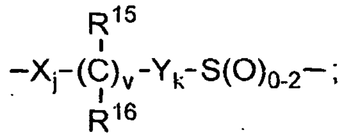;

M is -O-, -S-, -S(O)- or $-S(O)_2-$;

X, Y and Z are independently selected from the group consisting of $-CH_2-$, $-CH(C_1-C_6)$alkyl- and $-C(di-(C_1-C_6)$alkyl);

$R^{10}$ and $R^{11}$ are independently selected from the group consisting of 1-3 substituents independently selected from the group consisting of $(C_1-C_6)$alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$ alkylene)-$COOR^{19}$, -CH=CH-$COOR^{19}$, $-CF_3$, -CN, $-NO_2$ and halogen;

$R^{15}$ and $R^{17}$ are independently selected from the group consisting of $-OR^{19}$, $-O(CO)R^{19}$, $-O(CO)OR^{21}$ and $-O(CO)NR^{19}R^{20}$;

$R^{16}$ and $R^{18}$ are independently selected from the group consisting of H, $(C_1-C_6)$alkyl and aryl; or $R^{15}$ and $R^{16}$ together are =O, or $R^{17}$ and $R^{18}$ together are =O;

d is 1, 2 or 3;

h is 0, 1, 2, 3 or 4;

s is 0 or 1; t is 0 or 1; m, n and p are independently 0-4;

provided that at least one of s and t is 1, and the sum of m, n, p, s and t is 1-6;

provided that when p is 0 and t is 1, the sum of m, s and n is 1-5; and provided that when p is 0 and s is 1, the sum of m, t and n is 1-5;

v is 0 or 1;

j and k are independently 1-5, provided that the sum of j, k and v is 1-5;

and when Q is a bond and $R^1$ is

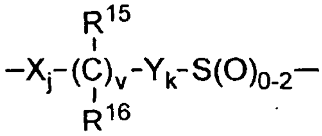,

$Ar^1$ can also be pyridyl, isoxazolyl, furanyl, pyrrolyl, thienyl, imidazolyl, pyrazolyl, thiazolyl, pyrazinyl, pyrimidinyl or pyridazinyl;
$R^{19}$ and $R^{20}$ are independently selected from the group consisting of H, $(C_1$-$C_6)$alkyl, aryl and aryl-substituted $(C_1$-$C_6)$ alkyl;
$R^{21}$ is $(C_1$-$C_6)$alkyl, aryl or $R^{24}$-substituted aryl;
$R^{22}$ is H, $(C_1$-$C_6)$alkyl, aryl $(C_1$-$C_6)$alkyl, -C(O)$R^{19}$ or -COO$R^{19}$;
$R^{23}$ and $R^{24}$ are independently 1-3 groups independently selected from the group consisting of H, $(C_1$-$C_6)$alkyl, $(C_1$-$C_6)$alkoxy, -COOH, $NO_2$, -N$R^{19}R^{20}$, -OH and halogeno; and
$R^{25}$ is H, -OH or $(C_1$-$C_6)$alkoxy.

$Ar^2$ is preferably phenyl or $R^{11}$-phenyl, especially (4-$R^{11}$)-substituted phenyl. Preferred definitions of $R^{11}$ are lower alkoxy, especially methoxy, and halogeno, especially fluoro.
$Ar^1$ is preferably phenyl or $R^{10}$-substituted phenyl, especially (4-$R^{10}$)-substituted phenyl. Preferably $R^{10}$ is halogeno, and more preferably fluoro.
There are several preferred definitions for the -$R^1$-Q- combination of variables:

Q is a bond and $R^1$ is lower alkylene, preferably propylene;
Q is a spiro group as defined above, wherein preferably $R^{13}$ and $R^{14}$ are each ethylene and $R^{12}$ is

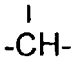 or 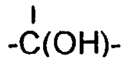 ,

and $R^1$ is $-(CH_2)q$ wherein q is 0-6;
Q is a bond and $R^1$ is

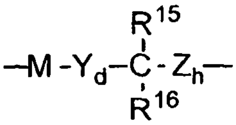

wherein the variables are chosen such that $R^1$ is $-O-CH_2-CH(OH)-$;
Q is a bond and $R^1$ is

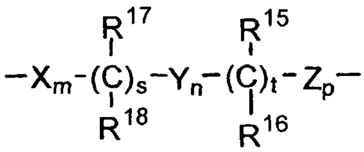

wherein the variables are chosen such that $R^1$ is $-CH(OH)-(CH_2)_2-$; and
Q is a bond and $R^1$ is

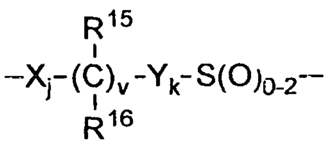

wherein the variables are chosen such that $R^1$ is $-CH(OH)-CH_2-S(O)_{0-2}-$.

A preferred compound of Formula (VII) therefore, is one wherein G and $G^1$ are as defined above and in which the remaining variables have the following definitions:

$Ar^1$ is phenyl or $R^{10}$-substituted phenyl, wherein $R^{10}$ is halogeno;
$Ar^2$ is phenyl or $R^{11}$-phenyl, wherein $R^{11}$ is 1 to 3 substituents independently selected from the group consisting of $C_1$-$C_6$ alkoxy and halogeno;
Q is a bond and $R^1$ is lower alkylene; Q, with the 3-position ring carbon of the azetidinone, forms the group

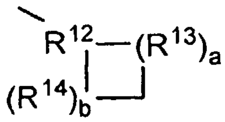

wherein preferably $R^{13}$ and $R^{14}$ are each ethylene and a and b are each 1, and wherein $R^{12}$ is

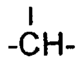 or 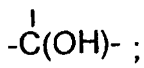

Q is a bond and $R^1$ is $-O-CH_2-CH(OH)-$; Q is a bond and $R^1$ is $-CH(OH)-(CH_2)_2-$; or Q is a bond and $R^1$ is- $CH(OH)-CH_2-S(O)_{0-2}-$.

Preferred variables for G and $G^1$ groups of the formulae

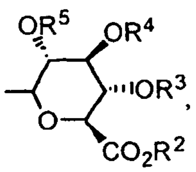, 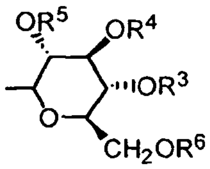 and 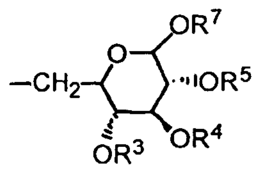

are as follows:

$R^2$, $R^3$, $R^4$, $R^5$, $R^6$ and $R^7$ are independently selected from the group consisting of H, $(C_1$-$C_6)$alkyl, benzyl and acetyl.
Preferred variables for group G or $G^1$ of the formula:

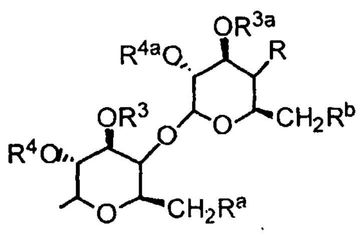

are as follows:

$R^3$, $R^{3a}$, $R^4$ and $R^{4a}$ are selected from the group consisting of H, $(C_1$-$C_6)$alkyl, benzyl and acetyl;
R, $R^a$ and $R^b$ are independently selected from the group consisting of H, -OH, halogeno, $-NH_2$, azido, $(C_1$-$C_6)$alkoxy$(C_1$-$C_6)$alkoxy and $-W-R^{30}$,

wherein W is -O-C(O)- or $-O-C(O)-NR^{31}-$, $R^{31}$ is H and $R^{30}$ is $(C_1$-$C_6)$alkyl, $-C(O)-(C_1$-$C_4)$alkoxy-$(C_1$-$C_6)$alkyl, T , T-$(C_1$-$C_6)$alkyl, or T or T-$(C_1$-$C_6)$alkyl wherein T is substituted by one or two halogeno or $(C_1$-$C_6)$alkyl groups.

Preferred $R^{30}$ substituents are selected from the group consisting of: 2-fluorophenyl, 2,4-difluoro-phenyl, 2,6-dichlorophenyl, 2-methylphenyl, 2-thienylmethyl, 2-methoxy-carbonylethyl, thiazol-2-yl-methyl, 2-furyl, 2-methoxycarbonylbutyl and phenyl.

Preferred combinations of R, $R^a$ and $R^b$ are as follows:

1) R, $R^a$ and $R^b$ are independently -OH or -O-C(O)-NH-$R^{30}$, especially wherein $R^a$ is -OH and R and $R^b$ are -O-C(O)-NH-$R^{30}$ and $R^{30}$ is selected from the preferred substituents identified above, or wherein R and $R^a$ are each -OH and $R^b$ is-O-C(O)-NH-$R^{30}$ wherein $R^{30}$ is 2-fluorophenyl, 2,4-difluoro-phenyl, 2,6-dichlorophenyl;

2) $R^a$ is -OH, halogeno, azido or ($C_1$-$C_6$)-alkoxy($C_1$-$C_6$)alkoxy, $R^b$ is H, halogeno, azido or ($C_1$-$C_6$)alkoxy($C_1$-$C_6$)-alkoxy, and R is -O-C(O)-NH-$R^{30}$, especially compounds wherein $R^a$ is -OH, $R^b$ is H and $R^{30}$ is 2-fluorophenyl;

3) R, $R^a$ and $R^b$ are independently -OH or -O-C(O)-$R^{30}$ and $R^{30}$ is ($C_1$-$C_6$)alkyl, T, or T substituted by one or two halogeno or ($C_1$-$C_6$)alkyl groups, especially compounds wherein R is -OH and $R^a$ and $R^b$ are -O-C(O)-$R^{30}$ wherein $R^{30}$ is 2-furyl; and

4) R, $R^a$ and $R^b$ are independently -OH or halogeno. Three additional classes of preferred compounds are those wherein the $C^{1'}$ anomeric oxy is beta, wherein the $C^{2'}$ anomeric oxy is beta, and wherein the R group is alpha. G and $G^1$ are preferably selected from:

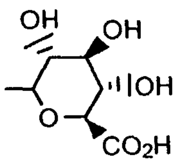, 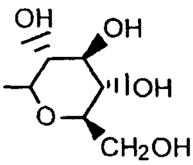, 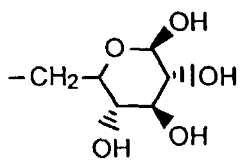, 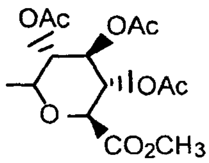,

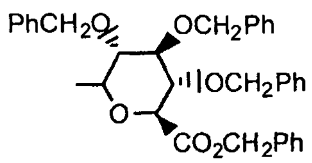, 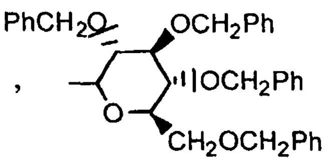, 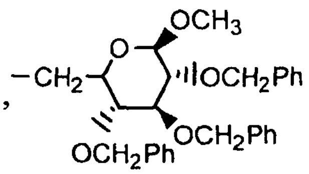,

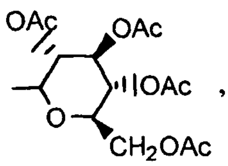, 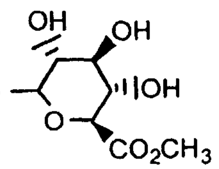, 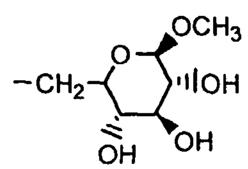,

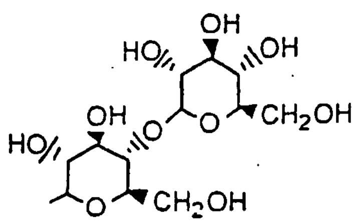, 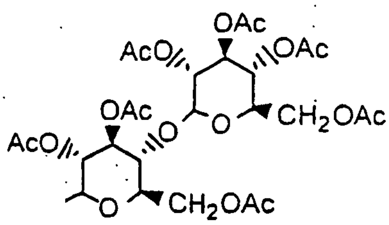

and

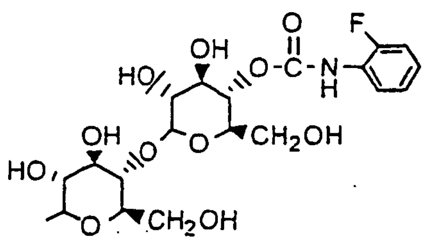

wherein Ac is acetyl and Ph is phenyl.

Preferably, $R^{26}$ is H or OH, more preferably H. The -O-G substituent is preferably in the 4-position of the phenyl ring to which it is attached.

In another embodiment, compounds used in the present invention are represented by Formula (VIII) below:

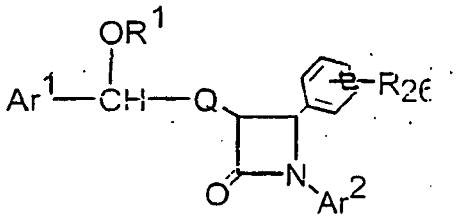

(VIII)

or a pharmaceutically acceptable salt or solvate thereof, wherein in Formula (VIII) above:

$R^{26}$ is selected from the group consisting of:

a) OH;
b) $OCH_3$;
c) fluorine and
d) chlorine.

$R^1$ is selected from the group consisting of H,

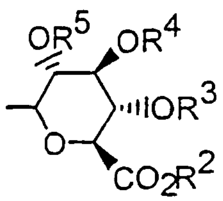 , 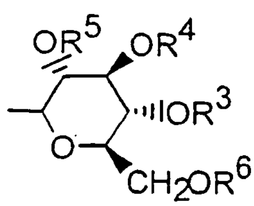 , 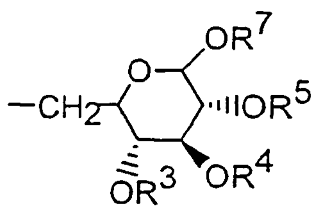 ,

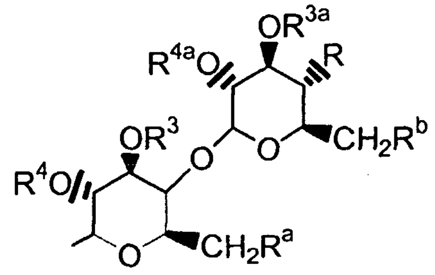 ,

$-SO_3H$; natural and unnatural amino acids.

R, $R^a$ and $R^b$ are independently selected from the group consisting of H, -OH, halogeno, $-NH_2$, azido, $(C_1-C_6)$alkoxy $(C_1-C_6)$-alkoxy and $-W-R^{30}$;

W is independently selected from the group consisting of -NH-C(O)-, -O-C(O)-, $-O-C(O)-N(R^{31})-$, $-NH-C(O)-N(R^{31})-$ and $-O-C(S)-N(R^{31})-$;

$R^2$ and $R^6$ are independently selected from the group consisting of H, $(C_1-C_6)$alkyl, aryl and aryl$(C_1-C_6)$alkyl;

$R^3$, $R^4$, $R^5$, $R^7$, $R^{3a}$ and $R^{4a}$ are independently selected from the group consisting of H, $(C_1-C_6)$alkyl, aryl$(C_1-C_6)$alkyl, $-C(O)(C_1-C_6)$alkyl and -C(O)aryl;

$R^{30}$ is independently selected form the group consisting of $R^{32}$-substituted T, $R^{32}$-substituted-T-$(C_1-C_6)$alkyl, $R^{32}$-substituted-$(C_2-C_4)$alkenyl, $R^{32}$-substituted-$(C_1-C_6)$alkyl, $R^{32}$-substituted-$(C_3$-C7)cycloalkyl and $R^{32}$-substituted-$(C_3$-C7)cycloalkyl$(C_1-C_6)$alkyl;

$R^{31}$ is independently selected from the group consisting of H and $(C_1-C_4)$alkyl;

T is independently selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;

$R^{32}$ is independently selected from 1-3 substituents independently selected from the group consisting of H, halogeno, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsulfinyl, $(C_1-C_4)$alkylsulfonyl, $-N(CH_3)_2$, $-C(O)-NH(C_1-C_4)$alkyt, $-C(O)-N((C_1-C_4)alkyl)_2$, $-C(O)-(C_1-C_4)$alkyl, $-C(O)-(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$alkoxycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^1$ -substituted aryl;

Q is $-(CH_2)q-$, wherein q is 2-6, or, with the 3-position ring carbon of the azetidinone, forms the spiro group

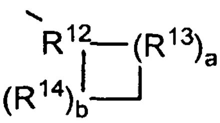 ;

$R^{12}$ is

$$-CH-,\ -C(C_1\text{-}C_6\ alkyl)-,\ -CF-,\ -C(OH)-,\ -C(C_6H_4\text{-}R^{23})-,\ -N-,\ or\ -^{+}NO^{-}\ ;$$

$R^{13}$ and $R^{14}$ are independently selected from the group consisting of $-CH_2-$, $-CH(C_1-C_6$ alkyl)-, $-C(di-(C_1-C_6)$ alkyl), -CH=CH- and $-C(C_1-C_6$ alkyl)=CH-; or $R^{12}$ together with an adjacent $R^{13}$, or $R^{12}$ together with an adjacent $R^{14}$, form a -CH=CH- or a $-CH=C(C_1-C_6$ alkyl)- group;

a and b are independently 0, 1, 2 or 3, provided both are not zero; provided that when $R^{13}$ is -CH=CH- or $-C(C_1-C_6$ alkyl)=CH-, a is 1; provided that when $R^{14}$ is -CH=CH- or $-C(C_1-C_6$ alkyl)=CH-, b is 1; provided that when a is 2 or 3, the $R^{13}$'s can be the same or different; and provided that when b is 2 or 3, the $R^{14}$'s can be the same or different;

$R^{10}$ and $R^{11}$ are independently selected from the group consisting of 1-3 substituents independently selected from the group consisting of $(C_1-C_6)$alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$,

$-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$ alkylene$)-COOR^{19}$, $-CH=CH-COOR^{19}$, $-CF_3$, -CN, $-NO_2$ and halogen;

$Ar^1$ can also be pyridyl, isoxazolyl, furanyl, pyrrolyl, thienyl, imidazolyl, pyrazolyl, thiazolyl, pyrazinyl, pyrimidinyl or pyridazinyl;

$R^{19}$ and $R^{20}$ are independently selected from the group consisting of H, $(C_1-C_6)$alkyl, aryl and aryl-substituted $(C_1-C_6)$ alkyl;

$R^{21}$ is $(C_1-C_6)$alkyl, aryl or $R^{24}$-substituted aryl;

$R^{22}$ is H, $(C_1-C_6)$alkyl, aryl $(C_1-C_6)$alkyl, $-C(O)R^{19}$ or $-COOR^{19}$;

$R^{23}$ and $R^{24}$ are independently 1-3 groups independently selected from the group consisting of H, $(C_1-C_6)$alkyl, $(C_1-C_6)$alkoxy, -COOH, $NO_2$, $-NR^{19}R^{20}$, -OH and halogeno; and

$R^{25}$ is H, -OH or $(C_1-C_6)$alkoxy.

$Ar^2$ is preferably phenyl or $R^{11}$-phenyl, especially $(4-R^{11})$-substituted phenyl. Preferred definitions of $R^{11}$ are lower alkoxy,

especially methoxy, and halogeno, especially fluoro.

$Ar^1$ is preferably phenyl or $R^{10}$-substituted phenyl, especially $(4-R^{10})$-substituted phenyl. A preferred definition of $R^{10}$ is halogeno, especially fluoro.

Preferably Q is a lower alkyl or a spiro group as defined above, wherein preferably $R^{13}$ and $R^{14}$ are each ethylene and $R^{12}$ is

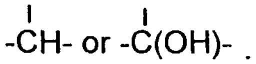

A preferred compound of formula VIII, therefore, is one wherein $R^1$ is as defined above and in which the remaining variables have the following definitions:

$Ar^1$ is phenyl or $R^{10}$-substituted phenyl, wherein $R^{10}$ is halogeno;

$Ar^2$ is phenyl or $R^{11}$-phenyl, wherein $R^{11}$ is 1 to 3 substituents independently selected from the group consisting of $C_1-C_6$ alkoxy and halogeno;

Q is a lower alkyl (i.e. C-1 to C-2) with Q = C-2 being preferred, or Q, with the 3-position ring carbon of the azetidinone, forms the group

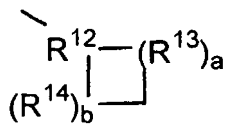

wherein preferably $R^{13}$ and $R^{14}$ are each ethylene and a and b are each 1, and wherein $R^{12}$ is

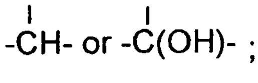

Preferred variables for $R^1$ groups of the formula

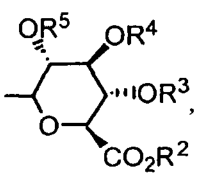, 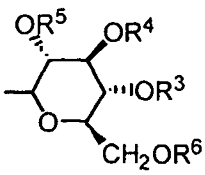 and 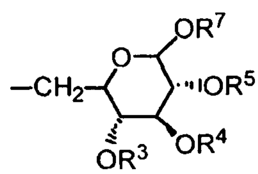

are as follows:

$R^2$, $R^3$, $R^4$, $R^5$, $R^6$ and $R^7$ are independently selected from the group consisting of H, $(C_1-C_6)$alkyl, benzyl and acetyl.

Preferred variables for group $R^1$ of the formula

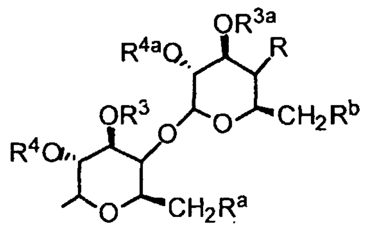

are as follows:

$R^3$, $R^{3a}$, $R^4$ and $R^{4a}$ are selected from the group consisting of H, $(C_{1-}C_6)$alkyl, benzyl and acetyl;
R, $R^a$ and $R^b$ are independently selected from the group consisting of H, -OH, halogeno, $-NH_2$, azido, $(C_1-C_6)$alkoxy $(C_1-C_6)$alkoxy and $-W-R^{30}$, wherein W is -O-C(O)- or $-O-C(O)-NR^{31}-$, $R^{31}$ is H and $R^{30}$ is $(C_1-C_6)$alkyl, $-C(O)-(C_1-C_4)$alkoxy-$(C_{1-}C_6)$alkyl, T , T-$(C_1-C_6)$alkyl, or T or T-$(C_1-C_6)$alkyl wherein T is substituted by one or two halogeno or $(C_1-C_6)$alkyl groups.

Preferred $R^{30}$ substituents are 2-fluorophenyl, 2,4-difluoro-phenyl, 2,6-dichlorophenyl, 2-methylphenyl, 2-thienylmethyl, 2-methoxy-carbonylethyl, thiazol-2-yl-methyl, 2-furyl, 2-methoxycarbonylbutyl and phenyl. Preferred combinations of R, $R^a$ and $R^b$ are as follows: 1) R, $R^a$ and $R^b$ are independently -OH or $-O-C(O)-NH-R^{30}$, especially wherein $R^a$ is -OH and R and $R^b$ are $-O-C(O)-NH-R^{30}$ and $R^{30}$ is selected from the preferred substituents identified above, or wherein R and $R^a$ are- OH and $R^b$ is-$O-C(O)-NH-R^{30}$ wherein $R^{30}$ is 2-fluorophenyl, 2,4-difluoro-phenyl, 2,6-dichlorophenyl; 2) $R^a$ is -OH, halogeno, azido or $(C_1-C_6)$-alkoxy$(C_1-C_6)$alkoxy, $R^b$ is H, halogeno, azido or $(C_1-C_6)$alkoxy $(C_1-C_6)$-alkoxy, and R is $-O-C(O)-NH-R^{30}$, especially compounds wherein $R^a$ is -OH, $R^b$ is H and $R^{30}$ is 2-fluorophenyl; 3) R, $R^a$ and $R^b$ are independently -OH or $-O-C(O)-R^{30}$ and $R^{30}$ is $(C_1-C_6)$alkyl, T , or T substituted by one or two halogeno or $(C_1-C_6)$alkyl groups, especially compounds wherein R is -OH and $R^a$ and $R^b$ are $-O-C(O)-R^{30}$ wherein $R^{30}$ is 2-furyl; and 4) R, $R^a$ and $R^b$ are independently -OH or halogeno. Three additional classes of preferred are compounds are those wherein the $C^{1'}$ anomeric oxy is beta, wherein the $C^{2'}$ anomeric oxy is beta, and wherein the R group is alpha.

$R^1$ is preferably selected from:

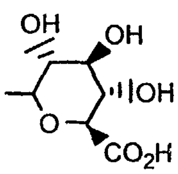 , 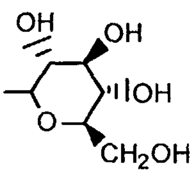 , 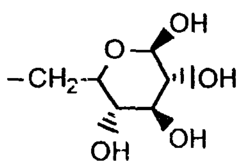 , 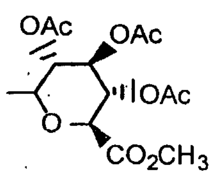 ,

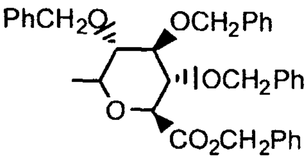 , 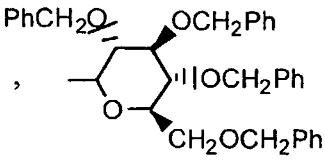 , 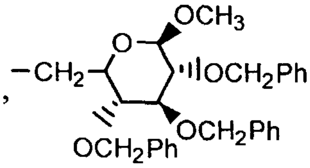 ,

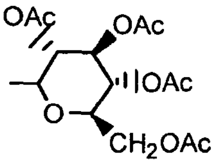 , 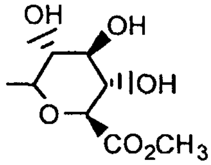 , 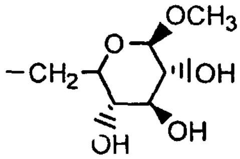 ,

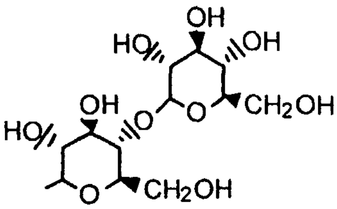 , 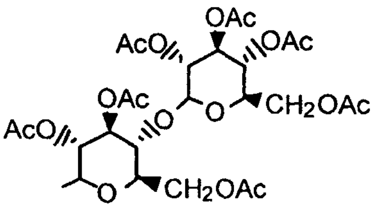

and

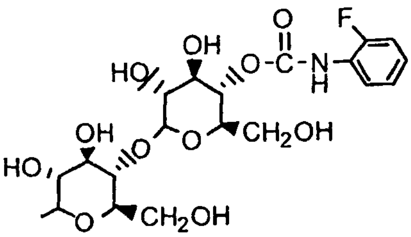

wherein Ac is acetyl and Ph is phenyl.

An example of a useful compound of this invention is one represented by the formula IX:

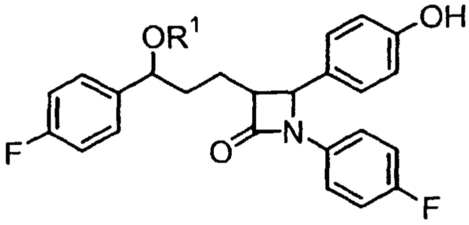

IX

wherein $R^1$ is defined as above, or a pharmaceutically acceptable salt or solvate thereof.

A more preferred compound is one represented by formula X:

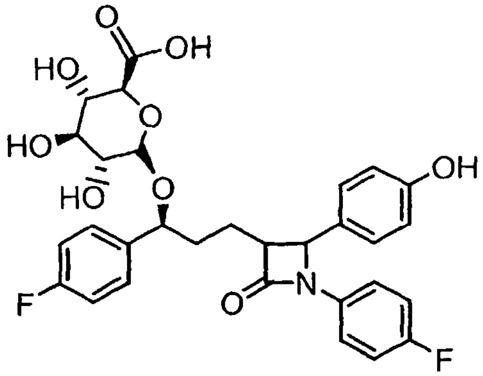

(X)

or a pharmaceutically acceptable salt or solvate thereof.

The compounds of Formulae I-X can be prepared by known methods, including the methods discussed above and, for example, WO 93/02048 describes the preparation of compounds wherein $-R^1$-Q- is alkylene, alkenylene or alkylene interrupted by a hetero atom, phenylene or cycloalkylene; WO 94/17038 describes the preparation of compounds wherein Q is a spirocyclic group; WO 95/08532 describes the preparation of compounds wherein $-R^1$-Q- is a hydroxy-substituted alkylene group; PCT/US95/03196 describes compounds wherein $-R^1$-Q- is a hydroxy-substituted alkylene attached to the $Ar^1$ moiety through an -O- or $S(O)_{0-2}$-group; and U.S. Serial No. 08/463,619, filed June 5, 1995, describes the preparation of compounds wherein $-R^1$-Q- is a hydroxy-substituted alkylene group attached the azetidinone ring by a $-S(O)_{0-2}$- group.

The daily dose of the compound of Formula I-X administered to the subject can range from about 0.1 to about 1000 mg per day, preferably about 0.25 to about 50 mg/day, and more preferably about 10 mg per day, given in a single dose or 2-4 divided doses. The exact dose, however, is determined by the attending clinician and is dependent on the potency of the compound administered, the age, weight, condition and response of the subject.

For administration of pharmaceutically acceptable salts of the above compounds, the weights indicated above refer to the weight of the acid equivalent or the base equivalent of the therapeutic compound derived from the salt.

In another alternative embodiment, the compositions used in the present invention can further comprise one or more Alzheimer's treatments different from compounds I-X discussed above (e.g., different in chemical structure) in combination with one or more of compounds I-X above.

Non-limiting examples of suitable treatments which can be useful in treating Alzheimer's Disease include administration of one or more of the following: cholinesterase inhibitors, muscarinic receptor agonists, M2 muscarinic receptor antagonists, acetylcholine release stimulators, choline uptake stimulators, nicotinic cholinergic receptor agonists, anti-Aβ vaccines, γ-secretase inhibitors, β-secretase inhibitors, amyloid aggregation inhibitors, amyloid precursor protein antisense oligonucleotides, monoamine reuptake inhibitors, human stem cells, gene therapy, nootropic agents, AMPA receptor ligands, growth factors or growth factor receptor agonists, anti-inflammatory agents, free radical scavengers, antioxidants, superoxide dismutase stimulators, calcium channel blockers, apoptosis inhibitors, caspase inhibitors, monoamine oxidase inhibitors, estrogens and estrogen receptor ligands, NMDA receptor antagonists, Jun N-terminal kinase (JNK) inhibitors, copper/zinc chelators, 5-HT1a receptor agonists, NGF stimulators, neuroprotective agents, H3 histamine receptor antagonists, calpain inhibitors, poly ADP ribose polymerase inhibitors, prolylendopeptidase

inhibitors, calcium modulators, corticortropin releasing factor receptor antagonists, corticortropin releasing factor binding protein inhibitors, GABA modulators, GABA-A receptor antagonists, GABA-B receptor antagonists, neuroimmunophilin ligands, sigma receptor ligands, galanin receptor ligands, imidazoline/alpha adrenergic receptor antagonists, vasoactive intestinal peptide receptor agonists, benzodiazepine receptor inverse agonists, cannabinoid receptor agonists, thyrotropin releasing hormone receptor agonists, protein kinase C inhibitors, 5-HT3 receptor antagonists, prostaglandin receptor antagonists, topoisomerase II inhibitors, steroid receptor ligand, nitric oxide modulators, RAGE inhibitors, dopamine receptor agonists, and combinations thereof.

Suitable cholinesterase inhibitors include donepezil hydrochloride (such as ARICEPT which is available from Pfizer), rivastigmine tartrate (such as EXELON which is available from Novartis), tacrine (such as COGNEX which is available from Parke-Davis), galanthamine derivatives available from Janssen, metrifonate available from Bayer Corp., ipidacrine available from Nikken Chemicals Co. Ltd., TAK-147, T-82 available from SS Pharmaceutical Co. Ltd., methanesulfonyl fluoride, CHF-2819, phenserine, physostigmine available from Forest Laboratories, Inc., huperzine, cymserine available from Anonyx Inc., tolserine available from National Institutes of Health, ER-127528 available from Eisai Co. Ltd., and combinations thereof.

Useful muscarinic receptor agonists include cevimeline, PD-151832 available from Pfizer Inc., YM-796 available from Yamanouchi Pharmaceutical Inc., P-58 available from Phytopharm plc and combinations thereof.

Suitable acetylcholine release stimulators include minaprine, montirelin available from Grunenthal GmbH, T-588 available from Toyama Chemical Co. Ltd., XE-991 and combinations thereof. Useful choline uptake stimulators include MKC-231 available from Mitsubishi-Tokyo Pharmaceuticals Inc.

Suitable nicotinic cholinergic receptor agonists include altinicline available from SIBIA Neurosciences Inc., SIB-1553A, ABT-089 (disclosed in U.S. Patent No. 5278176 and available from Abbott Laboratories), nicotine patch, GRS-21, TC-2403 and combinations thereof.

Suitable anti-Aβ vaccines include AN-1792.

Suitable amyloid aggregation inhibitors include reumacon available from Conpharm AB, NC-531 available from Neurochem Inc., PPI-1019 available from Praecis Pharmaceuticals Inc. and combinations thereof.

Suitable monoamine reuptake inhibitors include NS-2330.

Suitable nootropic agents include oxiracetam available from ISF Societa Per Azioni, pramiracetam available from Warner Lambert Co., idebenone available from Takeda Chemical Inds. Ltd., anapsos available from ASAC Pharmaceuticals International, nebracetam available from Boehringer Ingelheim Corp., JTP-2942 available from Japan Tobacco Inc., fasoracetam available from Nippon Shinyaku Co. Ltd., bacosides available from Central Drug Research Institute, alzene available from Bar-Ilan University, KA-672 available from Dr. Willmar Schwabe GmbH & Co., alaptid available from VUFB, IQ-200, ALE-26015 available from Allelix Pharm-Eco LP and combinations thereof.

Useful AMPA receptor ligands include CX-516, CX-691 available from Cortex Pharmaceuticals Inc. and combinations thereof.

Suitable growth factors or growth factor receptor agonists include leteprinim.

Suitable anti-inflammatory agents include COX2 inhibitors (such as VIOXX rofecoxib available from Merck & Co., Inc. and CELEBREX celecoxib available from Pfizer), cytokine inhibitors (such as thalidomide disclosed in WO 95/04533 and dexanabinol) complement inhibitors, leukotriene receptor antagonists and combinations thereof.

Useful free radical scavengers/antioxidants include EGb-761 available from Yuyu Industrial Co., CPI-22, dexanabinol and combinations thereof.

Suitable calcium channel blockers include tamolarizine available from Nippon Chemiphar Co., Ltd., nimodipine available from Bayer AG, PD-176078 available from Elan Pharmaceuticals, Inc., and combinations thereof.

Suitable apoptosis inhibitors include acetyl-L-carnitine, CEP-1347 available from Cephalon, Inc., TCH-346 available from Novartis AG and combinations thereof.

A useful caspase inhibitor is pralnacasan.

Suitable monoamine oxidase inhibitors include moclobemide available from Roche Holding AG, selegiline, rasagiline available from Teva Pharmaceutical Inds. Ltd., SL-25.1188, Ro-41-1049 available from Roche Holding AG, and combinations thereof.

Suitable NMDA receptor antagonists include memantine, ipenoxazone available from Nippon Chemiphar Co. Ltd. and combinations thereof.

Suitable copper/zinc chelators include clioquinol available from PN Gerolymatos SA.

A useful 5-HT1a receptor agonist is AP-159 available from Asahi Kasei Corp.; a suitable NGF stimulator is xaliprodene available from Sanofi-Synthelabo.

Suitable neuroprotective agents include citicholine, GS-1590 available from Leo Pharmaceutical Products Ltd. A/S, CPI-1189 available from Centaur Pharmaceuticals Inc., SR-57667 available from Sanofi-Synthelabo and combinations thereof.

Suitable H3 histamine receptor antagonists include GT-2016 and GT-2331 (both available from Gliatech, Inc.) and combinations thereof.

Useful prolylendopeptidase inhibitors include ONO-1603 available from Ono Pharmaceutical Co. Ltd., Z-321 available from Zeria Pharmaceutical Co. Ltd. and combinations thereof.

A useful calcium modulator includes neurocalc available from Apollo Biopharmaceuticals Inc.

A suitable corticortropin releasing factor receptor antagonist includes NBt-113 available from Neurocrine Biosciences, Inc.

A useful GABA modulator includes NGD 97-1 available from Neurogen Corp.

A suitable sigma receptor ligand is igmesine available from Pfizer Inc.

A useful imidazoline/alpha adrenergic receptor antagonist is efaroxan available from Reckitt & Colman PLC.

A suitable vasoactive intestinal peptide receptor agonist is stearyl-Nle-VIP.

A useful benzodiazepine inverse agonist is S-8510 available from Shionogi & Co. Ltd.

A suitable cannabinoid receptor agonist is dronabinol available from Unimed Pharmaceuticals Inc.

Useful thyrotropin releasing hormone receptor agonists include taltireline available from Tanabe Seiyaku Co. Ltd. and protirelin available from Takeda Chemical Inds., Inc.

A suitable 5-HT3 antagonist is GYKI-46903.

A useful topoisomerase II inhibitor is iododoxorubicin available from Pharmacia & Upjohn AB.

A suitable steroid receptor agonist is GL-701 available from Leland Stanford Junior University.

A useful corticosteroid receptor antagonist is anticort.

A suitable nitric oxide modulator is GL-701.

A suitable RAGE inhibitor is ALT-711 available from Alteon Inc.

A useful dopamine receptor agonist is speramine.

Generally, a total daily dosage of Alzheimer's treatment(s) different from compounds I-X as discussed above can range from about 0.01 to about 1000 mg/day, and preferably about 0.5 to about 20 mg/kg body weight/day in single or divided doses.

In another embodiment of the present invention, the compositions used in the present invention can further comprise one or more pharmacological or therapeutic agents or drugs such as cholesterol biosynthesis inhibitors and/or other lipid-lowering agents, as discussed below.

Non-limiting examples of suitable cholesterol biosynthesis inhibitors include competitive inhibitors of HMG CoA reductase, the rate-limiting step in cholesterol biosynthesis, squalene synthase inhibitors, squalene epoxidase inhibitors and mixtures thereof. Non-limiting examples of suitable HMG CoA reductase inhibitors include statins such as lovastatin (for example MEVACOR® which is available from Merck & Co.), pravastatin (for example PRAVACHOL® which is available from Bristol Meyers Squibb), fluvastatin, simvastatin (for example ZOCOR® which is available from Merck & Co.), atorvastatin, cerivastatin, CI-981 and pitavastatin (such as NK-104 of Negma Kowa of Japan), rosuvastatin; HMG CoA synthetase inhibitors, for example L-659,699 ((E,E)-11-[3'R-(hydroxy-methyl)-4'-oxo-2'R-oxetanyl]-3,5,7R-trimethyl-2,4-undecadienoic acid); squalene synthesis inhibitors, for example squalestatin 1; and squalene epoxidase inhibitors, for example, NB-598 ((E)-N-ethyl-N-(6,6-dimethyl-2-hepten-4-ynyl)-3-[(3,3'-bithiophen-5-yl)methoxy]benzene-methanamine hydrochloride) and other sterol biosynthesis inhibitors such as DMP-565. Preferred HMG CoA reductase inhibitors include lovastatin, pravastatin and simvastatin. The most preferred HMG CoA reductase inhibitor is simvastatin.

Generally, a total daily dosage of cholesterol biosynthesis inhibitor(s) can range from about 0.1 to about 160 mg per day, and preferably about 0.2 to about 80 mg/day in single or 2-3 divided doses.

In another embodiment, the compositions used in the present invention can further comprise one or more other lipid lowering agents as discussed below. For example, one or more PPAR activators, can be coadministered with or in combination with the compound(s) of Formulae I-X discussed above.

Also useful in the present invention are compositions or therapeutic combinations that further comprise at least one (one or more) activators for peroxisome proliferator-activated receptors (PPAR). The activators act as agonists for the peroxisome proliferator-activated receptors. Three subtypes of PPAR have been identified, and these are designated as peroxisome proliferator-activated receptor alpha (PPAR$\alpha$), peroxisome proliferator-activated receptor gamma (PPAR$\gamma$) and peroxisome proliferator-activated receptor delta (PPAR$\delta$). It should be noted that PPAR$\delta$ is also referred to in the literature as PPAR$\beta$ and as NUC 1, and each of these names refers to the same receptor.

PPAR$\alpha$ regulates the metabolism of lipids. PPAR$\alpha$ is activated by fibrates and a number of medium and long-chain fatty acids, and it is involved in stimulating $\beta$-oxidation of fatty acids. The PPAR$\gamma$ receptor subtypes are involved in activating the program of adipocyte differentiation and are not involved in stimulating peroxisome proliferation in the liver. PPAR$\delta$ has been identified as being useful in increasing high density lipoprotein (HDL) levels in humans. See, e.g., WO 97/28149.

PPAR$\alpha$ activator compounds are useful for, among other things, lowering triglycerides, moderately lowering LDL levels and increasing HDL levels. Useful examples of PPAR$\alpha$ activators include fibrates.

Non-limiting examples of suitable fibric acid derivatives ("fibrates") include clofibrate (such as ethyl 2-(p-chlorophenoxy)-2-methyl-propionate, for example ATROMID-S® Capsules which are commercially available from Wyeth-Ayerst); gemfibrozil (such as.5-(2,5-dimethylphenoxy)-2,2-dimethylpentanoic acid, for example LOPID® tablets which

are commercially available from Parke Davis); ciprofibrate (C.A.S. Registry No. 52214-84-3, see U.S. Patent No. 3,948,973 which is incorporated herein by reference); bezafibrate (C.A.S. Registry No. 41859-67-0, see U.S. Patent No. 3,781,328 which is incorporated herein by reference); clinofibrate (C.A.S. Registry No. 30299-08-2, see U.S. Patent No. 3,716,583 which is incorporated herein by reference); binifibrate (C.A.S. Registry No. 69047-39-8, see BE 884722 which is incorporated herein by reference); lifibrol (C.A.S. Registry No. 96609-16-4); fenofibrate (such as TRICOR® micronized fenofibrate (2-[4-(4-chlorobenzoyl) phenoxy]-2-methyl-propanoic acid, 1-methylethyl ester) which is commercially available from Abbott Laboratories or LIPANTHYL® micronized fenofibrate which is commercially available from Labortoire Founier, France) and mixtures thereof. These compounds can be used in a variety of forms, including but not limited to acid form, salt form, racemates, enantiomers, zwitterions and tautomers.

Other examples of PPARα activators useful in the practice of the present invention include suitable fluorophenyl compounds as disclosed in U.S. No. 6,028,109 which is incorporated herein by reference; certain substituted phenylpropionic compounds as disclosed in WO 00/75103 which is incorporated herein by reference; and PPARα activator compounds as disclosed in WO 98/43081 which is incorporated herein by reference.

Non-limiting examples of suitable PPARγ activators include derivatives of glitazones or thiazolidinediones, such as, troglitazone (such as REZULIN® troglitazone (-5-[[4-[3,4-dihydro-6-hyd roxy-2,5,7,8-tetramethyl-2H-1-benzopyran-2-yl)methoxy]phenyl] methyl]-2,4-thiazolidinedione) commercially available from Parke-Davis); rosiglitazone (such as AVANDIA® rosiglitazone maleate (-5-[[4-[2-(methyl-2-pyridinylamino)ethoxy] phenyl] methyl]-2,4-thiazolidinedione, (Z)-2-butenedioate) (1:1) commercially available from SmithKline Beecham) and pioglitazone (such as ACTOS™ pioglitazone hydrochloride (5-[[4-[2-(5-ethyl-2-pyridinyl)ethoxy]phenyl]methyl]-2,4-] thiazolidinedione monohydrochloride) commercially available from Takeda Pharmaceuticals). Other useful thiazolidinediones include ciglitazone, englitazone, darglitazone and BRL 49653 as disclosed in WO 98/05331 which is incorporated herein by reference; PPARγ activator compounds disclosed in WO 00/76488 which is incorporated herein by reference; and PPARy activator compounds disclosed in U.S. Patent No. 5,994,554 which is incorporated herein by reference.

Other useful PPARγ activator compounds include certain acetylphenols as disclosed in U.S. Patent No. 5,859,051 which is incorporated herein by reference; certain quinoline phenyl compounds as disclosed in WO 99/20275 which is incorporated herein by reference; aryl compounds as disclosed by WO 99/38845 which is incorporated herein by reference; certain 1,4-disubstituted phenyl compounds as disclosed in WO 00/63161; certain aryl compounds as disclosed in WO 01/00579 which is incorporated herein by reference; benzoic acid compounds as disclosed in WO 01/12612 & WO 01/12187 which are incorporated herein by reference; and substituted 4-hydroxy-phenylalconic acid compounds as disclosed in WO 97/31907 which is incorporated herein by reference.

PPARδ compounds are useful for, among other things, lowering triglyceride levels or raising HDL levels. Non-limiting examples of PPARδ activators include suitable thiazole and oxazole derivatives, such as C.A.S. Registry No. 317318-32-4, as disclosed in WO 01/00603 which is incorporated herein by reference); certain fluoro, chloro or thio phenoxy phenylacetic acids as disclosed in WO 97/28149 which is incorporated herein by reference; suitable non-β-oxidizable fatty acid analogues as disclosed in U.S. Patent No. 5,093,365 which is incorporated herein by reference; and PPARδ compounds as disclosed in WO 99/04815 which is incorporated herein by reference.

Moreover, compounds that have multiple functionality for activating various combinations of PPARα, PPARγ and PPARδ are also useful with the practice of the present invention. Non-limiting examples include certain substituted aryl compounds as disclosed in U.S. Patent No. 6,248,781; WO 00/23416; WO 00/23415; WO 00/23425; WO 00/23445; WO 00/23451; and WO 00/63153, all of which are incorporated herein by reference, are described as being useful PPARα and/or PPARγ activator compounds. Other non-limiting examples of useful PPARα and/or PPARγ activator compounds include activator compounds as disclosed in WO 97/25042 which is incorporated herein by reference; activator compounds as disclosed in WO 00/63190 which is incorporated herein by reference; activator compounds as disclosed in WO 01/21181 which is incorporated herein by reference; biaryl-oxa(thia)zole compounds as disclosed in WO 01/16120 which is incorporated herein by reference; compounds as disclosed in WO 00/63196 and WO 00/63209 which are incorporated herein by reference; substituted 5-aryl-2,4-thiazolidinediones compounds as disclosed in U.S. Patent No. 6,008,237 which is incorporated herein by reference; arylthiazolidinedione and aryloxazolidinedione compounds as disclosed in WO 00/78312 and WO 00/78313G which are incorporated herein by reference; GW2331 or (2-(4-[difluorophenyl]-1heptylureido)ethyl]phenoxy)-2-methylbutyric compounds as disclosed in WO 98/05331 which is incorporated herein by reference; aryl compounds as disclosed in U.S. Patent No. 6,166,049 which is incorporated wherein by reference; oxazole compounds as disclosed in WO 01/17994 which is incorporated herein by reference; and dithiolane compounds as disclosed in WO 01/25225 and WO 01/25226 which are incorporated herein by reference.

Other useful PPAR activator compounds include substituted benzylthiazolidine-2,4-dione compounds as disclosed in WO 01/14349, WO 01/14350 and WO/01/04351 which are incorporated herein by reference; mercaptocarboxylic compounds as disclosed in WO 00/50392 which is incorporated herein by reference; ascofuranone compounds as disclosed in WO 00/53563 which is incorporated herein by reference; carboxylic compounds as disclosed in WO 99/46232 which is incorporated herein by reference; compounds as disclosed in WO 99/12534 which is incorporated herein by reference; benzene compounds as disclosed in WO99/15520 which is incorporated herein by reference; o-antsamide

compounds as disclosed in WO 01/21578 which is incorporated herein by reference: and PPAR activator compounds as disclosed in WO 01/40192 which is incorporated herein by reference.

The peroxisome proliferator-activated receptor(s) activator(s) are administered in a therapeutically effective amount to treat the specified condition, for example in a daily dose preferably ranging from about 50 to about 3000 mg per day, and more preferably about 50 to about 2000 mg per day, given in a single dose or 2-4 divided doses. The exact dose, however, is determined by the attending clinician and is dependent on such factors as the potency of the compound administered, the age, weight, condition and response of the subject.

In another alternative embodiment, the compositions used in the present invention can further comprise one or more bile acid sequestrants (insoluble anion exchange resins), coadministered with or in combination with the compound(s) of Formulae I-X discussed above.

Bile acid sequestrants bind bile acids in the intestine, interrupting the enterohepatic circulation of bile acids and causing an increase in the faecal excretion of steroids. Use of bile acid sequestrants is desirable because of their non-systemic mode of action. Bile acid sequestrants can lower intrahepatic cholesterol and promote the synthesis of apo B/E (LDL) receptors which bind LDL from plasma to further reduce cholesterol levels in the blood.

Non-limiting examples of suitable bile acid sequestrants include cholestyramine (a styrene-divinylbenzene copolymer containing quaternary ammonium cationic groups capable of binding bile acids, such as QUESTRAN® or QUESTRAN LIGHT® cholestyramine which are available from Bristol-Myers Squibb), colestipol (a copolymer of diethylenetriamine and 1-chloro-2,3-epoxypropane, such as COLESTID® tablets which are available from Pharmacia), colesevelam hydrochloride (such as WelChol® Tablets (poly(allylamine hydrochloride) cross-linked with epichlorohydrin and alkylated with 1-bromodecane and (6-bromohexyl)-trimethylammonium bromide) which are available from Sankyo), water soluble derivatives such as 3,3-ioene, N-(cycloalkyl) alkylamines and poliglusam, insoluble quaternized polystyrenes, saponins and mixtures thereof. Other useful bile acid sequestrants are disclosed in PCT Patent Applications Nos. WO 97/11345 and WO 98/57652, and U.S. Patents Nos. 3,692,395 and 5,703,188 which are incorporated herein by reference. Suitable inorganic cholesterol sequestrants include bismuth salicylate plus montmorillonite clay, aluminum hydroxide and calcium carbonate antacids.

Generally, a total daily dosage of bile acid sequestrant(s) can range from about 1 to about 50 grams per day, and preferably about 2 to about 16 grams per day in single or 2-4 divided doses.

In an alternative embodiment, the compositions used in the present invention can further comprise one or more ileal bile acid transport ("IBAT") inhibitors (or apical sodium co-dependent bile acid transport ("ASBT") inhibitors) coadministered with or in combination with the compound(s) of Formulae I-X discussed above. The IBAT inhibitors can inhibit bile acid transport to reduce LDL cholesterol levels. Non-limiting examples of suitable IBAT inhibitors include benzothiepines such as therapeutic compounds comprising a 2,3,4,5-tetrahydro-1-benzothiepine 1,1-dioxide structure such as are disclosed in PCT Patent Application WO 00/38727 which is incorporated herein by reference.

Generally, a total daily dosage of IBAT inhibitor(s) can range from about 0.01 to about 1000 mg/day, and preferably about 0.1 to about 50 mg/day in single or 2-4 divided doses.

In another alternative embodiment, the compositions used in the present invention can further comprise nicotinic acid (niacin) and/or derivatives thereof coadministered with or in combination with the compound(s) of Formulae I-X discussed above.

As used herein, "nicotinic acid derivative" means a compound comprising a pyridine-3-carboxylate structure or a pyrazine-2-carboxylate structure, including acid forms, salts, esters, zwitterions and tautomers, where available. Examples of nicotinic acid derivatives include niceritrol, nicofuranose and acipimox (5-methyl pyrazine-2-carboxylic acid 4-oxide). Nicotinic acid and its derivatives inhibit hepatic production of VLDL and its metabolite LDL and increases HDL and apo A-1 levels. An example of a suitable nicotinic acid product is NIASPAN® (niacin extended-release tablets) which are available from Kos.

Generally, a total daily dosage of nicotinic acid or a derivative thereof can range from about 500 to about 10,000 mg/day, preferably about 1000 to about 8000 mg/day, and more preferably about 3000 to about 6000 mg/day in single or divided doses.

In another alternative embodiment, the compositions used in the present invention can further comprise one or more AcylCoA:Cholesterol *O*-acyltransferase ("ACAT") Inhibitors, which can reduce LDL and VLDL levels, coadministered with or in combination with the compound(s) of Formulae I-X discussed above. ACAT is an enzyme responsible for esterifying excess intracellular cholesterol and may reduce the synthesis of VLDL, which is a product of cholesterol esterification, and overproduction of apo B-100-containing lipoproteins.

Non-limiting examples of useful ACAT inhibitors include avasimibe ([[2,4,6-tris(1-methylethyl)phenyl]acetyl] sulfamic acid, 2,6-bis(1-methylethyl)phenyl ester, formerly known as Cl-1011), HL-004, lecimibide (DuP-128) and CL-277082 (N-(2,4-difluorophenyl)-*N*-[[4-(2,2-dimethylpropyl)phenyl]methyl]-*N*-heptylurea). <u>See</u> P. Chang et al., "Current, New and Future Treatments in Dyslipidaemia and Atherosclerosis", <u>Drugs</u> 2000 Jul;60(1); 55-93, which is incorporated by reference herein.

Generally, a total daily dosage of ACAT inhibitor(s) can range from about 0.1 to about 1000 mg/day in single

or 2-4 divided doses.

In another alternative embodiment, the compositions used in the present invention can further comprise one or more Cholesteryl Ester Transfer Protein ("CETP") Inhibitors coadministered with or in combination with the compound(s) of Formulae I-X discussed above. CETP is responsible for the exchange or transfer of cholesteryl ester carrying HDL and triglycerides in VLDL.

Non-limiting examples of suitable CETP inhibitors are disclosed in PCT Patent Application No. WO 00/38721 and U.S. Patent No. 6,147,090, which are incorporated herein by reference. Pancreatic cholesteryl ester hydrolase (pCEH) inhibitors such as WAY-121898 also can be coadministered with or in combination with the fibric acid derivative(s) and sterol absorption inhibitor(s) discussed above.

Generally, a total daily dosage of CETP inhibitor(s) can range from about 0.01 to about 1000 mg/day, and preferably about 0.5 to about 20 mg/kg body weight/day in single or divided doses.

In another alternative embodiment, the compositions used in the present invention can further comprise probucol or derivatives thereof (such as AGI-1067 and other derivatives disclosed in U.S. Patents Nos. 6,121,319 and 6,147,250), which can reduce LDL and HDL levels, coadministered with or in combination with the compound(s) of Formula I-X discussed above.

Generally, a total daily dosage of probucol or derivatives thereof can range from about 10 to about 2000 mg/day, and preferably about 500 to about 1500 mg/day in single or 2-4 divided doses.

In another alternative embodiment, the compositions used in the present invention can further comprise one or more low-density lipoprotein (LDL) receptor activators, coadministered with or in combination with the compound(s) of Formulae I-X discussed above. Non-limiting examples of suitable LDL-receptor activators include HOE-402, an imidazolidinyl-pyrimidine derivative that directly stimulates LDL receptor activity. See M. Huettinger et al., "Hypolipidemic activity of HOE-402 is Mediated by Stimulation of the LDL Receptor Pathway", Arterioscler. Thromb. 1993; 13:1005-12.

Generally, a total daily dosage of LDL receptor activator(s) can range from about 1 to about 1000 mg/day in single or 2-4 divided doses.

In another alternative embodiment, the compositions used in the present invention can further comprise fish oil, which contains Omega 3 fatty acids (3-PUFA), which can reduce VLDL and triglyceride levels, coadministered with or in combination with the compound(s) of Formulae I-X discussed above. Generally, a total daily dosage of fish oil or Omega 3 fatty acids can range from about 1 to about 30 grams per day in single or 2-4 divided doses.

In another alternative embodiment, the compositions used in the present invention can further comprise natural water soluble fibers, such as psyllium, guar, oat and pectin, which can reduce cholesterol levels, coadministered with or in combination with the compound(s) of Formulae I-X discussed above. Generally, a total daily dosage of natural water soluble fibers can range from about 0.1 to about 10 grams per day in single or 2-4 divided doses.

In another alternative embodiment, the compositions used in the present invention can further comprise plant sterols, plant stanols and/or fatty acid esters of plant stanols, such as sitostanol ester used in BENECOL® margarine, which can reduce cholesterol levels, coadministered with or in combination with the compound(s) of Formulae I-X discussed above. Generally, a total daily dosage of plant sterols, plant stanols and/or fatty acid esters of plant stanols can range from about 0.5 to about 20 grams per day in single or 2-4 divided doses.

In another alternative embodiment, the compositions used in the present invention can further comprise one or more antioxidants, such as probucol, tocopherol, ascorbic acid, β-carotene and selenium, or vitamins such as vitamin $B_6$ or vitamin $B_{12}$. coadministered with or in combination with the compound(s) of Formulae I-X discussed above. Generally, a total daily dosage of antioxidants or vitamins can range from about 0.05 to about 10 grams per day in single or 2-4 divided doses.

In another alternative embodiment, the compositions used in the present invention can further comprise monocyte and macrophage inhibitors such as polyunsaturated fatty acids (PUFA), thyroid hormones including throxine analogues such as CGS-26214 (a thyroxine compound with a fluorinated ring), gene therapy and use of recombinant proteins such as recombinant apo E, coadministered with or in combination with the compound(s) of Formulae I-X discussed above. Generally, a total daily dosage of these agents can range from about 0.01 to about 1000 mg/day in single or 2-4 divided doses.

Also useful with the present invention are compositions or therapeutic combinations that further comprise hormone replacement agents and compositions. Useful hormone agents and compositions include androgens, estrogens, progestins, their pharmaceutically acceptable salts and derivatives. Combinations of these agents and compositions also are useful.

The dosage of androgen and estrogen combinations vary, desirably from about 1 mg to about 4 mg androgen and from about 1 mg to about 3 mg estrogen. Examples include, but are not limited to, androgen and estrogen combinations such as the combination of esterified estrogens (sodium estrone sulfate and sodium equilin sulfate) and methyltestosterone (17-hydroxy-17-methyl-, (17B)- androst-4-en-3-one) available from Solvay Pharmaceuticals, Inc., Marietta, GA, under the tradename ESTRATEST.

Estrogens and estrogen combinations may vary in dosage from about 0.01 mg up to 8 mg, desirably from about

0.3 mg to about 3.0 mg. Examples of useful estrogens and estrogen combinations include:

(a) the blend of nine (9) synthetic estrogenic substances including sodium estrone sulfate, sodium equilin sulfate, sodium 17 α -dihydroequilin sulfate, sodium 17 α -estradiol sulfate, sodium 17 β-dihydroequilin sulfate, sodium 17 α-dihydroequilenin sulfate, sodium 17 β -dihydroequilenin sulfate, sodium equilenin sulfate and sodium 17 β -estradiol sulfate; available from Duramed Pharmaceuticals, Inc., Cincinnati, OH, under the tradename CENESTIN;
(b) ethinyl estradiol (19-nor-17α-pregna-1,3,5(10)-trien-20-yne-3,17-diol; available by Schering Plough Corporation, Kenilworth, NJ, under the tradename ESTINYL;
(c) esterified estrogen combinations such as sodium estrone sulfate and sodium equilin sulfate; available from Solvay under the tradename ESTRATAB and from Monarch Pharmaceuticals, Bristol, TN, under the tradename MENEST;
(d) estropipate (piperazine estra-1,3,5(10)-trien-17-one, 3-(sulfooxy)-estrone sulfate); available from Pharmacia & Upjohn, Peapack, NJ, under the tradename OGEN and from Women First Health Care, Inc., San Diego, CA, under the tradename ORTHO-EST; and
(e) conjugated estrogens (17 α-dihydroequilin, 17 α-estradiol, and 17 β-dihydroequilin); available from Wyeth-Ayerst Pharmaceuticals, Philadelphia, PA, under the tradename PREMARIN.

Progestins and estrogens may also be administered with a variety of dosages, generally from about .05 to about 2.0 mg progestin and about .001 mg to about 2 mg estrogen, desirably from about .1 mg to about 1 mg progestin and about 0.01 mg to about .5 mg estrogen. Examples of progestin and estrogen combinations that may vary in dosage and regimen include:

(a) the combination of estradiol (estra-1, 3, 5 (10)-triene-3, 17 β-diol hemihydrate) and norethindrone (17 β-acetoxy-19-nor-17 α-pregn-4-en-20-yn-3-one); which is available from Pharmacia & Upjohn, Peapack, NJ, under the tradename ACTIVELLA;
(b) the combination of levonorgestrel (d(-)-13 β-ethyl-17 α-ethinyl-17 β-hydroxygon- 4-en-3-one) and ethinyl estradial; available from Wyeth-Ayerst under the tradename ALESSE, from Watson Laboratories, Inc., Corona, CA, under the tradenames LEVORA and TRIVORA, Monarch Pharmaceuticals, under the tradename NORDETTE, and from Wyeth-Ayerst under the tradename TRIPHASIL;
(c) the combination of ethynodiol diacetate (19-nor-17 α-pregn-4-en-20-yne-3 β, 17-diol diacetate) and ethinyl estradiol; available from G.D. Searle & Co., Chicago, IL, under the tradename DEMULEN and from Watson under the tradename ZOVIA;
(d) the combination of desogestrel (13-ethyl-11- methylene-18,19-dinor-17 α-pregn- 4-en- 20-yn-17-ol) and ethinyl estradiol; available from Organon under the tradenames DESOGEN and MIRCETTE, and from Ortho-McNeil Pharmaceutical, Raritan, NJ, under the tradename ORTHO-CEPT;
(e) the combination of norethindrone and ethinyl estradiol; available from Parke-Davis, Morris Plains, NJ, under the tradenames ESTROSTEP and FEMHRT, from Watson under the tradenames MICROGESTIN, NECON, and TRI-NORINYL, from Ortho-McNeil under the tradenames MODICON and ORTHO-NOVUM, and from Warner Chilcott Laboratories, Rockaway, NJ, under the tradename OVCON;
(f) the combination of norgestrel ((±)-13-ethyl-17-hydroxy-18, 19-dinor-17 α-preg-4-en-20-yn-3-one) and ethinyl estradiol; available from Wyeth-Ayerst under the tradenames OVRAL and LO/OVRAL, and from Watson under the tradenames OGESTREL and LOW-OGESTREL;
(g) the combination of norethindrone, ethinyl estradiol, and mestranol (3-methoxy-19-nor-17 α-pregna-1,3,5(10)-trien-20-yn-17-ol); available from Watson under the tradenames BREVICON and NORINYL;
(h) the combination of 17 β-estradiol (estra-1,3,5(10)-triene-3,17 β-diol) and micronized norgestimate (17 α-17-(Acetyloxyl)-13-ethyl-18,19-dinorpregn-4-en-20-yn-3-one3-oxime); available from Ortho-McNeil under the tradename ORTHO-PREFEST;
(i) the combination of norgestimate (18,19-dinor-17-pregn-4-en-20-yn-3-one, 17-(acetyloxy)-13-ethyl-,oxime, (17 (α)-(+)-) and ethinyl estradiol; available from Ortho-McNeil under the tradenames ORTHO CYCLEN and ORTHO TRI-CYCLEN; and
(j) the combination of conjugated estrogens (sodium estrone sulfate and sodium equilin sulfate) and medroxyprogesterone acetate (20-dione, 17-(acetyloxy)-6-methyl-, (6(α))- pregn-4-ene-3); available from Wyeth-Ayerst under the tradenames PREMPHASE and PREMPRO.

In general, a dosage of progestins may vary from about .05 mg to about 10 mg or up to about 200 mg if microsized progesterone is administered. Examples of progestins include norethindrone; available from ESI Lederle, Inc., Philadelphia, PA, under the tradename AYGESTIN, from Ortho-McNeil under the tradename MICRONOR, and from Watson under the tradename NOR-QD; norgestrel; available from Wyeth-Ayerst under the tradename OVRETTE;

micronized progesterone (pregn-4-ene-3, 20-dione); available from Solvay under the tradename PROMETRIUM; and medroxyprogesterone acetate; available from Pharmacia & Upjohn under the tradename PROVERA.

The compositions or therapeutic combinations used in the present invention can further comprise one or more obesity control medications. Useful obesity control medications include, but are not limited to, drugs that reduce energy intake or suppress appetite, drugs that increase energy expenditure and nutrient-partitioning agents. Suitable obesity control medications include, but are not limited to, noradrenergic agents (such as diethylpropion, mazindol, phenylpropanolamine, phentermine, phendimetrazine, phendamine tartrate, methamphetamine, phendimetrazine and tartrate); serotonergic agents (such as sibutramine, fenfluramine, dexfenfluramine, fluoxetine, fluvoxamine and paroxtine); thermogenic agents (such as ephedrine, caffeine, theophylline, and selective 3-adrenergic agonists); an alpha-blocking agent; a kainite or AMPA receptor antagonist; a leptin-lipolysis stimulated receptor; a phosphodiesterase enzyme inhibitor; a compound having nucleotide sequences of the mahogany gene; a fibroblast growth factor-10 polypeptide; a monoamine oxidase inhibitor (such as befloxatone, moclobemide, brofaromine, phenoxathine, esuprone, befol, toloxatone, pirlindol, amiflamine, sercloremine, bazinaprine, lazabemide, mitacemide and caroxazone); a compound for increasing lipid metabolism (such as evodiamine compounds); and a lipase inhibitor (such as orlistat). Generally, a total dosage of the above-described obesity control medications can range from 1 to 3,000 mg/day, desirably from about 1 to 1,000 mg/day and more desirably from about 1 to 200 mg/day in single or 2-4 divided doses.

The compositions or therapeutic combinations used in the present invention can further comprise one or more blood modifiers. Useful blood modifiers include but are not limited to anti-coagulants (argatroban, bivalirudin, dalteparin sodium, desirudin, dicumarol, lyapolate sodium, nafamostat mesylate, phenprocoumon, tinzaparin sodium, warfarin sodium); antithrombotic (anagrelide hydrochloride, bivalirudin, cilostazol, dalteparin sodium, danaparoid sodium, dazoxiben hydrochloride, efegatran sulfate, enoxaparin sodium, fluretofen, ifetroban, ifetroban sodium, lamifiban, lotrafiban hydrochloride, napsagatran, orbofiban acetate, roxifiban acetate, sibrafiban, tinzaparin sodium, trifenagrel, abciximab, zolimomab aritox); fibrinogen receptor antagonists (roxifiban acetate, fradafiban, orbofiban, lotrafiban hydrochloride, tirofiban, xemilofiban, monoclonal antibody 7E3, sibrafiban); platelet inhibitors (cilostazol, clopidogrel bisulfate, epoprostenol, epoprostenol sodium, ticlopidine hydrochloride, aspirin, ibuprofen, naproxen, sulindae, idomethacin, mefenamate, droxicam, diclofenac, sulfinpyrazone, piroxicam, dipyridamole); platelet aggregation inhibitors (acadesine, beraprost, beraprost sodium, ciprostene calcium, itazigrel, lifarizine, lotrafiban hydrochloride, orbofiban acetate, oxagrelate, fradafiban, orbofiban, tirofiban, xemilofiban); hemorrheologic agents (pentoxifylline); lipoprotein associated coagulation inhibitor, Factor VIIa inhibitors (4H-31-benzoxazin-4-ones, 4H-3,1-benzoxazin-4-thiones, quinazolin-4-ones, quinazolin-4-thiones, benzothiazin-4-ones, imidazolyl-boronic acid-derived peptide analogues TFPI-derived peptides, naphthalene-2-sulfonic acid {1-[3-(aminoiminomethyl)-benzyl]-2-oxo-pyrrolidin-3-(S)-yl} amide trifluoroacetate, dibenzofuran-2-sulfonic acid {1-[3-(aminomethyl)-benzyl]-5-oxo-pyrrolidin-3-yl}-amide, tolulene-4-sulfonic acid {1-[3-(aminoirninomethyl)-benzyl]-2-oxo-pyrrolidin-3-(S)-yl}-amide trifluoroacetate, 3,4-dihydro-1 H-isoquinoline-2-sulfonic acid {1-[3-(aminoiminomethyl)-benzyl]-2-oxo-pyrrolin-3-(S)-yl}-amide trifluoroacetate); Factor Xa inhibitors (disubstituted pyrazolines, disubstituted triazolines, substituted n-[(aminoiminomethyl)phenyl] propylamides, substituted n-[(aminomethyl)phenyl] propylamides, tissue factor pathway inhibitor (TFPI), low molecular weight heparins, heparinoids, benzimidazolines, benzoxazolinones, benzopiperazinones, indanones, dibasic (amidinoaryl) propanoic acid derivatives, amidinophenyl-pyrrolidines, amidinophenyl-pyrrolines, amidinophenyl-isoxazolidines, amidinoindoles, amidinoazoles, bis-arlysulfonylaminobenzamide derivatives, peptidic Factor Xa inhibitors).

The compositions or therapeutic combinations used in the present invention can further comprise one or more cardiovascular agents. Useful cardiovascular agents include but are not limited to calcium channel blockers (clentiazem maleate, amlodipine besylate, isradipine, nimodipine, felodipine, nilvadipine, nifedipine, teludipine hydrochloride, diltiazem hydrochloride, belfosdil, verapamil hydrochloride, fostedil); adrenergic blockers (fenspiride hydrochloride, labetalol hydrochloride, proroxan, alfuzosin hydrochloride, acebutolol, acebutolol hydrochloride, alprenolol hydrochloride, atenolol, bunolol hydrochloride, carteolol hydrochloride, celiprolol hydrochloride, cetamolol hydrochloride, cicloprolol hydrochloride, dexpropranolol hydrochloride, diacetolol hydrochloride, dilevalol hydrochloride, esmolol hydrochloride, exaprolol hydrochloride, flestolol sulfate, labetalol hydrochloride, levobetaxolol hydrochloride, levobunolol hydrochloride, metalol hydrochloride, metoprolol, metoprolol tartrate, nadolol, pamatolol sulfate, penbutolol sulfate, practolol, propranolol hydrochloride, sotalol hydrochloride, timolol, timolol maleate, tiprenolol hydrochloride, tolamolol, bisoprolol, bisoprolol fumarate, nebivolol); adrenergic stimulants; angiotensin converting enzyme (ACE) inhibitors (benazepril hydrochloride, benazeprilat, captopril, delapril hydrochloride, fosinopril sodium, libenzapril, moexipril hydrochloride, pentopril, perindopril, quinapril hydrochloride, quinaprilat, ramipril, spirapril hydrochloride, spiraprilat, teprotide, enalapril maleate, lisinopril, zofenopril calcium, perindopril erbumine); antihypertensive agents (althiazide, benzthiazide, captopril, carvedilol, chlorothiazide sodium, clonidine hydrochloride, cyclothiazide, delapril hydrochloride, dilevalol hydrochloride, doxazosin mesylate, fosinopril sodium, guanfacine hydrochloride, methyldopa, metoprolol succinate, moexipril hydrochloride, monatepil maleate, pelanserin hydrochloride, phenoxybenzamine hydrochloride, prazosin hydrochloride, primidolol, quinapril hydrochloride, quinaprilat, ramipril, terazosin hydrochloride, candesartan, candesartan cilexetil, telmisartan, amlodipine besylate, amlodipine maleate, bevantolol hydrochloride); angiotensin II receptor antagonists (candesartan, irbesartan, losartan potassium, candesartan cilexetil, telmisartan); anti-anginal agents (amlodipine besylate, amlodipine maleate, betaxolol hydrochloride, bevantolol hydrochloride, butoprozine hydrochloride, carvedilol, cinepazet maleate, metoprolol succinate, molsidomine, monatepil maleate, primidolol, ranolazine hydrochoride, tosifen, verapamil hydrochloride); coronary vasodilators (fostedil, azaclorzine hydrochloride, chromonar hydrochloride, clonitrate, diltiazem hydrochloride, dipyridamole, droprenilamine, erythrityl tetranitrate, isosorbide dinitrate, isosorbide mononitrate, lidoflazine, mioflazine hydrochloride, mixidine, molsidomine, nicorandil, nifedipine, nisoldipine, nitroglycerine, oxprenolol hydrochloride, pentrinitrol, perhexiline maleate, prenylamine, propatyl nitrate, terodiline hydrochloride, tolamolol, verapamil); diuretics (the combination product of hydrochlorothiazide and spironolactone and the combination product of hydrochlorothiazide and triamterene).

The compositions or therapeutic combinations used in the present invention can further comprise one or more antidiabetic medications for reducing blood glucose levels in a human. Useful antidiabetic medications include, but are not limited to, drugs that reduce energy intake or suppress appetite, drugs that increase energy expenditure and nutrient-partitioning agents. Suitable antidiabetic medications include, but are not limited to, sulfonylurea (such as acetohexamide, chlorpropamide, gliamilide, gliclazide, glimepiride, glipizide, glyburide, glibenclamide, tolazamide, and tolbutamide), meglitinide (such as repaglinide and nateglinide), biguanide (such as metformin and buformin), thiazolidinedione (such as troglitazone, rosiglitazone, pioglitazone, ciglitazone, englitazone, and darglitazone), alpha-glucosidase inhibitor (such as acarbose, miglitol, camiglibose, and voglibose), certain peptides (such as amlintide, pramlintide, exendin, and GLP-1 agonistic peptides), and orally administrable insulin or insulin composition for intestinal delivery thereof. Generally, a total dosage of the above-described antidiabetic medications can range from 0.1 to 1,000 mg/day in single or 2-4 divided doses.

Mixtures of any of the pharmacological or therapeutic agents described above can be included in the compositions used in the methods of the present invention.

The compositions used in the present invention can be administered to a subject in need of such treatment in a therapeutically effective amount to treat AD, regulate levels of amyloid β (Aβ) peptides and/or regulate the amount of ApoE isoform 4 in the bloodstream and/or brain. The compositions can be administered by any suitable means that produce contact of these compounds with the site of action in the body, for example in the plasma, liver, brain or small intestine of a subject.

The daily dosage for the various compositions and therapeutic combinations described above can be administered to a subject in a single dose or in multiple subdoses, as desired. Subdoses can be administered 2 to 6 times per day, for example. Sustained release dosages can be used. Where the compound(s) of Formulae I-X and cholesterol biosynthesis inhibitor(s) or lipid-lowering agent(s) are administered in separate dosages, the number of doses of each component given per day may not necessarily be the same, e.g., one component may have a greater duration of activity and will therefore need to be administered less frequently.

The compositions and therapeutic combinations used in the present invention can further comprise one or more pharmaceutically acceptable carriers, one or more excipients and/or one or more additives. The pharmaceutical compositions can comprise about 1 to about 99 weight percent of active ingredient (one or more compounds of Formula I-X), and preferably about 5 to about 95 percent active ingredient.

Useful pharmaceutically acceptable carriers can be either solid, liquid or gas. Non-limiting examples of pharmaceutically acceptable carriers include solids and/or liquids such as magnesium carbonate, magnesium stearate, talc, sugar, lactose, ethanol, glycerol, water and the like. The amount of carrier in the treatment composition or therapeutic combination can range from about 5 to about 99 weight percent of the total weight of the treatment composition or therapeutic combination. Non-limiting examples of suitable pharmaceutically acceptable excipients and additives include non-toxic compatible fillers, binders such as starch, polyvinyl pyrrolidone or cellulose ethers, disintegrants such as sodium starch glycolate, crosslinked polyvinyl pyrrolidone or croscarmellose sodium, buffers, preservatives, anti-oxidants, lubricants, flavorings, thickeners, coloring agents, wetting agents such as sodium lauryl sulfate, emulsifiers and the like. The amount of excipient or additive can range from about 0.1 to about 95 weight percent of the total weight of the treatment composition or therapeutic combination. One skilled in the art would understand that the amount of carrier (s), excipients and additives (if present) can vary. Further examples of pharmaceutically acceptable carriers and methods of manufacture for various compositions can be found in A. Gennaro (ed.), Remington: The Science and Practice of Pharmacy, $20^{th}$ Edition, (2000), Lippincott Williams & Wilkins, Baltimore, MD.

Useful solid form preparations include powders, tablets, dispersible granules, capsules, cachets and suppositories. An example of a preparation of a preferred solid form dosage formulation is provided below.

Useful liquid form preparations include solutions, suspensions and emulsions. As an example may be mentioned water or water-propylene glycol solutions for parenteral injection or addition of sweeteners and opacifiers for oral solutions suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also useful are solid form preparations which are intended to be converted, shortly before use, to liquid form

preparations for either oral or parenteral administration. Such liquid forms include solutions, suspensions and emulsions.

The compounds used in the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is.administered orally.

The following formulation exemplifies one of the dosage forms of this invention. In the formulation, the term "Active Compound I" designates any of the compounds of Formulas I-X described herein above.

EXAMPLE

Tablets

| No. | Ingredient | mg/tablet |
|---|---|---|
| 1 | Active Compound I | 10 |
| 2 | Lactose monohydrate NF | 55 |
| 3 | Microcrystalline cellulose NF | 20 |
| 4 | Povidone USP (K29-32) | 4 |
| 5 | Croscarmellose sodium NF | 8 |
| 6 | Sodium lauryl sulfate NF | 2 |
| 7 | Magnesium stearate NF | 1 |
| | Total | 100 |

Method of Manufacture

Mix Item No. 4 with purified water in suitable mixer to form binder solution. Spray the binder solution and then water over Items 1, 2, 6 and a portion of Item 5 in a fluidized bed processor to granulate the ingredients. Continue fluidization to dry the damp granules. Screen the dried granules and blend with Item No. 3 and the remainder of Item 5. Add Item No. 7 and mix. Compress the mixture to appropriate size and weight on a suitable tablet machine.

For coadministration in separate tablets or capsules, representative formulations comprising a compound of Formulae I-X such as are discussed above are well known in the art and representative formulations comprising a cholesterol absorption inhibitor and/or lipid lowering agent such as are discussed above are well known in the art. It is contemplated that where the two active ingredients are administered as a single composition, the dosage forms disclosed above for compounds of Formulae I-X may readily be modified using the knowledge of one skilled in the art.

Since one aspect of the present invention relates to treating AD, regulating production of or levels of amyloid β (Aβ) peptides and/or regulating the amount of ApoE isoform 4 in the bloodstream and/or brain by treatment with a combination of active ingredients wherein the active ingredients may be administered separately, the invention also relates to combining separate pharmaceutical compositions in kit form. That is, a kit is contemplated wherein two separate units are combined: a pharmaceutical composition comprising at least compound of Formulae I-X and a separate pharmaceutical composition comprising at least one cholesterol biosynthesis inhibitor or lipid-lowering agent as described above. The kit will preferably include directions for the administration of the separate components. The kit form is particularly advantageous when the separate components must be administered in different dosage forms (e.g., oral and parenteral) or are administered at different dosage intervals.

The uses of the present invention can be used for one or more of the following: to prevent, to treat AD, or ameliorate symptoms of AD, to regulate production of or levels of amyloid β (Aβ) peptides and/or regulate the amount of ApoE isoform 4 in the bloodstream and/or brain of a subject.

In one alternative embodiment, the human carries one or more mutations in the genes that encode β-amyloid precursor protein, presenilin-1 or presenilin-2.

In another alternative embodiment, the human carries the Apolipoprotein ε4 gene.

In another alternative embodiment, the human has a family history of Alzheimer's Disease or dementia illness.

In another alternative embodiment, the human has trisomy 21 (Down's Syndrome).

In another alternative embodiment, the subject has a normal or low serum total blood cholesterol level. In another embodiment, the serum total blood cholesterol level is less than 200 mg/dl, more preferably less than 180 and can range from 150 to 200 mg/dl. In another embodiment, the total LDL cholesterol level is less than 100 mg/dl, more preferably less than 90 mg/dl and can range from 30 to 100 mg/dl. Methods of measuring serum total blood cholesterol and total LDL cholesterol are well known to those skilled in the art and for example include those disclosed in PCT WO

99/38498 at page 11, incorporated by reference herein. Methods of determining levels of other sterols in serum are disclosed in H. Gylling et al., "Serum Sterols During Stanol Ester Feeding in a Mildly Hypercholesterolemic Population", J. Lipid Res. 40: 593-600 (1999), incorporated by reference herein.

In another alternative embodiment, the subject has an elevated serum total blood cholesterol level. In another embodiment, the serum total cholesterol level is at least 200 mg/dl, more preferably at least 220 mg/dl and can range from 200 to 1000 mg/dl. In another alternative embodiment, the subject has an elevated total LDL cholesterol level. In another embodiment, the total LDL cholesterol level is greater than 100 mg/dl, more preferably greater than about 110 mg/dl and can range from 100 to 1000 mg/dl.

In another alternative embodiment, the human is at least about 40 years of age. In another alternative embodiment, the human is at least about 60 years of age. In another embodiment, the human is at least about 70 years of age. Preferably the human is between about 60 and 100 years of age.

In another embodiment, the subject exhibits no symptoms of Alzheimer's Disease. In another embodiment, the subject is a human who is at least 40 years of age and exhibits no symptoms of Alzheimer's Disease. In another embodiment, the subject is a human who is at least 40 years of age and exhibits one or more symptoms of Alzheimer's Disease.

By the uses of the present invention, the levels of amyloid β (Aβ) peptides in a subject's brain or blood can be reduced from levels prior to treatment from 10 to 100 percent, and preferably 50 to 100 percent.

In an alternative embodiment, the subject can have an elevated level of amyloid Aβ-42 peptide in the blood prior to treatment according to the present methods of greater than 30 picomoles/liter (pM), preferably greater than 35 pM, and more preferably greater than 40 pM. In another embodiment, the elevated level of amyloid Aβ-42 peptide can range from about 30 pM to 80 pM. One skilled in the art would understand that as the AD progresses, the measurable levels of amyloid β peptide may decrease slightly from elevated levels present before onset of the disease.

In an alternative embodiment, the subject can have an elevated level of amyloid Aβ-40 peptide in the blood prior to treatment according to the present methods of greater than 200 picomoles/liter (pM), preferably greater than 300 pM, and more preferably greater than 400 pM. In another embodiment, the elevated level of amyloid Aβ-40 peptide can range from 200 pM to 800 pM.

In another embodiment, the subject can have an elevated level of amyloid Aβ-42 peptide in the brain prior to treatment according to the present methods of greater than 50 picomoles per gram (pmol/g) of wet brain tissue weight, preferably greater than 200 pmol/g, and more preferably greater than 500 pmol/g. In another embodiment, the level of amyloid β peptide can range from 50 pmol/g to 10,000 pmol/g, and preferably about 500 pmol/g to 10,000 pmol/g.

In another embodiment, the subject can have an elevated level of amyloid Aβ-40 peptide in the brain prior to treatment according to the present methods of greater than 10 picomoles per gram (pmol/g) of wet brain tissue weight, preferably greater than 50 pmol/g, and more preferably greater than 100 pmol/g. In another embodiment, the level of amyloid β peptide can range from 10 pmol/g to 15,000 pmol/g.

The amount of amyloid β (Aβ) peptide in the brain or blood of a subject can be evaluated by enzyme-linked immunosorbent assay (ELISA) or quantitative immunolblotting test methods which are well known to those skilled in the art, such as is disclosed by Zhang et al., J. Biol. Chem. 274:8966-8972 (1999) and Zhang et al., Biochemistry 40: 5049-5055 (2001). These tests are performed on samples of the brain or blood which have been prepared in a manner well known to one skilled in the art, for example as disclosed in the Example below. Another example of a useful method for measuring levels of amyloid β peptides is by Europium immunoassay (EIA), such as is disclosed in PCT WO 99/38498 at page 11, incorporated herein by reference.

In another embodiment, the amount of total ApoE in the bloodstream and/or brain of a subject can be reduced from levels prior to treatment by 5 to 75 percent, and preferably 5 to 50 percent. The amount of total ApoE can be measured in a manner well known to one skilled in the art, for example using an ELISA test kit such as Apo-Tek ApoE test kit that is available from Organon Teknica.

Illustrating the invention are the following examples which, however, are not to be considered as limiting the invention to their details. Unless otherwise indicated, all parts and percentages in the following examples, as well as throughout the specification, are by weight.

## EXAMPLES

### PREPARATION OF COMPOUND OF FORMULA (II)

Step 1): To a solution of (S)-4-phenyl-2-oxazolidinone (41 g, 0.25 mol) in $CH_2Cl_2$ (200 ml), was added 4-dimethylaminopyridine (2.5 g, 0.02 mol) and triethylamine (84.7 ml, 0.61 mol) and the reaction mixture was cooled to 0°C. Methyl-4-(chloroformyl)butyrate (50 g; 0.3 mol) was added as a solution in $CH_2Cl_2$ (375 ml) dropwise over 1 h, and the reaction was allowed to warm to 22°C. After 17 h, water and $H_2SO_4$ (2N, 100 ml), was added the layers were separated, and the organic layer was washed sequentially with NaOH (10%), NaCl (sat'd) and water. The organic layer

was dried over $MgSO_4$ and concentrated to obtain a semicrystalline product.

Step 2): To a solution of $TiCl_4$ (18.2 ml, 0.165 mol) in $CH_2Cl_2$ (600 ml) at 0°C, was added titanium isopropoxide (16.5 ml, 0.055 mol). After 15 min, the product of Step 1 (49.0 g, 0.17 mol) was added as a solution in $CH_2Cl_2$ (100 ml). After 5 min., diisopropylethylamine (DIPEA) (65.2 ml, 0.37 mol) was added and the reaction mixture was stirred at 0°C for 1 h, the reaction mixture was cooled to -20°C, and 4-benzyloxybenzylidine(4-fluoro)aniline (114.3 g, 0.37 mol) was added as a solid. The reaction mixture was stirred vigorously for 4 h at -20°C, then acetic acid was added as a solution in $CH_2Cl_2$ dropwise over 15 min, the reaction mixture was allowed to warm to 0°C, and $H_2SO_4$ (2N) was added. The reaction mixture was stirred an additional 1 h, the layers were separated, washed with water, separated and the organic layer was dried. The crude product was crystallized from ethanol/water to obtain the pure intermediate.

Step 3): To a solution of the product of Step 2 (8.9 g, 14.9 mmol) in toluene (100 ml) at 50°C, was added N,O-bis(trimethylsilyl)acetamide (BSA) (7.50 ml, 30.3 mmol). After 0.5 h, solid TBAF (0.39 g, 1.5 mmol) was added and the reaction mixture stirred at 50°C for an additional 3 h. The reaction mixture was cooled to 22°C, $CH_3OH$ (10 ml), was added. The reaction mixture was washed with HCl (1N), $NaHCO_3$ (1N) and NaCl (sat'd.), and the organic layer was dried over $MgSO_4$.

Step 4): To a solution of the product of Step 3 (0.94 g, 2.2 mmol) in $CH_3OH$ (3 ml), was added water (1 ml) and $LiOH \cdot H_2O$ (102 mg, 2.4 mmole). The reaction mixture was stirred at 22°C for 1 h and then additional $LiOH \cdot H_2O$ (54 mg, 1.3 mmole) was added. After a total of 2 h, HCl (1N) and EtOAc was added, the layers were separated, the organic layer was dried and concentrated in *vacuo.* To a solution of the resultant product (0.91 g, 2.2 mmol) in $CH_2Cl_2$ at 22°C, was added ClCOCOCl (0.29 ml, 3.3 mmol) and the mixture stirred for 16 h. The solvent was removed in *vacuo.*

Step 5): To an efficiently stirred suspension of 4-fluorophenylzinc chloride (4.4 mmol) prepared from 4-fluorophenylmagnesium bromide (1M in THF, 4.4 ml, 4.4 mmol) and $ZnCl_2$ (0.6 g, 4.4 mmol) at 4°C, was added tetrakis (triphenylphosphine)palladium (0.25 g, 0.21 mmol) followed by the product of Step 4 (0.94 g, 2.2 mmol) as a solution in THF (2 ml). The reaction was stirred for 1 h at 0°C and then for 0.5 h at 22°C. HCl (1N, 5 ml) was added and the mixture was extracted with EtOAc. The organic layer was concentrated to an oil and purified by silica gel chromatography to obtain 1-(4-fluorophenyl)-4(S)-(4-hydroxyphenyl)-3(R)-(3-oxo-3-phenylpropyl)-2-azetidinone:
HRMS calc'd for $C_{24}H_{19}F_2NO_3$ = 408.1429, found 408.1411.

Step 6): To the product of Step 5 (0.95 g, 1.91 mmol) in THF (3 ml), was added (R)-tetrahydro-1-methyl-3,3-diphenyl-1H,3H-pyrrolo-[1 ,2-c][1 ,3,2] oxazaborole (120 mg, 0.43 mmol) and the mixture was cooled to -20°C. After 5 min, borohydride-dimethylsulfide complex (2M in THF, 0.85 ml, 1.7 mmol) was added dropwise over 0.5 h. After a total of 1.5 h , $CH_3OH$ was added followed by HCl (1 N) and the reaction mixture was extracted with EtOAc to obtain 1-(4-fluorophenyl)-3(R)-[3(S)-(4-fluorophenyl)-3-hydroxypropyl)]-4(S)-[4-(phenylmethoxy)phenyl]-2-azetidinone (compound 6A-1) as an oil. $^1H$ in $CDCl_3$ d H3 = 4.68. J = 2.3 Hz. Cl ($M^+H$) 500.

Use of (S)-tetra-hydro-1-methyl-3,3-diphenyl-1H,3H-pyrrolo-[1,2-c][1,3,2] oxazaborole gives the corresponding 3(R)-hydroxypropyl azetidinone (compound 6B-1). $^1H$ in $CDCl_3$ d H3 = 4.69. J = 2.3 Hz. Cl ($M^+H$) 500.

To a solution of compound 6A-1 (0.4 g, 0.8 mmol) in ethanol (2 ml), was added 10% Pd/C (0.03 g) and the reaction mixture was stirred under a pressure (60 psi) of $H_2$ gas for 16 h. The reaction mixture was filtered and the solvent was concentrated to obtain compound 6A. Mp 164-166°C; Cl ($M^+H$) 410. $[\alpha]_D^{25} = -28.1^o$ (c 3, $CH_3OH$). Elemental analysis calc'd for $C_{24}H_{21}F_2NO_3$: C 70.41; H 5.17; N 3.42; found C 70.25; H 5.19; N 3.54.

Similarly treat compound 6B-1 to obtain compound 6B.
Mp 129.5-132.5°C; Cl ($M^+H$) 410. Elemental analysis calc'd for $C_{24}H_{21}F_2NO_3$: C 70.41; H 5.17; N 3.42; found C 70.30; H 5.14; N 3.52.

Step 6' (Alternative): To a solution of the product of Step 5 (0.14 g, 0.3 mmol) in ethanol (2 ml), was added 10% Pd/C (0.03 g) and the reaction was stirred under a pressure (60 psi) of $H_2$ gas for 16 h. The reaction mixture was filtered and the solvent was concentrated to afford a 1:1 mixture of compounds 6A and 6B.

## Hypothetical In Vivo Evaluation

The compound of Formula VIa is administered to transgenic mice (about 5 weeks of age) overexpressing the β-amyloid precursor protein. The mice are CRND8 mice available from University of Toronto, described in Janus et al., Nature (Dec. 2000) and Chishti et al., JBC Online 2001. The compound is administered at a dosage of 30 mg/kg/day in the diet over a period of 5-11 weeks. After the specified period of compound administration, mice are sacrificed by $CO_2$ asphyxiation and the brains are removed and placed in phosphate buffered saline. One cortex is dissected away from the remainder of one hemisphere and is homogenized in 300 μl of sucrose homogenization buffer (20 mM Tris base, 250 mM sucrose, 1 mM EDTA (ethylene diamine tetraacetic acid) and 1 mM EGTA). The residual brain hemisphere (minus cortex) is used to measure CNS cholesterol and triglycerides using methods discussed above. The remaining intact hemisphere is snap-frozen on dry ice and cryostat sectioned. Sections are stained for Aβ-containing plaques using

monoclonal anti-human Aβ antibody 6F/3D (Dako) and Cy-3 conjugated secondary antibody (available from Jackson Immunological Research). Plaque number and size are quantitated from microscopic images of stained sections visualized with fluorescent light using Image-Pro Plus (available from Media Cybernetics).

Soluble Aβ peptide is extracted by mixing 50 μl of the cortex homogenate with 50 μl of sucrose homogenization buffer and centrifuging the mixture at 100,000 g for 1 hour at 4°C. The pellet is then resuspended in 100 μl of cell lysis buffer (1% sodium dodecyl sulfate, 10 mM Tris pH 7.4,150 mM NaCl, 5 mM EDTA) and the resulting mixture is centrifuged at 100,000 g for 1 hour at 4°C. The supernatant is then removed, tested for protein concentration and used for measurement of Aβ peptide as described below.

Aβ peptide deposited in plaques is extracted by mixing 100 μl of the cortex homogenate with 233 ml of cold (4°C) formic acid (70% final concentration of formic acid) and sonicating the mixture for 1 minute on ice using a Fisher Sonic Dismembrator Model F60 at Setting 5. The mixture is then centrifuged at 100,000 g for 1 hour at 4°C and 210 μl of the supernatant is then diluted with 210 μl of formic acid neutralization solution (1M Tris base, 0.5 M $Na_2HPO_4$). This solution is used for measurement of Aβ peptide as described below.

Plasma Aβ peptide is measured directly without further treatment of plasma samples. Cholesterol and triglyceride levels are measured from aliquots of these plasma samples.

Aβ40 and Aβ42 peptides are measured independently by enzyme-linked immunosorbent assay. Antibodies 4G8 and G2-10 are used for the measurement of Aβ40 and antibodies 4G8 and G2-11 are used for the measurement of Aβ42 (Ida et al., J. Biol. Chem. 271:22908-22914, 1996). Detection of ELISA assays is accomplished by electrochemiluminescent detection using Igen M8 which is available from Igen International, Gaithersburg, MD).

## Claims

1. Use of a composition comprising at least one compound represented by Formula (I):

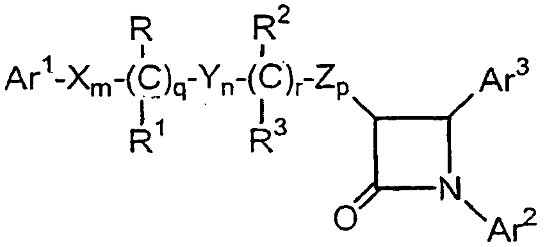

(I)

or a pharmaceutically acceptable salt thereof or solvate thereof,
wherein:

$Ar^1$ and $Ar^2$ are independently selected from the group consisting of aryl and $R^4$-substituted aryl;
$Ar^3$ is aryl or $R^5$-substituted aryl;
X, Y and Z are independently selected from the group consisting of $-CH_2-$, -CH(lower alkyl)- and -C(dilower alkyl)-;
R and $R^2$ are independently selected from the group consisting of $-OR^6$, $-O(CO)R^6$, $-O(CO)OR^9$ and $-O(CO)NR^6R^7$;
$R^1$ and $R^3$ are independently selected from the group consisting of hydrogen, lower alkyl and aryl;
q is 0 or 1;
r is 0 or 1;
m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NF^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$, $-CH=CH-COOR^6$, $-CF_3$, -CN, $-NO_2$ and halogen;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$,

$-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene) $COOR^6$ and $-CH=CH-COOR^6$;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl

for the manufacture of a medicament for treating, preventing or ameliorating symptoms of Alzheimer's Disease in the subject.

**2.** The use according to claim 1, wherein the subject exhibits no symptoms of Alzheimer's Disease.

**3.** The use according to claim 1, wherein the subject has Alzheimer's Disease.

**4.** The use according to claim 1, wherein the subject has a family history of Alzheimer's Disease or dementia illness.

**5.** The use according to claim 1, wherein the subject is a human and has trisomy 21 (Down's Syndrome).

**6.** The use according to claim 1, wherein the subject is a human.

**7.** The use according to claim 6, wherein the human carries one or more mutations in the genes that encode β-amyloid precursor protein, presenilin-1 or presenilin-2.

**8.** The use according to claim 6, wherein the human carries the Apolipoprotein E4 gene.

**9.** The use according to claim 1, wherein the subject has an elevated level of amyloid β peptide in the bloodstream and/or the brain.

**10.** The use according to claim 9, wherein the subject has an elevated level of amyloid β 42 peptide in the bloodstream and/or the brain.

**11.** The use according to claim 9, wherein the level of amyloid β (Aβ) peptides in the bloodstream is reduced from 10 to 100 percent from a level of amyloid β (Aβ) peptides prior to administration of the composition.

**12.** The use according to claim 9, wherein the subject has an level of amyloid Aβ-42 peptide greater than 30 pM in the bloodstream.

**13.** The use according to claim 12, wherein the subject has an level of amyloid Aβ-42 peptide greater than 40 pM in the bloodstream.

**14.** The use according to claim 12, wherein the subject has an level of amyloid Aβ-42 peptide ranging from 30 pM to about 80 pM in the bloodstream.

**15.** The use according to claim 9, wherein the subject has an level of amyloid Aβ-40 peptide greater than 200 pM in the bloodstream.

**16.** The use according to claim 15, wherein the subject has an level of amyloid Aβ-40 peptide greater than 400 pM in the bloodstream.

**17.** The use according to claim 15, wherein the subject has an level of amyloid Aβ-40 peptide ranging from 200 pM to 800 pM in the bloodstream.

**18.** The use according to claim 9, wherein the subject has an level of amyloid Aβ-42 peptide of greater than 50 pmol/gram of wet brain tissue.

**19.** The use according to claim 9, wherein the subject has an level of amyloid Aβ-40 peptide of greater than 10 pmol/gram of wet brain tissue.

**20.** The use according to claim 1, wherein the subject has an elevated blood cholesterol level.

**21.** The use according to claim 1, wherein the total serum cholesterol level of the subject is at least 200 mg/dl.

**22.** The use according to claim 1, wherein the total LDL cholesterol level of the subject is greater than 100 mg/dl.

**23.** The use according to claim 6, wherein the human is greater than about 40 years of age.

**24.** The use according to claim 7, wherein the human is greater than about 60 years of age.

**25.** The use according to claim 1, wherein the compound is represented by Formula (II) below:

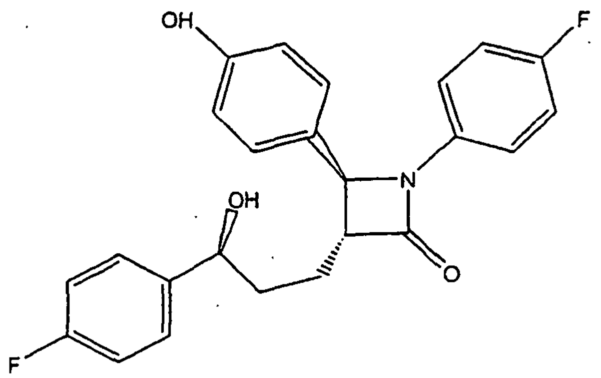

(II).

**26.** The use according to claim 1, wherein the compound is represented by Formula (VIa) below:

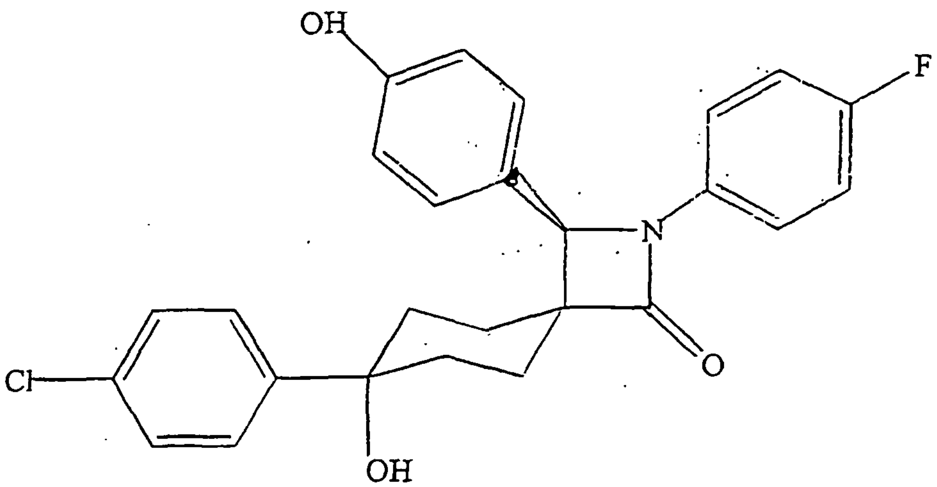

(VIa).

**27.** The use according to claim 1, wherein the compound is administered to the subject in an amount ranging from 0.1 to 1000 milligrams of compound per day.

**28.** The use according to claim 1, wherein the composition further comprises at least one cholesterol biosynthesis inhibitor.

**29.** The use according to claim 28, wherein the at least one cholesterol biosynthesis inhibitor comprises at least one HMG CoA reductase inhibitor.

**30.** The use according to claim 29, wherein the at least one HMG CoA reductase inhibitor is selected from lovastatin, pravastatin, fluvastatin, simvastatin, atorvastatin, cerivastatin, rosuvastatin or mixtures thereof.

**31.** The use according to claim 30, wherein the at least one HMG CoA reductase inhibitor is simvastatin.

**32.** The use according to claim 1, wherein the composition further comprises at least one fibric acid derivative.

**33.** The use according to claim 1, wherein the composition further comprises at least one bile acid sequestrant.

**34.** The use according to claim 1, wherein the composition further comprises nicotinic acid or a derivative thereof.

**35.** The use according to claim 1, wherein the composition further comprises at least one AcylCoA:Cholesterol *O*-acyltransferase Inhibitor.

**36.** The use according to claim 1, wherein the composition further comprises probucol or a derivative thereof.

**37.** The use according to claim 1, wherein the composition further comprises at least one low-density lipoprotein receptor activator.

**38.** The use according to claim 1, wherein the composition further comprises at least one Omega 3 fatty acid.

**39.** The use according to claim 1, wherein the composition further comprises at least one natural water soluble fiber.

**40.** The use according to claim 1, wherein the composition further comprises at least one of plant sterols, plant stanols or fatty acid esters of plant stanols.

**41.** The use according to claim 1, wherein the composition further comprises at least one antioxidant or vitamin.

**42.** The use according to claim 1, wherein the composition further comprises at least one Alzheimer's treatment different from compound I above.

**43.** The use according to claim 1, wherein the composition further comprises at least one Alzheimer's treatment different from compound I above selected from the group consisting of cholinesterase inhibitors, muscarinic receptor antagonists, M2 muscarinic receptor antagonists, acetylcholine release stimulators, choline uptake stimulators, nicotinic cholinergic receptor antagonists, anti-Aβ vaccines, γ-secreiase inhibitors, β-secretase inhibitors, amyloid aggregation inhibitors, amyloid precursor protein antisense oligonucleotides, monoamine reuptake inhibitors, human stem cells, gene therapy, nootropic agents, AMPA receptor ligands, growth factors or growth factor receptor agonists, anti-inflammatory agents, free radical scavengers/antioxidants, superoxide dismutase stimulators, calcium channel blockers, apoptosis inhibitors, caspase inhibitors, monoamine oxidase inhibitors, estrogens, NMDA receptor antagonists, Jun N-terminal kinase (JNK) inhibitors, copper/zinc chelators, 5-HT1a receptor agonists, NGF stimulators, neuroprotective agents, H3 histamine receptor antagonists, calpain inhibitors, poly ADP ribose polymerase inhibitors, prolylendopeptidase inhibitors, calcium modulators, corticortropin releasing factor antagonists, corticortropin releasing factor binding protein inhibitor, GABA modulators, GABA-A receptor antagonists, GABA-B receptor antagonists, neuroimmunophilin ligands, sigma receptor ligands, galanin receptor ligands, imidazoline/alpha adrenergic receptor antagonists, vasoactive intestinal peptide receptor agonists, benzodiazepine inverse agonists, cannabinoid receptor agonists, thyrotropin releasing hormone receptor agonists, protein kinase C inhibitors, 5-HT3 antagonists, prostaglandin receptor antagonists, topoisomerase II inhibitors, steroid receptor agonists, corticosteroid receptor antagonists, nitric oxide modulators, RAGE inhibitors, dopamine receptor agonists, and combinations thereof.

**44.** Use of a composition comprising a compound represented by Formula (II) below:

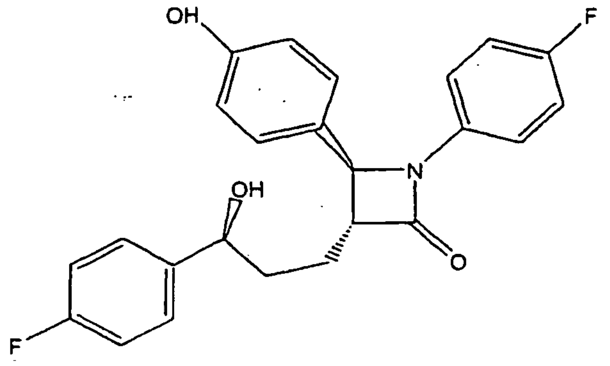

(II)

for the manufacture of a medicament for preventing, treating, or ameliorating symptoms of Alzheimer's Disease in the subject.

**45.** Use of a composition comprising at least one compound represented by Formula (I):

by Formula (I):

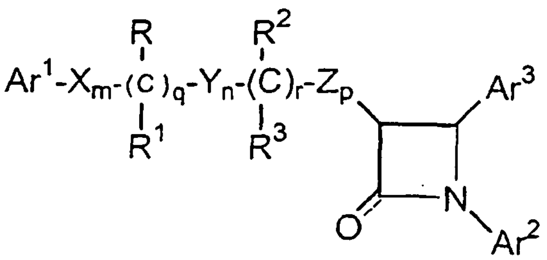

(I)

or a pharmaceutically acceptable salt thereof or solvate thereof,
wherein:

$Ar^1$ and $Ar^2$ are independently selected from the group consisting of aryl and $R^4$-substituted aryl;
$Ar^3$ is aryl or $R^5$-substituted aryl;
X, Y and Z are independently selected from the group consisting of $-CH_2-$, -CH(lower alkyl)- and -C(dilower alkyl)-;
R and $R^2$ are independently selected from the group consisting of $-OR^6$, $-O(CO)R^6$, $-O(CO)OR^9$ and $-O(CO)NR^6R^7$;
$R^1$ and $R^3$ are independently selected from the group consisting of hydrogen, lower alkyl and aryl;
q is 0 or 1;
r is 0 or 1;
m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
$R^4$ is 1-5 substituents independently selected from the group consisting of lower alkyl, $-OR^6$, $-O(CO)R^6$, $-O(CO)OR^9$, $O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$, $-CH=CH-COOR^6$, $-CF_3$, -CN, $-NO_2$ and halogen;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$ and $-CH=CH-COOR^6$;
$R^6$, $R^7$ and $R^8$ are independently selected from the group consisting of hydrogen, lower alkyl, aryl and aryl-substituted lower alkyl; and

$R^9$ is lower alkyl, aryl or aryl-substituted lower alkyl, for the manufacture of a medicament for regulating the production or level of at least one amyloid β peptide in the subject.

**46.** Use of a composition comprising at least one compound represented by Formula (I):

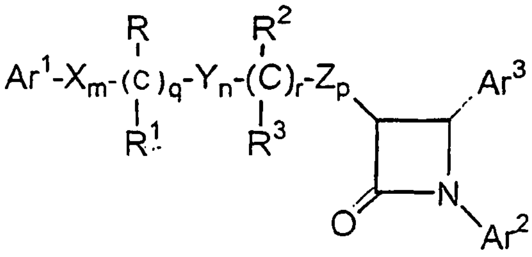

(I)

or a pharmaceutically acceptable salt thereof or solvate thereof, wherein:

$Ar^1$ and $Ar^2$ are independently selected from the group consisting of aryl and $R^4$-substituted aryl;
$Ar^3$ is aryl or $R^5$-substituted aryl;
X, Y and Z are independently selected from the group consisting of $-CH_2-$, -CH(lower alkyl)- and -C(dilower alkyl)-;
R and $R^2$ are independently selected from the group consisting of $-OR^6$, $-O(CO)R^6$, $-O(CO)OR^9$ and $-O(CO)NR^6R^7$;
$R^1$ and $R^3$ are independently selected from the group consisting of hydrogen, lower alkyl and aryl;
q is 0 or 1 ;
r is 0 or 1 ;
m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$, $-CH=CH-COOR^6$, $-CF_3$, -CN, $-NO_2$ and halogen;
$R^5$ is 1-5 substituents independently selected from the group consisting of $-OR^6$, $-O(CO)R^6$, $-O(CO)OR^9$, $O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene) $COOR^6$ and $-CH=CH-COOR^6$;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl, for the manufacture of a medicament for regulating the amount of ApoE isoform 4 in the bloodstream and/or brain of the subject.

**47.** Use of a composition comprising at least one compound represented by Formula (III):

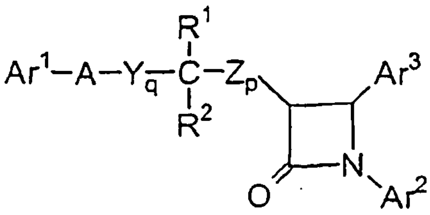

(III)

or a pharmaceutically acceptable salt thereof or solvate thereof, wherein, in Formula (III) above:

$Ar^1$ is $R^3$-substituted aryl;
$Ar^2$ is $R^4$-substituted aryl;
$Ar^3$ is $R^5$-substituted aryl;
Y and Z are independently selected from the group consisting of $-CH_2-$, -CH(lower alkyl)- and -C(dilower alkyl)-;
A is selected from -O-, -S-, -S(O)- or $-S(O)_2-$;
$R^1$ is selected from the group consisting of $-OR^6$, $-O(CO)R^6$, $-O(CO)OR^9$ and $-O(CO)NR^6R^7$; $R^2$ is selected from the group consisting of hydrogen, lower alkyl and aryl; or $R^1$ and $R^2$ together are =O;
q is 1, 2 or 3;
p is 0, 1, 2, 3 or 4;
$R^5$ is 1-3 substituents independently selected from the group consisting of $-OR^6$, $-O(CO)R^6$, $-O(CO)OR^9$, $-O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-lower alkyl, $-NR^6SO_2$-aryl, $-CONR^6R^7$, $-COR^6$, $-SO_2NR^6R^7$, $S(O)_{0-2}$-alkyl, $S(O)_{0-2}$-aryl, $-O(CH_2)_{1-10}-COOR^6$, $-O(CH_2)_{1-10}CONR^6R^7$, o-halogeno, m-halogeno, o-lower alkyl, m-lower alkyl, -(lower alkylene)-$COOR^6$, and $-CH=CH-COOR^6$;
$R^3$ and $R^4$ are independently 1-3 substituents independently selected from the group consisting of $R^5$, hydrogen, p-lower alkyl, aryl, $-NO_2$, $-CF_3$ and p-halogeno;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl for the manufacture of a medicament for preventing, treating, or ameliorating symptoms of Alzheimer's Disease in the subject.

**48.** Use of a composition comprising at least one compound represented by Formula (III):

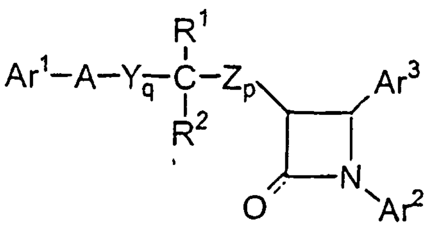

(III)

or a pharmaceutically acceptable salt thereof or solvate thereof, wherein, in Formula (III) above:

$Ar^1$ is $R^3$-substituted aryl;
$Ar^2$ is $R^4$-substituted aryl;
$Ar^3$ is $R^5$-substituted aryl;
Y and Z are independently selected from the group consisting of $-CH_2-$, -CH(lower alkyl)- and -C(dilower alkyl)-;
A is selected from -O-, -S-, -S(O)- or $-S(O)_2-$;
$R^1$ is selected from the group consisting of $-OR^6$, $-O(CO)R^6$, $-O(CO)OR^9$ and $-O(CO)NR^6R^7$; $R^2$ is selected from the group consisting of hydrogen, lower alkyl and aryl; or $R^1$ and $R^2$ together are =O;

q is 1, 2 or 3;
p is 0, 1, 2, 3 or 4; .
$R^5$ is 1-3 substituents independently selected from the group consisting of $-OR^6$, $-O(CO)R^6$, $-O(CO)OR^9$, $O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-lower alkyl, $-NR^6SO_2$-aryl, $-CONR^6R^7$, $-COR^6$, $-SO_2NR^6R^7$, $S(O)_{0-2}$-alkyl, $S(O)_{0-2}$-aryl, $-O(CH_2)_{1-10}-COOR^6$, $-O(CH_2)_{1-10}CONR^6R^7$, o-halogeno, m-halogeno, o-lower alkyl, m-lower alkyl, -(lower alkylene)-$COOR^6$, and $-CH=CH-COOR^6$;
$R^3$ and $R^4$ are independently 1-3 substituents independently selected from the group consisting of $R^5$, hydrogen, p-lower alkyl, aryl, $-NO_2$, $-CF_3$ and p-halogeno;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl, for the manufacture of a medicament for regulating the production or level of at least one amyloid β peptide in the subject.

**49.** Use of a composition comprising at least one compound represented by Formula (III):

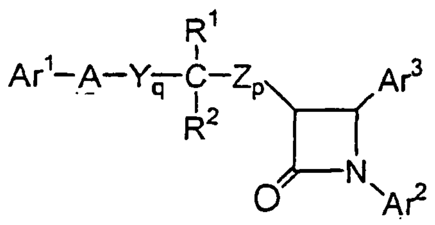

(III)

or a pharmaceutically acceptable salt thereof or solvate thereof, wherein, in Formula (III) above:

$Ar^1$ is $R^3$-substituted aryl;
$Ar^2$ is $R^4$-substituted aryl;
$Ar^3$ is $R^5$-substituted aryl;
Y and Z are independently selected from the group consisting of $-CH_2-$, -CH(lower alkyl)- and -C(dilower alkyl)-;
A is selected from -O-, -S-, -S(O)- or $-S(O)_2-$;
$R^1$ is selected from the group consisting of $-OR^6$, $-O(CO)R^6$, $-O(CO)OR^9$ and $-O(CO)NR^6R^7$; $R^2$ is selected from the group consisting of hydrogen, lower alkyl and aryl; or $R^1$ and $R^2$ together are =O;
q is 1, 2 or 3;
p is 0, 1, 2, 3 or 4;
$R^5$ is 1-3 substituents independently selected from the group consisting of $-OR^6$, $-O(CO)R^6$, $-O(CO)OR^9$, $-O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-lower alkyl,$-NR^5SO_2$-aryl, $-CONR^6R^7$, $-COR^6$, $-SO_2NR^6R^7$, $S(O)_{0-2}$-alkyl, $S(O)_{0-2}$-aryl, $-O(CH_2)_{1-10}-COOR^6$, $-O(CH_2)_{1-10}CONR^6R^7$, o-halogeno, m-halogeno, o-lower alkyl, m-lower alkyl, -(lower alkylene)-$COOR^6$, and $-CH=CH-COOR^6$;
$R^3$ and $R^4$ are independently 1-3 substituents independently selected from the group consisting of $R^5$, hydrogen, p-lower alkyl, aryl, $-NO_2$, $-CF_3$ and p-halogeno;
$R^6$, $R^7$ and $R^8$ are independently selected from the group consisting of hydrogen, lower alkyl, aryl and aryl-substituted lower alkyl; and
$R^9$ is lower alkyl, aryl or aryl-substituted lower alkyl, for the manufacture of a medicament for regulating the amount of ApoE isoform 4 in the bloodstream and/or brain of the subject.

**50.** Use a composition comprising at least one compound represented by Formula (IV):

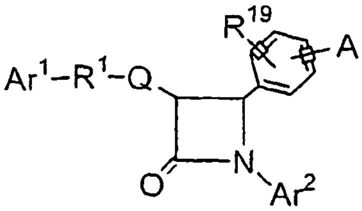

(IV)

or a pharmaceutically acceptable salt thereof or solvate thereof, wherein, in Formula (IV) above:

A is selected from the group consisting of $R^2$-substituted heterocycloalkyl, $R^2$-substituted heteroaryl, $R^2$-substituted benzofused heterocycloalkyl, and $R^2$-substituted benzofused heteroaryl;
$Ar^1$ is aryl or $R^3$-substituted aryl;
$Ar^2$ is aryl or $R^4$-substituted aryl;
Q is a bond or, with the 3-position ring carbon of the azetidinone, forms the spiro group

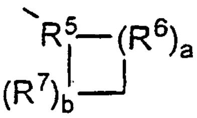

;

and
$R^1$ is selected from the group consisting of:

- $(CH_2)_q$-, wherein q is 2-6, provided that when Q forms a spiro ring, q can also be zero or 1;
- $(CH_2)_e$-G-$(CH_2)_r$-, wherein G is -O-, -C(O)-, phenylene, -$NR^8$- or -$S(O)_{0-2}$-, e is 0-5 and r is 0-5, provided that the sum of e and r is 1-6;
- ($C_2$-$C_6$ alkenylene)-; and
- $(CH_2)_f$-V-$(CH_2)_g$-, wherein V is $C_3$-$C_6$ cycloalkylene, f is 1-5 and g is 0-5, provided that the sum of f and g is 1-6;

$R^5$ is selected from:

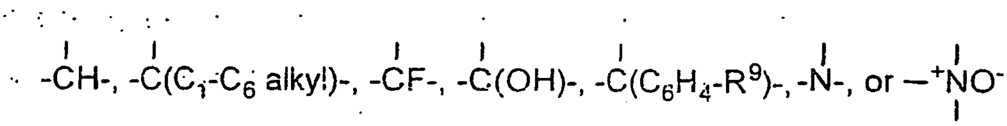

;

$R^6$ and $R^7$ are independently selected from the group consisting of -$CH_2$-, -CH($C_1$-$C_6$ alkyl)-, -C(di-($C_1$-$C_6$) alkyl), -CH=CH- and -C($C_1$-$C_6$ alkyl)=CH-; or $R^5$ together with an adjacent $R^6$, or $R^5$ together with an adjacent $R^7$, form a -CH=CH- or a -CH=C($C_1$-$C_6$ alkyl)- group;
a and b are independently 0, 1, 2 or 3, provided both are not zero; provided that when $R^6$ is -CH=CH- or -C($C_1$-$C_6$ alkyl)=CH-, a is 1; provided that when $R^7$ is -CH=CH- or -C($C_1$-$C_6$ alkyl)=CH-, b is 1; provided that when a is 2 or 3, the $R^6$'s can be the same or different; and provided that when b is 2 or 3, the $R^7$'s can be the same or different;
and when Q is a bond, $R^1$ also can be selected from:

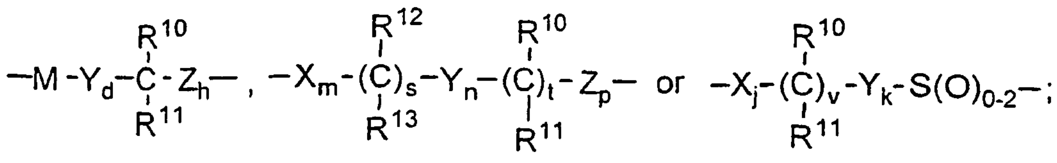

where M is -O-, -S-, -S(O)- or $-S(O)_2-$;

X, Y and Z are independently selected from the group consisting of- $CH_2$-, -CH($C_1$-$C_6$ alkyl)- and -C(di-($C_1$-$C_6$) alkyl);

$R^{10}$ and $R^{12}$ are independently selected from the group consisting of $-OR^{14}$, $-O(CO)R^{14}$, $-O(CO)OR^{16}$ and $-O(CO)NR^{14}R^{15}$;

$R^{11}$ and $R^{13}$ are independently selected from the group consisting of hydrogen, ($C_1$-$C_6$)alkyl and aryl; or $R^{10}$ and $R^{11}$ together are =O, or $R^{12}$ and $R^{13}$ together are =O;

d is 1, 2 or 3;

h is 0, 1, 2, 3 or 4;

s is 0 or 1; t is 0 or 1; m, n and p are independently 0-4; provided that at least one of s and t is 1, and the sum of m, n, p, s and t is 1-6; provided that when p is 0 and t is 1, the sum of m, s and n is 1-5; and provided that when p is 0 and s is 1, the sum of m, t and n is 1-5;

v is 0 or 1;

j and k are independently 1-5, provided that the sum of j, k and v is 1-5;

$R^2$ is 1-3 substituents on the ring carbon atoms selected from the group consisting of hydrogen, ($C_1$-$C_{10}$)alkyl, ($C_2$-$C_{10}$)alkenyl, ($C_2$-$C_{10}$)alkynyl, ($C_3$-$C_6$)cycloalkyl, ($C_3$-$C_6$)cycloalkenyl, $R^{17}$-substituted aryl, $R^{17}$-substituted benzyl, $R^{17}$-substituted benzyloxy, $R^{17}$-substituted aryloxy, halogeno, $-NR^{14}R^{15}$, $NR^{14}R^{15}$($C_1$-$C_6$ alkylene)-, $NR^{14}R^{15}C(O)$($C_1$-$C_6$ alkylene)-,$-NHC(O)R^{16}$, OH, $C_1$-$C_6$ alkoxy, $=OC(O)R^{16}$, $-COR^{14}$, hydroxy($C_1$-$C_6$)alkyl, ($C_1$-$C_6$)alkoxy($C_1$-$C_6$)alkyl, $NO_2$, $-S(O)_{0-2}R^{16}$, $-SO_2NR^{14}R^{15}$ and $-(C_1$-$C_6$alkylene)$COOR^{14}$; when $R^2$ is a substituent on a heterocycloalkyl ring, $R^2$ is as defined, or is =O or

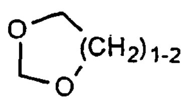

;

and, where $R^2$ is a substituent on a substitutable ring nitrogen, it is hydrogen, ($C_1$-$C_6$)alkyl, aryl, ($C_1$-$C_6$)alkoxy, aryloxy, ($C_1$-$C_6$)alkylcarbonyl, arylcarbonyl, hydroxy, $-(CH_2)_{1-6}CONR^{18}R^{18}$,

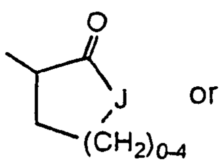 or 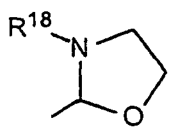 ;

wherein J is -O-, -NH-, $-NR^{18}$- or $-CH_2-$;

$R^3$ and $R^4$ are independently selected from the group consisting of 1-3 substituents independently selected from the group consisting of ($C_1$-$C_6$)alkyl, $-OR^{14}$, $-O(CO)R^{14}$, $-O(CO)OR^{16}$, $-O(CH_2)_{1-5}OR^{14}$, $-O(CO)NR^{14}R^{15}$, $-NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $S(O)_{0-2}R^{16}$, $-O(CH_2)_{1-10}-COOR^{14}$, $-O(CH_2)_{1-10}CONR^{14}R^{15}$, $-(C_1$-$C_6$ alkylene)$-COOR^{14}$, $-CH=CH-COOR^{14}$, $-CF_3$, -CN, $-NO_2$ and halogen;

$R^8$ is hydrogen, ($C_1$-$C_6$)alkyl, aryl ($C_1$-$C_6$)alkyl, $-C(O)R^{14}$ or $-COOR^{14}$;

$R^9$ and $R^{17}$ are independently 1-3 groups independently selected from the group consisting of hydrogen, ($C_1$-$C_6$) alkyl, ($C_1$-$C_6$)alkoxy, -COOH, $NO_2$, $-NR R^{15}$, OH and halogeno;

$R^{14}$ and $R^{15}$ are independently selected from the group consisting of hydrogen, ($C_1$-$C_6$)alkyl, aryl and aryl-substituted ($C_1$-$C_6$)alkyl;

$R^{16}$ is ($C_1$-$C_6$)alkyl, aryl or $R^{17}$-substituted aryl;

$R^{18}$ is hydrogen or ($C_1$-$C_6$)alkyl; and

$R^{19}$ is hydrogen, hydroxy or ($C_1$-$C_6$)alkoxy,

for the manufacture of a medicament for preventing, treating, or ameliorating symptoms of Alzheimer's Disease in the subject.

**51.** Use of a composition comprising at least one compound represented by Formula (IV):

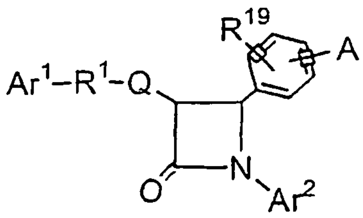

(IV)

or a pharmaceutically acceptable salt thereof or solvate thereof, wherein, in Formula (IV) above:

A is selected from the group consisting of $R^2$-substituted heterocycloalkyl, $R^2$-substituted heteroaryl, $R^2$-substituted benzofused heterocycloalkyl, and $R^2$-substituted benzofused heteroaryl;
$Ar^1$ is aryl or $R^3$-substituted aryl;
$Ar^2$ is aryl or $R^4$-substituted aryl;
Q is a bond or, with the 3-position ring carbon of the azetidinone, forms the spiro group

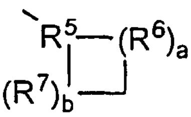
;

and
$R^1$ is selected from the group consisting of:

- $(CH_2)_q$-, wherein q is 2-6, provided that when Q forms a spiro ring, q can also be zero or 1;
- $(CH_2)_e$-G-$(CH_2)_r$-, wherein G is -O-, -C(O)-, phenylene, -$NR^8$- or -$S(O)_{0-2}$-, e is 0-5 and r is 0-5, provided that the sum of e and r is 1-6;
- ($C_2$-$C_6$ alkenylene)-; and
- $(CH_2)_f$-V-$(CH_2)_g$-, wherein V is $C_3$-$C_6$ cycloalkylene, f is 1-5 and g is 0-5, provided that the sum of f and g is 1-6;

$R^5$ is selected from:

-CH-, -C($C_1$-$C_6$ alkyl)-, -CF-, -C(OH)-, -C($C_6H_4$-$R^9$)-, -N-, or $-^{+}NO^{-}$ ;

$R^6$ and $R^7$ are independently selected from the group consisting of -$CH_2$-, -CH($C_1$-$C_6$ alkyl)-, -C(di-($C_1$-$C_6$) alkyl), -CH=CH- and -C($C_1$-$C_6$ alkyl)=CH-; or $R^5$ together with an adjacent $R^6$, or $R^5$ together with an adjacent $R^7$, form a -CH=CH- or a -CH=C($C_1$-$C_6$ alkyl)- group;
a and b are independently 0, 1, 2 or 3, provided both are not zero; provided that when $R^6$ is -CH=CH- or -C($C_1$-$C_6$ alkyl)=CH-, a is 1; provided that when $R^7$ is -CH=CH- or -C($C_1$-$C_6$ alkyl)=CH-, b is 1; provided that when a is 2 or 3, the $R^6$'s can be the same or different; and provided that when b is 2 or 3, the $R^7$'s can be the same or different;
and when Q is a bond, $R^1$ also can be selected from:

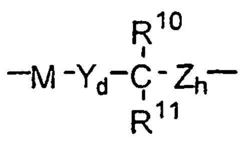 , 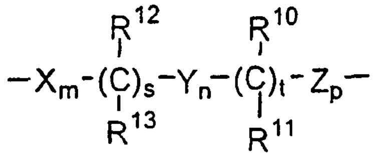 or 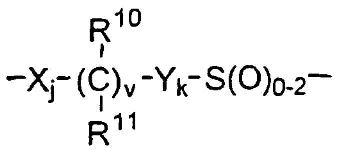 ;

where M is -O-, -S-, -S(O)- or $-S(O)_2-$;
X, Y and Z are independently selected from the group consisting of $-CH_2-$, $-CH(C_1-C_6$ alkyl)- and $-C(di-(C_1-C_6$ alkyl);
$R^{10}$ and $R^{12}$ are independently selected from the group consisting of $-OR^{14}$, $-O(CO)R^{14}$, $-O(CO)OR^{16}$ and $-O(CO)NR^{14}R^{15}$;
$R^{11}$ and $R^{13}$ are independently selected from the group consisting of hydrogen, $(C_1-C_6)$alkyl and aryl; or $R^{10}$ and $R^{11}$ together are =O, or $R^{12}$ and $R^{13}$ together are =O;
d is 1, 2 or 3;
h is 0, 1, 2, 3 or 4;
s is 0 or 1; t is 0 or 1; m, n and p are independently 0-4; provided that at least one of s and t is 1, and the sum of m, n, p, s and t is 1-6; provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
$R^2$ is 1-3 substituents on the ring carbon atoms selected from the group consisting of hydrogen, $(C_1-C_{10})$alkyl, $(C_2-C_{10})$alkenyl, $(C_2-C_{10})$alkynyl, $(C_3-C_6)$cycloalkyl, $(C_3-C_6)$cycloalkenyl, $R^{17}$-substituted aryl, $R^{17}$-substituted benzyl, $R^{17}$-substituted benzyloxy, $R^{17}$-substituted aryloxy, halogeno, $-NR^{14}R^{15}$, $NR^{14}R^{15}(C_{1-}C_6$ alkylene)-, $NR^{14}R^{15}C(O)(C_1-C_6$ alkylene)-,$-NHC(O)R^{16}$, OH, $C_1-C_6$ alkoxy, $-OC(O)R^{16}$, $-COR^{14}$, hydroxy$(C_1-C_6)$alkyl, $(C_1-C_6)$alkoxy$(C_1-C_6)$alkyl, $NO_2$, $-S(O)_{0-2}R^{16}$, $-SO_2NR^{14}R^{15}$ and- $(C_1-C_6$ alkylene)$COOR^{14}$; when $R^2$ is a substituent on a heterocycloalkyl ring, $R^2$ is as defined, or is =O or

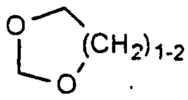 ;

and, where $R^2$ is a substituent on a substitutable ring nitrogen, it is hydrogen, $(C_1-C_6)$alkyl, aryl, $(C_1-C_6)$alkoxy, aryloxy, $(C_1-C_6)$alkylcarbonyl, arylcarbonyl, hydroxy, $-(CH_2)_{1-6}CONR^{18}R^{18}$,

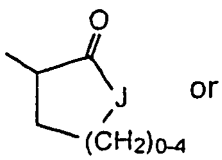 or 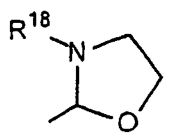 ;

wherein J is -O-, -NH-, $-NR^{18}-$ or $-CH_2-$;
$R^3$ and $R^4$ are independently selected from the group consisting of 1-3 substituents independently selected from the group consisting of $(C_1-C_6)$alkyl, $-OR^{14}$, $-O(CO)R^{14}$ $-O(CO)OR^{16}$, $-O(CH_2)_{1-5}OR^{14}$, $-O(CO)NR^{14}R^{15}$, $-NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $S(O)_{0-2}R^{16}$, $-O(CH_2)_{1-10}-COOR^{14}$, $-O(CH_2)_{1-10}CONF^{14}R^{15}$, $-(C_1-C_6$ alkylene)-$COOR^{14}$, $-CH=CH-COOR^{14}$, $-CF_3$, -CN, $-NO_2$ and halogen;
$R^8$ is hydrogen, $(C_1-C_6)$alkyl, aryl $(C_1-C_6)$alkyl, $-C(O)R^{14}$ or $-COOR^{14}$;
$R^9$ and $R^{17}$ are independently 1-3 groups independently selected from the group consisting of hydrogen, $(C_1-C_6)$alkyl, $(C_1-C_6)$alkoxy, -COOH, $NO_2$, $-NR^{14}R^{15}$, OH and halogeno;
$R^{14}$ and $R^{15}$ are independently selected from the group consisting of hydrogen, $(C_1-C_6)$alkyl, aryl and aryl-substituted $(C_1-C_6)$alkyl;
$R^{16}$ is $(C_1-C_6)$alkyl, aryl or $R^{17}$-substituted aryl;
$R^{18}$ is hydrogen or $(C_1-C_6)$alkyl; and

$R^{19}$ is hydrogen, hydroxy or $(C_1-C_6)$alkoxy,

for the manufacture of a medicament for regulating the production or level of at least one amyloid β peptide in the subject.

**52.** Use of a composition comprising at least one compound represented by Formula (IV):

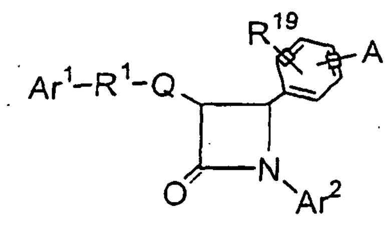

(IV)

or a pharmaceutically acceptable salt thereof or solvate thereof, wherein, in Formula (IV) above:

A is selected from the group consisting of $R^2$-substituted heterocycloalkyl, $R^2$-substituted heteroaryl, $R^2$-substituted benzofused heterocycloalkyl, and $R^2$-substituted benzofused heteroaryl;
$Ar^1$ is aryl or $R^3$-substituted aryl;
$Ar^2$ is aryl or $R^4$-substituted aryl;
Q is a bond or, with the 3-position ring carbon of the azetidinone, forms the spiro group

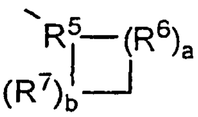

;

and
$R^1$ is selected from the group consisting of:

- $(CH_2)_q$-, wherein q is 2-6, provided that when Q forms a spiro ring, q can also be zero or 1;
- $(CH_2)_e$-G-$(CH_2)_r$-, wherein G is -O-, -C(O)-, phenylene, -$NR^8$- or -$S(O)_{0-2}$-, e is 0-5 and r is 0-5, provided that the sum of e and r is 1-6;
- ($C_2$-$C_6$ alkenylene)-; and
- $(CH_2)_f$-V-$(CH_2)_g$-, wherein V is $C_3$-$C_6$ cycloalkylene, f is 1-5 and g is 0-5, provided that the sum of f and g is 1-6;

$R^5$ is selected from:

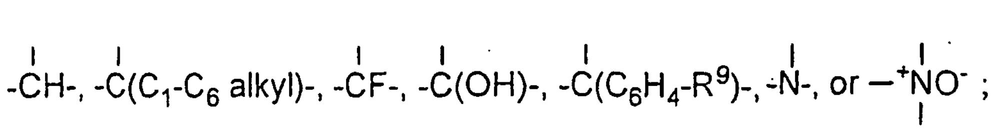

$R^6$ and $R^7$ are independently selected from the group consisting of -$CH_2$-, -$CH(C_1$-$C_6$ alkyl)-, -C(di-($C_1$-$C_6$) alkyl), -CH=CH- and -C($C_1$-$C_6$ alkyl)=CH-; or $R^5$ together with an adjacent $R^6$, or $R^5$ together with an adjacent $R^7$, form a -CH=CH- or a -CH=C($C_1$-$C_6$ alkyl)- group;
a and b are independently 0, 1, 2 or 3, provided both are not zero; provided that when $R^6$ is -CH=CH- or -C($C_1$-$C_6$ alkyl)=CH-, a is 1; provided that when $R^7$ is -CH=CH- or -C($C_1$-$C_6$ alkyl)=CH-, b is 1; provided that when a is 2 or 3, the $R^6$'s can be the same or different; and provided that when b is 2 or 3, the $R^7$'s can be the same or different;

and when Q is a bond, $R^1$ also can be selected from:

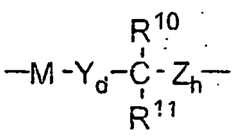 , 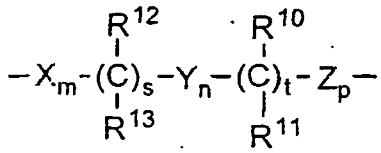 or 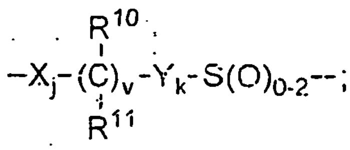 ;

where M is -O-, -S-, -S(O)- or $-S(O)_2-$;
X, Y and Z are independently selected from the group consisting of $-CH_2-$, $-CH(C_1-C_6$ alkyl)- and $-C(di-(C_1-C_6)$ alkyl);
$R^{10}$ and $R^{12}$ are independently selected from the group consisting of $-OR^{14}$, $-O(CO)R^{14}$, $-O(CO)OR^{16}$ and $-O(CO)NR^{14}R^{15}$;
$R^{11}$ and $R^{13}$ are independently selected from the group consisting of hydrogen, $(C_1-C_6)$alkyl and aryl; or $R^{10}$ and $R^{11}$ together are =O, or $R^{12}$ and $R^{13}$ together are =O;
d is 1, 2 or 3;
h is 0, 1, 2, 3 or 4;
s is 0 or 1; t is 0 or 1; m, n and p are independently 0-4; provided that at least one of s and t is 1, and the sum of m, n, p, s and t is 1-6; provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
$R^2$ is 1-3 substituents on the ring carbon atoms selected from the group consisting of hydrogen, $(C_1-C_{10})$alkyl, $(C_2-C_{10})$alkenyl, $(C_2-C_{10})$alkynyl, $(C_3-C_6)$cycloalkyl, $(C_3-C_6)$cycloalkenyl, $R^{17}$-substituted aryl, $R^{17}$-substituted benzyl, $R^{17}$-substituted benzyloxy, $R^{17}$-substituted aryloxy, halogeno, $-NR^{14}R^{15}$, $NR^{14}R^{15}(C_1-C_6$ alkylene)-, $NR^{14}R^{15}C(O)(C_1-C_6$ alkylene)-,$-NHC(O)R^{16}$, OH, $C_1-C_6$ alkoxy, $-OC(O)R^{16}$, $-COR^{14}$, hydroxy$(C_1-C_6)$alkyl, $(C_1-C_6)$alkoxy$(C_1-C_6)$alkyl, $NO_2$, $-S(O)_{0-2}R^{16}$, $-SO_2NR^{14}R^{15}$ and $-(C_1-C_6$ alkylene)$COOR^{14}$; when R is a substituent on a heterocycloalkyl ring, $R^2$ is as defined, or is =O or

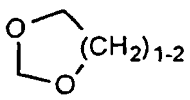 ;

f and, where $R^2$ is a substituent on a substitutable ring nitrogen, it is hydrogen, $(C_1-C_6)$alkyl, aryl, $(C_1-C_6)$alkoxy, aryloxy, $(C_1-C_6)$alkylcarbonyl, arylcarbonyl, hydroxy, $-(CH_2)_{1-6}CONR^{18}R^{18}$,

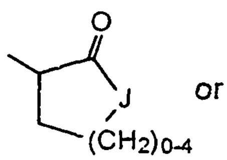 or 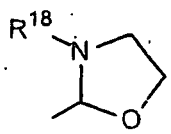 ;

wherein J is -O-, -NH-, $-NR^{18}-$ or $-CH_2-$;
$R^3$ and $R^4$ are independently selected from the group consisting of 1-3 substituents independently selected from the group consisting of $(C_1-C_6)$alkyl, $-OR^{14}$, $-O(CO)R^{14}$, $-O(CO)OR^{16}$, $-O(CH_2)_{1-5}OR^{14}$, $-O(CO)NR^{14}R^{15}$, $-NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $S(O)_{0-2}R^{16}$, $-O(CH_2)_{1-10}-COOR^{14}$, $-O(CH_2)_{1-10}CONR^{14}R^{15}$, $-(C_1-C_6$ alkylene)-$COOR^{14}$, $-CH=CH-COOR^{14}$, $-CF_3$, -CN, $-NO_2$ and halogen;
$R^8$ is hydrogen, $(C_1-C_6)$alkyl, aryl $(C_1-C_6)$alkyl, $-C(O)R^{14}$ or $-COOR^{14}$;
$R^9$ and $R^{17}$ are independently 1-3 groups independently selected from the group consisting of hydrogen, $(C_1-C_6)$ alkyl, $(C_1-C_6)$alkoxy, -COOH, $NO_2$, $-NR\ R^{15}$, OH and halogeno;
$R^{14}$ and $R^{15}$ are independently selected from the group consisting of hydrogen, $(C_1-C_6)$alkyl, aryl and aryl-substituted $(C_1-C_6)$alkyl;

$R^{16}$ is ($C_1$-$C_6$)alkyl, aryl or $R^{17}$-substituted aryl;
$R^{18}$ is hydrogen or ($C_1$-$C_6$)alkyl; and
$R^{19}$ is hydrogen, hydroxy or ($C_1$-$C_6$)alkoxy,

for the manufacture of a medicament for regulating amount of ApoE isoform 4 in the bloodstream and/or brain of the subject.

**53.** Use of a composition comprising at least one compound represented by Formula (V):

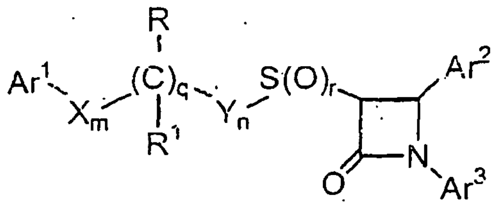

(V)

or a pharmaceutically acceptable salt thereof or solvate thereof, wherein, in Formula (V) above:

$Ar^1$ is aryl, $R^{10}$-substituted aryl or heteroaryl;
$Ar^2$ is aryl or $R^4$-substituted aryl;
$Ar^3$ is aryl or $R^5$-substituted aryl;
X and Y are independently selected from the group consisting of $-CH_2-$, -CH(lower alkyl)- and -C(dilower alkyl)-;
R is $-OR^6$, $-O(CO)R^6$, $-O(CO)OR^9$ or $-O(CO)NR^6R^7$; $R^1$ is hydrogen, lower alkyl or aryl; or R and $R^1$ together are =O;
q is 0 or 1;
r is 0, 1 or 2;
m and n are independently 0, 1, 2, 3, 4 or 5; provided that the sum of m, n and q is 1,2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$ and $-CH=CH-COOR^6$;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, $-CF_3$, -CN, $-NO_2$, halogen, -(lower alkylene)$COOR^6$ and $-CH=CH-COOR^6$;
$R^6$, $R^7$ and $R^8$ are independently selected from the group consisting of hydrogen, lower alkyl, aryl and aryl-substituted lower alkyl;
$R^9$ is lower alkyl, aryl or aryl-substituted lower alkyl; and
$R^{10}$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}-COOR^6$, $-O(CH_2)_{1-10}CONR^6R^7$, $-CF_3$, -CN, $-NO_2$ and halogen

for the manufacture of a medicament for preventing, treating, of ameliorating symptoms of Alzheimer's Disease in the subject.

**54.** Use of a composition comprising at least one compound represented by Formula (V):

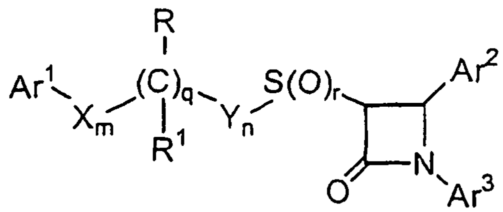

(V)

or a pharmaceutically acceptable salt thereof or solvate thereof, wherein, in Formula (V) above:

$Ar^1$ is aryl, $R^{10}$-substituted aryl or heteroaryl;
$Ar^2$ is aryl or $R^4$-substituted aryl;
$Ar^3$ is aryl or $R^5$-substituted aryl;
X and Y are independently selected from the group consisting of $-CH_2-$, -CH(lower alkyl)- and -C(dilower alkyl)-;
R is $-OR^6$, $-O(CO)R^6$, $-O(CO)OR^9$ or $-O(CO)NR^6R^7$; $R^1$ is hydrogen, lower alkyl or aryl; or R and $R^1$ together are =O,
q is 0 or 1;
r is 0, 1 or 2;
m and n are independently 0, 1, 2, 3, 4 or 5; provided that the sum of m, n and q is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$ $S(O)_{0-2}R^9$, $-O(CH_2)_{1-10}-COOR^6$, $-O(CH_2)_{1-10}CONR^6R^7$, -(lower alkylene)$COOR^6$ and $-CH=CH-COOR^6$;
$R^5$ is 1-5 substituents independently selected from the group consisting of $-OR^6$, $O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, $-CF_3$, -CN, $-NO_2$, halogen, -(lower alkylene)$COOR^6$ and $-CH=CH-COOR^6$;
$R^6$, $R^7$ and $R^8$ are independently selected from the group consisting of hydrogen, lower alkyl, aryl and aryl-substituted lower alkyl;
$R^9$ is lower alkyl, aryl or aryl-substituted lower alkyl; and
$R^{10}$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$,$-NR^6(CO)NR^7R^8$, $-NR^6SO_2R^9$,$-COOR^6$, $-CONR^6R^7$, $-COR^6$, $-SO_2NR^6R^7$, $-S(O)_{0-2}R^9$, $-O(CH_2)_{1-10}-COOR^6$, $-O(CH_2)_{1-10}CONR^6R^7$, $-CF_3$, -CN, $-NO_2$ and halogen

for the manufacture of a medicament for regulating the production or level of at least one amyloid β peptide in the subject.

**55.** Use of a composition comprising at least one compound represented by Formula (V):

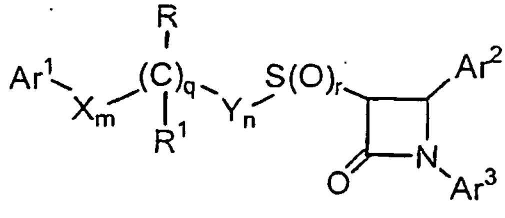

(V)

or a pharmaceutically acceptable salt thereof or solvate thereof, wherein, in Formula (V) above:

$Ar^1$ is aryl, $R^{10}$-substituted aryl or heteroaryl;
$Ar^2$ is aryl or $R^4$-substituted aryl;
$Ar^3$ is aryl or $R^5$-substituted aryl;
X and Y are independently selected from the group consisting of $-CH_2-$, -CH(lower alkyl)- and -C(dilower alkyl)-;
R is $-OR^6$, $-O(CO)R^6$, $-O(CO)OR^9$ or $-O(CO)NR^6R^7$; $R^1$ is hydrogen, lower alkyl or aryl; or R and $R^1$ together are =O;
q is 0 or 1;
r is 0, 1 or 2;
m and n are independently 0, 1, 2, 3, 4 or 5; provided that the sum of m, n and q is 1, 2, 3, 4 or 5;
$R^4$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^7SO_2R^9$ $-COOR^6$, $-CONR^6R^7$, $-COR^6$, $SO_2NR^6R^7$, $S(O)_{0-2}R^9$, $-O(CH_2)_{1-10}-COOR^6$, $-O(CH_2)_{1-10}CONR^6R^7$, -(lower alkylene)$COOR^6$ and $-CH=CH-COOR^6$;
$R^5$ is 1-5 substituents independently selected from the group consisting of $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, $-CF_3$, -CN, $-NO_2$, halogen, -(lower alkylene)$COOR^6$ and $-CH=CH-COOR^6$;
$R^6$, $R^7$ and $R^8$ are independently selected from the group consisting of hydrogen, lower alkyl, aryl and aryl-substituted lower alkyl;
$R^9$ is lower alkyl, aryl or aryl-substituted lower alkyl; and
$R^{10}$ is 1-5 substituents independently selected from the group consisting of lower alkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, $-CF_3$, -CN, $-NO_2$ and halogen

for the manufacture of a medicament for regulating the amount of ApoE isoform 4 in the bloodstream and/or brain of the subject.

**56.** Use of a composition comprising at least one compound represented by Formula (VI):

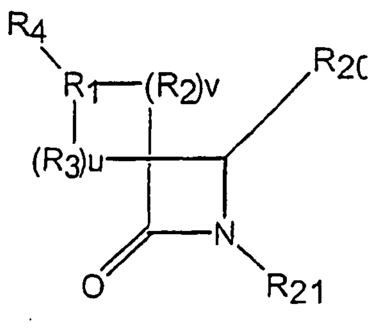

(VI)

or a pharmaceutically acceptable salt thereof or solvate thereof, wherein:

$R_1$ is

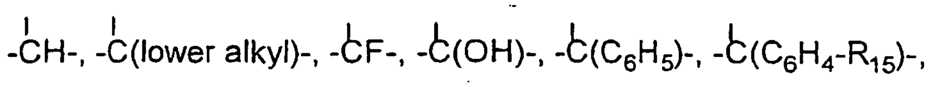

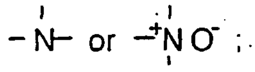

$R_2$ and $R_3$ are independently selected from the group consisting of: $-CH_2-$, -CH(lower alkyl)-, -C(di-lower alkyl)-,

-CH=CH-, and -C(lower alkyl)=CH-; or $R_1$ together with an adjacent $R_2$, or $R_1$ together with an adjacent $R_3$, form a -CH=CH- or a -CH=C(lower alkyl)- group;

u and v are independently 0, 1, 2 or 3, provided both are not zero; provided that when $R_2$ is -CH=CH- or -C (lower alkyl)=CH-, v is 1; provided that when $R_3$ is -CH=CH- or -C(lower alkyl)=CH-, u is 1; provided that when v is 2 or 3, the $R_2$'s can be the same or different; and provided that when u is 2 or 3, the $R_3$'s can be the same or different;

$R_4$ is selected from B-$(CH_2)_m$C(O)-, wherein m is 0, 1, 2, 3, 4 or 5;

B-$(CH_2)_q$-, wherein q is 0, 1, 2, 3, 4, 5 or 6;

B-$(CH_2)_e$-Z-$(CH_2)_r$-, wherein Z is -O-, -C(O)-, phenylene, -N($R_8$)- or -S$(O)_{0-2}$-, e is 0, 1, 2, 3, 4 or 5 and r is 0, 1, 2, 3, 4 or 5, provided that the sum of e and r is 0, 1, 2, 3, 4, 5 or 6;

B-($C_2$-$C_6$ alkenylene)-;

B-($C_4$-$C_6$ alkadienylene)-;

B-$(CH_2)_t$-Z-($C_2$-$C_6$ alkenylene)-, wherein Z is as defined above, and wherein t is 0, 1, 2 or 3, provided that the sum of t and the number of carbon atoms in the alkenylene chain is 2, 3, 4, 5 or 6;

B-$(CH_2)_f$-V-$(CH_2)_g$-, wherein V is $C_3$-$C_6$ cycloalkylene, f is 1, 2, 3, 4 or 5 and g is 0, 1, 2, 3, 4 or 5, provided that the sum of f and g is 1, 2, 3, 4, 5 or 6;

B-$(CH_2)_t$-V-($C_2$-$C_6$ alkenylene)- or B-($C_2$-$C_6$ alkenylene)-V-$(CH_2)_t$-, wherein V and t are as defined above, provided that the sum of t and the number of carbon atoms in the alkenylene chain is 2, 3, 4, 5 or 6;

B-$(CH_2)_a$-Z-$(CH_2)_b$-V-$(CH_2)_d$-, wherein Z and V are as defined above and a, b and d are independently 0, 1, 2, 3, 4, 5 or 6, provided that the sum of a, b and d is 0, 1, 2, 3, 4, 5 or 6; or

T-$(CH_2)_s$-, wherein T is cycloalkyl of 3-6 carbon atoms and s is 0, 1, 2, 3, 4, 5 or 6; or

$R_1$ and $R_4$ together form the group

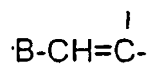 ;

B is selected from indanyl, indenyl, naphthyl, tetrahydronaphthyl, heteroaryl or W-substituted heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, or

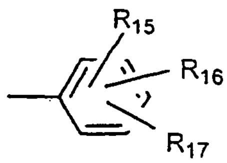

W is 1 to 3 substituents independently selected from the group consisting of lower alkyl, hydroxy lower alkyl, lower alkoxy, alkoxyalkyl, alkoxyalkoxy, alkoxycarbonylalkoxy, (lower alkoxyimino)-lower alkyl, lower alkanedioyl, lower alkyl lower alkanedioyl, allyloxy, -$CF_3$, -$OCF_3$, benzyl, $R_7$-benzyl, benzyloxy, $R_7$-benzyloxy, phenoxy, $R_7$-phenoxy, dioxolanyl, $NO_2$, -N($R_8$)($R_9$), N($R_8$)($R_9$)-lower alkylene-, N($R_8$)($R_9$)-lower alkylenyloxy-, OH, halogeno, -CN, -$N_3$, -NHC(O)O$R_{10}$, -NHC(O)$R_{10}$, $R_{11}O_2$SNH-, $(R_{11}O_2S)_2$N-, -$S(O)_2NH_2$, -S$(O)_{0-2}R_8$, tert-butyldimethyl-silyloxymethyl, -C(O)$R_{12}$,- COO$R_{19}$, -CON($R_8$)($R_9$), -CH=CHC(O)$R_{12}$, -lower alkylene-C(O)$R_{12}$, $R_{10}$C(O)(lower alkylenyloxy)-, N($R_8$)($R_9$)C(O)(lower alkylenyloxy)- and

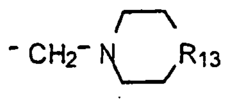

for substitution on ring carbon atoms, and the substituents on the substituted heteroaryl ring nitrogen atoms, when present, are selected from the group consisting of lower alkyl, lower alkoxy, -C(O)O$R_{10}$, -C(O)$R_{10}$, OH, N($R_8$)($R_9$)-lower alkylene-, N($R_8$)($R_9$)-lower alkylenyloxy-, -$S(O)_2NH_2$ and 2-(trimethylsilyl)-ethoxymethyl;

$R_7$ is 1-3 groups independently selected from the group consisting of lower alkyl, lower alkoxy, -COOH, $NO_2$, -N($R_8$)($R_9$), OH, and halogeno;

$R_8$ and Rg are independently selected from H or lower alkyl;

$R_{10}$ is selected from lower alkyl, phenyl, $R_7$-phenyl, benzyl or $R_7$-benzyl;

$R_{11}$ is selected from OH, lower alkyl, phenyl, benzyl, $R_7$-phenyl or $R_7$-benzyl;

$R_{12}$ is selected from H, OH, alkoxy, phenoxy, benzyloxy,

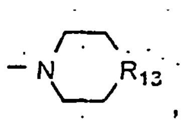

-N($R_8$)($R_9$), lower alkyl, phenyl or $R_7$-phenyl;

$R_{13}$ is selected from -O-, -$CH_2$-, -NH-, -N(lower alkyl)- or -NC(O)$R_{19}$;

$R_{15}$, $R_{16}$ and $R_{17}$ are independently selected from the group consisting of H and the groups defined for W; or $R_{15}$ is hydrogen and $R_{16}$ and $R_{17}$, together with adjacent carbon atoms to which they are attached, form a dioxolanyl ring;

$R_{19}$ is H, lower alkyl, phenyl or phenyl lower alkyl; and

$R_{20}$ and $R_{21}$ are independently selected from the group consisting of phenyl, W-substituted phenyl, naphthyl, W-substituted naphthyl, indanyl, indenyl, tetrahydronaphthyl, benzodioxolyl, heteroaryl, W-substituted heteroaryl, benzofused heteroaryl, W-substituted benzofused heteroaryl and cyclopropyl, wherein heteroaryl is as defined above,

for the manufacture of a medicament for preventing, treating, or ameliorating symptoms of Alzheimer's Disease in the subject.

**57.** Use of a composition comprising at least one compound represented by Formula (VI):

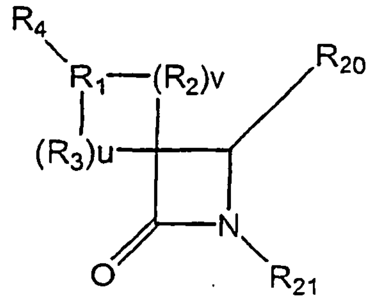

(VI)

or a pharmaceutically acceptable salt thereof or solvate thereof, wherein:

$R_1$ is

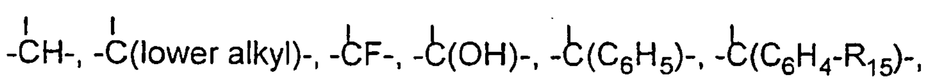

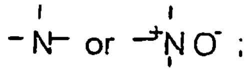

$R_2$ and $R_3$ are independently selected from the group consisting of: -$CH_2$-, -CH(lower alkyl)-, -C(di-lower alkyl)-, -CH=CH- and -C(lower alkyl)=CH-; or $R_1$ together with an adjacent $R_2$, or $R_1$ together with an adjacent $R_3$, form a -CH=CH- or a -CH=C(lower alkyl)- group;

u and v are independently 0, 1, 2 or 3, provided both are not zero; provided that when $R_2$ is -CH=CH- or -C(lower alkyl)=CH-, v is 1; provided that when $R_3$ is -CH=CH- or -C(lower alkyl)=CH-, u is 1; provided that when

v is 2 or 3, the $R_2$'s can be the same or different; and provided that when u is 2 or 3, the $R_3$'s can be the same or different;

$R_4$ is selected from B-$(CH_2)_mC(O)$-, wherein m is 0, 1, 2, 3, 4 or 5;

B-$(CH_2)_q$-, wherein q is 0, 1, 2, 3, 4, 5 or 6;

B-$(CH_2)_e$-Z-$(CH_2)_r$-, wherein Z is -O-, -C(O)-, phenylene, -N($R_8$)- or -$S(O)_{0-2}$-, e is 0, 1, 2, 3, 4 or 5 and r is 0, 1, 2, 3, 4 or 5, provided that the sum of e and r is 0, 1, 2, 3, 4, 5 or 6;

B-($C_2$-$C_6$ alkenylene)-;

B-($C_4$-$C_6$ alkadienylene)-;

B-$(CH_2)_t$-Z-($C_2$-$C_6$ alkenylene)-, wherein Z is as defined above, and wherein t is 0, 1, 2 or 3, provided that the sum of t and the number of carbon atoms in the alkenylene chain is 2, 3, 4, 5 or 6;

B-$(CH_2)_f$-V-$(CH_2)_g$-, wherein V is $C_3$-$C_6$ cycloalkylene, f is 1, 2, 3, 4 or 5 and g is 0, 1, 2, 3, 4 or 5, provided that the sum of f and g is 1, 2, 3, 4, 5 or 6;

B-$(CH_2)_t$-V-(C2-C6 alkenylene)- or

B-($C_2$-$C_6$ alkenylene)-V-$(CH_2)_t$-, wherein V and t are as defined above, provided that the sum of t and the number of carbon atoms in the alkenylene chain is 2, 3, 4, 5 or 6; B-$(CH_2)_a$-Z-$(CH_2)_b$-V-$(CH_2)_d$-, wherein Z and V are as defined above and a, b and d are independently 0, 1, 2, 3, 4, 5 or 6, provided that the sum of a, b and d is 0, 1, 2, 3, 4, 5 or 6; or

T-$(CH_2)_s$-, wherein T is cycloalkyl of 3-6 carbon atoms and s is 0, 1, 2, 3, 4, 5 or 6; or

$R_1$ and $R_4$ together form the group

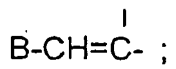

B is selected from indanyl, indenyl, naphthyl, tetrahydronaphthyl, heteroaryl or W-substituted heteroaryl, wherein heteroaryl is selected from the group consisting of pyrrolyl, pyridinyl, pyrimidinyl, pyrazinyl, triazinyl, imidazolyl, thiazolyl, pyrazolyl, thienyl, oxazolyl and furanyl, and for nitrogen-containing heteroaryls, the N-oxides thereof, or

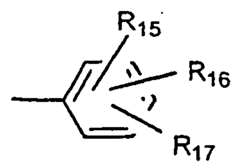

W is 1 to 3 substituents independently selected from the group consisting of lower alkyl, hydroxy lower alkyl, lower alkoxy, alkoxyalkyl, alkoxyalkoxy, alkoxycarbonylalkoxy, (lower alkoxyimino)-lower alkyl, lower alkanedioyl, lower alkyl lower alkanedioyl, allyloxy, -$CF_3$, -$OCF_3$, benzyl, $R_7$-benzyl, benzyloxy, $R_7$-benzyloxy, phenoxy, $R_7$-phenoxy, dioxolanyl, $NO_2$, -N($R_8$)($R_9$), N($R_8$)($R_9$)-lower alkylene-, N($R_8$)($R_9$)-lower alkylenyloxy-, OH, halogeno, -CN, -$N_3$, -NHC(O)O$R_{10}$, -NHC(O)$R_{10}$, $R_{11}O_2$SNH-, $(R_{11}O_2S)_2$N-, -$S(O)_2NH_2$, -$S(O)_{0-2}R_8$, tert-butyldimethyl-silyloxymethyl, -C(O)$R_{12}$,- COO$R_{19}$, -CON($R_8$)($R_9$), -CH=CHC(O)$R_{12}$, -lower alkylene-C(O)$R_{12}$, $R_{10}$C(O)(lower alkylenyloxy)-, N($R_8$)($R_9$)C(O)(lower alkylenyloxy)- and

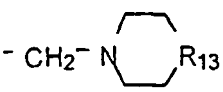

for substitution on ring carbon atoms, and the substituents on the substituted heteroaryl ring nitrogen atoms, when present, are selected from the group consisting of lower alkyl, lower alkoxy, -C(O)O$R_{10}$, -C(O)$R_{10}$, OH, N($R_8$)($R_9$)-lower alkylene-, N($R_8$)($R_9$)-lower alkylenyloxy-, -$S(O)_2NH_2$ and 2-(trimethylsilyl)-ethoxymethyl;

$R_7$ is 1-3 groups independently selected from the group consisting of lower alkyl, lower alkoxy, -COOH, $NO_2$, -N($R_8$)($R_9$), OH, and halogeno;

$R_8$ and Rg are independently selected from H or lower alkyl;

$R_{10}$ is selected from lower alkyl, phenyl, $R_7$-phenyl, benzyl or $R_7$-benzyl;

$R_{11}$ is selected from OH, lower alkyl, phenyl, benzyl, $R_7$-phenyl or $R_7$-benzyl;

$R_{12}$ is selected from H, OH, alkoxy, phenoxy, benzyloxy,

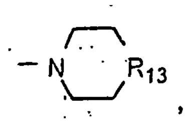

$-N(R_8)(R_9)$, lower alkyl, phenyl or $R_7$-phenyl;
$R_{13}$ is selected from -O-, $-CH_2-$, -NH-, -N(lower alkyl)- or $-NC(O)R_{19}$;
$R_{15}$, $R_{16}$ and $R_{17}$ are independently selected from the group consisting of H and the groups defined for W; or $R_{15}$ is hydrogen and $R_{16}$ and $R_{17}$, together with adjacent carbon atoms to which they are attached, form a dioxolanyl ring;
$R_{19}$ is H, lower alkyl, phenyl or phenyl lower alkyl; and
$R_{20}$ and $R_{21}$ are independently selected from the group consisting of phenyl, W-substituted phenyl, naphthyl, W-substituted naphthyl, indanyl, indenyl, tetrahydronaphthyl, benzodioxolyl, heteroaryl, W-substituted heteroaryl, benzofused heteroaryl, W-substituted benzofused heteroaryl and cyclopropyl, wherein heteroaryl is as defined above,

for the manufacture of a medicament for regulating the production or level of at least one amyloid β peptide in the subject.

**58.** Use of a composition comprising at least one compound represented by Formula (VI):

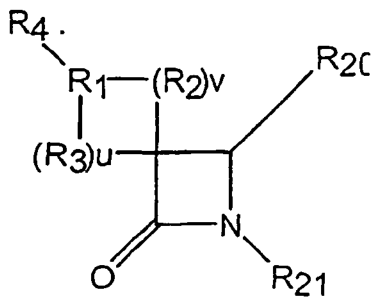

(VI)

or a pharmaceutically acceptable salt thereof or solvate thereof, wherein:

$R_1$ is

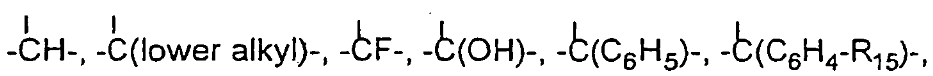

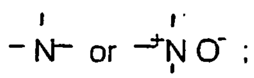

$R_2$ and $R_3$ are independently selected from the group consisting of: $-CH_2-$, -CH(lower alkyl)-, -C(di-lower alkyl)-, -CH=CH- and -C(lower alkyl)=CH-; or $R_1$ together with an adjacent $R_2$, or $R_1$ together with an adjacent $R_3$, form a -CH=CH- or a -CH=C(lower alkyl)- group;
u and v are independently 0, 1, 2 or 3, provided both are not zero; provided that when $R_2$ is -CH=CH- or -C (lower alkyl)=CH-, v is 1; provided that when $R_3$ is -CH=CH- or -C(lower alkyl)=CH-, u is 1; provided that when v is 2 or 3, the $R_2$'s can be the same or different; and provided that when u is 2 or 3, the $R_3$'s can be the same or different;
$R_4$ is selected from $B-(CH_2)_mC(O)-$, wherein m is 0, 1, 2, 3, 4 or 5;
$B-(CH_2)_q-$, wherein q is 0, 1, 2, 3, 4, 5 or 6;

B-$(CH_2)_e$-Z-$(CH_2)_r$-, wherein Z is -O-, -C(O)-, phenylene, -N($R_8$)- or -S(O)$_{0-2}$-, e is 0, 1, 2, 3, 4 or 5 and r is 0, 1, 2, 3, 4 or 5, provided that the sum of e and r is 0, 1, 2, 3, 4, 5 or 6;
B-($C_2$-$C_6$ alkenylene)-;
B-($C_4$-$C_6$ alkadienylene)-;
B-$(CH_2)_t$-Z-($C_2$-$C_6$ alkenylene)-, wherein Z is as defined above, and wherein t is 0, 1, 2 or 3, provided that the sum of t and the number of carbon atoms in the alkenylene chain is 2, 3, 4, 5 or 6;
B-$(CH_2)_f$-V-$(CH_2)_g$-, wherein V is $C_3$-$C_6$ cycloalkylene, f is 1, 2, 3, 4 or 5 and g is 0, 1, 2, 3, 4 or 5, provided that the sum of f and g is 1, 2, 3, 4, 5 or 6;
B-$(CH_2)_t$-V-($C_2$-$C_6$ alkenylene)- or
B-($C_2$-$C_6$ alkenylene)-V-$(CH_2)_t$-, wherein V and t are as defined above, provided that the sum of t and the number of carbon atoms in the alkenylene chain is 2, 3, 4, 5 or 6; B-$(CH_2)_a$-Z-(CH2)$_b$-V-$(CH_2)_d$-, wherein Z and V are as defined above and a, b and d are independently 0, 1, 2, 3, 4, 5 or 6, provided that the sum of a, b and d is 0, 1, 2, 3, 4, 5 or 6; or
T-$(CH_2)_s$-, wherein T is cycloalkyl of 3-6 carbon atoms and s is 0, 1, 2, 3, 4, 5 or 6; or
$R_1$ and $R_4$ together form the group B-CH=C- ;
B is selected from indanyl, indenyl, naphthyl, tetrahydronaphthyl, heteroaryl or W-substituted heteroaryl, wherein heteroaryl is selected from the group consisting of pyrrolyl, pyridinyl, pyrimidinyl, pyrazinyl, triazinyl, imidazolyl, thiazolyl, pyrazolyl; thienyl, oxazolyl and furanyl, and for nitrogen-containing heteroaryls, the N-oxides thereof, or

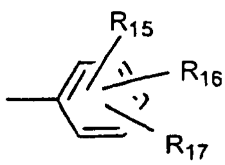

W is 1 to 3 substituents independently selected from the group consisting of lower alkyl, hydroxy lower alkyl, lower alkoxy, alkoxyalkyl, alkoxyalkoxy, alkoxycarbonylalkoxy, (lower alkoxyimino)-lower alkyl, lower alkanedioyl, lower alkyl lower alkanedioyl, allyloxy, -$CF_3$, -$OCF_3$, benzyl, $R_7$-benzyl, benzyloxy, $R_7$-benzyloxy, phenoxy, $R_7$-phenoxy, dioxolanyl, $NO_2$, -N($R_8$)($R_9$), N($R_8$)($R_9$)-lower alkylene-, N($R_8$)($R_9$)-lower alkylenyloxy-, OH, halogeno, -CN, -$N_3$, -NHC(O)O$R_{10}$, -NHC(O)$R_{10}$, $R_{11}O_2$SNH-, $(R_{11}O_2S)_2$N-, -S(O)$_2NH_2$, -S(O)$_{0-2}R_8$, tert-butyldimethyl-silyloxymethyl, -C(O)$R_{12}$,- COO$R_{19}$, -CON($R_8$)($R_9$), -CH=CHC(O)$R_{12}$, -lower alkylene-C(O)$R_{12}$, $R_{10}$C (O)(lower alkylenyloxy)-, N($R_8$)($R_9$)C(O)(lower alkylenyloxy)- and

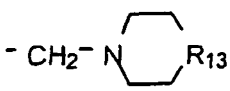

for substitution on ring carbon atoms, and the substituents on the substituted heteroaryl ring nitrogen atoms, when present, are selected from the group consisting of lower alkyl, lower alkoxy, -C(O)O$R_{10}$, -C(O)$R_{10}$, OH, N($R_8$)($R_9$)-lower alkylene-, N($R_8$)($R_9$)-lower alkylenyloxy-, -S(O)$_2NH_2$ and 2-(trimethylsilyl)-ethoxymethyl;
$R_7$ is 1-3 groups independently selected from the group consisting of lower alkyl, lower alkoxy, -COOH, $NO_2$, -N($R_8$)($R_9$), OH, and halogeno;
$R_8$ and Rg are independently selected from H or lower alkyl;
$R_{10}$ is selected from lower alkyl, phenyl, $R_7$-phenyl, benzyl or $R_7$-benzyl;
$R_{11}$ is selected from OH, lower alkyl, phenyl, benzyl, $R_7$-phenyl or $R_7$-benzyl:
$R_{12}$ is selected from H, OH, alkoxy, phenoxy, benzyloxy,

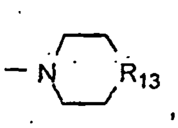

-N($R_8$)($R_9$), lower alkyl, phenyl or $R^7$-phenyl;
$R_{13}$ is selected from -O-, -$CH_2$-, -NH-, -N(lower alkyl)- or -NC(O)$R_{19}$;
$R_{15}$, $R_{16}$ and $R_{17}$ are independently selected from the group consisting of H and the groups defined for W; or
$R_{15}$ is hydrogen and $R_{16}$ and $R_{17}$, together with adjacent carbon atoms to which they are attached, form a

dioxolanyl ring;

$R_{19}$ is H, lower alkyl, phenyl or phenyl lower alkyl; and

$R_{20}$ and $R_{21}$ are independently selected from the group consisting of phenyl, W-substituted phenyl, naphthyl, W-substituted naphthyl, indanyl, indenyl, tetrahydronaphthyl, benzodioxolyl, heteroaryl, W-substituted heteroaryl, benzofused heteroaryl, W-substituted benzofused heteroaryl and cyclopropyl, wherein heteroaryl is as defined above,

for the manufacture of a medicament for regulating the amount of ApoE isoform 4 in the bloodstream and/or brain of the subject.

**59.** Use of a composition comprising at least one compound represented by Formula (VII):

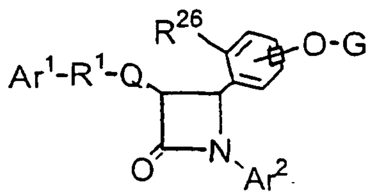

(VII)

or a pharmaceutically acceptable salt thereof or solvate thereof, wherein, in Formula (VII) above,

$R^{26}$ is H or $OG^1$;

G and $G^1$ are independently selected from the group consisting of H,

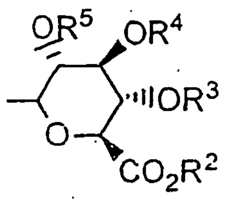

,

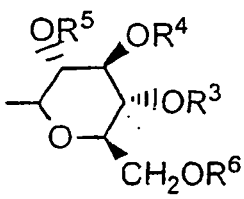

,

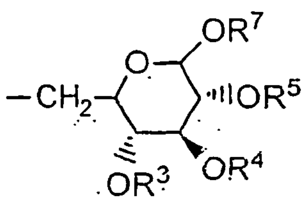

and

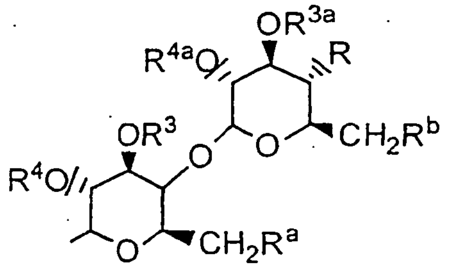

;

provided that when $R^{26}$ is H or OH, G is not H;

R, $R^a$ and $R^b$ are independently selected from the group consisting of H, -OH, halogeno, $-NH_2$, azido, $(C_1-C_6)$alkoxy$(C_1-C_6)$-alkoxy or $-W-R^{30}$;

W is independently selected from the group consisting of -NH-C(O)-, -O-C(O)-, $-O-C(O)-N(R^{31})-$, $-NH-C(O)-N(R^{31})-$ and $-O-C(S)-N(R^{31})-$;

$R^2$ and $R^6$ are independently selected from the group consisting of H, $(C_1-C_6)$alkyl, aryl and aryl$(C_1-C_6)$alkyl;

$R^3$, $R^4$, $R^5$, $R^7$, $R^{3a}$ and $R^{4a}$ are independently selected from the group consisting of H, $(C_1-C_6)$alkyl, aryl$(C_1-C_6)$alkyl, $-C(O)(C_1-C_6)$alkyl and -C(O)aryl;

$R^{30}$ is selected from the group consisting of $R^{32}$-substituted T, $R^{32}$-substituted-T-($C_1$-$C_6$)alkyl, $R^{32}$-substituted-($C_2$-$C_4$)alkenyl, $R^{32}$-substituted-($C_1$-$C_6$)alkyl, $R^{32}$-substituted-($C_3$-C7)cycloalkyl and $R^{32}$-substituted-($C_3$-$C_7$)cycloalkyl($C_1$-$C_6$)alkyl;

$R^{31}$ is selected from the group consisting of H and ($C_1$-$C_4$)alkyl;

T is selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;

$R^{32}$ is independently selected from 1-3 substituents independently selected from the group consisting of halogeno, ($C_1$-$C_4$)alkyl, -OH, phenoxy, -$CF_3$, -$NO_2$, ($C_1$-$C_4$)alkoxy, methylenedioxy, oxo, ($C_1$-$C_4$)alkylsulfanyl, ($C_1$-$C_4$)alkylsulfinyl, ($C_1$-$C_4$)alkylsulfonyl, -$N(CH_3)_2$, -C(O)-NH($C_1$-$C_4$)alkyl, -C(O)-N(($C_1$-$C_4$)alkyl)$_2$, -C(O)-($C_1$-$C_4$)alkyl, -C(O)-($C_1$-$C_4$)alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a ($C_1$-$C_4$)alkoxycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^{11}$-substituted aryl;

Q is a bond or, with the 3-position ring carbon of the azetidinone, forms the spiro group

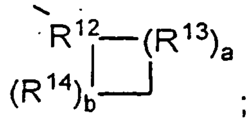

and

$R^1$ is selected from the group consisting of

-$(CH_2)_q$-, wherein q is 2-6, provided that when Q forms a spiro ring, q can also be zero or 1;

-$(CH_2)_e$-E-$(CH_2)_r$-, wherein E is -O-, -C(O)-, phenylene, -$NR^{22}$- or -$S(O)_{0-2}$-, e is 0-5 and r is 0-5, provided that the sum of e and r is 1-6;

-($C_2$-$C_6$)alkenylene-; and

-$(CH_2)_f$-V-$(CH_2)_g$-, wherein V is $C_3$-$C_6$ cycloalkylene, f is 1-5 and g is 0-5, provided that the sum of f and g is 1-6;

$R^{12}$ is

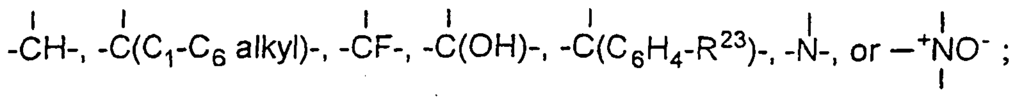

$R^{13}$ and $R^{14}$ are independently selected from the group consisting of -$CH_2$-, -CH($C_1$-$C_6$ alkyl)-, -C(di-($C_1$-$C_6$) alkyl), -CH=CH- and -C($C_1$-$C_6$ alkyl)=CH-; or $R^{12}$ together with an adjacent $R^{13}$, or $R^{12}$ together with an adjacent $R^{14}$, form a -CH=CH- or a -CH=C($C_1$-$C_6$ alkyl)- group;

a and b are independently 0, 1, 2 or 3, provided both are not zero;

provided that when $R^{13}$ is -CH=CH- or -C($C_1$-$C_6$ alkyl)=CH-, a is 1;

provided that when $R^{14}$ is -CH=CH- or -C($C_1$-$C_6$ alkyl)=CH-, b is 1;

provided that when a is 2 or 3, the $R^{13}$'s can be the same or different; and

provided that when b is 2 or 3, the $R^{14}$'s can be the same or different; :

and when Q is a bond, $R^1$ also can be:

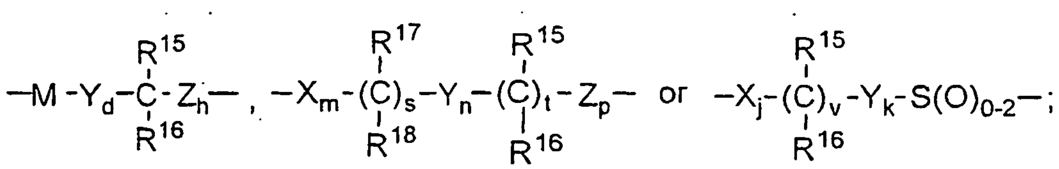

M is -O-, -S-, -S(O)- or -$S(O)_2$-;

X, Y and Z are independently selected from the group consisting of -$CH_2$-, -CH($C_1$-$C_6$)alkyl- and -C(di-($C_1$-$C_6$) alkyl);

$R^{10}$ and $R^{11}$ are independently selected from the group consisting of 1-3 substituents independently selected from the group consisting of $(C_1-C_6)$alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-S02NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$ alkylene)-$COOR^{19}$, $-CH=CH-COOR^{19}$, $-CF_3$, -CN, $-NO_2$ and halogen;

$R^{15}$ and $R^{17}$ are independently selected from the group consisting of $-OR^{19}$, $-O(CO)R^{19}$, $-O(CO)OR^{21}$ and -O (CO)$NR^{19}R^{20}$;

$R^{16}$ and $R^{18}$ are independently selected from the group consisting of H, $(C_1-C_6)$alkyl and aryl; or $R^{15}$ and $R^{16}$ together are =O, or $R^{17}$ and $R^{18}$ together are =O;

d is 1, 2 or 3;

h is 0, 1, 2, 3 or 4;

s is 0 or 1; t is 0 or 1; m, n and p are independently 0-4;

provided that at least one of s and t is 1, and the sum of m, n, p, s and t is 1-6;

provided that when p is 0 and t is 1, the sum of m, s and n is 1-5; and provided that when p is 0 and s is 1, the sum of m, t and n is 1-5;

v is 0 or 1;

j and k are independently 1-5, provided that the sum of j, k and v is 1-5;

and when Q is a bond and $R^1$ is

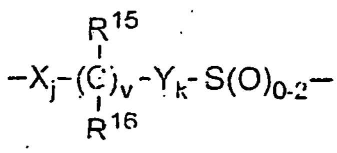

,

Ar[1] can also be pyridyl, isoxazolyl, furanyl, pyrrolyl, thienyl, imidazolyl, pyrazolyl, thiazolyl, pyrazinyl, pyrimidinyl or pyridazinyl;

$R^{19}$ and $R^{20}$ are independently selected from the group consisting of H, $(C_{1-}C_6)$alkyl, aryl and aryl-substituted $(C_1-C_6)$alkyl;

$R^{21}$ is $(C_1-C_6)$alkyl, aryl or $R^{24}$-substituted aryl;

$R^{22}$ is H, $(C_1-C_6)$alkyl, aryl $(C_1-C_6)$alkyl, $-C(O)R^{19}$ or $-COOR^{19}$;

R23 and $R^{24}$ are independently 1-3 groups independently selected from the group consisting of H, $(C_1-C_6)$alkyl, $(C_1-C_6)$alkoxy, -COOH, $NO_2$, $-NR^{19}R^{20}$, -OH and halogeno; and

$R^{25}$ is H, -OH or $(C_1-C_6)$alkoxy,

for the manufacture of a medicament for preventing, treating, or ameliorating symptoms of Alzheimer's Disease in the subject.

**60.** Use of a composition comprising at least one compound represented by Formula (VII):

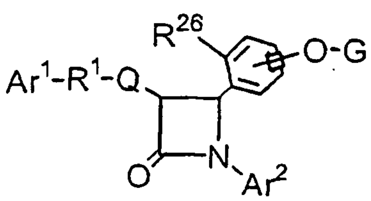

(VII)

or a pharmaceutically acceptable salt thereof or solvate thereof, wherein, in Formula (VII) above,

$R^{26}$ is H or $OG^1$;

G and $G^1$ are independently selected from the group consisting of H,

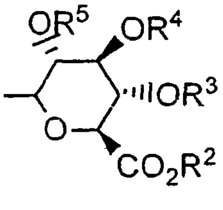 , 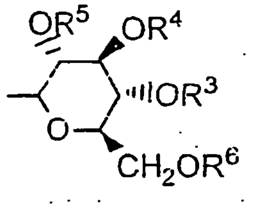 , 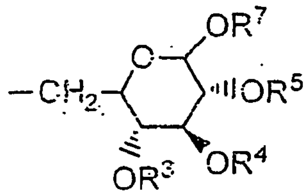

and

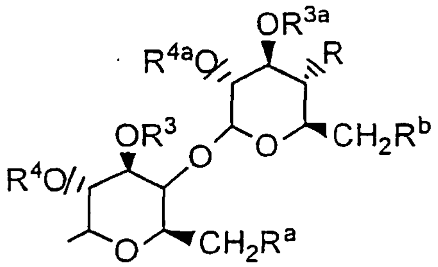 ;

provided that when $R^{26}$ is H or OH, G is not H;

R, $R^a$ and $R^b$ are independently selected from the group consisting of H, -OH, halogeno, $-NH_2$, azido, $(C_1-C_6)$ alkoxy$(C_1-C_6)$-alkoxy or $-W-R^{30}$;

W is independently selected from the group consisting of -NH-C(O)-, -O-C(O)-, $-O-C(O)-N(R^{31})-$, $-NH-C(O)-N(R^{31})-$ and $-O-C(S)-N(R^{31})-$;

$R^2$ and $R^6$ are independently selected from the group consisting of H, $(C_{1-}C_6)$alkyl, aryl and aryl$(C_1-C_6)$alkyl;

$R^3$, $R^4$, $R^5$, $R^7$, $R^{3a}$ and $R^{4a}$ are independently selected from the group consisting of H, $(C_1-C_6)$alkyl, aryl$(C_1-C_6)$alkyl, $-C(O)(C_1-C_6)$alkyl and- C(O)aryl;

$R^{30}$ is selected from the group consisting of $R^{32}$-substituted T, $R^{32}$-substituted-T-$(C_1-C_6)$alkyl, $R^{32}$-substituted-$(C_2-C_4)$alkenyl, $R^{32}$-substituted-$(C_1-C_6)$alkyl, $R^{32}$-substituted-$(C_3$-C7)cycloalkyl and $R^{32}$-substituted-$(C_3-C_7)$cycloalkyl$(C_1-C_6)$alkyl;

$R^{31}$ is selected from the group consisting of H and $(C_1-C_4)$alkyl;

T is selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;

$R^{32}$ is independently selected from 1-3 substituents independently selected from the group consisting of halogeno, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsulfinyl, $(C_1-C_4)$alkylsulfonyl, $-N(CH_3)_2$, $-C(O)-NH(C_1-C_4)$alkyl, $-C(O)-N((C_1-C_4)\text{alkyl})_2$, $-C(O)-(C_1-C_4)$alkyl, $-C(O)-(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$alkoxycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^{11}$-substituted aryl;

Q is a bond or, with the 3-position ring carbon of the azetidinone, forms the spiro group

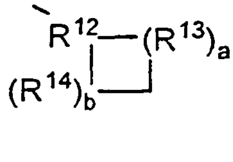 ;

and

$R^1$ is selected from the group consisting of

$-(CH_2)_q-$, wherein q is 2-6, provided that when Q forms a spiro ring, q can also be zero or 1;

$-(CH_2)_e-E-(CH_2)_r-$, wherein E is -O-, -C(O)-, phenylene, $-NR^{22}-$ or$-S(O)_{0-2}-$, e is 0-5 and r is 0-5, provided that

the sum of e and r is 1-6;
-(C2-$C_6$)alkenylene-; and
$-(CH_2)_f-V-(CH_2)_g-$, wherein V is $C_3$-$C_6$ cycloalkylene, f is 1-5 and g is 0-5, provided that the sum of f and g is 1-6;
$R^{12}$ is

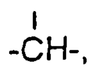, 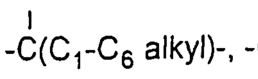, 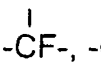, 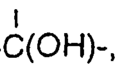, 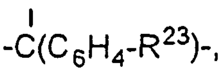, 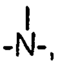, or 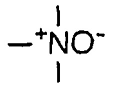 ;

$R^{13}$ and $R^{14}$ are independently selected from the group consisting of $-CH_2-$, -CH($C_1$-$C_6$ alkyl)-, -C(di-($C_1$-$C_6$) alkyl), -CH=CH- and -C($C_1$-$C_6$ alkyl)=CH-; or $R^{12}$ together with an adjacent $R^{13}$, or $R^{12}$ together with an adjacent $R^{14}$, form a -CH=CH- or a -CH=C($C_1$-$C_6$ alkyl)- group;
a and b are independently 0, 1, 2 or 3, provided both are not zero;
provided that when $R^{13}$ is -CH=CH- or -C($C_1$-$C_6$ alkyl)=CH-, a is 1;
provided that when $R^{14}$ is -CH=CH- or -C($C_1$-$C_6$ alkyl)=CH-, b is 1;
provided that when a is 2 or 3, the $R^{13}$'s can be the same or different; and
provided that when b is 2 or 3, the $R^{14}$'s can be the same or different;
and when Q is a bond, $R^1$ also can be:

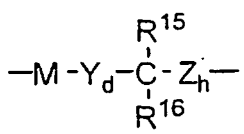 , 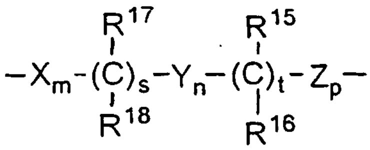 or 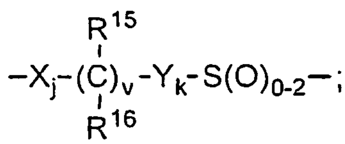;

M is -O-, -S-, -S(O)- or $-S(O)_2-$;
X, Y and Z are independently selected from the group consisting of $-CH_2-$, -CH($C_1$-$C_6$)alkyl- and -C(di-($C_1$-$C_6$) alkyl);
$R^{10}$ and $R^{11}$ are independently selected from the group consisting of 1-3 substituents independently selected from the group consisting of ($C_1$-$C_6$)alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, -($C_1$-$C_6$ alkylene)-$COOR^{19}$, $-CH=CH-COOR^{19}$, $-CF_3$, -CN, $-NO_2$ and halogen;
$R^{15}$ and $R^{17}$ are independently selected from the group consisting of $-OR^{19}$, $-O(CO)R^{19}$, $-O(CO)OR^{21}$ and -O (CO)$NR^{19}R^{20}$;
$R^{16}$ and $R^{18}$ are independently selected from the group consisting of H, ($C_1$-$C_6$)alkyl and aryl; or $R^{15}$ and $R^{16}$ together are =O, or $R^{17}$ and $R^{18}$ together are =O;
d is 1, 2 or 3;
h is 0, 1, 2, 3 or 4;
s is 0 or 1; t is 0 or 1; m, n and p are independently 0-4;
provided that at least one of s and t is 1, and the sum of m, n, p, s and t is 1-6;
provided that when p is 0 and t is 1, the sum of m, s and n is 1-5; and provided that when p is 0 and s is 1, the sum of m, t and n is 1-5;
v is 0 or 1;
j and k are independently 1-5, provided that the sum of j, k and v is 1-5;
and when Q is a bond and $R^1$ is

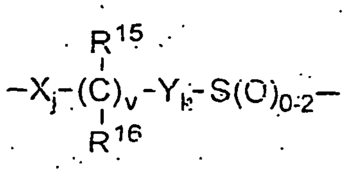 ,

$Ar^1$ can also be pyridyl, isoxazolyl, furanyl, pyrrolyl, thienyl, imidazolyl, pyrazolyl, thiazolyl, pyrazinyl, pyrimidinyl or pyridazinyl;

$R^{19}$ and $R^{20}$ are independently selected from the group consisting of H, ($C_1$-$C_6$)alkyl, aryl and aryl-substituted ($C_1$-$C_6$)alkyl;
$R^{21}$ is ($C_1$-$C_6$)alkyl, aryl or $R^{24}$-substituted aryl;
$R^{22}$ is H, ($C_1$-$C_6$)alkyl, aryl ($C_1$-$C_6$)alkyl, -C(O)$R^{19}$ or -COOR$^{19}$;
$R^{23}$ and $R^{24}$ are independently 1-3 groups independently selected from the group consisting of H, ($C_1$-$C_6$)alkyl, ($C_1$-$C_6$)alkoxy, -COOH, $NO_2$, -NR$^{19}$R$^{20}$, -OH and halogeno; and
$R^{25}$ is H, -OH or ($C_1$-$C_6$)alkoxy,

for the manufacture of a medicament for regulating the production or level of at least one amyloid β peptide in the subject.

**61.** Use of a composition comprising at least one compound represented by Formula (VII):

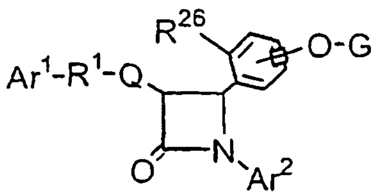

(VII)

or a pharmaceutically acceptable salt thereof or solvate thereof, wherein, in Formula (VII) above,

$R^{26}$ is H or OG$^1$;
G and G$^1$ are independently selected from the group consisting of H.

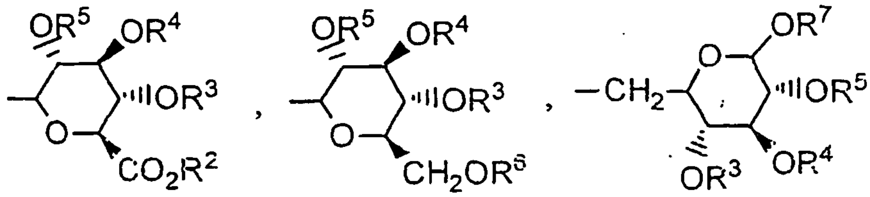

and

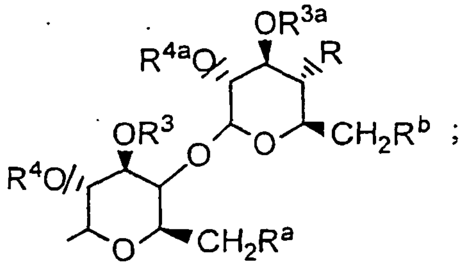

provided that when $R^{26}$ is H or OH, G is not H;
R, $R^a$ and $R^b$ are independently selected from the group consisting of H, -OH, halogeno, -$NH_2$, azido, ($C_1$-$C_6$)alkoxy($C_1$-$C_6$)-alkoxy or -W-$R^{30}$;
W is independently selected from the group consisting of -NH-C(O)-, -O-C(O)-, -O-C(O)-N($R^{31}$)-, -NH-C(O)-N($R^{31}$)- and -O-C(S)-N($R^{31}$)-;
$R^2$ and $R^6$ are independently selected from the group consisting of H, ($C_1$-$C_6$)alkyl, aryl and aryl($C_1$-$C_6$)alkyl;
$R^3$, $R^4$, $R^5$, $R^7$, $R^{3a}$ and $R^{4a}$ are independently selected from the group consisting of H, ($C_1$-$C_6$)alkyl, aryl($C_1$-$C_6$)

alkyl, $-C(O)(C_1-C_6)$alkyl and -C(O)aryl;

$R^{30}$ is selected from the group consisting of $R^{32}$-substituted T, $R^{32}$-substituted-T-$(C_1-C_6)$alkyl, $R^{32}$-substituted-$(C_2-C_4)$alkenyl, $R^{32}$-substituted-$(C_1-C_6)$alkyl, $R^{32}$-substituted-$(C_3$-C7)cycloalkyl and $R^{32}$-substituted-$(C_3$-C7)cycloalkyl$(C_1-C_6)$alkyl;

$R^{31}$ is selected from the group consisting of H and $(C_1-C_4)$alkyl;

T is selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;

$R^{32}$ is independently selected from 1-3 substituents independently selected from the group consisting of halogeno, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsulfinyl, $(C_1-C_4)$alkylsulfonyl, $-N(CH_3)_2$, $-C(O)-NH(C_1-C_4)$alkyl, $-C(O)-N((C_1-C_4)$alkyl$)_2$, -C(O)-$(C_1-C_4)$alkyl, -C(O)-$(C_1$-$C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$alkoxycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^{11}$-substituted aryl;

Q is a bond or, with the 3-position ring carbon of the azetidinone, forms the spiro group

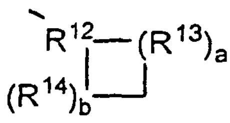

;

and

$R^1$ is selected from the group consisting of

$-(CH_2)_q-$, wherein q is 2-6, provided that when Q forms a spiro ring, q can also be zero or 1;

$-(CH_2)_e-E-(CH_2)_r-$, wherein E is -O-, -C(O)-, phenylene, $-NR^{22}$- or $-S(O)_{0-2}-$, e is 0-5 and r is 0-5, provided that the sum of e and r is 1-6;

$-(C_2-C_6)$alkenylene-; and

$-(CH_2)_f-V-(CH_2)_g-$, wherein V is $C_3-C_6$ cycloalkylene, f is 1-5 and g is 0-5, provided that the sum of f and g is 1-6;

$R^{12}$ is

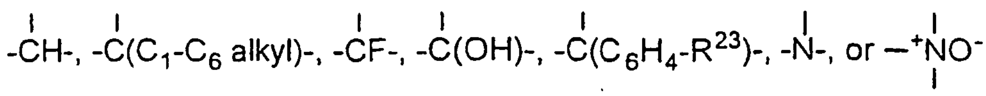

;

$R^{13}$ and $R^{14}$ are independently selected from the group consisting of $-CH_2-$, $-CH(C_1-C_6$ alkyl)-, -C(di-$(C_1-C_6)$ alkyl), -CH=CH- and $-C(C_1-C_6$ alkyl)=CH-; or $R^{12}$ together with an adjacent $R^{13}$, or $R^{12}$ together with an adjacent $R^{14}$, form a -CH=CH- or a $-CH=C(C_1-C_6$ alkyl)- group;

a and b are independently 0, 1, 2 or 3, provided both are not zero;

provided that when $R^{13}$ is -CH=CH- or-$C(C_1-C_6$ alkyl)=CH-, a is 1;

provided that when $R^{14}$ is -CH=CH- or $-C(C_1-C_6$ alkyl)=CH-, b is 1;

provided that when a is 2 or 3, the $R^{13}$'s can be the same or different; and

provided that when b is 2 or 3, the $R^{14}$'s can be the same or different;

and when Q is a bond, $R^1$ also can be:

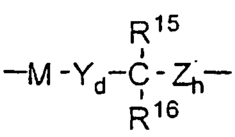

,

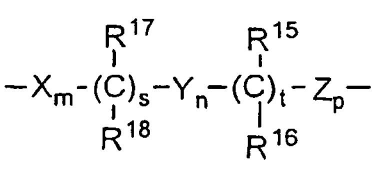

or

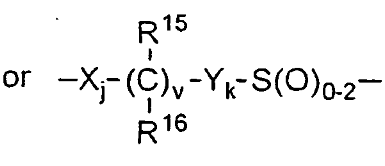

;

M is -O-, -S-, -S(O)- or $-S(O)_2-$;

X, Y and Z are independently selected from the group consisting of $-CH_2-$, $-CH(C_1-C_6)alkyl-$ and $-C(di-(C_1-C_6)alkyl)$;

$R^{10}$ and $R^{11}$ are independently selected from the group consisting of 1-3 substituents independently selected from the group consisting of $(C_1-C_6)$alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$ alkylene)$-COOR^{19}$, $-CH=CH-COOR^{19}$, $-CF_3$, -CN, $-NO_2$ and halogen;

$R^{15}$ and $R^{17}$ are independently selected from the group consisting of $-OR^{19}$, $-O(CO)R^{19}$, $-O(CO)OR^{21}$ and $-O(CO)NR^{19}R^{20}$;

$R^{16}$ and $R^{18}$ are independently selected from the group consisting of H, $(C_1-C_6)$alkyl and aryl; or $R^{15}$ and $R^{16}$ together are =O, or $R^{17}$ and $R^{18}$ together are =O;

d is 1, 2 or 3;

h is 0, 1, 2, 3 or 4;

s is 0 or 1; t is 0 or 1; m, n and p are independently 0-4;

provided that at least one of s and t is 1, and the sum of m, n, p, s and t is 1-6;

provided that when p is 0 and t is 1, the sum of m, s and n is 1-5; and provided that when p is 0 and s is 1, the sum of m, t and n is 1-5;

v is 0 or 1;

j and k are independently 1-5, provided that the sum of j, k and v is 1-5;

and when Q is a bond and $R^1$ is

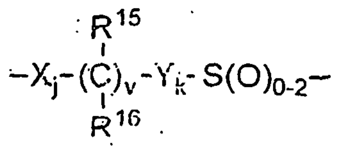

,

$Ar^1$ can also be pyridyl, isoxazolyl, furanyl, pyrrolyl, thienyl, imidazolyl, pyrazolyl, thiazolyl, pyrazinyl, pyrimidinyl or pyridazinyl;

$R^{19}$ and $R^{20}$ are independently selected from the group consisting of H, $(C_1-C_6)$alkyl, aryl and aryl-substituted $(C_1-C_6)$alkyl;

$R^{21}$ is $(C_1-C_6)$alkyl, aryl or $R^{24}$-substituted aryl;

$R^{22}$ is H, $(C_1-C_6)$alkyl, aryl $(C_1-C_6)$alkyl, $-C(O)R^{19}$ or $-COOR^{19}$;

$R^{23}$ and $R^{24}$ are independently 1-3 groups independently selected from the group consisting of H, $(C_1-C_6)$alkyl, $(C_1-C_6)$alkoxy, -COOH, $NO_2$, $-NR^{19}R^{20}$, -OH and halogeno; and

$R^{25}$ is H, -OH or $(C_1-C_6)$alkoxy,

for the manufacture of a medicament for regulating the amount of ApoE isoform 4 in the bloodstream and/or brain of the subject.

**62.** Use of a composition comprising at least one compound represented by Formula (VIII):

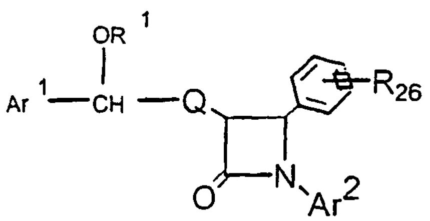

(VIII)

or a pharmaceutically acceptable salt or solvate thereof, wherein

$R^{26}$ is selected from the group consisting of:

a) OH;
b) $OCH_3$;
c) fluorine and
d) chlorine.

$R^1$ is selected from the group consisting of H,

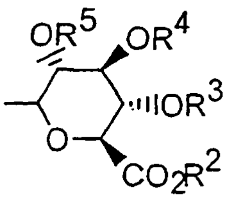 , 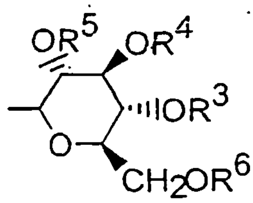 , 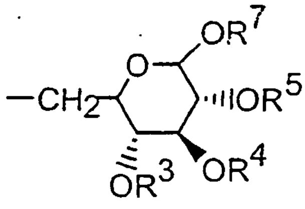 ,

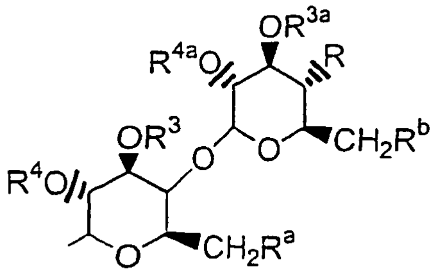 ,

$-SO_3H$; natural and unnatural amino acids.

R, $R^a$ and $R^b$ are independently selected from the group consisting of H, -OH, halogeno, $-NH_2$, azido, $(C_1-C_6)$alkoxy$(C_1-C_6)$-alkoxy and $-W-R^{30}$;

W is independently selected from the group consisting of -NH-C(O)-, -O-C(O)-, $-O-C(O)-N(R^{31})-$, $-NH-C(O)-N(R^{31})-$ and $-O-C(S)-N(R^{31})-$;

$R^2$ and $R^6$ are independently selected from the group consisting of H, $(C_1-C_6)$alkyl, aryl and aryl$(C_1-C_6)$alkyl;

$R^3$, $R^4$, $R^5$, $R^7$, $R^{3a}$ and $R^{4a}$ are independently selected from the group consisting of H, $(C_1-C_6)$alkyl, aryl$(C_1-C_6)$alkyl, $-C(O)(C_1-C_6)$alkyl and -C(O)aryl;

$R^{30}$ is independently selected form the group consisting of $R^{32}$-substituted T, $R^{32}$-substituted-T-$(C_1-C_6)$alkyl, $R^{32}$-substituted-$(C_2-C_4)$alkenyl, $R^{32}$-substituted-$(C_1-C_6)$alkyl, $R^{32}$-substituted-$(C_3-C_7)$cycloalkyl and $R^{32}$-substituted-$(C_3$-C7)cycloalkyl$(C_1-C_6)$alkyl;

$R^{31}$ is independently selected from the group consisting of H and $(C_1-C_4)$alkyl;

T is independently selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;

$R^{32}$ is independently selected from 1-3 substituents independently selected from the group consisting of H, halogeno, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsulfinyl, $(C_1-C_4)$alkylsulfonyl, $-N(CH_3)_2$, $-C(O)-NH(C_1-C_4)$alkyl, $-C(O)-N((C_1-C_4)$alkyl$)_2$, $-C(O)-(C_1-C_4)$alkyl, $-C(O)-(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$alkoxycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^{11}$-substituted aryl;

Q is $-(CH_2)_q-$, wherein q is 2-6, or, with the 3-position ring carbon of the azetidinone, forms the spiro group

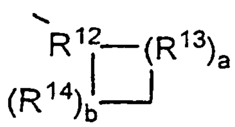 ;

$R^{12}$ is

$$-CH-, -C(C_1-C_6\ alkyl)-, -CF-, -C(OH)-, -C(C_6H_4-R^{23})-, -N-, \text{ or } -^{+}NO^{-};$$

$R^{13}$ and $R^{14}$ are independently selected from the group consisting of $-CH_2-$, $-CH(C_1-C_6\ alkyl)-$, $-C(di-(C_1-C_6)\ alkyl)$, $-CH=CH-$ and $-C(C_1-C_6\ alkyl)=CH-$; or $R^{12}$ together with an adjacent $R^{13}$, or $R^{12}$ together with an adjacent $R^{14}$, form a $-CH=CH-$ or a $-CH=C(C_1-C_6\ alkyl)-$ group;

a and b are independently 0, 1, 2 or 3, provided both are not zero; provided that when $R^{13}$ is $-CH=CH-$ or $-C(C_1-C_6\ alkyl)=CH-$, a is 1; provided that when $R^{14}$ is $-CH=CH-$ or $-C(C_1-C_6\ alkyl)=CH-$, b is 1; provided that when a is 2 or 3, the $R^{13}$'s can be the same or different; and provided that when b is 2 or 3, the $R^{14}$'s can be the same or different;

$R^{10}$ and $R^{11}$ are independently selected from the group consisting of 1-3 substituents independently selected from the group consisting of $(C_1-C_6)$alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6\ alkylene)-COOR^{19}$, $-CH=CH-COOR^{19}$, $-CF_3$, $-CN$, $-NO_2$ and halogen;

$R^{19}$ and $R^{20}$ are independently selected from the group consisting of H, $(C_1-C_6)$alkyl, aryl and aryl-substituted $(C_1-C_6)$alkyl;

$R^{21}$ is $(C_1-C_6)$alkyl, aryl or $R^{24}$-substituted aryl;

$R^{22}$ is H, $(C_1-C_6)$alkyl, aryl $(C_1-C_6)$alkyl, $-C(O)R^{19}$ or $-COOR^{19}$;

R23 and $R^{24}$ are independently 1-3 groups independently selected from the group consisting of H, $(C_1-C_6)$alkyl, $(C_1-C_6)$alkoxy, -COOH, $NO_2$, $-NR^{19}R^{20}$, -OH and halogeno; and

$R^2$5 is H, -OH or $(C_1-C_6)$alkoxy,

for the manufacture of a medicament for preventing, treating, or ameliorating symptoms of Alzheimer's Disease in the subject.

**63.** Use a composition comprising at least one compound represented by Formula (VIII):

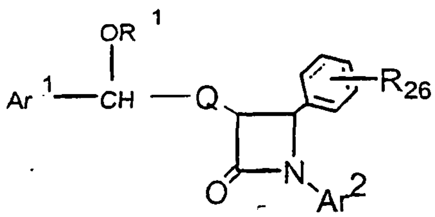

(VIII)

or a pharmaceutically acceptable salt or solvate thereof, wherein

$R^{26}$ is selected from the group consisting of:

a) OH;
b) $OCH_3$;
c) fluorine and
d) chlorine.

$R^1$ is selected from the group consisting of H,

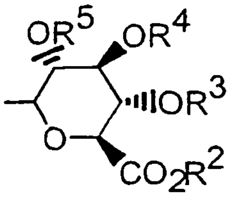 , 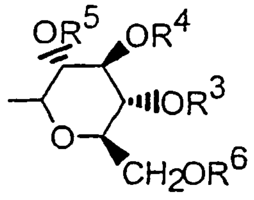 , 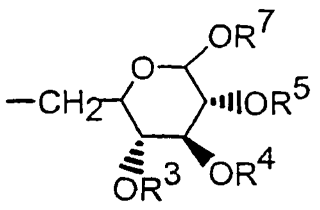 ,

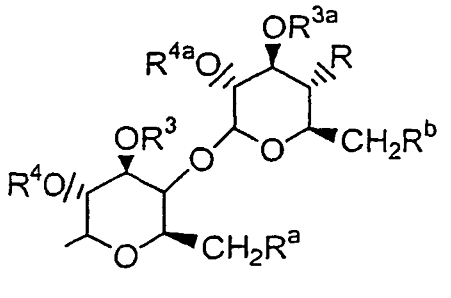 ,

$-SO_3H$; natural and unnatural amino acids.

R, $R^a$ and $R^b$ are independently selected from the group consisting of H, -OH, halogeno, $-NH_2$, azido, $(C_1-C_6)$alkoxy$(C_1-C_6)$-alkoxy and $-W-R^{30}$;

W is independently selected from the group consisting of- NH-C(O)-, -O-C(O)-, $-O-C(O)-N(R^{31})-$, $-NH-C(O)-N(R^{31})-$ and $-O-C(S)-N(R^{31})-$;

$R^2$ and $R^6$ are independently selected from the group consisting of H, $(C_{1-}C_6)$alkyl, aryl and aryl$(C_1-C_6)$alkyl;

$R^3$,$R^4$, $R^5$, $R^7$, $R^{3a}$ and $R^{4a}$ are independently selected from the group consisting of H, $(C_1-C_6)$alkyl, aryl$(C_1-C_6)$alkyl, $-C(O)(C_1-C_6)$alkyl and -C(O)aryl;

$R^{30}$ is independently selected form the group consisting of $R^{32}$-substituted T, $R^{32}$-substituted-T-$(C_1-C_6)$alkyl, $R^{32}$-substituted-$(C_2-C_4)$alkenyl, $R^{32}$-substituted-$(C_1-C_6)$alkyl, $R^{32}$-substituted-$(C_3$-C7)cycloalkyl and $R^{32}$-substituted-$(C_3$-C7)cycloalkyl$(C_1-C_6)$alkyl;

$R^{31}$ is independently selected from the group consisting of H and $(C_1-C_4)$alkyl;

T is independently selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;

$R^{32}$ is independently selected from 1-3 substituents independently selected from the group consisting of H, halogeno, $(C_1-C_4)$alkyl, -OH, phenoxy, $-CF_3$, $-NO_2$, $(C_1-C_4)$alkoxy, methylenedioxy, oxo, $(C_1-C_4)$alkylsulfanyl, $(C_1-C_4)$alkylsulfinyl, $(C_{1-}C_4)$alkylsulfonyl, $-N(CH_3)_2$, $-C(O)-NH(C_1-C_4)$alkyl, $-C(O)-N((C_1-C_4)alkyl)_2$, $-C(O)-(C_1-C_4)$alkyl, $-C(O)-(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_{1-}C_4)$alkoxycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^{11}$-substituted aryl;

Q is $-(CH_2)q-$, wherein q is 2-6, or, with the 3-position ring carbon of the azetidinone, forms the spiro group

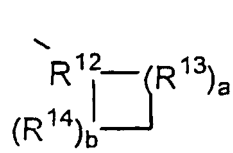 ;

$R^{12}$ is

$-CH-$, $-C(C_1-C_6\ alkyl)-$, $-CF-$, $-C(OH)-$, $-C(C_6H_4-R^{23})-$, $-N-$, or $-^{+}NO^{-}$ ;

$R^{13}$ and $R^{14}$ are independently selected from the group consisting of $-CH_2-$, $-CH(C_1-C_6$ alkyl)-, -C(di-($C_1-C_6$) alkyl), -CH=CH- and -C($C_1-C_6$ alkyl)=CH-; or $R^{12}$ together with an adjacent $R^{13}$, or $R^{12}$ together with an adjacent $R^{14}$, form a -CH=CH- or a -CH=C($C_1-C_6$ alkyl)- group;

a and b are independently 0, 1, 2 or 3, provided both are not zero; provided that when $R^{13}$ is -CH=CH- or -C ($C_1-C_6$ alkyl)=CH-, a is 1; provided that when $R^{14}$ is -CH=CH- or -C($C_1-C_6$ alkyl)=CH-, b is 1; provided that when a is 2 or 3, the $R^{13}$'s can be the same or different; and provided that when b is 2 or 3, the $R^{14}$'s can be the same or different;

$R^{10}$ and $R^{11}$ are independently selected from the.group consisting of 1-3 substituents independently, selected from the group consisting of ($C_1-C_6$)alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, -($C_1-C_6$ alkylene)-$COOR^{19}$, $-CH=CH-COOR^{19}$, $-CF_3$, -CN, $-NO_2$ and halogen;

$R^{19}$ and $R^{20}$ are independently selected from the group consisting of H, ($C_1-C_6$)alkyl, aryl and aryl-substituted ($C_1-C_6$)alkyl;

R21 is ($C_1-C_6$)alkyl, aryl or $R^{24}$-substituted aryl;

$R^{22}$ is H, ($C_1-C_6$)alkyl, aryl ($C_1-C_6$)alkyl, $-C(O)R^{19}$ or $-COOR^{19}$;

$R^{23}$ and $R^{24}$ are independently 1-3 groups independently selected from the group consisting of H, ($C_1-C_6$)alkyl, ($C_1-C_6$)alkoxy, -COOH, $NO_2$, $-NR^{19}R^{20}$, -OH and halogeno; and

$R^{25}$ is H, -OH or ($C_1-C_6$)alkoxy,

for the manufacture of a medicament for regulating the production or level of at least one amyloid β peptide in the subject.

**64.** Use of a composition comprising at least one compound represented by Formula (VIII):

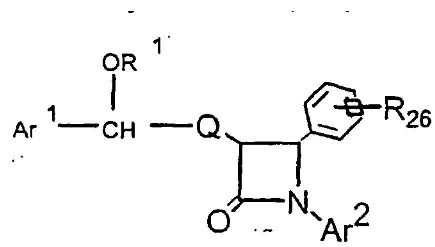

(VIII)

or a pharmaceutically acceptable salt or solvate thereof, wherein

$R^{26}$ is selected from the group consisting of:

a) OH;
b) $OCH_3$;
c) fluorine and
d) chlorine.

$R^1$ is selected from the group consisting of H,

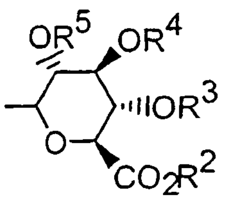 , 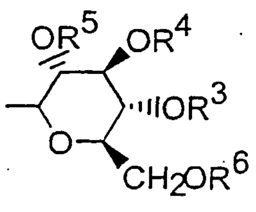 , 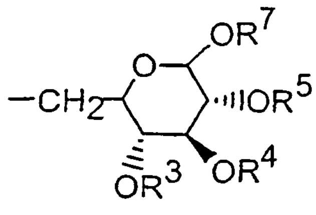 ,

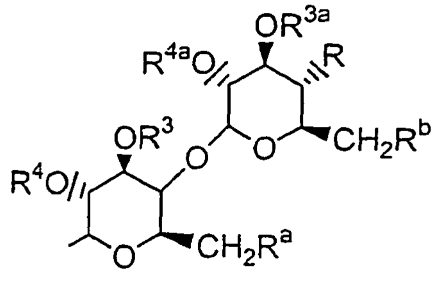 ,

$-SO_3H$; natural and unnatural amino acids.

R, $R^a$ and $R^b$ are independently selected from the group consisting of H, -OH, halogeno, $-NH_2$, azido, $(C_1-C_6)$alkoxy$(C_1-C_6)$-alkoxy and $-W-R^{30}$;

W is independently selected from the group consisting of- NH-C(O)-, -O-C(O)-, $-O-C(O)-N(R^{31})-$, $-NH-C(O)-N(R^{31})-$ and $-O-C(S)-N(R^{31})-$;

$R^2$ and $R^6$ are independently selected from the group consisting of H, $(C_1-C_6)$alkyl, aryl and aryl$(C_1-C_6)$alkyl;

$R^3$, $R^4$, $R^5$, $R^7$, $R^{3a}$ and $R^{4a}$ are independently selected from the group consisting of H, $(C_1-C_6)$alkyl, aryl$(C_1-C_6)$alkyl, $-C(O)(C_1-C_6)$alkyl and -C(O)aryl;

$R^{30}$ is independently selected form the group consisting of $R^{32}$-substituted T, $R^{32}$-substituted-T-$(C_1-C_6)$alkyl, $R^{32}$-substituted-$(C_2-C_4)$alkenyl, $R^{32}$-substituted-$(C_1-C_6)$alkyl, $R^{32}$-substituted-$(C_3-C_7)$cycloalkyl and $R^{32}$-substituted-$(C_3-C_7)$cycloalkyl$(C_1-C_6)$alkyl;

$R^{31}$ is independently selected from the group consisting of H and $(C_1-C_4)$alkyl;

T is independently selected from the group consisting of phenyl, furyl, thienyl, pyrrolyl, oxazolyl, isoxazolyl, thiazolyl, iosthiazolyl, benzothiazolyl, thiadiazolyl, pyrazolyl, imidazolyl and pyridyl;

$R^{32}$ is independently selected from 1-3 substituents independently selected from the group consisting of H, halogeno, $(C_1-C_4)$alkyl, -OH, phenoxy, $-CF_3$, $-NO_2$, $(C_1-C_4)$alkoxy; methylenedioxy, oxo, $(C_1-C_4)$alkylsulfanyl, $(C_1-C_4)$alkylsulfinyl, $(C_1-C_4)$alkylsulfonyl, $-N(CH_3)_2$, $-C(O)-NH(C_1-C_4)$alkyl, $-C(O)-N((C_1-C_4)alkyl)_2$, $-C(O)-(C_1-C_4)$alkyl, $-C(O)-(C_1-C_4)$alkoxy and pyrrolidinylcarbonyl; or $R^{32}$ is a covalent bond and $R^{31}$, the nitrogen to which it is attached and $R^{32}$ form a pyrrolidinyl, piperidinyl, N-methyl-piperazinyl, indolinyl or morpholinyl group, or a $(C_1-C_4)$alkoxycarbonyl-substituted pyrrolidinyl, piperidinyl, N-methylpiperazinyl, indolinyl or morpholinyl group;

$Ar^1$ is aryl or $R^{10}$-substituted aryl;

$Ar^2$ is aryl or $R^{11}$-substituted aryl;

Q is $-(CH_2)_q-$, wherein q is 2-6, or, with the 3-position ring carbon of the azetidinone, forms the spiro group

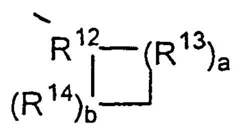 ;

$R^{12}$ is

$$-\overset{|}{C}H-,\ -\overset{|}{C}(C_1-C_6\ alkyl)-,\ -\overset{|}{C}F-,\ -\overset{|}{C}(OH)-,\ -\overset{|}{C}(C_6H_4-R^{23})-,\ -\overset{|}{N}-,\ or\ -{}^{+}\underset{|}{\overset{|}{N}}O^{-}\ ;$$

$R^{13}$ and $R^{14}$ are independently selected from the group consisting of $-CH_2-$, $-CH(C_1-C_6$ alkyl$)-$, $-C(di-(C_1-C_6)$ alkyl), $-CH=CH-$ and $-C(C_1-C_6$ alkyl$)=CH-$; or $R^{12}$ together with an adjacent $R^{13}$, or $R^{12}$ together with an adjacent $R^{14}$, form a $-CH=CH-$ or a $-CH=C(C_1-C_6$ alkyl$)-$ group;

a and b are independently 0, 1, 2 or 3, provided both are not zero; provided that when $R^{13}$ is $-CH=CH-$ or $-C(C_1-C_6$ alkyl$)=CH-$, a is 1; provided that when $R^{14}$ is $-CH=CH-$ or $-C(C_1-C_6$ alkyl$)=CH-$, b is 1; provided that when a is 2 or 3, the $R^{13}$'s can be the same or different; and provided that when b is 2 or 3, the $R^{14}$'s can be the same or different;

$R^{10}$ and $R^{11}$ are independent!y selected from the group consisting of 1-3 substituents independently selected from the group consisting of $(C_1-C_6)$alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6$ alkylene$)-COOR^{19}$, $-CH=CH-COOR^{19}$, $-CF_3$, $-CN$, $-NO_2$ and halogen;

R19 and $R^{20}$ are independently selected from the group consisting of H, $(C_{1-}C_6)$alkyl, aryl and aryl-substituted $(C_1-C_6)$alkyl;

$R^{21}$ is $(C_1-C_6)$alkyl, aryl or $R^{24}$-substituted aryl;

$R^{22}$ is H, $(C_1-C_6)$alkyl, aryl $(C_1-C_6)$alkyl, $-C(O)R^{19}$ or $-COOR^{19}$;

$R^{23}$ and $R^{24}$ are independently 1-3 groups independently selected from the group consisting of H, $(C_1-C_6)$alkyl, $(C_1-C_6)$alkoxy, $-COOH$, $NO_2$, $-NR^{19}R^{20}$, $-OH$ and halogeno; and

$R^{25}$ is H, $-OH$ or $(C_1-C_6)$alkoxy,

for the manufacture of a medicament for regulating the amount of ApoE isoform 4 in the bloodstream and/or brain of the subject.

## Patentansprüche

**1.** Verwendung einer Zusammensetzung, umfassend zumindest eine Verbindung gemäß Formel (I):

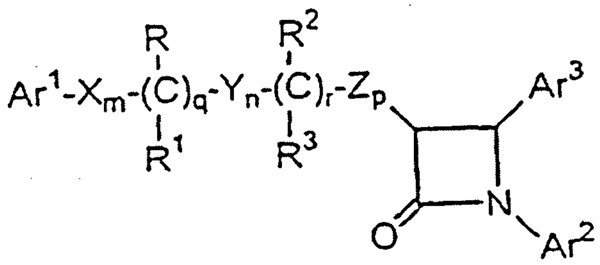

(I)

oder ein pharmazeutisch annehmbares Salz oder Solvat davon, wobei

$Ar^1$ und $Ar^2$ unabhängig voneinander ausgewählt sind aus der Gruppe, bestehend aus Aryl und $R^4$-substituiertem Aryl;

$Ar^3$ Aryl oder $R^5$-substituiertes Aryl ist;

X, Y und Z unabhängig voneinander ausgewählt sind aus der Gruppe, bestehend aus $-CH_2-$, -CH(Niederalkyl)- und -C(Diniederalkyl)-;

R und $R^2$ unabhängig voneinander ausgewählt sind aus der Gruppe, bestehend aus $-OR^6$, $-O(CO)R^6$, $-O(CO)OR^9$ und $-O(CO)NR^6R^7$;

$R^1$ und $R^3$ unabhängig voneinander ausgewählt sind aus der Gruppe, bestehend aus Wasserstoff, Niederalkyl und Aryl;

q ist 0 oder 1;

r ist 0 oder 1;

m, n und p sind unabhängig voneinander ausgewählt aus 0, 1, 2, 3 oder 4; vorausgesetzt, dass zumindest eines von q und r 1 ist und die Summe von m, n, p, q und r 1, 2, 3, 4, 5 oder 6 ist; und vorausgesetzt, dass wenn p 0 und r 1 ist, die Summe von m, q und n 1, 2, 3, 4 oder 5 ist;

$R^4$ ist 1-5 Substituenten, unabhängig voneinander ausgewählt aus der Gruppe, bestehend aus Niederalkyl, $-OR^6$, $-O(CO)R^6$, $-O(CO)OR^9$, $-(O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(Niederalkylen)$COOR^6$, $-CH=CH-COOR^6$, $-CF_3$, $-CN$, $-NO_2$ und Halogen;

$R^5$ ist 1-5 Substituenten, unabhängig voneinander ausgewählt aus der Gruppe, bestehend aus $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}-CONR^6R^7$, -(Niederalkylen)$COOR^6$ und $-CH=CH-COOR^6$;
$R^6$, $R^7$ und $R^8$ sind unabhängig voneinander ausgewählt aus der Gruppe, bestehend aus Wasserstoff,Niederalkyl, Aryl und Aryl-substituiertes Niederalkyl und
$R^9$ ist Niederalkyl, Aryl oder Aryl-substituiertes Niederalkyl

für die Herstellung eines Medikaments zur Behandlung, Verhinderung oder Verbesserung von Symptomen der Alzheimer-Krankheit in einem Individuum.

**2.** Verwendung gemäß Anspruch 1, worin das Individuum keine Symptome der Alzheimer-Krankheit zeigt.

**3.** Verwendung gemäß Anspruch 1, worin das Individuum Alzheimer-Krankheit hat.

**4.** Verwendung gemäß Anspruch 1, worin das Individuum eine familiäre Geschichte von Alzheimer-Krankheit oder Demenzerkrankung hat.

**5.** Verwendung gemäß Anspruch 1, worin das Individuum ein Mensch ist und Trisomy 21 (Down-Syndrom) hat.

**6.** Verwendung gemäß Anspruch 1, worin das Individuum ein Mensch ist.

**7.** Verwendung gemäß Anspruch 6, worin der Mensch eine oder mehrere Mutationen in den Genen trägt, die β-Amyloid-Vorläuferprotein, Presenilin-1 oder Presenilin-2 codieren.

**8.** Verwendung gemäß Anspruch 6, worin der Mensch das Apolipoprotein-E4-Gen trägt.

**9.** Verwendung gemäß Anspruch 1, worin das Individuum einen erhöhten Spiegel von Amyloid β Peptid im Blutstrom und/oder dem Hirn hat.

**10.** Verwendung gemäß Anspruch 9, worin das Individuum einen erhöhten Spiegel von Amyloid β 42 Peptid im Blutstrom und/oder dem Hirn hat.

**11.** Verwendung gemäß Anspruch 9, worin der Spiegel von Amyloid β (Aβ) Peptiden im Blutstrom zwischen 10 und 100 % verringert ist von dem Spiegel von Amyloid β (Aβ) Peptiden vor Verabreichung der Zusammensetzung.

**12.** Verwendung gemäß Anspruch 9, worin das Individuum einen Spiegel von Amyloid Aβ-42 Peptid größer als 30 pM im Blutstrom hat.

**13.** Verwendung gemäß Anspruch 12, worin das Individuum einen Spiegel von Amyloid Aβ-42 Peptid von größer als 40 pM im Blutstrom hat.

**14.** Verwendung gemäß Anspruch 12, worin das Individuum einen Spiegel von Amyloid Aβ-42 Peptid im Bereich von 30 pM bis etwa 80 pM im Blutstrom hat.

**15.** Verwendung gemäß Anspruch 9, worin das Individuum einen Spiegel vom Amyloid Aβ-40 Peptid von größer als 200 pM im Blutstrom hat.

**16.** Verwendung gemäß Anspruch 15, worin das Individuum einen Spiegel vom Amyloid Aβ-40 Peptid von größer als 400 pM im Blutstrom hat.

**17.** Verwendung gemäß Anspruch 15, worin das Individuum einen Spiegel vom Amyloid Aβ-40 Peptid im Bereich von 200 pM bis 800 pM im Blutstrom hat.

**18.** Verwendung gemäß Anspruch 9, worin das Individuum einen Spiegel vom Amyloid Aβ-42 Peptid von mehr als 50 pmol/g nassem Hirngewebe hat.

**19.** Verwendung gemäß Anspruch 9, worin das Individuum einen Spiegel vom Amyloid Aβ-40 Peptid von größer als 10

pmol/g nassem Hirngewebe hat.

**20.** Verwendung gemäß Anspruch 1, worin das Individuum einen erhöhten Blut-Cholesterinspiegel hat.

**21.** Verwendung gemäß Anspruch 1, worin der gesamte Serum-Cholesterinspiegel des Individuums zumindest 200 mg/dl ist.

**22.** Verwendung gemäß Anspruch 1, worin der Gesamt-LDL-Cholesterinspiegel des Individuums größer als 100 mg/dl ist.

**23.** Verwendung gemäß Anspruch 6, worin der Mensch älter als etwa 40 Jahre ist.

**24.** Verwendung gemäß Anspruch 7, worin der Mensch älter als etwa 60 Jahre ist.

**25.** Verwendung gemäß Anspruch 1, worin die Verbindung dargestellt wird durch die nachstehende Formel (II):

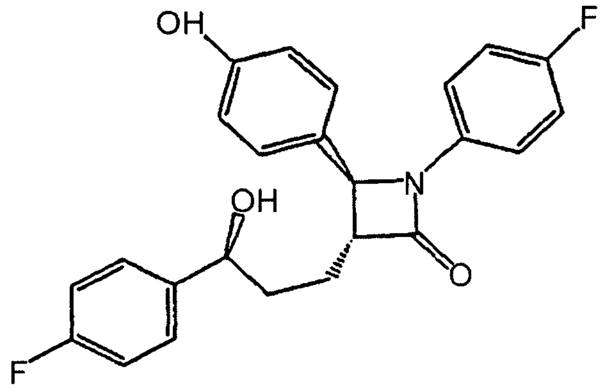

(II).

**26.** Verwendung gemäß Anspruch 1, worin die Verbindung dargestellt wird durch die nachstehende Formel (VIa):

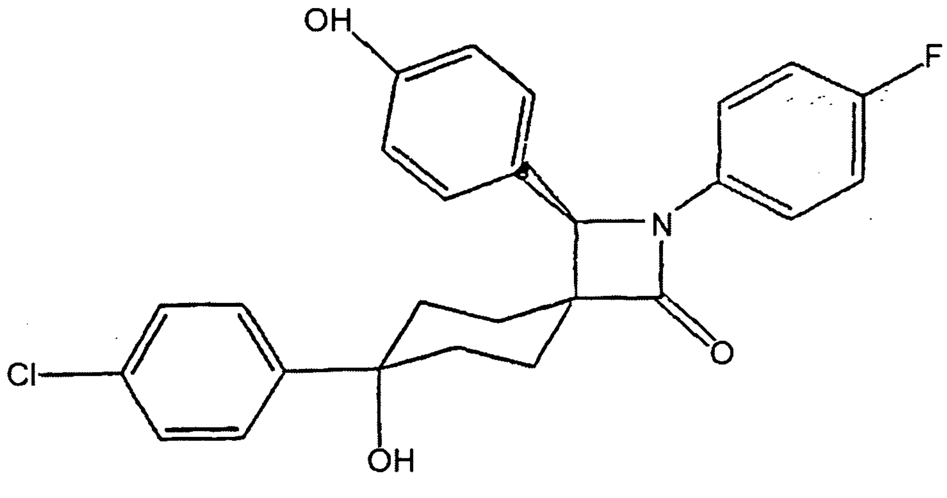

(VIa).

**27.** Verwendung gemäß Anspruch 1, worin die Verbindung dem Individuum in einer Menge zwischen 0,1 und 1.000 mg Verbindung pro Tag verabreicht wird.

**28.** Verwendung gemäß Anspruch 1, worin die Zusammensetzung des Weiteren zumindeste einen Cholesterin-Biosynthese-Inhibitor umfasst.

**29.** Verwendung gemäß Anspruch 28, worin der zumindest eine Cholesterin-Biosynthese-Inhibitor zumindest einen

HMG CoA-Reduktase-Inhibitor umfasst.

**30.** Verwendung gemäß Anspruch 29, worin der zumindest eine HMG CoA-Reduktase-Inhibitor ausgewählt ist aus Lovastatin, Pravastatin, Fluvastatin, Simvastatin, Atorvastatin, Cerivastatin, Rosuvastatin oder Mischungen davon.

**31.** Verwendung gemäß Anspruch 30, worin der zumindest eine HMG CoA-Reduktase-Inhibitor Simvastatin ist.

**32.** Verwendung gemäß Anspruch 1, worin die Zusammensetzung des Weiteren zumindest ein Fibrat (fibric acid)-Derivat umfasst.

**33.** Verwendung gemäß Anspruch 1, worin die Zusammensetzung des Weiteren zumindest ein Gallensäure-Sequestriermittel umfasst.

**34.** Verwendung gemäß Anspruch 1, worin die Zusammensetzung des Weiteren Nicotinsäure oder ein Derivat davon umfasst.

**35.** Verwendung gemäß Anspruch 1, worin die Zusammensetzung des Weiteren zumindest einen Acyl-CoA:Cholesterin-O-Acyltransferase-Inhibitor umfasst.

**36.** Verwendung gemäß Anspruch 1, worin die Zusammensetzung des Weiteren Probucol oder ein Derivat davon umfasst.

**37.** Verwendung gemäß Anspruch 1, worin die Zusammensetzung des Weiteren zumindest einen Aktivator von dem Rezeptor für niedrigdichtes Lipoprotein (low-density lipoprotein) umfasst.

**38.** Verwendung gemäß Anspruch 1, worin die Zusammensetzung des Weiteren zumindest eine Omega 3-Fettsäure umfasst.

**39.** Verwendung gemäß Anspruch 1, worin die Zusammensetzung des Weiteren zumindest eine natürliche wasserlösliche Faser umfasst.

**40.** Verwendung gemäß Anspruch 1, worin die Zusammensetzung des Weiteren zumindest eine Verbindung, ausgewählt aus Pflanzensterolen, Pflanzenstanolen oder Fettsäureestern von Pflanzenstanolen umfasst.

**41.** Verwendung gemäß Anspruch 1, worin die Zusammensetzung weiter zumindest ein Antioxidans oder Vitamin umfasst.

**42.** Verwendung gemäß Anspruch 1, worin die Zusammensetzung des Weiteren zumindest einen Alheimer-Wirkstoff, der sich von der Verbindung I oben unterscheidet, umfasst.

**43.** Verwendung gemäß Anspruch 1, worin die Zusammensetzung des Weiteren zumindest einen Alheimer-Wirkstoff, der sich von der obigen Verbindung I unterscheidet, umfasst, ausgewählt aus der Gruppe, bestehend aus Cholinesteraseinhibitoren, muscarinische Rezeptor-Antagonisten, M2-muscarinischer Rezeptor-Antagonisten, Acetylcholin-Freisetzungsstimulatoren, Cholin-Aufnahmestimulatoren, Antagonisten des nicotinischen cholinergen Rezeptors, anti-Aβ-Impfungen, γ-Sekretaseinhibitoren, β-Sekretaseinhibitoren, Amyloidaggregationsinbibitoren, Amyloid-Vorläuferprotein-Antisens-Oligonukleotide, Monoamin-Wiederaufnahme-Inhibitoren, menschliche Stammzellen, Gentherapie, nootrope Mittel, AMPA-Rezeptorliganden, Wachstumsfaktoren oder Wachstumsfaktor Rezeptoragonisten, anti-entzündliche Mittel, freie Radikalfänger/Antioxidantien, Superoxid-Dismutase-Stimulatoren, Calciumkanalblocker, Apoptoseinhibitoren, Caspaseinhibitoren, Monoaminoxidaseinhibitoren, Östrogene, NMDA-Rezeptor-Antagonisten, Jun N-terminale Kinase (JNK)-Inhibotoren, Kupfer/Zink-Chelatoren, 5-HT1a-Rezeptor-Agonisten, NGF-Stimulatoren, neuroprotektive Mittel, H3-Histaminrezeptor-Antagonisten, Calpaininhibitoren, Poly-ADP-Ribose-Polymerase-Inhibitoren, Prolylendopeptidaseinhibitoren, Calciummodulatoren, Corticotropin-Freisetzungsfaktor-Antagonisten, Corticotropin-Freisetzungsfaktor-Bindungsproteininhibitor, GABA-Modulatoren, GABA-A-Rezeptor-Antagonisten, GABA-B-Rezeptor-Antagonisten, Neuroimmunophilinliganden, Sigma-Rezeptorliganden, Galanin-Rezeptorlinganden, Imidazolin/α-adrenergische Rezeptor-Antagonisten, vasoaktive intestinale Peptidrezeptor-Agonisten, Benzodiazepin inverse Agonisten, Cannabinoidrezeptor-Agonisten, Thyrotropin-Freisetzungshormonrezeptor-Agonisten, Proteinkinase C-Inhibitoren, 5-HT3-Antagonisten, Prostaglandinrezeptor-Antagonisten, Topoisomerase II-Inhibitoren, Steroidrezeptor-Agonisten, Corticosteroidrezeptor-Antagonisten, Stickstoffmonoxidmodulatoren, RAGE-Inhibitoren, Dopaminrezeptor-Antagonisten und Kombinationen davon.

**44.** Verwendung einer Zusammensetzung, umfassend eine Verbindung, dargestellt durch die untenstehende Formel (II) :

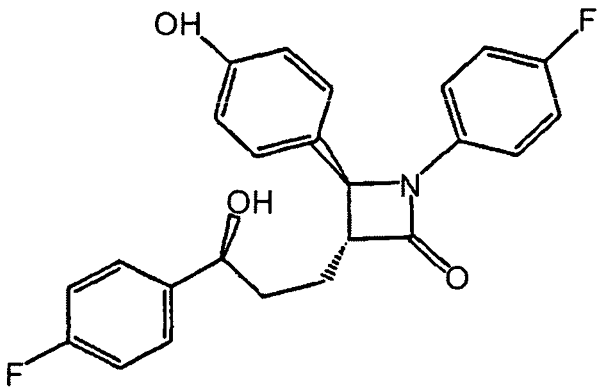

(II)

für die Herstellung eines Medikaments zum Verhindern, Behandeln oder Verbessern von Symptomen der Alzheimer-Krankheit in einem Individuum.

**45.** Verwendung einer Zusammensetzung, umfassend zumindest eine Verbindung, dargestellt durch die Formel (I):

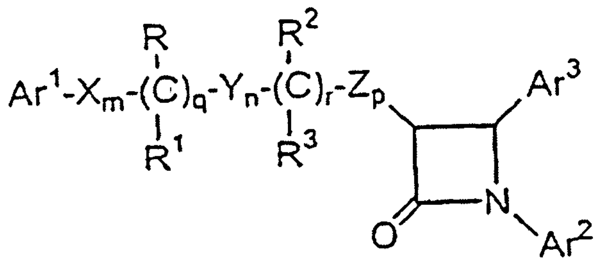

(I)

oder ein pharmazeutisch annehmbares Salz oder Solvat davon, worin

$Ar^1$ und $Ar^2$ unabhängig voneinander ausgewählt sind aus der Gruppe, bestehend aus Aryl und $R^4$-substituiertem Aryl;
$Ar^3$ Aryl oder $R^5$-substituiertes Aryl ist;
X, Y und Z unabhängig voneinander ausgewählt sind aus der Gruppe, bestehend aus $-CH_2-$, -CH(Niederalkyl)- und -C(Diniederalkyl)-;
R und $R^2$ unabhängig voneinander ausgewählt sind aus der Gruppe, bestehend aus $-OR^6$, $-O(CO)R^6$, $-O(CO)OR^9$ und $-O(CO)NR^6R^7$;
$R^1$ und $R^3$ unabhängig voneinander ausgewählt sind aus der Gruppe, bestehend aus Wasserstoff, Niederalkyl und Aryl;
q ist 0 oder 1;
r ist 0 oder 1;
m, n und p sind unabhängig voneinander ausgewählt aus 0, 1, 2, 3 oder 4; vorausgesetzt, dass zumindest eines von q und r 1 ist und die Summe von m, n, p, q und r 1, 2, 3, 4, 5 oder 6 ist; und vorausgesetzt, dass wenn p 0 und r 1 ist, die Summe von m, q und n 1, 2, 3, 4 oder 5 ist;
$R^4$ ist 1-5 Substituenten, unabhängig voneinander ausgewählt aus der Gruppe, bestehend aus Niederalkyl, $-OR^6$, $-O(CO)R^6$, $-O(CO)OR^9$, $-(O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$,- (Niederalkylen)$COOR^6$, $-CH=CH-COOR^6$, $-CF_3$, -CN, $-NO_2$ und Halogen;
$R^5$ ist 1-5 Substituenten, unabhängig voneinander ausgewählt aus der Gruppe, bestehend aus $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COO_R{}^6$, $-CONR^6R^7$, $-COR^6$, $-SO_2NR^6R^7$, $S(O)_{0-2}R^9$, $-O(CH_2)_{1-10}-COOR^6$, -O

$(CH_2)_{1-10}$-$CONR^6R^7$, -(Niederalkylen)$COOR^6$ und -CH=CH-$COOR^6$;
$R^6$, $R^7$ und $R^8$ sind unabhängig voneinander ausgewählt aus der Gruppe, bestehend aus Wasserstoff,Niederalkyl, Aryl und Aryl-substituiertes Niederalkyl und
$R^9$ ist Niederalkyl, Aryl oder Aryl-substituiertes Niederalkyl

für die Herstellung eines Medikaments zum Regulieren der Herstellung oder des Spiegels von zumindest einem Amyloid-β-Peptid in dem Individuum.

**46.** Verwendung einer Zusammensetzung, umfassend zumindest eine Verbindung, dargestellt durch die Formel (I):

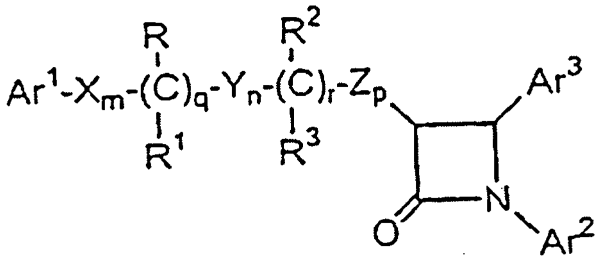

(I)

oder ein pharmazeutisch annehmbares Salz oder Solvat davon, worin

$Ar^1$ und $Ar^2$ unabhängig voneinander ausgewählt sind aus der Gruppe, bestehend aus Aryl und $R^4$-substituiertem Aryl;
$Ar^3$ Aryl oder $R^5$-substituiertes Aryl ist;
X, Y und Z unabhängig voneinander ausgewählt sind aus der Gruppe, bestehend aus -$CH_2$-, -CH(Niederalkyl)- und -C(Diniederalkyl)-;
R und $R^2$ unabhängig voneinander ausgewählt sind aus der Gruppe, bestehend aus -$OR^6$, -O(CO)$R^6$, -O(CO)$OR^9$ und -O(CO)$NR^6R^7$;
$R^1$ und $R^3$ unabhängig voneinander ausgewählt sind aus der Gruppe, bestehend aus Wasserstoff, Niederalkyl und Aryl; q ist 0 oder 1;
r ist 0 oder 1;
m, n und p sind unabhängig voneinander ausgewählt aus 0, 1, 2, 3 oder 4; vorausgesetzt, dass zumindest eines von q und r 1 ist und die Summe von m, n, p, q und r 1, 2, 3, 4, 5 oder 6 ist; und vorausgesetzt, dass wenn p 0 und r 1 ist, die Summe von m, q und n 1, 2, 3, 4 oder 5 ist;
$R^4$ ist 1-5 Substituenten, unabhängig voneinander ausgewählt aus der Gruppe, bestehend aus Niederalkyl, -$OR^6$, -O(CO)$R^6$, -O(CO)$OR^9$, -O$(CH_2)_{1-5}$$OR^6$, -O(CO)$NR^6R^7$, -$NR^6R^7$, -$NR^6$(CO)$R^7$, -$NR^6$(CO)$OR^9$, -$NR^6$(CO)$NR^7R^8$, -$NR^6SO_2R^9$, -$COOR^6$, -$CONR^6R^7$, -$COR^6$, -$SO_2NR^6R^7$, $S(O)_{0-2}R^9$, -O$(CH_2)_{1-10}$-$COOR^6$, -O$(CH_2)_{1-10}$$CONR^6R^7$, -(Niederalkylen)$COOR^6$, -CH=CH-$COOR^6$, -$CF_3$, -CN, -$NO_2$ und Halogen;
$R^5$ ist 1-5 Substituenten, unabhängig voneinander ausgewählt aus der Gruppe, bestehend aus -$OR^6$, -O(CO)$R^6$, -O(CO)$OR^9$, -O$(CH_2)_{1-5}$$OR^6$, -O(CO)$NR^6R^7$, -$NR^6R^7$, -$NR^6$(CO)$R^7$, -$NR^6$(CO)$OR^9$, -$NR^6$(CO)$NR^7R^8$, -$NR^6SO_2R^9$, -$COOR^6$, -$CONR^6R^7$, -$COR^6$, -$SO_2NR^6R^7$, $S(O)_{0-2}R^9$, -O$(CH_2)_{1-10}$-$COOR^6$, -O$(CH_2)_{1-10}$-$CONR^6R^7$, -(Niederalkylen)$COOR^6$ und -CH=CH-$COOR^6$;
$R^6$, $R^7$ und $R^8$ sind unabhängig voneinander ausgewählt aus der Gruppe, bestehend aus Wasserstoff,Niederalkyl, Aryl und Aryl-substituiertes Niederalkyl und
$R^9$ ist Niederalkyl, Aryl oder Aryl-substituiertes Niederalkyl

für die Herstellung eines Medikaments zur Regulation der Menge von ApoE-Isoform 4 im Blutstrom und/oder Hirn des Individuums.

**47.** Verwendung einer Zusammensetzung, umfassend zumindest eine Verbindung, dargestellt durch die Formel (III):

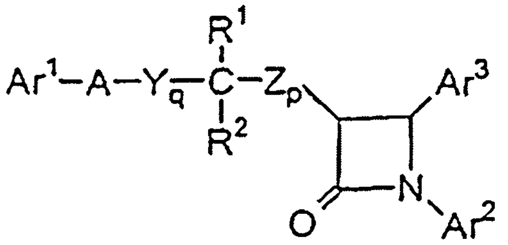

(III)

oder ein pharmazeutisch annehmbares Salz oder Solvat davon, wobei in der obenstehenden Formel (III)

$Ar^1$ $R^3$-substituiertes Aryl ist;
$Ar^2$ $R^4$-substituiertes Aryl ist;
$Ar^3$ $R^5$-substituiertes Aryl ist;
Y und Z unabhängig voneinander ausgewählt sind aus der Gruppe, bestehend aus $-CH_2-$, -CH(Niederalkyl)- und -C(Diniederalkyl)-;
A ausgewählt ist aus -O-, -S-, -S(O)- oder $-S(O)_2-$;
$R^1$ ausgewählt ist aus der Gruppe, bestehend aus $-OR^6$, $-O(CO)R^6$, $-O(CO)OR^9$ und $-O(CO)NR^6R^7$;
$R^2$ ausgewählt ist aus der Gruppe, bestehend aus Wasserstoff, Niederalkyl und Aryl; oder
$R^1$ und $R^2$ zusammen = O sind;
q ist 1, 2 oder 3;
p ist 0, 1, 2, 3 oder 4;
$R^5$ 1-3 Substituenten, unabhängig voneinander ausgewählt aus der Gruppe, bestehend aus $-OR^6$, $-O(CO)R^6$, $-O(CO)OR^9$, $-O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-Niederalkyl, $-NR^6SO_2$-Aryl, $-CONR^6R^7$, $-COR^6$, $-SO_2NR^6R^7$, $S(O)_{0-2}$-Alkyl, $S(O)_{0-2}$-Aryl, $-O(CH_2)_{1-10}-COOR^6$, $-O(CH_2)_{1-10}CONR^6R^7$, o-Halogeno, m-Halogeno, o-Niederalkyl, m-Niederalkyl, -(Niederalkylen)-COOR6 und $-CH=CH-COOR^6$;
$R^3$ und $R^4$ unabhängig voneinander 1-3 Substituenten unabhängig voneinander ausgewählt aus der Gruppe, bestehend aus $R^5$, Wasserstoff, p-Niederalkyl, Aryl, $-NO_2$, $-CF_3$ und p-Halogeno;
$R^6$, $R^7$ und $R^8$ unabhängig voneinander ausgewählt sind aus der Gruppe, bestehend aus Wasserstoff, Niederalkyl, Aryl und Aryl-substituiertes Niederalkyl; und
$R^9$ Niederalkyl, Aryl oder Aryl-substituiertes Niederalkyl ist,

für die Herstellung eines Medikaments zur Verhinderung, Behandlung und Verbesserung der Symptome der Alzheimer-Krankheit in einem Individuum.

**48.** Verwendung einer Zusammensetzung, umfassend zumindest eine Verbindung, dargestellt durch Formel (III):

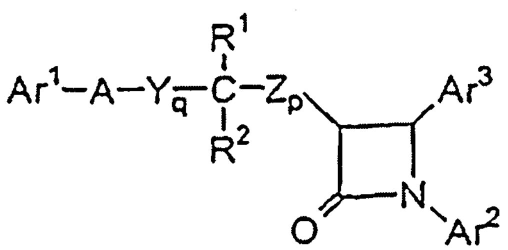

(III)

oder ein pharmazeutisch annehmbares Salz oder Solvat davon, wobei in der obenstehenden Formel (III)

$Ar^1$ $R^3$-substituiertes Aryl ist;
$Ar^2$ $R^4$-substituiertes Aryl ist;
$Ar^3$ $R^5$-substituiertes Aryl ist;
Y und Z unabhängig voneinander ausgewählt sind aus der Gruppe, bestehend aus $-CH_2-$, -CH(Niederalkyl)-

und -C(Diniederalkyl)-;
A ausgewählt ist aus -O-, -S-, -S(O)- oder $-S(O)_2-$;
$R^1$ ausgewählt ist aus der Gruppe, bestehend aus $-OR^6$, $-O(CO)R^6$, $-O(CO)OR^9$ und $-O(CO)NR^6R^7$;
$R^2$ ausgewählt ist aus der Gruppe, bestehend aus Wasserstoff, Niederalkyl und Aryl; oder
$R^1$ und $R^2$ zusammen = O sind;
q ist 1, 2 oder 3;
p ist 0, 1, 2, 3 oder 4;
$R^5$ 1-3 Substituenten, unabhängig voneinander ausgewählt aus der Gruppe, bestehend aus $-OR^6$, $-O(CO)R^6$, $-O(CO)OR^9$, $-O(CH_2)_{1-5}OR^9$, $-O(CO)NR^6R^7$, $-NR^6R^7$, $-NR^6(CO)R^7$,- $NR^6(CO)OR^9$, $-NR^6(CO)NR^7R^8$, $-NR^6SO_2$-Niederalkyl, $-NR^6SO_2$-Aryl, $-CONR^6R^7$, $-COR^6$, $-SO_2NR^6R^7$, $S(O)_{0-2}$-Alkyl, $S(O)_{0-2}$-Aryl, -O $(CH_2)_{1-10}-COOR^6$, $-O(CH_2)_{1-10}CONR^6R^7$, o-Halogeno, m-Halogeno, O-Niederalkyl, m-Niederalkyl, -(Niederalkylen)-COOR6 und $-CH=CH-COOR^6$;
$R^3$ und $R^4$ unabhängig voneinander 1-3 Substituenten unabhängig voneinander ausgewählt aus der Gruppe, bestehend aus $R^5$, Wasserstoff, p-Niederalkyl, Aryl, $-NO_2$, $-CF_3$ und p-Halogeno;
$R^6$, $R^7$ und $R^8$ unabhängig voneinander ausgewählt sind aus der Gruppe, bestehend aus Wasserstoff, Niederalkyl, Aryl und Aryl-substituiertes Niederalkyl; und
$R^9$ Niederalkyl, Aryl oder Aryl-substituiertes Niederalkyl ist,

zur Herstellung eines Medikaments zur Regulation der Produktion oder des Spiegels von zumindest einem Amyloid-β-Peptid in dem Individuum.

**49.** Verwendung einer Zusammensetzung, umfassend zumindest eine Verbindung, dargestellt durch Formel (III):

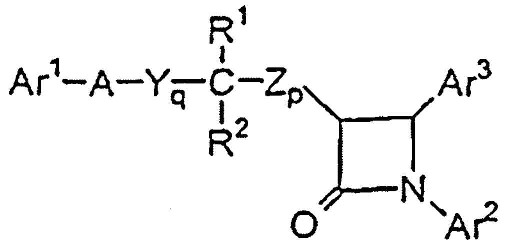
(III)

oder ein pharmazeutisch annehmbares Salz oder Solvat davon, wobei in der obenstehenden Formel (III)

$Ar^1$ $R^3$-substituiertes Aryl ist;
$Ar^2$ $R^4$-substituiertes Aryl ist;
$Ar^3$ $R^5$-substituiertes Aryl ist;
Y und Z unabhängig voneinander ausgewählt sind aus der Gruppe, bestehend aus $-CH_2-$, -CH(Niederalkyl)- und -C(Diniederalkyl)-;
A ausgewählt ist aus -O-, -S-, -S(O)- oder $-S(O)_2-$;
$R^1$ ausgewählt ist aus der Gruppe, bestehend aus $-OR^6$, $-O(CO)R^6$, $-0(CO)OR^9$ und $-O(CO)NR^6R^7$;
$R^2$ ausgewählt ist aus der Gruppe, bestehend aus Wasserstoff, Niederalkyl und Aryl; oder
$R^1$ und $R^2$ zusammen = O sind;
q ist 1, 2 oder 3;
p ist 0, 1, 2, 3 oder 4;
$R^5$ 1-3 Substituenten, unabhängig voneinander ausgewählt aus der Gruppe, bestehend aus $-OR^6$, $-O(CO)R^6$, $-O(CO)OR^9$, $-O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-Niederalkyl, $-NR^6SO_2$-Aryl, $-CONR^6R^7$, $-COR^6$, $-SO_2NR^6R^7$, $S(O)_{0-2}$-Alkyl, $S(O)_{0-2}$-Aryl, -O $(CH_2)_{1-10}-COOR^6$, $-O(CH_2)_{1-10}CONR^6R^7$, o-Halogeno, m-Halogeno, o-Niederalkyl, m-Niederalkyl, -(Niederalkylen)-$COOR^6$ und $-CH=CH-COOR^6$;
$R^3$ und $R^4$ unabhängig voneinander 1-3 Substituenten unabhängig voneinander, ausgewählt aus der Gruppe, bestehend aus $R^5$, Wasserstoff, p-Niederalkyl, Aryl, $-NO_2$, $-CF_3$ und p-Halogeno;
$R^6$, $R^7$ und $R^8$ unabhängig voneinander ausgewählt sind aus der Gruppe, bestehend aus Wasserstoff, Niederalkyl, Aryl und Aryl-substituiertes Niederalkyl; und
$R^9$ Niederalkyl, Aryl oder Aryl-substituiertes Niederalkyl ist,

zur Herstellung eines Medikaments zur Regulation der Menge von ApoE-Isoform 4 im Blutstrom und/oder Hirn des Individuums.

**50.** Verwendung einer Zusammensetzung, umfassend zumindest eine Verbindung, dargestellt durch Formel (IV):

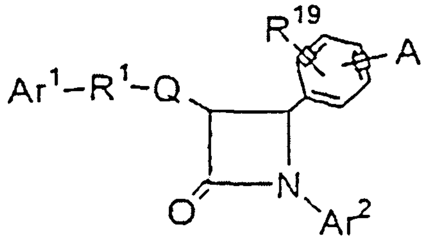

(IV)

oder ein pharmazeutisch annehmbares Salz oder Solvat davon, worin in der obenstehenden Formel (IV):

A ausgewählt ist aus der Gruppe, bestehend aus $R^2$-substituiertem Heterocycloalkyl, $R^2$-substituiertem Heteroaryl, $R^2$-substituiertem benzofusioniertem Heterocycloalkyl und $R^2$-substituiertem benzofusioniertem Heteroaryl;
$Ar^1$ Aryl oder $R^3$-substituiertes Aryl ist;
$Ar^2$ Aryl oder $R^4$-substituiertes Aryl ist;
Q eine Bindung ist oder mit dem Ring-Kohlenstoffatom an Position 3 von Azetidinon die Spirogruppe

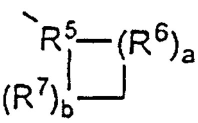

bildet; und
$R^1$ ausgewählt ist aus der Gruppe, bestehend aus:

- $(CH_2)_q$-, worin q 2-6 ist, vorausgesetzt, dass wenn Q einen Spiroring bildet, q auch 0 oder 1 sein kann;
- $(CH_2)_e$-G-$(CH_2)_r$-, worin G -O-, -C(O)-, Phenylen, -$NR^8$- oder -$S(O)_{0-2}$- ist, e 0-5 ist und r 0-5 ist, vorausgesetzt, dass die Summe von e und r 1-6 ist;
- ($C_2$-$C_6$-Alkenylen)-; und
- $(CH_2)_f$-V-$(CH_2)_g$-, worin V $C_3$-$C_6$-Cycloalkylen ist, f 1-5 und g 0-5 ist, vorausgesetzt, dass die Summe von f und g 1-6 ist;

$R^5$ ausgewählt ist aus:

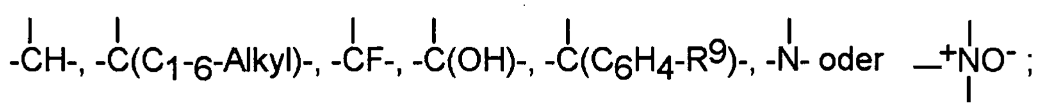

$R^6$ und $R^7$ unabhängig voneinander ausgewählt sind aus der Gruppe, bestehend aus -$CH_2$-, -CH($C_1$-$C_6$-Alkyl)-, -C(Di-($C_1$-$C_6$)-alkyl); -CH=CH- und -C($C_1$-$C_6$-Alkyl)=CH- oder $R^5$ zusammen mit einem benachbarten $R^6$ oder $R^5$ gemeinsam mit einem benachbarten $R^7$ eine -CH=CH- oder -CH=C($C_1$-$C_6$-Alkyl)-Gruppe bilden;
a und b unabhängig voneinander 0, 1, 2 oder 3 sind, vorausgesetzt, dass nicht beide null sind;
vorausgesetzt, dass wenn $R^6$ -CH=CH- oder -C($C_1$-$C_6$-Alkyl)=CH- ist, a 1 ist; vorausgesetzt, dass wenn $R^7$ -CH=CH- oder -C($C_1$-$C_6$-Alkyl)=CH- ist, b 1 ist;
vorausgesetzt, dass wenn a 2 oder 3 ist, die $R^6$-Reste gleich oder verschieden sein können und vorausgesetzt, dass wenn b 2 oder 3 ist, die $R^7$-Reste gleich oder verschieden sein können;
und wenn Q eine Bindung ist, kann $R^1$ auch ausgewählt werden aus:

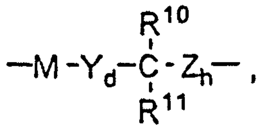, 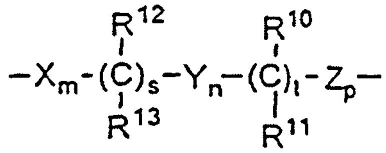 oder 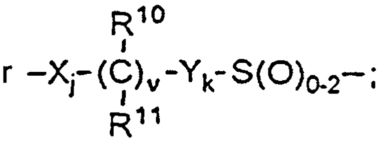;

worin M -O-, -S-, -S(O)- oder $-S(O)_2-$ ist;
X, Y und Z unabhängig voneinander ausgewählt sind aus der Gruppe, bestehend aus $-CH_2-$, $-CH(C_1-C_6\text{-Alkyl})-$ und $-C(Di-(C_1-C_6)alkyl)$;
$R^{10}$ und $R^{12}$ unabhängig voneinander ausgewählt sind aus der Gruppe, bestehend aus $-OR^{14}$, $-O(CO)R^{14}$, -O (CO)$OR^{16}$ und $-O(CO)NR^{14}R^{15}$;
$R^{11}$ und $R^{13}$ unabhängig voneinader ausgewählt sind aus der Gruppe, bestehend aus Wasserstoff, $(C_1-C_6)$-Alkyl und Aryl; oder $R^{10}$ und $R^{11}$ zusammen =O sind, oder $R^{12}$ und $R^{13}$ zusammen =0 sind;
d ist 1, 2 oder 3;
h ist 0, 1, 2, 3 oder 4;
s ist 0 oder 1; t ist 0 oder 1; m, n und p unabhängig voneinader 0-4 sind; vorausgesetzt, dass zumindest einer von s und t 1 ist, und die Summe von m, n, p, s und t 1-6 ist, vorausgesetzt, dass wenn p 0 und t 1 ist, die Summe von m, s und n 1-5 ist; und vorausgesetzt, dass wenn p 0 ist und s 1 ist, die Summe von m, t und n 1-5 ist;
v ist 0 oder 1;
j und k sind unabhängig voneinander 1-5, vorausgesetzt, dass die Summe von j, k und v 1-5 ist;
$R^2$ 1-3 Substituenten auf den Ring-Kohlenstoffatomen ist, ausgewählt aus der Gruppe, bestehend aus Wasserstoff, $(C_1-C_{10})$-Alkyl, $(C_2-C_{10})$-Alkenyl, $(C_2-C_{10})$-Alkinyl, $(C_3-C_6)$-Cycloalkyl, $(C_3-C_6)$-Cycloalkenyl, $R^{17}$-substituiertes Aryl, $R^{17}$-substituiertes Benzyl, $R^{17}$-substituiertes Benzyloxy, $R^{17}$-substituiertes Aryloxy, Halogeno, $-NR^{14}R^{15}$, $NR^{14}R^{15}-(C_1-C_6\text{-Alkylen})-$, $NR^{14}R^{15}C(O)(C_1-C_6)\text{-Alkylen})-$, $-NHC(O)R^{16}$, OH, $C_1-C_6$-Alkoxy, -OC(O) $R^{16}$, $-COR^{14}$, Hydroxy$(C_1-C_6)$alkyl, $(C_1-C_6)$-Alkoxy$(C_1-C_6)$alkyl, $NO_2$, $-S(O)_{0-2}R^{16}$, $-SO_2NR^{14}R^{15}$ und $-(C_1-C_6\text{-Alkylen})COOR^{14}$; wenn $R^2$ ein Substituent auf einem Heterocycloalkylring ist, ist $R^2$ wie definiert oder ist =O

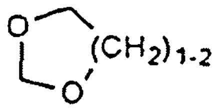 ;

und wenn $R^2$ ein Substituent auf einem substituierbaren Ring-Stickstoff ist, ist er Wasserstoff, $(C_1-C_6)$Alkyl, Aryl, $(C_1-C_6)$Alkoxy, Aryloxy, $(C_1-C_6)$Alkylcarbonyl, Arylcarbonyl, Hydroxy, $-(CH_2)_{1-6}CONR^{18}R^{18}$,

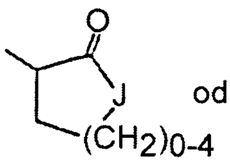 oder 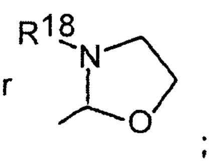 ;

worin J -O-, -NH-, $-NR^{18}-$ oder $-CH_2-$ ist;
$R^3$ und $R^4$ unabhängig voneinander ausgewählt sind aus der Gruppe, bestehend aus 1-3 Substituenten, unabhängig ausgewählt aus der Gruppe, bestehend aus $(C_1-C_6)$Alkyl, $-OR^{14}$, $-O(CO)R^{14}$, $-O(CO)OR^{16}$, $-O(CH_2)_{1-5}OR^{14}$, $-O(CO)NR^{14}R^{15}$, $-NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $S(O)_{0-2}R^{16}$, $-O(CH_2)_{1-10}-COOR^{14}$, $-O(CH_2)_{1-10}CONR^{14}R^{15}$, $-(C_1-C_6)$Alkylen$)-COOR^{14}$, $-CH=CH-COOR^{14}$, $-CF_3$, -CN, $-NO_2$ und Halogen;
$R^8$ ist Wasserstoff, $(C_1-C_6)$Alkyl, Aryl$(C_1-C_6)$alkyl, $-C(O)R^{14}$ oder $-COOR^{14}$;
$R^9$ und $R^{17}$ sind unabhängig voneinander 1-3 Gruppen, unabhängig voneinander ausgewählt aus der Gruppe, bestehend aus Wasserstoff, $(C_1-C_6)$Alkyl, $(C_1-C_6)$Alkoxy, -COOH, $NO_2$, $-NR^{14}R^{15}$, OH und Halogeno;
$R^{14}$ und $R^{15}$ unabhängig voneinander ausgewählt sind aus der Gruppe, bestehend aus Wasserstoff, $(C_1-C_6)$

Alkyl, Aryl und Aryl-substituiertem $(C_1\text{-}C_6)$Alkyl;
$R^{16}$ $(C_1\text{-}C_6)$Alkyl, Aryl oder $R^{17}$-substituiertes Aryl;
$R^{18}$ ist H oder $(C_1\text{-}C_6)$Alkyl; und
$R^{19}$ ist H, -OH oder $(C_1\text{-}C_6)$Alkoxy,

für die Herstellung eines Medikaments für das Verhindern, Behandeln oder Verbessern der Symptome der Alzheimerschen Krankheit in einem Individuum.

**51.** Verwendung einer Zusammensetzung, umfassend zumindest eine Verbindung dargestellt durch die Formel (IV):

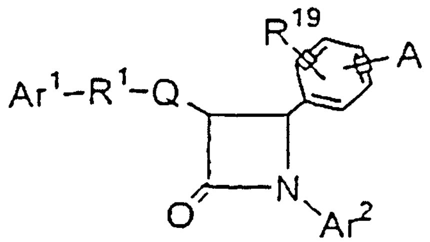

(IV)

oder ein pharmazeutisch annehmbares Salz oder Solvat davon, worin in der obenstehenden Formel (IV):

A ausgewählt ist aus der Gruppe, bestehend aus $R^2$-substituiertem Heterocycloalkyl, $R^2$-substituiertem Heteroaryl, $R^2$-substituiertem benzofusioniertem Heterocycloalkyl und $R^2$-substituiertem benzofusioniertem Heteroaryl;
$Ar^1$ Aryl oder $R^3$-substituiertes Aryl ist;
$Ar^2$ Aryl oder $R^4$-substituiertes Aryl ist;
Q eine Bindung ist oder mit dem Ring-Kohlenstoffatom an Position 3 von Azetidinon die Spirogruppe

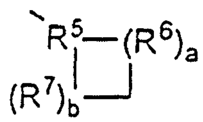

bildet; und
$R^1$ ausgewählt ist aus der Gruppe, bestehend aus:

- $-(CH_2)_q-$, worin q 2-6 ist, vorausgesetzt, dass wenn Q einen Spiroring bildet, q auch 0 oder 1 sein kann;
- $-(CH_2)_e-G-(CH_2)_r-$, worin G -O-, -C(O)-, Phenylen, $-NR^8-$ oder $-S(O)_{0\text{-}2}-$ ist, e 0-5 ist und r 0-5 ist, vorausgesetzt, dass die Summe von e und r 1-6 ist;
- $(C_2\text{-}C_6\text{-Alkenylen})$-; und
- $-(CH_2)_f-V-(CH_2)g-$, worin V $C_3\text{-}C_6$-Cycloalkylen ist, f 1-5 und g 0-5 ist, vorausgesetzt, dass die Summe von f und g 1-6 ist;

$R^5$ ausgewählt ist aus:

$$-CH-,\ -C(C_{1\text{-}6}\text{-Alkyl})-,\ -CF-,\ -C(OH)-,\ -C(C_6H_4\text{-}R^9)-,\ -N-\ \text{oder}\ -^{+}NO^{-};$$

$R^6$ und $R^7$ unabhängig voneinander ausgewählt sind aus der Gruppe, bestehend aus $-CH_2-$, $-CH(C_1\text{-}C_6\text{-Alkyl})-$, $-C(Di\text{-}(C_1\text{-}C_6)\text{-alkyl})$; -CH=CH- und $-C(C_1\text{-}C_6\text{-Alkyl})=CH-$ oder $R^5$ zusammen mit einem benachbarten $R^6$ oder $R^5$ gemeinsam mit einem benachbarten $R^7$ eine -CH=CH- oder $-CH=C(C_1\text{-}C_6\text{-Alkyl})$-Gruppe bilden;

a und b unabhängig voneinander 0, 1, 2 oder 3 sind, vorausgesetzt, dass nicht beide null sind;
vorausgesetzt, dass wenn $R^6$ -CH=CH- oder -C($C_1$-$C_6$-Alkyl)=CH- ist, a 1 ist; vorausgesetzt, dass wenn $R^7$ -CH=CH- oder -C($C_1$-$C_6$-Alkyl)=CH- ist, b 1 ist;
vorausgesetzt, dass wenn a 2 oder 3 ist, die $R^6$-Reste gleich oder verschieden sein können und vorausgesetzt, dass wenn b 2 oder 3 ist, die $R^7$-Reste gleich oder verschieden sein können;
und wenn Q eine Bindung ist, kann $R^1$ auch ausgewählt werden aus:

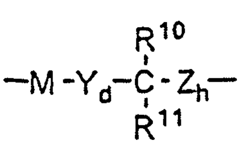 , 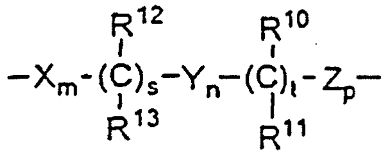 oder 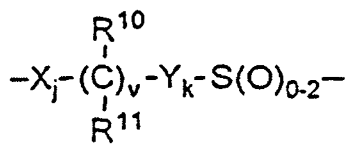 ;

worin M -O-, -S-, -S(O)- oder -$S(O)_2$- ist;
X, Y und Z unabhängig voneinander ausgewählt sind aus der Gruppe, bestehend aus -$CH_2$-, -CH($C_1$-$C_6$-Alkyl)- und -C(Di-($C_1$-$C_6$)alkyl);
$R^{10}$ und $R^{12}$ unabhängig voneinander ausgewählt sind aus der Gruppe, bestehend aus -$OR^{14}$, -O(CO)$R^{14}$, -O (CO)$OR^{16}$ und -O(CO)$NR^{14}R^{15}$;
$R^{11}$ und $R^{13}$ unabhängig voneinader ausgewählt sind aus der Gruppe, bestehend aus Wasserstoff, ($C_1$-$C_6$)-Alkyl und Aryl; oder $R^{10}$ und $R^{11}$ zusammen =O sind, oder $R^{12}$ und $R^{13}$ zusammen =O sind;
d ist 1, 2 oder 3;
h ist 0, 1, 2, 3 oder 4;
s ist 0 oder 1; t ist 0 oder 1; m, n und p unabhängig voneinader 0-4 sind; vorausgesetzt, dass zumindest einer von s und t 1 ist, und die Summe von m, n, p, s und t 1-6 ist, vorausgesetzt, dass wenn p 0 und t 1 ist, die Summe von m, s und n 1-5 ist; und vorausgesetzt, dass wenn p 0 ist und s 1 ist, die Summe von m, t und n 1-5 ist;
v ist 0 oder 1;
j und k sind unabhängig voneinander 1-5, vorausgesetzt, dass die Summe von j, k und v 1-5 ist;
$R^2$ 1-3 Substituenten auf den Ring-Kohlenstoffatomen ist, ausgewählt aus der Gruppe, bestehend aus Wasserstoff, ($C_1$-$C_{10}$)-Alkyl, ($C_2$-$C_{10}$)-Alkenyl, ($C_2$-$C_{10}$)-Alkinyl, ($C_3$-$C_6$)-Cycloalkyl, ($C_3$-$C_6$)-Cycloalkenyl, $R^{17}$-substituiertes Aryl, $R^{17}$-substituiertes Benzyl, $R^{17}$-substituiertes Benzyloxy, $R^{17}$-substituiertes Aryloxy, Halogeno, -$NR^{14}R^{15}$, $NR^{14}R^{15}$-($C_1$-$C_6$-Alkylen)-, $NR^{14}R^{15}$C(O)($C_1$-$C_6$)-Alkylen)-, -NHC(O)$R^{16}$, OH, $C_1$-$C_6$-Alkoxy, -OC(O) $R^{16}$, -$COR^{14}$, Hydroxy($C_1$-$C_6$)alkyl, ($C_1$-$C_6$)-Alkoxy($C_1$-$C_6$)alkyl, $NO_2$, -$S(O)_{0-2}R^{16}$,- $SO_2NR^{14}R^{15}$ und -($C_1$-$C_6$-Alkylen)$COOR^{14}$; wenn $R^2$ ein Substituent auf einem Heterocycloalkylring ist, ist $R^2$ wie definiert oder ist =O

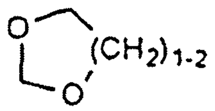 ;

und wenn $R^2$ ein Substituent auf einem substituierbaren Ring-Stickstoff ist, ist er Wasserstoff, ($C_1$-$C_6$)Alkyl, Aryl, ($C_1$-$C_6$)Alkoxy, Aryloxy, ($C_1$-$C_6$)Alkylcarbonyl, Arylcarbonyl, Hydroxy, -$(CH_2)_{1-6}CONR^{18}R^{18}$,

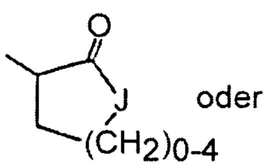 oder 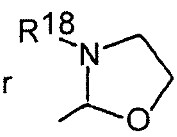 ;

worin J -O-, -NH-, -$NR^{18}$- oder -$CH_2$- ist;
$R^3$ und $R^4$ unabhängig voneinander ausgewählt sind aus der Gruppe, bestehend aus 1-3 Substituenten, unabhängig ausgewählt aus der Gruppe, bestehend aus ($C_1$-$C_6$)Alkyl, -$OR^{14}$, -O(CO)$R^{14}$, -O(CO)$OR^{16}$, -O $(CH_2)_{1-5}OR^{14}$, -O(CO)$NR^{14}R^{15}$, -$NR^{14}R^{15}$, -$NR^{14}$(CO)$R^{15}$, -$NR^{14}$(CO)$OR^{16}$, -$NR^{14}$(CO)$NR^{15}R^{19}$,

$-NR^{14}SO_2R^{16}$, $-COOR^{14}$, $-CONR^{14}R^{15}$, $-COR^{14}$, $-SO_2NR^{14}R^{15}$, $S(O)_{0-2}R^{16}$, $-O(CH_2)_{1-10}-COOR^{14}$, $-O(CH_2)_{1-10}CONR^{14}R^{15}$, $-(C_1-C_6)$Alkylen)-$COOR^{14}$, $-CH=CH-COOR^{14}$, $-CF_3$, -CN, $-NO_2$ und Halogen;
$R^8$ ist Wasserstoff, $(C_1-C_6)$Alkyl, Aryl$(C_1-C_6)$alkyl, $-C(O)R^{14}$ oder $-COOR^{14}$;
$R^9$ und $R^{17}$ sind unabhängig voneinander 1-3 Gruppen, unabhängig voneinander ausgewählt aus der Gruppe, bestehend aus Wasserstoff, $(C_1-C_6)$Alkyl, $(C_1-C_6)$Alkoxy, -COOH, $NO_2$, $-NR^{14}R^{15}$, OH und Halogeno;
$R^{14}$ und $R^{15}$ unabhängig voneinander ausgewählt sind aus der Gruppe, bestehend aus Wasserstoff, $(C_1-C_6)$Alkyl, Aryl und Aryl-substituiertem $(C_1-C_6)$Alkyl;
$R^{16}$ $(C_1-C_6)$Alkyl, Aryl oder $R^{17}$-substituiertes Aryl;
$R^{18}$ ist H oder $(C_1-C_6)$Alkyl; und
$R^{19}$ ist H, -OH oder $(C_1-C_6)$Alkoxy,

für die Herstellung eines Medikaments für das Regulieren der Produktion oder des Spiegels von zumindest einem Amyloid β Peptid in dem Individuum.

**52.** Verwendung einer Zusammensetzung umfassend zumindest eine Verbindung dargestellt durch die Formel (IV):

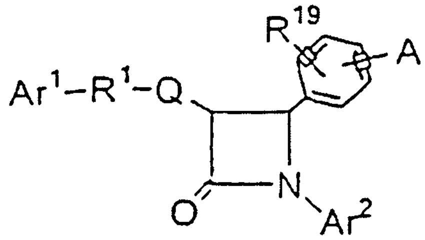

(IV)

oder ein pharmazeutisch annehmbares Salz oder Solvat davon, worin in der obenstehenden Formel (IV):

A ausgewählt ist aus der Gruppe, bestehend aus $R^2$-substituiertem Heterocycloalkyl, $R^2$-substituiertem Heteroaryl, $R^2$-substituiertem benzofusioniertem Heterocycloalkyl und $R^2$-substituiertem benzofusioniertem Heteroaryl;
$Ar^1$ Aryl oder $R^3$-substituiertes Aryl ist;
$Ar^2$ Aryl oder $R^4$-substituiertes Aryl ist;
Q eine Bindung ist oder mit dem Ring-Kohlenstoffatom an Position 3 von Azetidinon die Spirogruppe

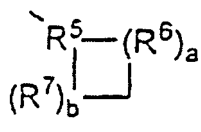

bildet; und
$R^1$ ausgewählt ist aus der Gruppe, bestehend aus:

- $(CH_2)_q$-, worin q 2-6 ist, vorausgesetzt, dass wenn Q einen Spiroring bildet, q auch 0 oder 1 sein kann;
- $(CH_2)_e$-G-$(CH_2)_r$-, worin G -O-, -C(O)-, Phenylen, $-NR^8$- oder $-S(O)_{0-2}$- ist, e 0-5 ist und r 0-5 ist, vorausgesetzt, dass die Summe von e und r 1-6 ist;
- $(C_2-C_6$-Alkenylen)-; und
- $(CH_2)_f$-V-$(CH_2)_g$-, worin V $C_3-C_6$-Cycloalkylen ist, f 1-5 und g 0-5 ist, vorausgesetzt, dass die Summe von f und g 1-6 ist;

$R^5$ ausgewählt ist aus:

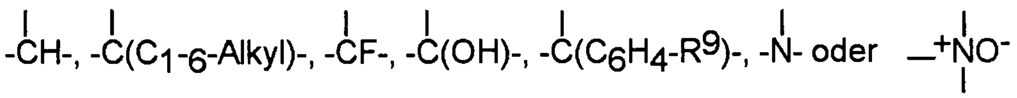 ;

$R^6$ und $R^7$ unabhängig voneinander ausgewählt sind aus der Gruppe, bestehend aus $-CH_2-$, $-CH(C_1-C_6\text{-Alkyl})-$, $-C(Di-(C_1-C_6)\text{-alkyl})$; -CH=CH- und $-C(C_1-C_6\text{-Alkyl})=CH-$ oder $R^5$ zusammen mit einem benachbarten $R^6$ oder $R^5$ gemeinsam mit einem benachbarten $R^7$ eine -CH=CH- oder $-CH=C(C_1-C_6\text{-Alkyl})$-Gruppe bilden;

a und b unabhängig voneinander 0, 1, 2 oder 3 sind, vorausgesetzt, dass nicht beide null sind;

vorausgesetzt, dass wenn $R^6$ -CH=CH- oder $-C(C_1-C_6\text{-Alkyl})=CH-$ ist, a 1 ist; vorausgesetzt, dass wenn $R^7$ -CH=CH- oder $-C(C_1-C_6\text{-Alkyl})=CH-$ ist, b 1 ist; vorausgesetzt, dass wenn a 2 oder 3 ist, die $R^6$-Reste gleich oder verschieden sein können und vorausgesetzt, dass wenn b 2 oder 3 ist, die $R^7$-Reste gleich oder verschieden sein können;

und wenn Q eine Bindung ist, kann $R^1$ auch ausgewählt werden aus:

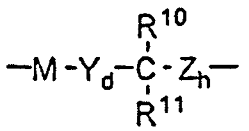 , 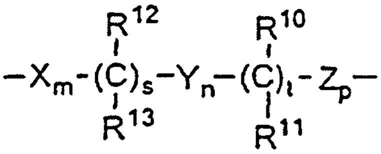 oder 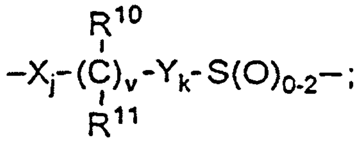 ;

worin M -O-, -S-, -S(O)- oder $-S(O)_2-$ ist;

X, Y und Z unabhängig voneinander ausgewählt sind aus der Gruppe, bestehend aus $-CH_2-$, $-CH(C_1-C_6\text{-Alkyl})-$ und $-C(Di-(C_1-C_6)alkyl)$;

$R^{10}$ und $R^{12}$ unabhängig voneinander ausgewählt sind aus der Gruppe, bestehend aus $-OR^{14}$, $-O(CO)R^{14}$, $-O(CO)OR^{16}$ und $-O(CO)NR^{14}R^{15}$;

$R^{11}$ und $R^{13}$ unabhängig voneinader ausgewählt sind aus der Gruppe, bestehend aus Wasserstoff, $(C_1-C_6)$-Alkyl und Aryl; oder $R^{10}$ und $R^{11}$ zusammen =O sind, oder $R^{12}$ und $R^{13}$ zusammen =O sind;

d ist 1, 2 oder 3;

h ist 0, 1, 2, 3 oder 4;

s ist 0 oder 1; t ist 0 oder 1; m, n und p unabhängig voneinader 0-4 sind; vorausgesetzt, dass zumindest eine von s und t 1 ist, und die Summe von m, n, p, s und t 1-6 ist, vorausgesetzt, dass wenn p 0 und t 1 ist, die Summe von m, s und n 1-5 ist; und vorausgesetzt, dass wenn p 0 ist und s 1 ist, die Summe von m, t und n 1-5 ist;

v ist 0 oder 1;

j und k sind unabhängig voneinander 1-5, vorausgesetzt, dass die Summe von j, k und v 1-5 ist;

$R^2$ 1-3 Substituenten auf den Ring-Kohlenstoffatomen ist, ausgewählt aus der Gruppe, bestehend aus Wasserstoff, $(C_1-C_{10})$-Alkyl, $(C_2-C_{10})$-Alkenyl, $(C_2-C_{10})$-Alkinyl, $(C_3-C_6)$-Cycloalkyl, $(C_3-C_6)$-Cycloalkenyl, $R^{17}$-substituiertes Aryl, $R^{17}$-substituiertes Benzyl, $R^{17}$-substituiertes Benzyloxy, $R^{17}$-substituiertes Aryloxy, Halogeno, $-NR^{14}R^{15}$, $NR^{14}R^{15}-(C_1-C_6\text{-Alkylen})-$, $NR^{14}R^{15}C(O)$ $(C_1-C_6)$-Alkylen)-, $-NHC(O)R^{16}$, OH, $C_1-C_6$-Alkoxy, $-OC(O)R^{16}$, $-COR^{14}$, Hydroxy$(C_1-C_6)$alkyl, $(C_1-C_6)$-Alkoxy$(C_1-C_6)$alkyl, $NO_2$, $-S(O)_{0-2}R^{16}$, $-SO_2NR^{14}R^{15}$ und $-(C_1-C_6\text{-Alkylen})COOR^{14}$; wenn $R^2$ ein Substituent auf einem Heterocycloalkylring ist, ist $R^2$ wie definiert oder ist =O

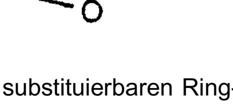 ;

und wenn $R^2$ ein Substituent auf einem substituierbaren Ring-Stickstoff ist, ist er Wasserstoff, $(C_1-C_6)$Alkyl, Aryl, $(C_1-C_6)$Alkoxy, Aryloxy, $(C_1-C_6)$Alkylcarbonyl, Arylcarbonyl, Hydroxy, $-(CH_2)_{1-6}CONR^{18}R^{18}$,

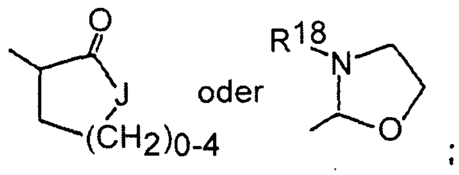

worin J -O-, -NH-, $-NR^{18}-$ oder $-CH_2-$ ist;

$R^3$ und $R^4$ unabhängig voneinander ausgewählt sind aus der Gruppe, bestehend aus 1-3 Substituenten, unabhängig ausgewählt aus der Gruppe, bestehend aus $(C_1-C_6)$Alkyl, $-OR^{14}$, $-O(CO)R^{14}$, $-O(CO)OR^{16}$, $-O(CH_2)_{1-5}OR^{14}$, $-O(CO)NR^{14}R^{15}$, $-NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $S(O)_{0-2}R^{16}$, $-O(CH_2)_{1-10}-COOR^{14}$, $-O(CH_2)_{1-10}CONR^{14}R^{15}$, $-(C_1-C_6)$Alkylen$)-COOR^{14}$, $-CH=CH-COOR^{14}$, $-CF_3$, -CN, $-NO_2$ und Halogen;

$R^8$ ist Wasserstoff, $(C_1-C_6)$Alkyl, Aryl$(C_1-C_6)$alkyl, $-C(O)R^{14}$ oder $-COOR^{14}$;

$R^9$ und $R^{17}$ sind unabhängig voneinander 1-3 Gruppen, unabhängig voneinander ausgewählt aus der Gruppe, bestehend aus Wasserstoff, $(C_1-C_6)$Alkyl, $(C_1-C_6)$Alkoxy, -COOH, $NO_2$, $-NR^{14}R^{15}$, OH und Halogeno;

$R^{14}$ und $R^{15}$ unabhängig voneinander ausgewählt sind aus der Gruppe, bestehend aus Wasserstoff, $(C_1-C_6)$ Alkyl, Aryl und Aryl-substituiertem $(C_1-C_6)$Alkyl;

$R^{16}$ $(C_1-C_6)$Alkyl, Aryl oder $R^{17}$-substituiertes Aryl;

$R^{18}$ ist H oder $(C_1-C_6)$Alkyl; und

$R^{19}$ ist H, -OH oder $(C_1-C_6)$Alkoxy,

für die Herstellung eines Medikaments für das Regulieren der Menge von ApoE Isoform 4 im Blutstrom und/oder Hirn des Individuums.

**53.** Verwendung einer Zusammensetzung umfassend zumindest eine Verbindung dargestellt durch die Formel (V):

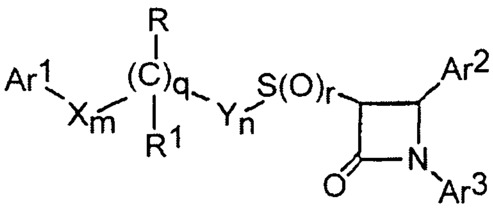

(V)

oder ein pharmazeutisch akzeptables Salz oder Solvat davon, worin in der obigen Formel (V):

$Ar^1$ Aryl, $R^{10}$ - substituiertes Aryl oder Heteroaryl ist;

$Ar^2$ Aryl oder $R^4$ -substiutiertes Aryl ist;

$Ar^3$ Aryl oder $R^5$ -substituiertes Aryl ist;

X und Y unabhängig voneinander ausgewählt sind aus der Gruppe bestehend aus $-CH_2-$, -CH(Niederalkyl)- und -C(Di-Niederalkyl)-;

R is $OR^6$, $-O(CO)R^6$, $-O(CO)OR^9$ oder $-O(CO)NR^6R^7$; $R^1$ ist H, Niederakyl oder Aryl; oder R und $R^1$ zusammen sind =O;

q ist 0 oder 1;

r ist 0, 1 oder 2;

m und n sind unabhängig voneinander 0, 1, 2, 3, 4, oder 5; vorausgesetzt dass die Summe von m, n und q 1, 2, 3, 4 oder 5 ist;

$R^4$ sind 1-5 Substituenten unabhängig voneinander ausgewählt aus der Gruppe bestehend aus Niederalkyl, $-OR^6$, $-O(CO)R^6$, $-O(CO)OR^9$, $-O(CH2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(Niederalkylen)$COOR^6$ und $-CH=CH-COOR^6$;

$R^5$ sind 1-5 Substituenten unabhängig voneinander ausgewählt aus der Gruppe bestehend aus $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$,

$-NR^6SO_2R^9$, $-COO_R{}^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, $-CF_3$, -CN, $-NO_2$, Halogen, -(Niederalkylen)$COOR^6$ und -CH=CH-$COOR^6$;
$R^6$, $R^7$ und $R^8$ sind unabhängig voneinander ausgewählt aus der Gruppe bestehend aus -H, Niederalkyl, Aryl oder Aryl-substituiertem Nideralkyl;
$R^9$ ist Niederalkyl, Aryl oder Aryl-substituiertes Niederalkyl; und
$R^{10}$ sind 1-5 Substituenten unabhängig voneinander ausgewählt aus der Gruppe bestehend aus Niederalkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}-COOR^6$, $-O(CH_2)_{1-10}CONR^6R^7$, -CF3, -CN, $-NO_2$ und Halogen

für die Herstellung eines Medikaments für das Verhindern, Behandeln oder Verbessern der Symptome der Alzheimer-Krankheit in einem Individuum.

**54.** Verwendung einer Zusammensetzung umfassend zumindest eine Verbindung dargestellt durch die Formel (V):

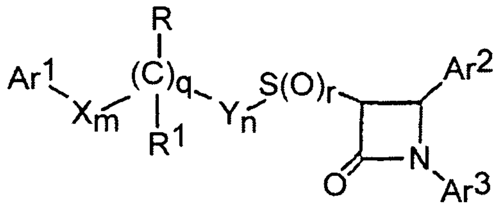

(V)

oder ein pharmazeutisch akzeptables Salz oder Solvat davon, worin in der obigen Formel (V):

$Ar^1$ Aryl, $R^{10}$ - substituiertes Aryl oder Heteroaryl ist;
$Ar^2$ Aryl oder $R^4$ -substiutiertes Aryl ist;
$Ar^3$ Aryl oder $R^5$ -substituiertes Aryl ist;
X und Y unabhängig voneinander ausgewählt sind aus der Gruppe bestehend aus $-CH_2-$, -CH(Niederalkyl)- und -C(Di-Niederalkyl)-;
R is $OR^6$, $-O(CO)R^6$, $-O(CO)OR^9$ oder $-O(CO)NR^6R^7$; $R^1$ ist H, Niederakyl oder Aryl; oder R und $R^1$ zusammen sind =O;
q ist 0 oder 1;
r ist 0, 1 oder 2;
m und n sind unabhängig voneinander 0, 1, 2, 3, 4, oder 5; vorausgesetzt dass die Summe von m, n und q 1, 2, 3, 4 oder 5 ist;
$R^4$ ist 1-5 Substituenten unabhängig voneinander, ausgewählt aus der Gruppe, bestehend aus Niederalkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(Niederalkylen)$COOR^6$ und -CH=CH-$COOR^6$;
$R^5$ sind 1-5 Substituenten unabhängig voneinander ausgewählt aus der Gruppe bestehend aus $-OR^6$, $-O(CO)R^6$, $-O(CO)OR^9$, $-O(CH^2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, $-CF_3$, -CN, $-NO_2$, Halogen, -(Niederalkylen)$COOR^6$ und -CH=CH-$COOR^6$;
$R^6$, $R^7$ und $R^8$ sind unabhängig voneinander ausgewählt aus der Gruppe bestehend aus -H, Niederalkyl, Aryl oder Aryl-substituiertem Nideralkyl;
$R^9$ ist Niederalkyl, Aryl oder Aryl-substituiertes Niederalkyl; und
$R^{10}$ sind 1-5 Substituenten unabhängig voneinander ausgewählt aus der Gruppe bestehend aus Niederalkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}-COOR^6$, $-O(CH_2)_{1-10}CONR^6R^7$, -CF3, -CN, $-NO_2$ und Halogen

für die Herstellung eines Medikaments für das Regulieren der Produktion oder des Spiegels von zumindest einem Amyloid β Peptid in dem Individuum.

**55.** Verwendung einer Zusammensetzung umfassend zumindest eine Verbindung dargestellt durch die Formel (V):

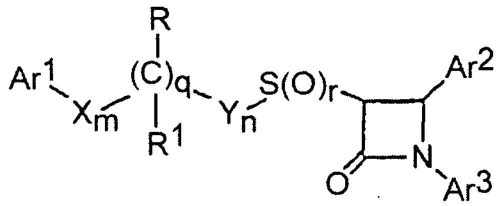

(V)

oder ein pharmazeutisch akzeptables Salz oder Solvat davon, worin in der obigen Formel (V):

$Ar^1$ Aryl, $R^{10}$ - substituiertes Aryl oder Heteroaryl ist;
$Ar^2$ Aryl oder $R^4$ -substiutiertes Aryl ist;
$Ar^3$ Aryl oder $R^5$ -substituiertes Aryl ist;
X und Y unabhängig voneinander ausgewählt sind aus der Gruppe bestehend aus $-CH_2-$, -CH(Niederalkyl)- und -C(Di-Niederalkyl)-;
R is $OR^6$, $-O(CO)R^6$, $-O(CO)OR^9$ oder $-O(CO)NR^6R^7$; $R^1$ ist H, Niederakyl oder Aryl; oder R und $R^1$ zusammen sind =O;
q ist 0 oder 1;
r ist 0, 1 oder 2;
m und n sind unabhängig voneinander 0, 1, 2, 3, 4, oder 5; vorausgesetzt dass die Summe von m, n und q 1, 2, 3, 4 oder 5 ist;
$R^4$ ist 1-5 Substituenten unabhängig voneinander, ausgewählt aus der Gruppe, bestehend aus Niederalkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, -(Niederalkylen)COOR6 und $-CH=CH-COOR^6$;
$R^5$ sind 1-5 Substituenten unabhängig voneinander ausgewählt aus der Gruppe bestehend aus $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $S(O)_{0-2}R^9$, $-O(CH_2)_{1-10}-COOR^6$, $-O(CH_2)_{1-10}CONR^6R^7$, $-CF_3$, -CN, $-NO_2$, Halogen, -(Niederalkylen)$COOR^6$ und $-CH=CH-COOR^6$;
$R^6$, $R^7$ und $R^8$ sind unabhängig voneinander ausgewählt aus der Gruppe bestehend aus -H, Niederalkyl, Aryl oder Aryl-substituiertem Nideralkyl;
$R^9$ ist Niederalkyl, Aryl oder Aryl-substituiertes Niederalkyl; und
$R^{10}$ sind 1-5 Substituenten unabhängig voneinander ausgewählt aus der Gruppe bestehend aus Niederalkyl, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}-COOR^6$, $-O(CH_2)_{1-10}CONR^6R^7$, -CF3, -CN, $-NO_2$ und Halogen

für die Herstellung eines Medikaments für das Regulieren der Menge von ApoE Isoform 4 im Blutstrom und/oder Hirn des Individuums.

**56.** Verwendung einer Zusammensetzung umfassend zumindest eine Verbindung dargestellt durch die Formel (VI):

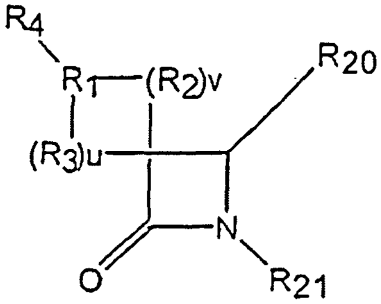

(VI)

oder ein pharmazeutisch akzeptables Salz oder Solvat davon, worin:

$R_1$ ist

-CH-, -C(Niederalkyl)-, -CF-, -C(OH)-, $-C(C_6H_5)-$, $-C(C_6H_4-R_{15})-$,

-N- oder $-^{+}NO^{-}$;

$R_2$ und $R_3$ unabhängig voneinander ausgewählt sind aus der Gruppe bestehend aus $-CH_2-$, -CH(Niederalkyl)- und -C(Di-Niederalkyl)-, -CH=CH- und -C(Niederalkyl)=CH-; oder $R_1$ zusammen mit einem benachbarten $R_2$, oder $R_1$ zusammen mit einem benachbarten $R_3$ ein -CH=CH- oder eine -CH=C(Niederalkyl)-Gruppe bilden;
u und v sind unabhängig voneinander 0, 1, 2, oder 3, vorausgesetzt es sind nicht beide 0; vorausgesetzt, dass wenn $R_2$ -CH=CH- oder -C(Niederalkyl)=CH- ist, v 1 ist; vorausgesetzt, dass wenn $R_3$ -CH=CH- oder -C(Niederalkyl)=CH- ist, u 1 ist; vorausgesetzt, dass wenn v 2 oder 3 ist, die $R_2$s gleich oder unterschiedlich sein können; und vorausgesetzt dass wenn u 2 oder 3 ist, die $R_3$s gleich oder unterschiedlich sein können;
$R_4$ ist ausgewählt aus $B-(CH_2)_mC(O)-$, worin m 0, 1, 2, 3, 4, oder 5 ist;
$B-(CH_2)_q-$, worin q 0, 1, 2, 3, 4, 5 oder 6 ist; $B-(CH_2)_e-Z-(CH_2)_r-$, wobei Z -O-, -C(O)-, Phenylen, $-N(R_8)-$ oder $S(O)_{0-2}-$ ist, e ist 0, 1, 2, 3, 4, oder 5 und r 0, 1, 2, 3, 4, oder 5 ist, vorausgesetzt dass die Summe von e und r 0, 1, 2, 3, 4, 5 oder 6 ist;
$B-(C_2-C_6$-Alkenylen)-;
$B-(C_4-C_6$-Alkadienylen)-;
$B-(CH_2)_t-Z-(C_2-C_6$-Alkenylen)-, wobei Z wie oben definiert ist, und wobei t 0, 1, 2 oder 3 ist, vorausgesetzt dass die Summe von t und der Anzahl von Kohlenstoffatomen in der Alkenylenkette 2, 3, 4, 5, oder 6 ist;
$B-(CH_2)_f-V-(CH_2)_g-$, wobei V $C_3-C_6$-Cycloalkylen ist, f 1, 2, 3, 4 oder 5 ist und g 0, 1, 2, 3, 4 oder 5 ist, vorausgesetzt, dass die Summe von f und g 1, 2, 3, 4, 5 oder 6 ist;
$B-(CH_2)_t-V(C_2-C_6$-Alkenylen)- oder
$B(C_2-C_6$-Alkenylen)$-V-(CH_2)_t-$, wobei V und t wie oben definiert sind, vorausgesetzt dass die Summe von t und der Anzahl der Kohlenstoffatome in der Alkenylenkette 2, 3, 4, 5 oder 6 ist;
$B-(CH_2)_a-Z-(CH_2)_b-V-(CH_2)_d-$, wobei Z und V wie oben definiert sind und a, b und d unabhängig voneinander 0, 1, 2, 3, 4, 5 oder 6 sind, vorausgesetzt, dass die Summe von a, b und d 0, 1, 2, 3, 4, 5 oder 6 ist; oder $T-(CH_2)_s-$, wobei T Cycloalkyl mit 3-6 Kohlenstoffatomen ist und s 0, 1, 2, 3, 4, 5 oder 6 ist; oder
$R_1$ und $R_4$ zusammen die Gruppe

B-CH=C-

bilden;
B ist ausgewählt aus Indanyl, Indenyl, Napthyl, Tetrahydronaphtyl, Heteroaryl oder W-substiutiertem Heteroaryl, wobei Heteroaryl ausgewählt ist aus der Gruppe bestehend aus Pyrrolyl, Pyridinyl, Pyrimidinyl, Pyrazinyl, Triazinyl, Imidazolyl, Thiazolyl, Pyrazolyl, Thienyl, Oxazolyl und Furanyl, und bei N-enthaltenden Heteroarylen die N-Oxide davon, oder

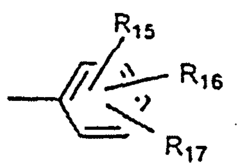

W sind 1 bis 3 Substituenten, unabhängig voneinander ausgewählt aus der Gruppe bestehend aus Niederalkyl, Hydroxyniederalkyl, Niederalkoxy, Alkoxyalkyl, Alkoxyalkoxy, Alkoxycarbonylalkoxy, (Niederalkoxyimino)-niederalkyl, Niederalkandioyl, Niederalkylniederalkandioyl, Allyloxy, $-CF_3$, $-OCF_3$, Benzyl, $R_7$-Benzyl, Benzyloxy,

$R_7$-Benzyloxy, Phenoxy, $R_7$-Phenoxy, Dioxolanyl, $NO_2$, $-N(R_8)(R_9)$, $N(R_8)(R_9)$-Niederalkylen-, $N(R_8)(R_9)$-Niederalkylenoxy-, OH, Halogeno, -CN, $-N_3$, $-NHC(O)OR_{10}$, $-NHC(O)R_{10}$, $R_{11}O_2SNH$-, $(R_{11}O_2S)_2N$-, $-S(O)_2NH_2$-, $S(O)_{0-2}R_8$, tert-Butyldimethyl-silyloxymethyl, $-C(O)R_{12}$, $-COOR_{19}$, $-CON(R_8)(R_9)$, $-CH=CHC(O)R_{12}$, -Niederalkylen-$C(O)R_{12}$,
$R_{10}C(O)$(Niederalkylenyloxy)-,
$N(R_8)(R_9)C(O)$(Niederalkylenoxy)- und

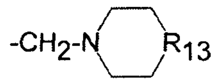

zur Substitution von Ring-Kohlenstoffatomen, und die Substituenten auf den substituierten Heteroaryl Ring Stickstoffatomen, falls vorhanden, sind ausgewählt aus der Gruppe bestehend aus Niederalkyl, Niederalkoxy, - $C(O)OR_{10}$-, $-C(O)R_{10}$, OH, $N(R_8)(R_9)$-Niederalkylen-, $N(R_8)(R_9)$-Niederaklylenyloxy-, $-S(O_2)NH_2$ und 2-(Trimethylsilyl)-Ethoxymethyl;
$R_7$ sind 1-3 Gruppen unabhängig voneinander ausgewählt aus der Gruppe bestehend aus Niederalkyl, Niederalkoxy, -COOH, $NO_2$, $-N(R_8)(R_9)$, OH und Halogeno;
$R_8$ und $R_9$ sind unabhängig voneinander ausgewählt aus H oder Niederalkyl,
$R_{10}$ ist ausgewählt aus Niederalkyl, Phenyl, $R_7$-Phenyl, Benzyl oder $R_7$-Benzyl;
$R_{11}$ ist ausgewählt aus OH, Niederalkyl, Phenyl, Benzyl, $R_7$-Phenyl oder $R_7$-Benzyl;
$R_{12}$ ist ausgewählt aus H, OH, Alkoxy, Phenoxy, Benzyloxy,

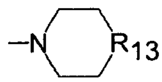 ,

$-N(R_8)(R_9)$, Niederalkyl, Phenyl oder $R_7$-Phenyl; $R_{13}$ ist ausgewählt aus -O-, $-CH_2$-, -NH-, -N(Niederalkyl)- oder $-NC(O)R_{19}$;
$R_{15}$, $R_{16}$ und $R_{17}$ sind unabhängig voneinander ausgewählt aus der Gruppe bestehend aus H und den für W definierten Gruppen; oder $R_{15}$ ist H und $R_{16}$ und $R_{17}$, zusammen mit benachbarten Kohlenstoffatomen mit welchen sie verbunden sind, bilden einen Dioxolanyl Ring;
$R_{19}$ ist H, Niederalkyl, Phenyl oder Phenylniederalkyl; und
$R_{20}$ und $R_{21}$ sind unabhängig voneinander ausgewählt aus der Gruppe bestehend aus Phenyl, W-substituiertem Phenyl, Naphtyl, W-substituiertem Naphtyl, Indanyl, Indenyl, Tetrahydronaphtyl, Benzodioxolyl, Heteroaryl, W-substituiertem Heteroaryl, Benzofusioniertem Heteroaryl, W-substituiertes Benzofusioniertem Heteroaryl und Cyclopropyl, wobei Heteroaryl wie oben definiert ist,

für die Herstellung eines Medikaments für die Verhinderung, Behandlung oder Verbesserung der Symptome der Alzheimer-Krankheit in einem Individuum.

**57.** Verwendung einer Zusammensetzung umfassend zumindest eine Verbindung dargestellt durch die Formel (VI):

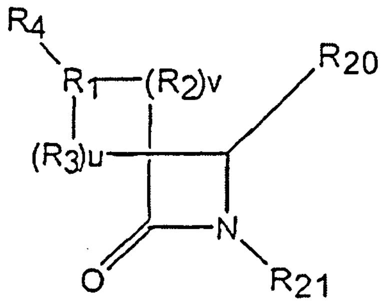

(VI)

oder ein pharmazeutisch akzeptables Salz oder Solvat davon, worin:

$R_1$ ist

$-CH-$, $-C(Niederalkyl)-$, $-CF-$, $-C(OH)-$, $-C(C_6H_5)-$, $-C(C_6H_4-R_{15})-$,

$-N-$ oder $-^{+}NO^{-}$;

$R_2$ und $R_3$ unabhängig voneinander ausgewählt sind aus der Gruppe bestehend aus $-CH_2-$, -CH(Niederalkyl)- und -C(Di-Niederalkyl)-, -CH=CH- und -C(Niederalkyl)=CH-; oder $R_1$ zusammen mit einem benachbarten $R_2$, oder $R_1$ zusammen mit einem benachbarten $R_3$ ein -CH=CH- oder eine -CH=C(Niederalkyl)-Gruppe bilden;
u und v sind unabhängig voneinander 0, 1, 2, oder 3, vorausgesetzt es sind nicht beide 0; vorausgesetzt, dass wenn $R_2$ -CH=CH- oder -C(Niederalkyl)=CH- ist, v 1 ist; vorausgesetzt, dass wenn $R_3$ -CH=CH- oder -C(Niederalkyl)=CH- ist, u 1 ist; vorausgesetzt, dass wenn v 2 oder 3 ist, die $R_2$s gleich oder unterschiedlich sein können; und vorausgesetzt dass wenn u 2 oder 3 ist, die $R_3$s gleich oder unterschiedlich sein können;
$R_4$ ist ausgewählt aus $B-(CH_2)_mC(O)-$, worin m 0, 1, 2, 3, 4, oder 5 ist;
$B-(CH_2)_q-$, worin q 0, 1, 2, 3, 4, 5 oder 6 ist;
$B-(CH_2)_e-Z-(CH_2)_r-$, wobei Z -O-, -C(O)-, Phenylen, $-N(R_8)-$ oder $-S(O)_{0-2}-$ ist, e ist 0, 1, 2, 3, 4, oder 5 und r 0, 1, 2, 3, 4, oder 5 ist, vorausgesetzt dass die Summe von e und r 0, 1, 2, 3, 4, 5 oder 6 ist;
$B-(C_2-C_6$-Alkenylen)-;
$B-(C_4-C_6$-Alkadienylen)-;
$B-(CH_2)_t-Z-(C_2-C_6$-Alkenylen)-, wobei Z wie oben definiert ist, und wobei t 0, 1, 2 oder 3 ist, vorausgesetzt dass die Summe von t und der Anzahl von Kohlenstoffatomen in der Alkenylenkette 2, 3, 4, 5, oder 6 ist;
$B-(CH_2)_f-V-(CH_2)_g-$, wobei V $C_3-C_6$-Cycloalkylen ist, f 1, 2, 3, 4 oder 5 ist und g 0, 1, 2, 3, 4 oder 5 ist, vorausgesetzt, dass die Summe von f und g 1, 2, 3, 4, 5 oder 6 ist;
$B-(CH_2)_t-V(C_2-C_6$-Alkenylen)- oder
$B(C_2-C_6$-Alkenylen)-V-$(CH_2)_t-$, wobei V und t wie oben definiert sind, vorausgesetzt dass die Summe von t und der Anzahl der Kohlenstoffatome in der Alkenylenkette 2, 3, 4, 5 oder 6 ist;
$B-(CH_2)_a-Z-(CH_2)_b-V-(CH_2)_d-$, wobei Z und V wie oben definiert sind und a, b und d unabhängig voneinander 0, 1, 2, 3, 4, 5 oder 6 sind, vorausgesetzt, dass die Summe von a, b und d 0, 1, 2, 3, 4, 5 oder 6 ist; oder
$T-(CH_2)_s-$, wobei T Cycloalkyl mit 3-6 Kohlenstoffatomen ist und s 0, 1, 2, 3, 4, 5 oder 6 ist; oder
$R_1$ und $R_4$ zusammen die Gruppe B-CH=C.bilden;
B ist ausgewählt aus Indanyl, Indenyl, Napthyl, Tetrahydronaphtyl, Heteroaryl oder W-substiutiertem Heteroaryl, wobei Heteroaryl ausgewählt ist aus der Gruppe bestehend aus Pyrrolyl, Pyridinyl, Pyrimidinyl, Pyrazinyl, Triazinyl, Imidazolyl, Thiazolyl, Pyrazolyl, Thienyl, Oxazolyl und Furanyl, und bei N-enthaltenden Heteroarylen die N-Oxide davon, oder

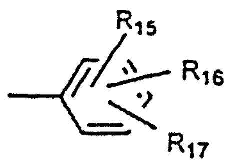

W sind 1 bis 3 Substituenten, unabhängig voneinander ausgewählt aus der Gruppe bestehend aus Niederalkyl, Hydroxyniederalkyl, Niederalkoxy, Alkoxyalkyl, Alkoxyalkoxy, Alkoxycarbonylalkoxy, (Niederalkoxyimino)-niederalkyl, Niederalkandioyl, Niederalkylniederalkandioyl, Allyloxy, $-CF_3$,$-OCF_3$, Benzyl, $R_7$-Benzyl, Benzyloxy, $R_7$-Benzyloxy, Phenoxy, $R_7$-Phenoxy, Dioxolanyl, $NO_2$, $-N(R_8)(R_9)$, $N(R_8)(R_9)$-Niederalkylen-, $N(R_8)(R_9)$-Niederalkylenoxy-, OH, Halogeno, -CN, $-N_3$, $-NHC(O)OR_{10}$, $-NHC(O)R_{10}$, $R_{11}O_2SNH-$, $(R_{11}O_2S)_2N-$, $-S(O)_2NH_2-$, $S(O)_{0-2}R_8$, tert-Butyldimethyl-silyloxymethyl, $-C(O)R_{12}$, $-COOR_{19}$, $-CON(R_8)(R_9)$, $-CH=CHC(O)R_{12}$, -Niederalkylen-$C(O)R_{12}$,
$R_{10}C(O)$(Niederalkylenyloxy)-,
$N(R_8)(R_9)C(O)$(Niederalkylenoxy)- und

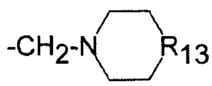

zur Substitution von Ring-Kohlenstoffatomen, und die Substituenten auf den substituierten Heteroaryl Ring Stickstoffatomen, falls vorhanden, sind ausgewählt aus der Gruppe bestehend aus Niederalkyl, Niederalkoxy, $-C(O)OR_{10}$-, $-C(O)R_{10}$, OH, $N(R_8)(R_9)$-Niederalkylen-, $N(R_8)(R_9)$-Niederaklylenyloxy-, $-S(O_2)NH_2$ und 2-(Trimethylsilyl)-Etoxymethyl;

$R_7$ sind 1-3 Gruppen unabhängig voneinander ausgewählt aus der Gruppe bestehend aus Niederalkyl, Niederalkoxy, -COOH, $NO_2$, $-N(R_8)(R_9)$, OH und Halogeno;

$R_8$ und $R_9$ sind unabhängig voneinander ausgewählt aus H oder Nideralkyl,

$R_{10}$ ist ausgewählt aus Niederalkyl, Phenyl, $R_7$-Phenyl, Benzyl oder $R_7$-Benzyl;

$R_{11}$ ist ausgewählt aus OH, Niederalkyl, Phenyl, Benzyl, $R_7$-Phenyl oder $R_7$-Benzyl;

$R_{12}$ ist ausgewählt aus H, OH, Alkoxy, Phenoxy, Benzyloxy,

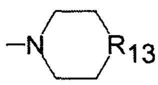 ,

$-N(R_8)(R_9)$, Niederalkyl, Phenyl oder $R_7$-Phenyl; $R_{13}$ ist ausgewählt aus -O-, $-CH_2$-, -NH-, -N(Niederalkyl)- oder $-NC(O)R_{19}$;

$R_{15}$, $R_{16}$ und $R_{17}$ sind unabhängig voneinander ausgewählt aus der Gruppe bestehend aus H und den für W definierten Gruppen; oder $R_{15}$ ist H und $R_{16}$ und $R_{17}$, zusammen mit benachbarten Kohlenstoffatomen mit welchen sie verbunden sind, bilden einen Dioxolanyl Ring;

$R_{19}$ ist H, Niederalkyl, Phenyl oder Phenylniederalkyl; und

$R_{20}$ und $R_{21}$ sind unabhängig voneinander ausgewählt aus der Gruppe bestehend aus Phenyl, W-substituiertem Phenyl, Naphtyl, W-substituiertem Naphtyl, Indanyl, Indenyl, Tetrahydronaphtyl, Benzodioxolyl, Heteroaryl, W-substituiertem Heteroaryl, Benzofusioniertem Heteroaryl, W-substituiertem Benzofusioniertem Heteroaryl und Cyclopropyl, wobei Heteroaryl wie oben definiert ist,

für die Herstellung eines Medikaments für das Regulieren der Produktion oder des Spiegels von zumindest einem Amyloid β Peptid in dem Individuum.

**58.** Verwendung einer Zusammensetzung umfassend zumindest eine Verbindung dargestellt durch die Formel (VI):

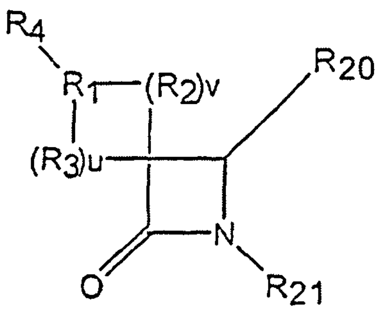

(VI)

oder ein pharmazeutisch akzeptables Salz oder Solvat davon, worin:

$R_1$ ist

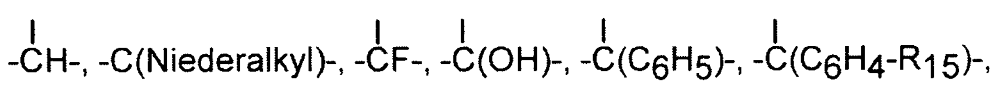

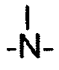 oder 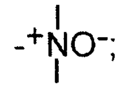

$R_2$ und $R_3$ unabhängig voneinander ausgewählt sind aus der Gruppe bestehend aus $-CH_2-$, -CH(Niederalkyl)- und -C(Di-Niederalkyl)-, -CH=CH- und -C(Niederalkyl)=CH-; oder $R_1$ zusammen mit einem benachbarten $R_2$, oder $R_1$ zusammen mit einem benachbarten $R_3$ ein -CH=CH- oder eine -CH=C(Niederalkyl)-Gruppe bilden;
u und v sind unabhängig voneinander 0, 1, 2, oder 3, vorausgesetzt es sind nicht beide 0; vorausgesetzt, dass wenn $R_2$ -CH=CH- oder -C(Niederalkyl)=CH- ist, v 1 ist; vorausgesetzt, dass wenn $R_3$ -CH=CH- oder -C(Niederalkyl)=CH- ist, u 1 ist; vorausgesetzt, dass wenn v 2 oder 3 ist, die $R_2$s gleich oder unterschiedlich sein können; und vorausgesetzt dass wenn u 2 oder 3 ist, die $R_3$s gleich oder unterschiedlich sein können;
$R_4$ ist ausgewählt aus $B-(CH_2)_mC(O)-$, worin m 0, 1, 2, 3, 4, oder 5 ist;
$B-(CH_2)_q-$, worin q 0, 1, 2, 3, 4, 5 oder 6 ist;
$B-(CH_2)_e-Z-(CH_2)_r-$, wobei Z -O-, -C(O)-, Phenylen, $-N(R_8)-$ oder $S(O)_{0-2}-$ ist, e ist 0, 1, 2, 3, 4, oder 5 und r 0, 1, 2, 3, 4, oder 5 ist, vorausgesetzt dass die Summe von e und r 0, 1, 2, 3, 4, 5 oder 6 ist;
B-($C_2$-$C_6$-Alkenylen)-;
B-($C_4$-$C_6$-Alkadienylen)-;
$B-(CH_2)_t-Z-$($C_2$-$C_6$-Alkenylen)-, wobei Z wie oben definiert ist, und wobei t 0, 1, 2 oder 3 ist, vorausgesetzt dass die Summe von t und der Anzahl von Kohlenstoffatomen in der Alkenylenkette 2, 3, 4, 5, oder 6 ist;
$B-(CH_2)_f-V-(CH_2)_g-$, wobei V $C_3$-$C_6$-Cycloalkylen ist, f 1, 2, 3, 4 oder 5 ist und g 0, 1, 2, 3, 4 oder 5 ist, vorausgesetzt, dass die Summe von f und g 1, 2, 3, 4, 5 oder 6 ist;
$B-(CH_2)_t-V$($C_2$-$C_6$-Alkenylen)- oder
B($C_2$-$C_6$-Alkenylen)-$V-(CH_2)_t-$, wobei V und t wie oben definiert sind, vorausgesetzt dass die Summe von t und der Anzahl der Kohlenstoffatome in der Alkenylenkette 2, 3, 4, 5 oder 6 ist;
$B-(CH_2)_a-Z-(CH_2)_b-V-(CH_2)_d-$, wobei Z und V wie oben definiert sind und a, b und d unabhängig voneinander 0, 1, 2, 3, 4, 5 oder 6 sind, vorausgesetzt, dass die Summe von a, b und d 0, 1, 2, 3, 4, 5 oder 6 ist; oder
$T-(CH_2)_s-$, wobei T Cycloalkyl mit 3-6 Kohlenstoffatomen ist und s 0, 1, 2, 3, 4, 5 oder 6 ist; oder
$R_1$ und $R_4$ zusammen die Gruppe B-CH=C. bilden;
B ist ausgewählt aus Indanyl, Indenyl, Napthyl, Tetrahydronaphtyl, Heteroaryl oder W-substiutiertem Heteroaryl, wobei Heteroaryl ausgewählt ist aus der Gruppe bestehend aus Pyrrolyl, Pyridinyl, Pyrimidinyl, Pyrazinyl, Triazinyl, Imidazolyl, Thiazolyl, Pyrazolyl, Thienyl, Oxazolyl und Furanyl, und bei N-enthaltenden Heteroarylen die N-Oxide davon, oder

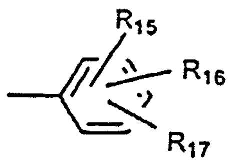

W sind 1 bis 3 Substituenten, unabhängig voneinander ausgewählt aus der Gruppe bestehend aus Niederalkyl, Hydroxyniederalkyl, Niederalkoxy, Alkoxyalkyl, Alkoxyalkoxy, Alkoxycarbonylalkoxy, (Niederalkoxyimino)-niederalkyl, Niederalkandioyl, Niederalkylniederalkandioyl, Allyloxy, $-CF_3$,$-OCF_3$, Benzyl, $R_7$-Benzyl, Benzyloxy, $R_7$-Benzyloxy, Phenoxy, $R_7$-Phenoxy, Dioxolanyl, $NO_2$, $-N(R_8)(R_9)$, $N(R_8)(R_9)$-Niederalkylen-, $N(R_8)(R_9)$-Niederalkylenoxy-, OH, Halogeno, -CN, $-N_3$, $-NHC(O)OR_{10}$, $-NHC(O)R_{10}$, $R_{11}O_2SNH-$, $(R_{11}O_2S)_2N-$, $-S(O)_2NH_2-$, $S(O)_{0-2}R_8$, tert-Butyldimethyl-silyloxymethyl,- $C(O)R_{12}$, $-COOR_{19}$, $-CON(R_8)(R_9)$, $-CH=CHC(O)R_{12}$, -Niederalkylen-$C(O)R_{12}$, $R_{10}C(O)$(Niederalkylenyloxy)-, $N(R_8)(R_9)C(O)$(Niederalkylenoxy)- und

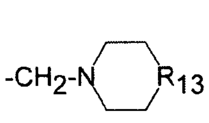

zur Substitution von Ring-Kohlenstoffatomen, und die Substituenten auf den substituierten Heteroaryl Ring Stickstoffatomen, falls vorhanden, sind ausgewählt aus der Gruppe bestehend aus Niederalkyl, Nideralkoxy,

-C(O)OR$_{10}$-, -C(O)R$_{10}$, OH, N(R$_8$)(R$_9$)-Niederalkylen-, N(R$_8$)(R$_9$)-Niederaklylenyloxy-, -S(O$_2$)NH$_2$ und 2-(Trimethylsilyl)-Etoxymethyl;

R$_7$ sind 1-3 Gruppen unabhängig voneinander ausgewählt aus der Gruppe bestehend aus Niederalkyl, Niederalkoxy, -COOH, NO$_2$, -N(R$_8$)(R$_9$), OH und Halogeno;

R$_8$ und R$_9$ sind unabhängig voneinander ausgewählt aus H oder Niederalkyl,

R$_{10}$ ist ausgewählt aus Niederalkyl, Phenyl, R$_7$-Phenyl, Benzyl oder R$_7$-Benzyl;

R$_{11}$ ist ausgewählt aus OH, Niederalkyl, Phenyl, Benzyl, R$_7$-Phenyl oder R$_7$-Benzyl;

R$_{12}$ ist ausgewählt aus H, OH, Alkoxy, Phenoxy, Benzyloxy,

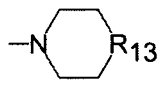 ,

-N(R$_8$)(R$_9$), Niederalkyl, Phenyl oder R$_7$-Phenyl; R$_{13}$ ist ausgewählt aus -O-, -CH$_2$-, -NH-, -N(Niederalkyl)- oder -NC(O)R$_{19}$;

R$_{15}$, R$_{16}$ und R$_{17}$ sind unabhängig voneinander ausgewählt aus der Gruppe bestehend aus H und den für W definierten Gruppen; oder R$_{15}$ ist H und R$_{16}$ und R$_{17}$, zusammen mit benachbarten Kohlenstoffatomen mit welchen sie verbunden sind, bilden einen Dioxolanyl Ring;

R$_{19}$ ist H, Niederalkyl, Phenyl oder Phenylniederalkyl; und

R$_{20}$ und R$_{21}$ sind unabhängig voneinander ausgewählt aus der Gruppe bestehend aus Phenyl, W-substituiertem Phenyl, Naphtyl, W-substituiertem Naphtyl, Indanyl, Indenyl, Tetrahydronaphtyl, Benzodioxolyl, Heteroaryl, W-substituiertem Heteroaryl, Benzofusioniertem Heteroaryl, W-substituiertem Benzofusioniertem Heteroaryl und Cyclopropyl, wobei Heteroaryl wie oben definiert ist, für die Herstellung eines Medikaments für das Regulieren der Menge von ApoE Isoform 4 im Blutstrom und/oder Hirn des Individuums.

**59.** Verwendung einer Zusammensetzung umfassend zumindest eine Verbindung dargestellt durch die Formel (VII):

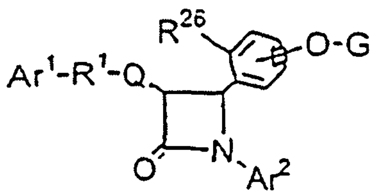 (VII)

oder ein pharmazeutisch annehmbares Salz oder Solvat davon, worin, in der obigen Formel (VII),

R$^{26}$ H oder OG$^1$ ist;

G und G$^1$ unabhängig voneinander ausgewählt sind aus der Gruppe bestehend aus H,

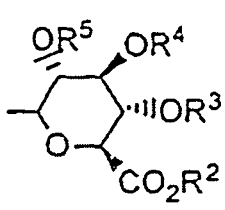 , 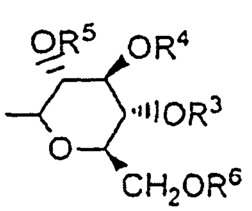 , 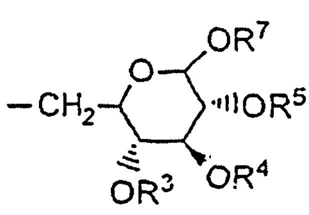

und

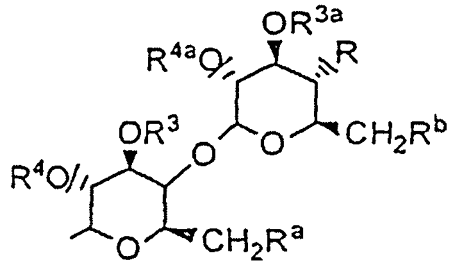 ;

vorausgesetzt, dass wenn $R^{26}$ H oder OH ist, G nicht H ist;

R, $R^a$ und $R^b$ sind unabhängig voneinander ausgewählt aus der Gruppe bestehend aus H, -OH, Halogen, $-NH_2$, Azid, $(C_1-C_6)$Alkoxy$(C_1-C_6)$-Alkoxy oder $-W-R^{30}$;

W ist unabhängig ausgewählt aus der Gruppe bestehend aus -NH-C(O)-, -O-C(O)-, $-O-C(O)-N(R^{31})-$, $-NH-C(O)-N(R^{31})-$ und $-O-C(S)-N(R^{31})-$;

$R^2$ und $R^6$ sind unabhängig voneinander ausgewählt aus der Gruppe bestehend aus H, $(C_1-C_6)$Alkyl, Aryl, und Aryl$(C_1-C_6)$Alkyl;

$R^3$, $R^4$, $R^5$, $R^7$, $R^{3a}$ und $R^{4a}$ sind unabhängig voneinander ausgewählt aus der Gruppe bestehend aus H, $(C_1-C_6)$Alkyl, Aryl$(C_1-C_6)$Alkyl, $-C(O)(C_1-C_6)$Alkyl und -C(O)Aryl;

$R^{30}$ ist ausgewählt aus der Gruppe bestehend aus $R^{32}$-substituiertem T, $R^{32}$-substituiertem $-T-(C_1-C_6)$Alkyl, $R^{32}$-substituiertem-$(C_2-C_4)$Alkenyl, $R^{32}$-substituiertem-$(C_1-C_6)$Alkyl, $R^{32}$-substituiertem $-(C_3-C_7)$Cycloalkyl und $R^{32}$-substituiertem $-(C_3-C_7)$Cycloalkyl$(C_1-C_6)$Alkyl;

$R^{31}$ ist ausgewählt aus der Gruppe bestehend aus H und $(C_1-C_4)$Alkyl;

T ist ausgewählt aus der Gruppe bestehend aus Phenyl, Furyl, Thienyl, Pyrrolyl, Oxazolyl, Isoxazolyl, Thiazolyl, Isothiazolyl, Benzothiazolyl, Thiadiazolyl, Pyrazolyl, Imidazolyl und Pyridyl;

$R^{32}$ ist unabhängig ausgewählt aus 1-3 Substituenten unabhängig voneinander ausgewählt aus der Gruppe bestehend aus Halogen, $(C_1-C_4)$Alkyl, -OH, Phenoxy, $-CF_3$, $-NO_2$, $(C_1-C_4)$Alkoxy, Methylendioxy, Oxo, $(C_1-C_4)$Alkylsulfanyl, $(C_1-C_4)$Alkylsulfinyl, $(C_1-C_4)$Alkylsulfonyl, $-N(CH_3)_2$, $-C(O)-NH(C_1-C_4)$Alkyl, $-C(O)-N((C_1-C_4)Alkyl)_2$, $-C(O)-(C_1-C_4)$Alkyl, $-C(O)-(C_1-C_4)$Alkoxy und Pyrrolidinylcarbonyl; oder $R^{32}$ ist eine kovalente Bindung und $R^{31}$, der Stickstoff an den es gebunden ist und $R^{32}$ bilden eine Pyrrolidinyl-, Piperidinyl-, N-Methyl-piperazinyl-, Indolinyl- oder Morpholinylgruppe oder eine $(C_1-C_4)$Alkoxycarbonyl-substituierte Pyrrolidinyl-, Piperidinyl-, N-Methylpiperazinyl-, Indolinyl- oder Morpholinylgruppe $Ar^1$ ist Aryl oder $R^{10}$-substituiertes Aryl; $Ar^2$ ist Aryl oder $R^{11}$-substituiertes Aryl;

Q ist eine Bindung oder, mit dem Ring-Kohlenstoff an Position 3 des Azetidinons, bildet die Spiro Gruppe

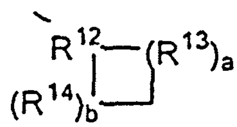 ;

und

$R^1$ ist ausgewählt aus der Gruppe bestehend aus

$-(CH_2)_q-$, worin q 2-6 ist, vorausgesetzt, dass wenn Q einen Spiro Ring bildet, q auch 0 oder 1 sein kann;

$-(CH_2)_e-E-(CH_2)_r$, wobei E -O-, -C(O)-, Phenylen, $-NR^{22}-$ oder $-S(O)_{0-2}-$ ist, e 0-5 ist und r 0-5 ist, vorausgesetzt, dass die Summe von e und r 1-6 ist;

$-(C_2-C_6)$Alkenylen; und

$-(CH_2)_f-V-(CH_2)_g-$, wobei V $C_3-C_6$ Cycloalkylen ist, f 1-5 ist und g 0-5 ist, vorausgesetzt, dass die Summe von f und g 1-6 ist;

$R^{12}$ ist

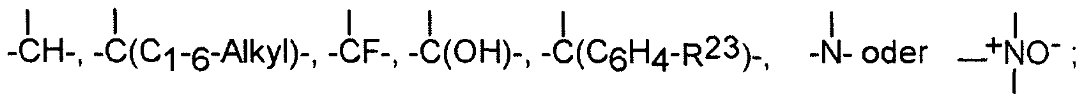

$R^{13}$ und $R^{14}$ sind unabhängig voneinander ausgewählt aus der Gruppe bestehend aus $-CH_2-$, $-CH(C_1-C_6\text{-Alkyl})-$, $-C(Di-(C_1-C_6)alkyl)$, -CH=CH- und $-C(C_1-C_6\text{-Alkyl})=CH-$; oder $R^{12}$ zusammen mit einem benachbarten $R^{13}$ oder $R^{12}$ zusammen mit einem benachbarten $R^{14}$ bilden eine -CH=CH- oder eine $-CH=C(C_1-C_6\text{-Alkyl})$-Gruppe;
a und b sind unabhängig voneinander 0, 1, 2 oder 3, vorausgesetzt es sind nicht beide 0;
vorausgesetzt, dass wenn $R^{13}$ -CH=CH- oder $-C(C_1-C_6\text{-Alkyl})=CH-$ ist, a 1 ist;
vorausgesetzt, dass wenn $R^{14}$ -CH=CH- oder $-C(C_1-C_6\text{-Alkyl})=CH-$ ist, b 1 ist;
vorausgesetzt, dass wenn a 2 oder 3 ist, die $R^{13}$ gleich oder verschieden sein können, und vorausgesetzt, dass wenn b 2 oder 3 ist, die $R^{14}$ gleich oder verschieden sein können;
und wenn Q eine Bindung ist, kann $R^1$ auch sein:

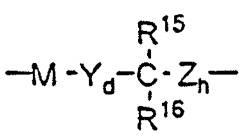 , 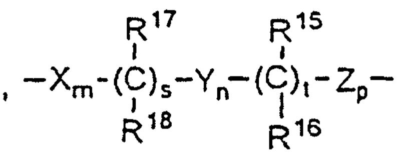 oder 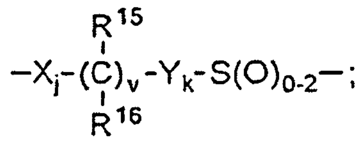 ;

M ist -O-, -S-, -S(O)- oder $-S(O)_2-$;
X, Y und Z sind unabhängig voneinander ausgewählt aus der Gruppe bestehend aus $-CH_2-$, $-CH(C_1-C_6)Alkyl-$ und $-C(di-(C_1-C_6)\text{-Alkyl})$;
$R^{10}$ und $R^{11}$ sind unabhängig voneinander ausgewählt aus der Gruppe bestehend aus 1-3 Substituenten, unabhängig voneinander ausgewählt aus der Gruppe bestehend aus $(C_1-C_6)$Alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_6\text{-Alkylen})-COOR^{19}$, $-CH=CH-COOR^{19}$, $-CF_3$, -CN, $-NO_2$ und Halogen;
$R^{15}$ und $R^{17}$ sind unabhängig voneinander ausgewählt aus der Gruppe bestehend aus $-OR^{19}-$, $-O(CO)R^{19}$, $-O(CO)OR^{21}$ und $-O(CO)NR^{19}R^{20}$;
$R^{16}$ und $R^{18}$ sind unabhängig voneinander ausgewählt aus der Gruppe bestehend aus H, $(C_1-C_6)$-Alkyl und Aryl; oder $R^{15}$ und $R^{16}$ zusammen sind =O, oder $R^{17}$ und $R^{18}$ zusammen sind =O;
d ist 1, 2 oder 3;
h ist 0, 1, 2, 3 oder 4;
s ist 0 oder 1; t ist 0 oder 1; m, n und p sind unabhängig voneinander 0-4;
vorausgesetzt, dass zumindest einer von s und t 1 ist, und die Summe von m, n, p, s und t 1-6 ist; vorausgesetzt, dass wenn p 0 ist und t 1 ist, die Summe von m, s und n 1-5 ist; und vorausgesetzt, dass wenn p 0 ist und s 1 ist, die Summe von m, t und n 1-5 ist;
v ist 0 oder 1;
j und k sind unabhängig voneinander 1-5, vorausgesetzt,dass die Summe von j, k und v 1-5 ist; und wenn Q eine Bindung ist und $R^1$

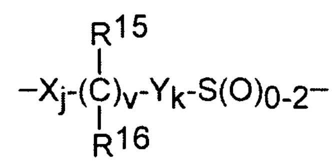

ist, kann $Ar^1$ auch Pyridyl, Isoxazolyl, Furanyl, Pyrrolyl, Thienyl, Imidazolyl, Pyrazolyl, Thiazolyl, Pyrazinyl, Pyrimidinyl oder Pyridazinyl sein;
$R^{19}$ und $R^{20}$ sind unabhängig voneinander ausgewählt aus der Gruppe bestehend aus H, $(C_1-C_6)$ Alkyl, Aryl und Aryl-substituiertem $(C_1-C_6)$-Alkyl;
$R^{21}$ ist $(C_1-C_6)$ Alkyl, Aryl oder $R^{24}$-substituiertes Aryl;
$R^{22}$ ist H, $(C_1-C_6)$ Alkyl, Aryl $(C_1-C_6)$ Alkyl, $-C(O)R^{19}$ oder $-COOR^{19}$;
$R^{23}$ und $R^{24}$ sind unabhängig 1-3 Gruppen ausgewählt aus der Gruppe bestehend aus H, $(C_1-C_6)$-Alkyl, $(C_1-C_6)$-Alkoxy, -COOH, $NO_2$, $-NR^{19}R^{20}$, -OH und Halogen; und $R^{25}$ ist H, -OH oder $(C_1-C_6)$-Alkoxy,

für die Herstellung eines Medikaments für die Verhinderung, Behandlung oder Verbesserung der Symptome der

Alzheimer-Krankheit in einem Individuum.

**60.** Verwendung einer Zusammensetzung umfassend zumindest eine Verbindung dargestellt durch die Formel (VII):

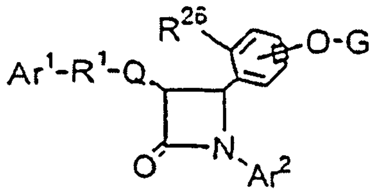

(VII)

oder ein pharmazeutisch annehmbares Salz oder Solvat davon, worin, in der obigen Formel (VII),

$R^{26}$ H oder $OG^1$ ist;
G und $G^1$ unabhängig voneinander ausgewählt sind aus der Gruppe bestehend aus H,

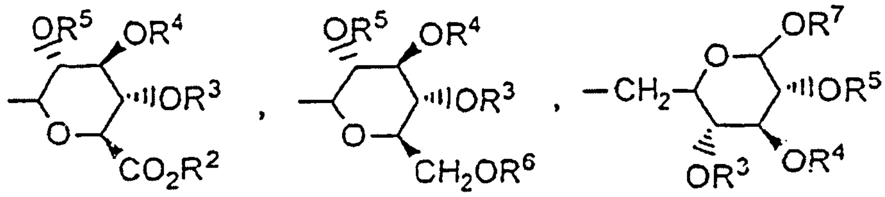

, , und

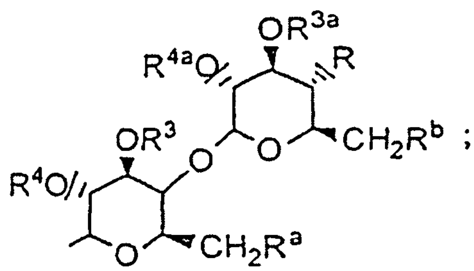

;

vorausgesetzt, dass wenn $R^{26}$ H oder OH ist, G nicht H ist;
R, $R^a$ und $R^b$ sind unabhängig voneinander ausgewählt aus der Gruppe bestehend aus H, -OH, Halogen, $-NH_2$, Azid, $(C_1\text{-}C_6)$Alkoxy$(C_1\text{-}C_6)$-Alkoxy oder $-W-R^{30}$;
W ist unabhängig ausgewählt aus der Gruppe bestehend aus -NH-C(O)-, -O-C(O)-, -O-C(O)-N($R^{31}$)-, -NH-C(O)-N($R^{31}$)- und -O-C(S)-N($R^{31}$)- ;
$R^2$ und $R^6$ sind unabhängig voneinander ausgewählt aus der Gruppe bestehend aus H, $(C_1\text{-}C_6)$Alkyl, Aryl, und Aryl$(C_1\text{-}C_6)$Alkyl;
$R^3$, $R^4$, $R^5$, $R^7$, $R^{3a}$ und $R^{4a}$ sind unabhängig voneinander ausgewählt aus der Gruppe bestehend aus H, $(C_1\text{-}C_6)$ Alkyl, Aryl$(C_1\text{-}C_6)$Alkyl, $-C(O)(C_1\text{-}C_6)$Alkyl und -C(O)Aryl;
$R^{30}$ ist ausgewählt aus der Gruppe bestehend aus $R^{32}$-substituiertem T, $R^{32}$-substituiertem $-T-(C_1\text{-}C_6)$Alkyl, $R^{32}$-substituiertem-$(C_2\text{-}C_4)$Alkenyl, $R^{32}$-substituiertem-$(C_1\text{-}C_6)$Alkyl, $R^{32}$-substituiertem $(C_3\text{-}C_7)$Cycloalkyl und $R^{32}$-substituiertem-$(C_3\text{-}C_7)$Cycloalkyl$(C_1\text{-}C_6)$Alkyl;
$R^{31}$ ist ausgewählt aus der Gruppe bestehend aus H und $(C_1\text{-}C_4)$Alkyl;
T ist ausgewählt aus der Gruppe bestehend aus Phenyl, Furyl, Thienyl, Pyrrolyl, Oxazolyl, Isoxazolyl, Thiazolyl, Isothiazolyl, Benzothiazolyl, Thiadiazolyl, Pyrazolyl, Imidazolyl und Pyridyl;
$R^{32}$ ist unabhängig ausgewählt aus 1-3 Substituenten unabhängig voneinander ausgewählt aus der Gruppe

bestehend aus Halogen, $(C_1\text{-}C_4)$Alkyl, -OH, Phenoxy, $-CF_3$, $-NO_2$, $(C_1\text{-}C_4)$Alkoxy, Methylendioxy, Oxo, $(C_1\text{-}C_4)$Alkylsulfanyl, $(C_1\text{-}C_4)$Alkylsulfinyl, $(C_1\text{-}C_4)$Alkylsulfonyl, $-N(CH_3)_2$, $-C(O)\text{-}NH(C_1\text{-}C_4)$Alkyl, $-C(O)\text{-}N((C_1\text{-}C_4)$Alkyl$_2$, $-C(O)\text{-}(C_1\text{-}C_4)$Alkyl, $-C(O)\text{-}(C_1\text{-}C_4)$Alkoxy und Pyrrolidinylcarbonyl; oder $R^{32}$ ist eine kovalente Bindung und $R^{31}$, der Stickstoff an den es gebunden ist und $R^{32}$ bilden eine Pyrrolidinyl-, Piperidinyl-, N-Methyl-piperazinyl-, Indolinyl- oder Morpholinylgruppe oder eine $(C_1\text{-}C_4)$Alkoxycarbonyl-substituierte Pyrrolidinyl-, Piperidinyl-, N-Methylpiperazinyl-, Indolinyl- oder Morpholinylgruppe $Ar^1$ ist Aryl oder $R^{10}$-substituiertes Aryl;
$Ar^2$ ist Aryl oder $R^{11}$-substituiertes Aryl;
Q ist eine Bindung oder, mit dem Ring-Kohlenstoff an Position 3 des Azetidinons, bildet die Spiro Gruppe

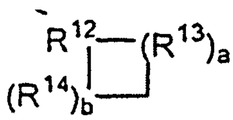 ;

und
$R^1$ ist ausgewählt aus der Gruppe bestehend aus
$-(CH_2)_q-$, worin q 2-6 ist, vorausgesetzt, dass wenn Q einen Spiro Ring bildet, q auch 0 oder 1 sein kann;
$-(CH_2)_e\text{-}E\text{-}(CH_2)_r$, wobei E -O-, -C(O)-, Phenylen, $-NR^{22}-$ oder $-S(O)_{0\text{-}2}-$ ist, e 0-5 ist und r 0-5 ist, vorausgesetzt, dass die Summe von e und r 1-6 ist;
$-(C_2\text{-}C_6)$Alkenylen; und
$-(CH_2)_f\text{-}V\text{-}(CH_2)_g-$, wobei V $C_3\text{-}C_6$ Cycloalkylen ist, f 1-5 ist und g 0-5 ist, vorausgesetzt, dass die Summe von f und g 1-6 ist;
$R^{12}$ ist

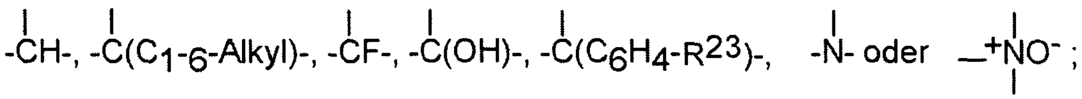

$R^{13}$ und $R^{14}$ sind unabhängig voneinander ausgewählt aus der Gruppe bestehend aus $-CH_2-$, $-CH(C_1\text{-}C_6\text{-Alkyl})-$, $-C(\text{Di-}(C_1\text{-}C_6)\text{alkyl})$, -CH=CH- und
$-C(C_1\text{-}C_6\text{-Alkyl})=CH-$; oder $R^{12}$ zusammen mit einem benachbarten $R^{13}$ oder $R^{12}$ zusammen mit einem benachbarten $R^{14}$ bilden eine -CH=CH- oder eine $-CH=C(C_1\text{-}C_6\text{-Alkyl})$-Gruppe;
a und b sind unabhängig voneinander 0, 1, 2 oder 3, vorausgesetzt es sind nicht beide 0;
vorausgesetzt, dass wenn $R^{13}$ -CH=CH- oder $-C(C_1\text{-}C_6\text{-Alkyl})=CH-$ ist, a 1 ist;
vorausgesetzt, dass wenn $R^{14}$ -CH=CH- oder $-C(C_1\text{-}C_6\text{-Alkyl})=CH-$ ist, b 1 ist;
vorausgesetzt, dass wenn a 2 oder 3 ist, die $R^{13}$ gleich oder verschieden sein können, und vorausgesetzt, dass wenn b 2 oder 3 ist, die $R^{14}$ gleich oder verschieden sein können;
und wenn Q eine Bindung ist, kann $R^1$ auch sein:

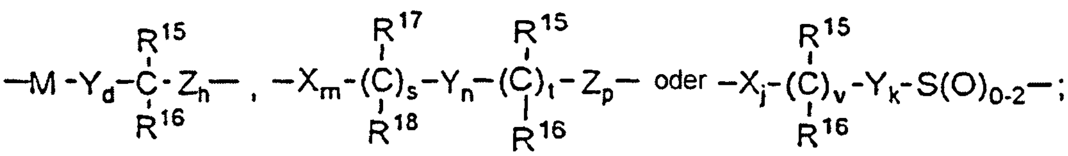

M ist -O-, -S-, -S(O)- oder $-S(O)_2-$;
X, Y und Z sind unabhängig voneinander ausgewählt aus der Gruppe bestehend aus $-CH_2-$, $-CH(C_1\text{-}C_6)$Alkyl- und $-C(\text{di-}(C_1\text{-}C_6)\text{-Alkyl})$;
$R^{10}$ und $R^{11}$ sind unabhängig voneinander ausgewählt aus der Gruppe bestehend aus 1-3 Substituenten, unabhängig voneinander ausgewählt aus der Gruppe bestehend aus $(C_1\text{-}C_6)$Alkyl, $-OR^{19}$, $-O(CO)R^{19}$, $-O(CO)OR^{21}$, $-O(CH_2)_{1\text{-}5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0\text{-}2}R^{21}$, $-O(CH_2)_{1\text{-}10}\text{-}COOR^{19}$, $-O(CH_2)_{1\text{-}10}CONR^{19}R^{20}$, $-(C_1\text{-}C_6\text{-Alkylen})\text{-}COOR^{19}$, $-CH=CH\text{-}COOR^{19}$, $-CF_3$, -CN, $-NO_2$ und Halogen;

$R^{15}$ und $R^{17}$ sind unabhängig voneinander ausgewählt aus der Gruppe bestehend aus $-OR^{19}$-, $-O(CO)R^{19}$, $-O(CO)OR^{21}$ und $-O(CO)NR^{19}R^{20}$;
$R^{16}$ und $R^{18}$ sind unabhängig voneinander ausgewählt aus der Gruppe bestehend aus H, $(C_1-C_6)$-Alkyl und Aryl; oder $R^{15}$ und $R^{16}$ zusammen sind =O, oder $R^{17}$ und $R^{18}$ zusammen sind =O;
d ist 1, 2 oder 3;
h ist 0, 1, 2, 3 oder 4;
s ist 0 oder 1; t ist 0 oder 1; m, n und p sind unabhängig voneinander 0-4;
vorausgesetzt, dass zumindest einer von s und t 1 ist, und die Summe von m, n, p, s und t 1-6 ist; vorausgesetzt, dass wenn p 0 ist und t 1 ist, die Summe von m, s und n 1-5 ist; und vorausgesetzt, dass wenn p 0 ist und s 1 ist, die Summe von m, t und n 1-5 ist;
v ist 0 oder 1;
j und k sind unabhängig voneinander 1-5, vorausgesetzt,dass die Summe von j, k und v 1-5 ist;
und wenn Q eine Bindung ist und $R^1$

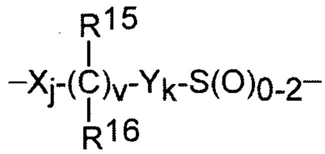

ist, kann $Ar^1$ auch Pyridyl, Isoxazolyl, Furanyl, Pyrrolyl, Thienyl, Imidazolyl, Pyrazolyl, Thiazolyl, Pyrazinyl, Pyrimidinyl oder Pyridazinyl sein;
$R^{19}$ und $R^{20}$ sind unabhängig voneinander ausgewählt aus der Gruppe bestehend aus H, $(C_1-C_6)$ Alkyl, Aryl und Aryl-substituiertem $(C_1-C_6)$-Alkyl;
$R^{21}$ ist $(C_1-C_6)$ Alkyl, Aryl oder $R^{24}$-substituiertes Aryl;
$R^{22}$ ist H, $(C_1-C_6)$ Alkyl, Aryl $(C_1-C_6)$ Alkyl, $-C(O)R^{19}$ oder $-COOR^{19}$;
$R^{23}$ und $R^{24}$ sind unabhängig 1-3 Gruppen ausgewählt aus der Gruppe bestehend aus H, $(C_1-C_6)$-Alkyl, $(C_1-C_6)$-Alkoxy,- COOH, $NO_2$, $-NR^{19}R^{20}$, -OH und Halogen; und $R^{25}$ ist H, -OH oder $(C_1-C_6)$-Alkoxy,

für die Herstellung eines Medikaments für das Regulieren der Produktion oder des Spiegels von zumindest einem Amyloid β Peptid in dem Individuum.

**61.** Verwendung einer Zusammensetzung umfassend zumindest eine Verbindung dargestellt durch die Formel (VII):

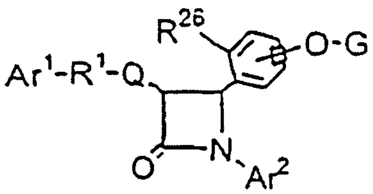

(VII)

oder ein pharmazeutisch annehmbares Salz oder Solvat davon, worin, in der obigen Formel (VII),

$R^{26}$ H oder $OG^1$ ist;
G und $G^1$ unabhängig voneinander ausgewählt sind aus der Gruppe bestehend aus H,

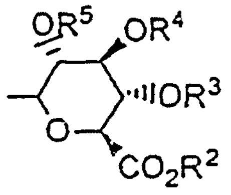 , 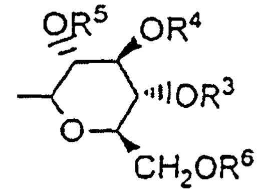 , 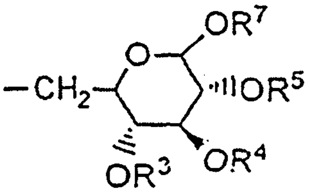

und

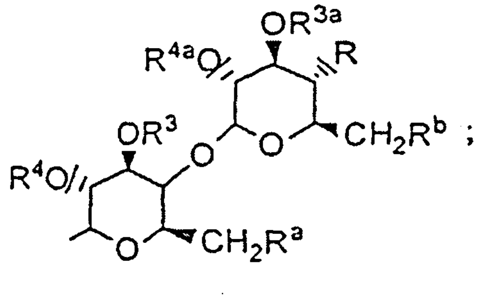

vorausgesetzt, dass wenn $R^{26}$ H oder OH ist, G nicht H ist;

R, $R^a$ und $R^b$ sind unabhängig voneinander ausgewählt aus der Gruppe bestehend aus H, -OH, Halogen, $-NH_2$, Azid, $(C_1\text{-}C_6)$Alkoxy$(C_1\text{-}C_6)$-Alkoxy oder $-W-R^{30}$;

W ist unabhängig ausgewählt aus der Gruppe bestehend aus -NH-C(O)-, -O-C(O)-, -O-C(O)-N($R^{31}$)-, -NH-C(O)-N($R^{31}$)- und -O-C(S)-N($R^{31}$)-;

$R^2$ und $R^6$ sind unabhängig voneinander ausgewählt aus der Gruppe bestehend aus H, $(C_1\text{-}C_6)$Alkyl, Aryl, und Aryl$(C_1\text{-}C_6)$Alkyl;

$R^3$, $R^4$, $R^5$, $R^7$, $R^{3a}$ und $R^{4a}$ sind unabhängig voneinander ausgewählt aus der Gruppe bestehend aus H, $(C_1\text{-}C_6)$ Alkyl, Aryl$(C_1\text{-}C_6)$Alkyl, $-C(O)(C_1\text{-}C_6)$Alkyl und -C(O)Aryl;

$R^{30}$ ist ausgewählt aus der Gruppe bestehend aus $R^{32}$-substituiertem T, $R^{32}$-substituiertem-T-$(C_1\text{-}C_6)$Alkyl, $R^{32}$-substituiertem-$(C_2\text{-}C_4)$Alkenyl, $R^{32}$-substituiertem-$(C_1\text{-}C_6)$Alkyl, $R^{32}$-substituiertem-$(C_3\text{-}C_7)$Cycloalkyl und $R^{32}$-substituiertem $(C_3\text{-}C_7)$Cycloalkyl$(C_1\text{-}C_6)$Alkyl;

$R^{31}$ ist ausgewählt aus der Gruppe bestehend aus H und $(C_1\text{-}C_4)$Alkyl;

T ist ausgewählt aus der Gruppe bestehend aus Phenyl, Furyl, Thienyl, Pyrrolyl, Oxazolyl, Isoxazolyl, Thiazolyl, Isothiazolyl, Benzothiazolyl, Thiadiazolyl, Pyrazolyl, Imidazolyl und Pyridyl;

$R^{32}$ ist unabhängig ausgewählt aus 1-3 Substituenten unabhängig voneinander ausgewählt aus der Gruppe bestehend aus Halogen, $(C_1\text{-}C_4)$Alkyl, -OH, Phenoxy, $-CF_3$, $-NO_2$, $(C_1\text{-}C_4)$Alkoxy, Methylendioxy, Oxo, $(C_1\text{-}C_4)$ Alkylsulfanyl, $(C_1\text{-}C_4)$Alkylsulfinyl, $(C_1\text{-}C_4)$Alkylsulfonyl, $-N(CH_3)_2$, $-C(O)-NH(C_1\text{-}C_4)$Alkyl, $-C(O)-N((C_1\text{-}C_4)$ Alkyl$_2$, $-C(O)-(C_1\text{-}C_4)$Alkyl, $-C(O)-(C_1\text{-}C_4)$Alkoxy und Pyrrolidinylcarbonyl; oder $R^{32}$ ist eine kovalente Bindung und $R^{31}$, der Stickstoff an den es gebunden ist und $R^{32}$ bilden eine Pyrrolidinyl-, Piperidinyl-, N-Methyl-piperazinyl-, Indolinyl- oder Morpholinylgruppe oder eine $(C_1\text{-}C_4)$Alkoxycarbonyl-substituierte Pyrrolidinyl-, Piperidinyl-, N-Methylpiperazinyl-, Indolinyl- oder Morpholinylgruppe $Ar^1$ ist Aryl oder $R^{10}$-substituiertes Aryl;

$Ar^2$ ist Aryl oder $R^{11}$-substituiertes Aryl;

Q ist eine Bindung oder, mit dem Ring-Kohlenstoff an Position 3 des Azetidinons, bildet die Spiro Gruppe

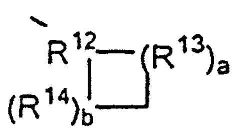 ;

und

$R^1$ ist ausgewählt aus der Gruppe bestehend aus

$-(CH_2)_q-$, worin q 2-6 ist, vorausgesetzt, dass wenn Q einen Spiro Ring bildet, q auch 0 oder 1 sein kann;

$-(CH_2)_e-E-(CH_2)_r$, wobei E -O-, -C(O)-, Phenylen, $-NR^{22}-$ oder $-S(O)_{0\text{-}2}-$ ist, e 0-5 ist und r 0-5 ist, vorausgesetzt,

dass die Summe von e und r 1-6 ist;
-($C_2$-$C_6$)Alkenylen; und
-$(CH_2)_f$-V-$(CH_2)_g$-, wobei V $C_3$-$C_6$ Cycloalkylen ist, f 1-5 ist und g 0-5 ist, vorausgesetzt, dass die Summe von f und g 1-6 ist;
$R^{12}$ ist

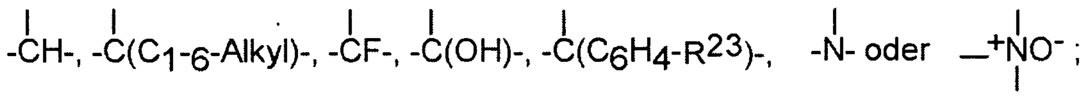

$R^{13}$ und $R^{14}$ sind unabhängig voneinander ausgewählt aus der Gruppe bestehend aus -$CH_2$-, -CH($C_1$-$C_6$-Alkyl)-, -C(Di-($C_1$-$C_6$)alkyl), -CH=CH- und -C($C_1$-$C_6$-Alkyl)=CH-; oder $R^{12}$ zusammen mit einem benachbarten $R^{13}$ oder $R^{12}$ zusammen mit einem benachbarten $R^{14}$ bilden eine -CH=CH- oder eine -CH=C($C_1$-$C_6$-Alkyl)-Gruppe;
a und b sind unabhängig voneinander 0, 1, 2 oder 3, vorausgesetzt es sind nicht beide 0;
vorausgesetzt, dass wenn $R^{13}$ -CH=CH- oder -C($C_1$-$C_6$-Alkyl)=CH- ist, a 1 ist;
vorausgesetzt, dass wenn $R^{14}$ -CH=CH- oder -C($C_1$-$C_6$-Alkyl)=CH- ist, b 1 ist;
vorausgesetzt, dass wenn a 2 oder 3 ist, die $R^{13}$ gleich oder verschieden sein können, und vorausgesetzt, dass wenn b 2 oder 3 ist, die $R^{14}$ gleich oder verschieden sein können;
und wenn Q eine Bindung ist, kann $R^1$ auch sein:

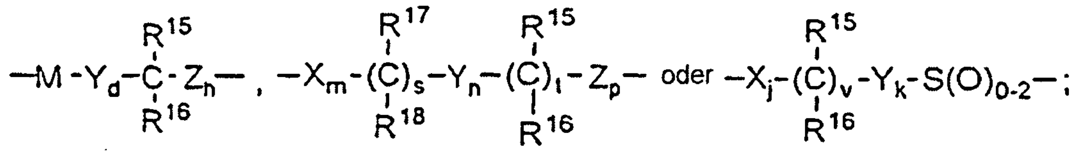

M ist -O-, -S-, -S(O)- oder -$S(O)_2$-;
X, Y und Z sind unabhängig voneinander ausgewählt aus der Gruppe bestehend aus -$CH_2$-, -CH($C_1$-$C_6$)Alkyl- und -C(di-($C_1$-$C_6$)-Alkyl);
$R^{10}$ und $R^{11}$ sind unabhängig voneinander ausgewählt aus der Gruppe bestehend aus 1-3 Substituenten, unabhängig voneinander ausgewählt aus der Gruppe bestehend aus ($C_1$-$C_6$)Alkyl, -$OR^{19}$, -O(CO)$R^{19}$, -O(CO)$OR^{21}$, -$O(CH_2)_{1-5}OR^{19}$, -O(CO)$NR^{19}R^{20}$, -$NR^{19}R^{20}$, -$NR^{19}$(CO)$R^{20}$, -$NR^{19}$(CO)$OR^{21}$, -$NR^{19}$(CO)$NR^{20}R^{25}$, -$NR^{19}SO_2R^{21}$, -$COOR^{19}$, -$CONR^{19}R^{20}$, -$COR^{19}$, -$SO_2NR^{19}R^{20}$, $S(O)_{0-2}R^{21}$, -$O(CH_2)_{1-10}$-$COOR^{19}$, -O$(CH_2)_{1-10}CONR^{19}R^{20}$, -($C_1$-$C_6$-Alkylen)-$COOR^{19}$, -CH=CH-$COOR^{19}$, -$CF_3$, -CN, -$NO_2$ und Halogen;
$R^{15}$ und $R^{17}$ sind unabhängig voneinander ausgewählt aus der Gruppe bestehend aus -$OR^{19}$-, -O(CO)$R^{19}$, -O(CO)$OR^{21}$ und -O(CO)$NR^{19}R^{20}$;
$R^{16}$ und $R^{18}$ sind unabhängig voneinander ausgewählt aus der Gruppe bestehend aus H, ($C_1$-$C_6$)-Alkyl und Aryl; oder $R^{15}$ und $R^{16}$ zusammen sind =O, oder $R^{17}$ und $R^{18}$ zusammen sind =O;
d ist 1, 2 oder 3;
h ist 0, 1, 2, 3 oder 4;
s ist 0 oder 1; t ist 0 oder 1; m, n und p sind unabhängig voneinander 0-4;
vorausgesetzt, dass zumindest einer von s und t 1 ist, und die Summe von m, n, p, s und t 1-6 ist; vorausgesetzt, dass wenn p 0 ist und t 1 ist, die Summe von m, s und n 1-5 ist; und vorausgesetzt, dass wenn p 0 ist und s 1 ist, die Summe von m, t und n 1-5 ist;
v ist 0 oder 1;
j und k sind unabhängig voneinander 1-5, vorausgesetzt,dass die Summe von j, k und v 1-5 ist; und wenn Q eine Bindung ist und $R^1$

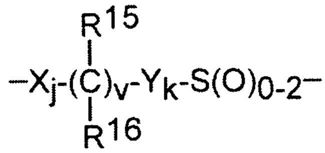

ist, kann $Ar^1$ auch Pyridyl, Isoxazolyl, Furanyl, Pyrrolyl, Thienyl, Imidazolyl, Pyrazolyl, Thiazolyl, Pyrazinyl, Pyrimidinyl oder Pyridazinyl sein;
$R^{19}$ und $R^{20}$ sind unabhängig voneinander ausgewählt aus der Gruppe bestehend aus H, $(C_1\text{-}C_6)$ Alkyl, Aryl und Aryl-substituiertem $(C_1\text{-}C_6)$-Alkyl;
$R^{21}$ ist $(C_1\text{-}C_6)$ Alkyl, Aryl oder $R^{24}$-substituiertes Aryl;
$R^{22}$ ist H, $(C_1\text{-}C_6)$ Alkyl, Aryl $(C_1\text{-}C_6)$ Alkyl, $-C(O)R^{19}$ oder $-COOR^{19}$;
$R^{23}$ und $R^{24}$ sind unabhängig 1-3 Gruppen ausgewählt aus der Gruppe bestehend aus H, $(C_1\text{-}C_6)$-Alkyl, $(C_1\text{-}C_6)$-Alkoxy, -COOH, $NO_2$, $-NR^{19}R^{20}$, -OH und Halogen, und $R^{25}$ ist H, -OH oder $(C_1\text{-}C_6)$-Alkoxy,

für die Herstellung eines Medikaments für das Regulieren der Menge von ApoE Isoform 4 im Blutstrom und/oder Hirn des Individuums.

**62.** Verwendung einer Zusammensetzung umfassend zumindest eine Verbindung dargestellt durch die Formel (VIII):

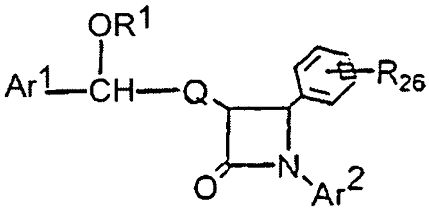

(VIII)

oder ein pharmazeutisch annembares Salz oder Solvat davon, wobei

$R^{26}$ ausgewählt ist aus der Gruppe bestehend aus:

a) OH;
b) $OCH_3$;
c) Fluor und
d) Chlor

$R^1$ ausgewählt ist aus der Gruppe bestehend aus H,

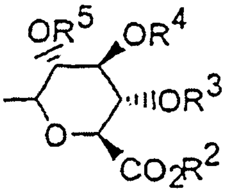

,

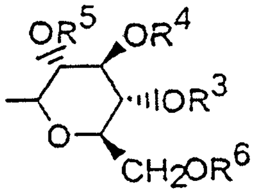

,

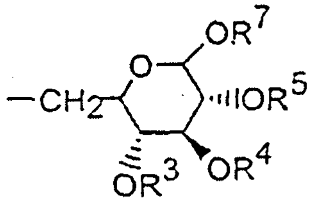

,

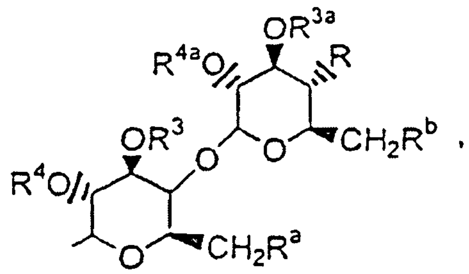

$-SO_3H$; natürlichen und nicht natürlichen Aminosäuren. R, $R^a$ und $R^b$ unabhängig voneinander ausgewählt sind aus der Gruppe bestehend aus H, -OH, Halogen, $-NH_2$, Azid, $(C_1-C_6)$Alkoxy$(C_1-C_6)$-Alkoxy und $-W-R^{30}$;

W ist unabhängig ausgewählt aus der Gruppe bestehend aus -NH-C(O)-, -O-C(O)-, $-O-C(O)-N(R^{31})-$, $-NH-C(O)-N(R^{31})-$ und $-O-C(S)-N(R^{31})-$;

$R^2$ und $R^6$ sind unabhängig voneinander ausgewählt aus der Gruppe bestehend aus H, $(C_1-C_6)$-Alkyl, Aryl und Aryl$(C_1-C_6)$Alkyl;

$R^3$, $R^4$, $R^5$, $R^7$, $R^{3a}$ und $R^{4a}$ sind unabhängig voneinander ausgewählt aus der Gruppe bestehend aus H, $(C_1-C_6)$ Alkyl, Aryl$(C_1-C_6)$Alkyl, $-C(O)(C_1-C_6)$Alkyl und -C(O)Aryl;

$R^{30}$ ist unabhängig ausgewählt aus der Gruppe bestehend aus $R^{32}$-substituiertem T, $R^{32}$-substituiertem T-$(C_1-C_6)$Alkyl, $R^{32}$-substituiertem-$(C_2-C_4)$Alkenyl, $R^{32}$-substiuiertem-$(C_1-C_6)$ Alkyl, $R^{32}$-substituiertem-$(C_3-C_7)$ Cycloalkyl und $R^{32}$-substituiertem-$(C_3-C_7)$ Cycloalkyl $(C_1-C_6)$ Alkyl;

$R^{31}$ ist unabhängig ausgewählt aus der Gruppe bestehend aus H und $(C_1-C_4)$ Alkyl;

T ist unabhängig ausgewählt aus der Gruppe bestehend aus Phenyl, Furyl, Thienyl, Pyrrolyl, Oxazolyl, Isoxazolyl, Thiazolyl, Isothiazolyl, Benzothiazolyl, Thiadiazolyl, Pyrazolyl, Imidazolyl und Pyridyl;

$R^{32}$ ist unabhängig ausgewählt aus 1-3 Substituenten unabhängig voneinander ausgewählt aus der Gruppe bestehend aus H, Halogeno, $(C_1-C_4)$-Alkyl, -OH, Phenoxy, $-CF_3$, $-NO_2$, $(C_1-C_4)$-Alkoxy, Methylendioxy, Oxo, $(C_1-C_4)$-Alkylsulfanyl, $(C_1-C_4)$-Alkylsulfinyl, $(C_1-C_4)$-Alkylsulfonyl, $-N(CH_3)_2$, $-C(O)-NH(C_1-C_4)$-Alkyl, $-C(O)-N((C_1-C_4)Alkyl)_2$, $-C(O)-(C_1-C_4)$-Alkyl, $-C(O)-(C_1-C_4)$-Alkoxy und Pyrrolidinylcarbonyl; oder $R^{32}$ ist eine kovalente Bindung und $R^{31}$, der Stickstoff an den es gebunden ist und $R^{32}$ bilden eine Pyrrolidinyl-, Piperidinyl-, N-Methyl-piperazinyl-, Indolinyl oder Morpholinylgruppe oder eine $(C_1-C_4)$-Alkoxycarbonyl-substituierte Pyrrolidinyl-, Piperidinyl-, N-Methylpiperazinyl-, Indolinyl- oder Morpholinylgruppe;

$Ar^1$ ist Aryl oder $R^{10}$-substituiertes Aryl;

$Ar^2$ ist Aryl oder $R^{11}$-substituiertes Aryl;

Q ist $-(CH_2)_q-$, wobei q 2-6 ist, oder, mit dem Ring-Kohlenstoff an Position 3 des Azetidinons, die Spiro Gruppe

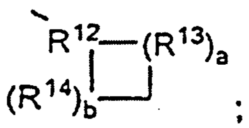

bildet;

$R^{12}$ ist

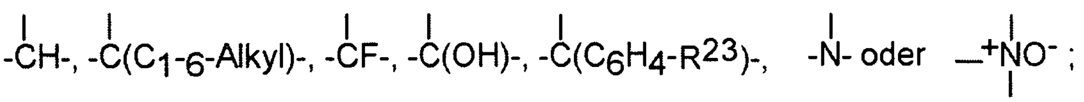

$R^{13}$ und $R^{14}$ sind unabhängig voneinander ausgewählt aus der Gruppe bestehend aus $-CH_2-$, $-CH(C_1-C_6$-Alkyl)-, -C(di-$(C_1-C_6)$-Alykl), -CH=CH- und $-C(C_1-C_6$-Alkyl)=CH-; oder $R^{12}$ zusammen mit einem benachbarten $R^{13}$, oder $R^{12}$ zusammen mit eimen benachbarten $R^{14}$ bilden eine -CH=CH- oder eine $-CH=C(C_1-C_6$-Alkyl)-Gruppe;

a und b sind unabhängig voneinander 0, 1, 2 oder 3, vorausgesetzt es sind nicht beide 0;
vorausgesetzt, dass wenn $R^{13}$ -CH=CH- oder -C($C_1$-$C_6$-Alkyl)=CH- ist, a 1 ist;
vorausgesetzt, dass wenn $R^{14}$ -CH=CH- oder -C($C_1$-$C_6$-Alkyl)=CH- ist, b 1 ist;
vorausgesetzt, dass wenn a 2 oder 3 ist, die $R^{13}$ gleich oder verschieden sein können; und vorausgesetzt, dass wenn b 2 oder 3 ist, die $R^{14}$ gleich oder verschieden sein können;
$R^{10}$ und $R^{11}$ sind unabhängig voneinander ausgewählt aus der Gruppe bestehend aus 1-3 Substituenten, unabhängig voneinander ausgewählt aus der Gruppe bestehend aus ($C_1$-$C_6$)Alkyl, -$OR^{19}$, -O(CO)$R^{19}$, -O(CO)$OR^{21}$, -O$(CH_2)_{1-5}$$OR^{19}$, -O(CO)$NR^{19}R^{20}$, $NR^{19}R^{20}$, -$NR^{19}$(CO)$R^{20}$, -$NR^{19}$(CO)$OR^{21}$, -$NR^{19}$(CO)$NR^{20}R^{25}$, -$NR^{19}SO_2R^{21}$, -$COOR^{19}$, -$CONR^{19}R^{20}$, -$COR^{19}$, -$SO_2NR^{19}R^{20}$, $S(O)_{0-2}R^{21}$, -O$(CH_2)_{1-10}$-$COOR^{19}$, -O$(CH_2)_{1-10}$$CONR^{19}R^{20}$, -($C_1$-$C_6$-Alkylen)-$COOR^{19}$, -CH=CH-$COOR^{19}$, -$CF_3$, -CN, -$NO_2$ und Halogen;
$R^{19}$ und $R^{20}$ sind unabhängig voneinander ausgewählt aus der Gruppe bestehend aus H, ($C_1$-$C_6$)-Alkyl, Aryl und Aryl-substituiertem ($C_1$-$C_6$)-Alkyl;
$R^{21}$ ist ($C_1$-$C_6$)-Alkyl, Aryl oder $R^{24}$-substituiertes Aryl;
$R^{22}$ ist H, ($C_1$-$C_6$)-Alkyl, Aryl($C_1$-$C_6$)-Alkyl, -C(O)$R^{19}$ oder -$COOR^{19}$;
$R^{23}$ und $R^{24}$ sind unabhängig 1-3 Gruppen ausgewählt aus der Gruppe bestehend aus H, ($C_1$-$C_6$)-Alkyl, ($C_1$-$C_6$)-Alkoxy, -COOH, $NO_2$, -$NR^{19}R^{20}$, -OH und Halogen; und
$R^{25}$ ist H, -OH oder ($C_1$-$C_6$)-Alkoxy,

für die Herstellung eines Medikaments für die Verhinderung, Behandlung oder Verbesserung der Symptome der Alzheimer-Krankheit in einem Individuum.

**63.** Verwendung einer Zusammensetzung umfassend zumindest eine Verbindung dargestellt durch die Formel (VIII):

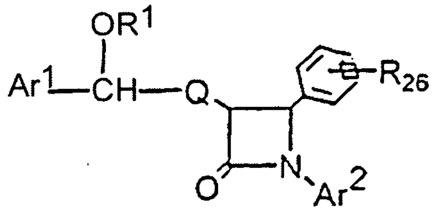

(VIII)

oder ein pharmazeutisch annehmbares Salz oder Solvat davon, wobei

$R^{26}$ ausgewählt ist aus der Gruppe bestehend aus:

e) OH;
f) $OCH_3$;
g) Fluor und
h) Chlor

$R^1$ ausgewählt ist aus der Gruppe bestehend aus H,

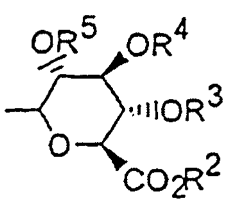 , 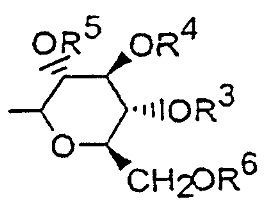 , 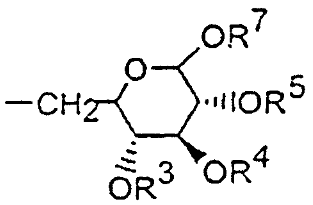 ,

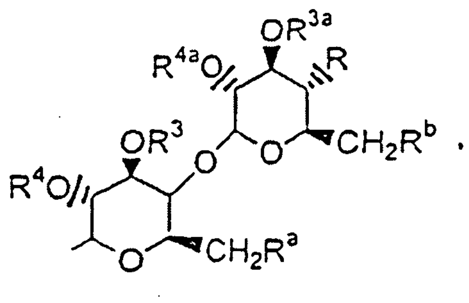

$-SO_3H$; natürlichen und nicht natürlichen Aminosäuren. R, $R^a$ und $R^b$ unabhängig voneinander ausgewählt sind aus der Gruppe bestehend aus H, -OH, Halogen, $-NH_2$, Azid, $(C_1-C_6)$Alkoxy$(C_1-C_6)$-Alkoxy und $-W-R^{30}$;
W ist unabhängig ausgewählt aus der Gruppe bestehend aus -NH-C(O)-, -O-C(O)-, $-O-C(O)-N(R^{31})-$, $-NH-C(O)-N(R^{31})-$ und $-O-C(S)-N(R^{31})-$;
$R^2$ und $R^6$ sind unabhängig voneinander ausgewählt aus der Gruppe bestehend aus H, $(C_1-C_6)$-Alkyl, Aryl und Aryl$(C_1-C_6)$Alkyl;
$R^3$, $R^4$, $R^5$, $R^7$, $R^{3a}$ und $R^{4a}$ sind unabhängig voneinander ausgewählt aus der Gruppe bestehend aus H, $(C_1-C_6)$ Alkyl, Aryl$(C_1-C_6)$Alkyl, $-C(O)(C_1-C_6)$Alkyl und -C(O)Aryl;
$R^{30}$ ist unabhängig ausgewählt aus der Gruppe bestehend aus $R^{32}$-substituiertem T, $R^{32}$-substituiertem T-$(C_1-C_6)$Alkyl, $R^{32}$-substituiertem-$(C_2-C_4)$Alkenyl, $R^{32}$-substiuiertem-$(C_1-C_6)$ Alkyl, $R^{32}$-substituiertem-$(C_3-C_7)$ Cycloalkyl und $R^{32}$-substituiertem-$(C_3-C_7)$ Cycloalkyl $(C_1-C_6)$ Alkyl;
$R^{31}$ ist unabhängig ausgewählt aus der Gruppe bestehend aus H und $(C_1-C_4)$ Alkyl;
T ist unabhängig ausgewählt aus der Gruppe bestehend aus Phenyl, Furyl, Thienyl, Pyrrolyl, Oxazolyl, Isoxazolyl, Thiazolyl, Isothiazolyl, Benzothiazolyl, Thiadiazolyl, Pyrazolyl, Imidazolyl und Pyridyl;
$R^{32}$ ist unabhängig ausgewählt aus 1-3 Substituenten unabhängig voneinander ausgewählt aus der Gruppe bestehend aus H, Halogeno, $(C_1-C_4)$-Alkyl, -OH, Phenoxy, $-CF_3$,$-NO_2$, $(C_1-C_4)$-Alkoxy, Methylendioxy, Oxo, $(C_1-C_4)$-Alkylsulfanyl, $(C_1-C_4)$-Alkylsulfinyl, $(C_1-C_4)$-Alkylsulfonyl, $-N(CH_3)_2$, $-C(O)-NH(C_1-C_4)$-Alkyl, $-C(O)-N((C_1-C_4)Alkyl)_2$, $-C(O)-(C_1-C_4)$-Alkyl, $-C(O)-(C_1-C_4)$-Alkoxy und Pyrrolidinylcarbonyl; oder $R^{32}$ ist eine kovalente Bindung und $R^{31}$, der Stickstoff an den es gebunden ist und $R^{32}$ bilden eine Pyrrolidinyl-, Piperidinyl-, N-Methyl-piperazinyl-, Indolinyl oder Morpholinylgruppe oder eine $(C_1-C_4)$-Alkoxycarbonyl-substituierte Pyrrolidinyl-, Piperidinyl-, N-Methylpiperazinyl-, Indolinyl- oder Morpholinylgruppe;
$Ar^1$ ist Aryl oder $R^{10}$-substituiertes Aryl;
$Ar^2$ ist Aryl oder $R^{11}$-substituiertes Aryl;
Q ist $-(CH_2)_q-$, wobei q 2-6 ist, oder, mit dem Ring-Kohlenstoff an Position 3 des Azetidinons, die Spiro Gruppe

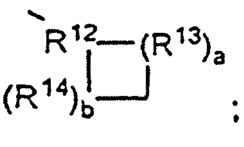

bildet;
$R^{12}$ ist

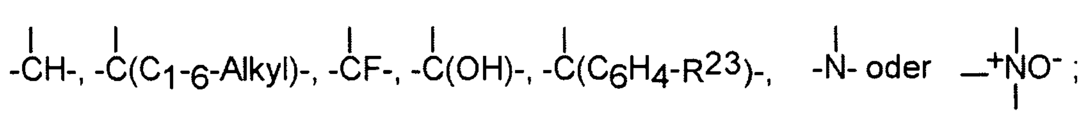

$R^{13}$ und $R^{14}$ sind unabhängig voneinander ausgewählt aus der Gruppe bestehend aus $-CH_2-$, $-CH(C_1-C_6\text{-Alkyl})-$, $-C(di-(C_1-C_6)\text{-Alykl})$, -CH=CH- und $-C(C_1-C_6\text{-Alkyl})=CH-$; oder $R^{12}$ zusammen mit einem benachbarten $R^{13}$, oder $R^{12}$ zusammen mit eimen benachbarten $R^{14}$ bilden eine -CH=CH- oder eine $-CH=C(C_1-C_6\text{-Alkyl})$-Gruppe;
a und b sind unabhängig voneinander 0, 1, 2 oder 3, vorausgesetzt es sind nicht beide 0;
vorausgesetzt, dass wenn $R^{13}$ -CH=CH- oder $-C(C_1-C_6\text{-Alkyl})=CH$ ist, a 1 ist;
vorausgesetzt, dass wenn $R^{14}$ -CH=CH- oder $-C(C_1-C_6\text{-Alkyl})=CH-$ ist, b 1 ist;

vorausgesetzt, dass wenn a 2 oder 3 ist, die $R^{13}$ gleich oder verschieden sein können; und vorausgesetzt, dass wenn b 2 oder 3 ist, die $R^{14}$ gleich oder verschieden sein können;

$R^{10}$ und $R^{11}$ sind unabhängig voneinander ausgewählt aus der Gruppe bestehend aus 1-3 Substituenten, unabhängig voneinander ausgewählt aus der Gruppe bestehend aus ($C_1$-$C_6$)Alkyl, -$OR^{19}$, -O(CO)$R^{19}$, -O(CO)$OR^{21}$, -O$(CH_2)_{1-5}OR^{19}$, -O(CO)$NR^{19}R^{20}$, -$NR^{19}R^{20}$, -$NR^{19}$(CO)$R^{20}$, -$NR^{19}$(CO)$OR^{21}$, -$NR^{19}$(CO)$NR^{20}R^{25}$, -$NR^{19}SO_2R^{21}$, -$COOR^{19}$, -$CONR^{19}R^{20}$, -$COR^{19}$, -$SO_2NR^{19}R^{20}$, $S(O)_{0-2}R^{21}$, -O$(CH_2)_{1-10}$-$COOR^{19}$, -O$(CH_2)_{1-10}CONR^{19}R^{20}$, -($C_1$-$C_{6}$-Alkylen)-$COOR^{19}$,- CH=CH-$COOR^{19}$, -$CF_3$, -CN, -$NO_2$ und Halogen;

$R^{19}$ und $R^{20}$ sind unabhängig voneinander ausgewählt aus der Gruppe bestehend aus H, ($C_1$-$C_6$)-Alkyl, Aryl und Aryl-substituiertem ($C_1$-$C_6$)-Alkyl;

$R^{21}$ ist ($C_1$-$C_6$)-Alkyl, Aryl oder $R^{24}$-substituiertes Aryl;

$R^{22}$ ist H, ($C_1$-$C_6$)-Alkyl, Aryl($C_1$-$C_6$)-Alkyl, -C(O)$R^{19}$ oder -$COOR^{19}$;

$R^{23}$ und $R^{24}$ sind unabhängig 1-3 Gruppen ausgewählt aus der Gruppe bestehend aus H, ($C_1$-$C_6$)-Alkyl, ($C_1$-$C_6$)-Alkoxy, -COOH, $NO_2$, -$NR^{19}R^{20}$, -OH und Halogen; und $R^{25}$ ist H, -OH oder ($C_1$-$C_6$)-Alkoxy,

für die Herstellung eines Medikaments für das Regulieren der Produktion oder des Spiegels von zumindest einem Amyloid β Peptid in dem Individuum.

**64.** Verwendung einer Zusammensetzung umfassend zumindest eine Verbindung dargestellt durch die Formel (VIII):

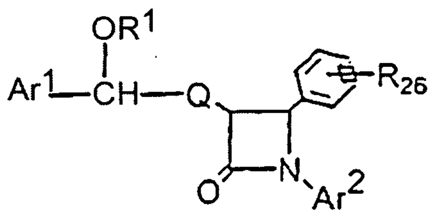

(VIII)

oder ein pharmazeutisch annehmbares Salz oder Solvat davon, wobei

$R^{26}$ ausgewählt ist aus der Gruppe bestehend aus:

i) OH;
j) $OCH_3$;
k) Fluor und
1) Chlor

$R^1$ ausgewählt ist aus der Gruppe bestehend aus H,

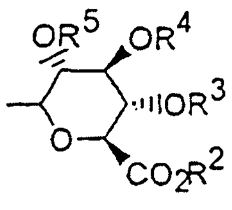
,
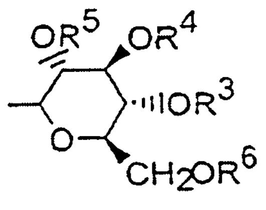
,
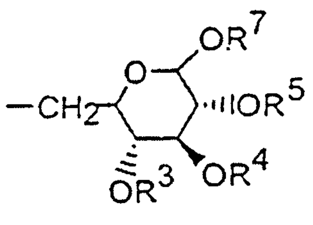
,

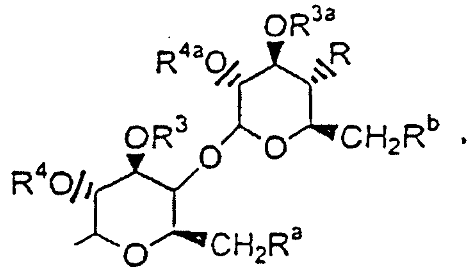

$-SO_3H$; natürlichen und nicht natürlichen Aminosäuren. R, $R^a$ und $R^b$ unabhängig voneinander ausgewählt sind aus der Gruppe bestehend aus H, -OH, Halogen, $-NH_2$, Azid, $(C_1-C_6)$Alkoxy$(C_1-C_6)$-Alkoxy und $-W-R^{30}$;
W ist unabhängig ausgewählt aus der Gruppe bestehend aus -NH-C(O)-, -O-C(O)-, $-O-C(O)-N(R^{31})-$, -NH-C (O)-$N(R^{31})$- und -O-C(S)-$N(R^{31})$-;
$R^2$ und $R^6$ sind unabhängig voneinander ausgewählt aus der Gruppe bestehend aus H, $(C_1-C_6)$-Alkyl, Aryl und Aryl$(C_1-C_6)$Alkyl;
$R^3$, $R^4$, $R^5$, $R^7$, $R^{3a}$ und $R^{4a}$ sind unabhängig voneinander ausgewählt aus der Gruppe bestehend aus H, $(C_1-C_6)$ Alkyl, Aryl$(C_1-C_6)$Alkyl, -C(O)$(C_1-C_6)$Alkyl und -C(O)Aryl;
$R^{30}$ ist unabhängig ausgewählt aus der Gruppe bestehend aus $R^{32}$-substituiertem T, $R^{32}$-substituiertem T-$(C_1-C_6)$Alkyl, $R^{32}$-substituiertem-$(C_2-C_4)$Alkenyl, $R^{32}$-substiuiertem-$(C_1-C_6)$ Alkyl, $R^{32}$-substituiertem-$(C_3-C_7)$ Cycloalkyl und $R^{32}$-substituiertem-$(C_3-C_7)$ Cycloalkyl $(C_1-C_6)$ Alkyl;
$R^{31}$ ist unabhängig ausgewählt aus der Gruppe bestehend aus H und $(C_1-C_4)$ Alkyl;
T ist unabhängig ausgewählt aus der Gruppe bestehend aus Phenyl, Furyl, Thienyl, Pyrrolyl, Oxazolyl, Isoxazolyl, Thiazolyl, Isothiazolyl, Benzothiazolyl, Thiadiazolyl, Pyrazolyl, Imidazolyl und Pyridyl;
$R^{32}$ ist unabhängig ausgewählt aus 1-3 Substituenten unabhängig voneinander ausgewählt aus der Gruppe bestehend aus H, Halogeno, $(C_1-C_4)$-Alkyl, -OH, Phenoxy, $-CF_3$,$-NO_2$, $(C_1-C_4)$-Alkoxy, Methylendioxy, Oxo, $(C_1-C_4)$-Alkylsulfanyl, $(C_1-C_4)$-Alkylsulfinyl, $(C_1-C_4)$-Alkylsulfonyl, $-N(CH_3)_2$, -C(O)-NH$(C_1-C_4)$-Alkyl, -C (O)-N($(C_1-C_4)$Alkyl$)_2$, -C(O)-$(C_1-C_4)$-Alkyl, -C(O)-$(C_1-C_4)$-Alkoxy und Pyrrolidinylcarbonyl; oder $R^{32}$ ist eine kovalente Bindung und $R^{31}$, der Stickstoff an den es gebunden ist und $R^{32}$ bilden eine Pyrrolidinyl-, Piperidinyl-, N-Methyl-piperazinyl-, Indolinyl oder Morpholinylgruppe oder eine $(C_1-C_4)$-Alkoxycarbonyl-substituierte Pyrrolidinyl-, Piperidinyl-, N-Methylpiperazinyl-, Indolinyl- oder Morpholinylgruppe;
$Ar^1$ ist Aryl oder $R^{10}$-substituiertes Aryl;
$Ar^2$ ist Aryl oder $R^{11}$-substituiertes Aryl;
Q ist $-(CH_2)q-$, wobei q 2-6 ist, oder, mit dem Ring-Kohlenstoff an Position 3 des Azetidinons, die Spiro Gruppe

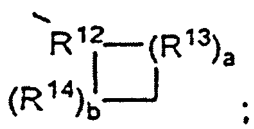

bildet;
$R^{12}$ ist

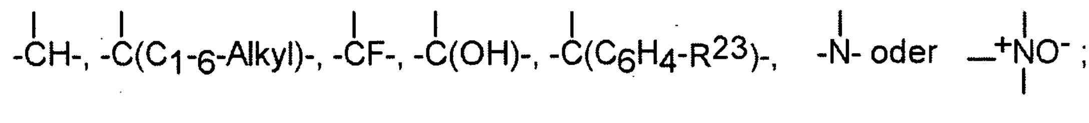

$R^{13}$ und $R^{14}$ sind unabhängig voneinander ausgewählt aus der Gruppe bestehend aus $-CH_2-$, -CH$(C_1-C_6$-Alkyl)-, -C(di-$(C_1-C_6)$-Alykl), -CH=CH- und -C$(C_1-C_6$-Alkyl)=CH-; oder $R^{12}$ zusammen mit einem benachbarten $R^{13}$, oder $R^{12}$ zusammen mit eimen benachbarten $R^{14}$ bilden eine -CH=CH- oder eine -CH=C$(C_1-C_6$-Alkyl)-Gruppe;
a und b sind unabhängig voneinander 0, 1, 2 oder 3, vorausgesetzt es sind nicht beide 0;
vorausgesetzt, dass wenn $R^{13}$ -CH=CH- oder -C$(C_1-C_6$-Alkyl)=CH ist, a 1 ist;
vorausgesetzt, dass wenn $R^{14}$ -CH=CH- oder -C$(C_1-C_6$-Alkyl)=CH- ist, b 1 ist;

vorausgesetzt, dass wenn a 2 oder 3 ist, die $R^{13}$ gleich oder verschieden sein können; und vorausgesetzt, dass wenn b 2 oder 3 ist, die $R^{14}$ gleich oder verschieden sein können;

$R^{10}$ und $R^{11}$ sind unabhängig voneinander ausgewählt aus der Gruppe bestehend aus 1-3 Substituenten, unabhängig voneinander ausgewählt aus der Gruppe bestehend aus $(C_1-C_6)$Alkyl, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $S(O)_{0-2}R^{21}$, $-O(CH_2)_{1-10}-COOR^{19}$, $-O(CH_2)_{1-10}CONR^{19}R^{20}$, $-(C_1-C_{6}$-Alkylen)-$COOR^{19}$, $-CH=CH-COOR^{19}$, $-CF_3$, -CN, $-NO_2$ und Halogen;

$R^{19}$ und $R^{20}$ sind unabhängig voneinander ausgewählt aus der Gruppe bestehend aus H, $(C_1-C_6)$-Alkyl, Aryl und Aryl-substituiertem $(C_1-C_6)$-Alkyl;

$R^{21}$ ist $(C_1-C_6)$-Alkyl, Aryl oder $R^{24}$-substituiertes Aryl;

$R^{22}$ ist H, $(C_1-C_6)$-Alkyl, Aryl$(C_1-C_6)$-Alkyl, $-C(O)R^{19}$ oder $-COOR^{19}$;

$R^{23}$ und $R^{24}$ sind unabhängig 1-3 Gruppen ausgewählt aus der Gruppe bestehend aus H, $(C_1-C_6)$-Alkyl, $(C_1-C_6)$-Alkoxy, -COOH, $NO_2$, $-NR^{19}R^{20}$, -OH und Halogen; und $R^{25}$ ist H, -OH oder $(C_1-C_6)$-Alkoxy,

für die Herstellung eines Medikaments für das Regulieren der Menge von ApoE Isoform 4 im Blutstrom und/oder Hirn des Individuums.

## Revendications

**1.** Emploi d'une composition comprenant au moins un composé représenté par la formule (I) :

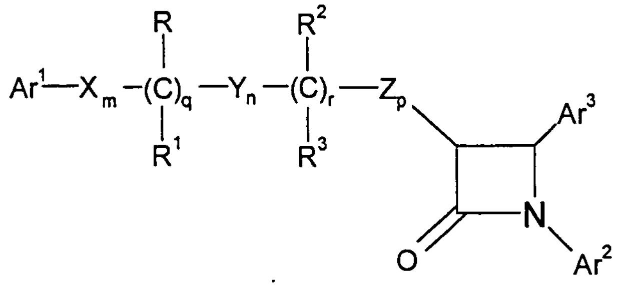

(I)

ou un sel pharmacologiquement admissible d'un tel composé, ou un solvat d'un tel composé ou sel, dans laquelle formule :

$Ar^1$ et $Ar^2$ représentent chacun, indépendamment, un groupe aryle ou un groupe aryle à substituant(s) $R^4$ ;

$Ar^3$ représente un groupe aryle ou un groupe aryle à substituant(s) $R^5$ ;

X, Y et Z représentent chacun, indépendamment, un chaînon $-CH_2-$, -CH(alkyle inférieur)- ou -C(alkyle inférieur)$_2$- ;

R et $R^2$ représentent chacun, indépendamment, un groupe de formule $-OR^6$, $-O(CO)R^6$, $-O(CO)OR^9$ ou $-O(CO)NR^6R^7$ ;

$R^1$ et $R^3$ représentent chacun, indépendamment, un atome d'hydrogène ou un groupe alkyle inférieur ou aryle ;

q vaut 0 ou 1 ;

r vaut 0 ou 1 ;

m, n et p valent chacun, indépendamment, 0, 1, 2, 3 ou 4 ;

sous réserve qu'au moins l'un des indices q et r vaille 1, et que la somme des indices m, n, p, q et r vaille 1, 2, 3, 4, 5 ou 6 ;

et sous réserve que, si p vaut 0 et r vaut 1, la somme des indices m, n et q vaille 1, 2, 3, 4 ou 5 ;

$R^4$ représente 1 à 5 substituants choisis indépendamment dans l'ensemble constitué par les groupes alkyle inférieur, les groupes de formules $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$,- $O(CH_2)_{1-10}CONR^6R^7$, -(alkylène inférieur)-$COOR^6$, $-CH=CH-COOR^6$, $-CF_3$, -CN et $NO_2$, et les atomes d'halogène ;

$R^5$ représente 1 à 5 substituants choisis indépendamment dans l'ensemble constitué par les groupes de formules $-OR^6$ $-O(CO)R^6$, $-O(CO)OR^9$, $-O(CH_2)_{1-5}OR^6$, $-O(CO)NR^6R^7$ $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, -(alkylène inférieur)-$COOR^6$ et $-CH=CH-COOR^6$;

$R^6$, $R^7$ et $R^8$ représentent chacun, indépendamment, un atome d'hydrogène ou un groupe alkyle inférieur, aryle, ou alkyle inférieur à substituant aryle; et $R^9$ représente un groupe alkyle inférieur, aryle, ou alkyle inférieur à substituant aryle ;

en vue de la fabrication d'un médicament destiné au traitement, à la prévention ou à l'amélioration des symptômes de la maladie d'Alzheimer chez un sujet.

**2.** Emploi conforme à la revendication 1, le sujet ne présentant pas de symptômes de la maladie d'Alzheimer.

**3.** Emploi conforme à la revendication 1, le sujet étant atteint de la maladie d'Alzheimer.

**4.** Emploi conforme à la revendication 1, le sujet ayant des antécédents familiaux de maladie d'Alzheimer ou de démence.

**5.** Emploi conforme à la revendication 1, le sujet étant un sujet humain atteint de trisomie 21 (syndrome de Down).

**6.** Emploi conforme à la revendication 1, le sujet étant un sujet humain.

**7.** Emploi conforme à la revendication 6, le sujet humain portant une ou plusieurs mutations dans les gènes qui codent la protéine précurseur de β-amyloïde, la préséniline-1 ou la préséniline-2.

**8.** Emploi conforme à la revendication 6, le sujet humain portant le gène d'apolipoprotéine E4.

**9.** Emploi conforme à la revendication 1, le sujet présentant un taux élevé de peptides β-amyloïdes dans le sang et/ou le cerveau.

**10.** Emploi conforme à la revendication 9, le sujet présentant un taux élevé de peptide β-amyloïde 42 dans le sang et/ou le cerveau.

**11.** Emploi conforme à la revendication 9, le taux de peptides β-amyloïdes (Aβ) dans le sang étant réduit de 10 à 100 %, par rapport au taux de peptides β-amyloïdes observé avant l'administration de la composition.

**12.** Emploi conforme à la revendication 9, le sujet présentant un taux sanguin de peptide β-amyloïde Aβ-42 supérieur à 30 pM.

**13.** Emploi conforme à la revendication 12, le sujet présentant un taux sanguin de peptide β-amyloïde Aβ-42 supérieur à 40 pM.

**14.** Emploi conforme à la revendication 12, le sujet présentant un taux sanguin de peptide β-amyloïde Aβ-42 de 30 à 80 pM.

**15.** Emploi conforme à la revendication 9, le sujet présentant un taux sanguin de peptide β-amyloïde Aβ-40 supérieur à 200 pM.

**16.** Emploi conforme à la revendication 15, le sujet présentant un taux sanguin de peptide β-amyloïde Aβ-40 supérieur à 400 pM.

**17.** Emploi conforme à la revendication 15, le sujet présentant un taux sanguin de peptide β-amyloïde Aβ-40 de 200 à 800 pM.

**18.** Emploi conforme à la revendication 9, le sujet présentant un taux cérébral de peptide β-amyloïde Aβ-42 supérieur à 50 pmol par gramme de tissu cérébral humide.

**19.** Emploi conforme à la revendication 9, le sujet présentant un taux cérébral de peptide β-amyloïde Aβ-40 supérieur à 10 pmol par gramme de tissu cérébral humide.

**20.** Emploi conforme à la revendication 1, le sujet présentant un taux sanguin élevé de cholestérol.

**21.** Emploi conforme à la revendication 1, le sujet présentant un taux sérique total de cholestérol d'au moins 200 mg/dl.

**22.** Emploi conforme à la revendication 1, le sujet présentant un taux total de LDL-cholestérol supérieur à 100 mg/dl.

**23.** Emploi conforme à la revendication 6, le sujet humain étant âgé de plus d'environ 40 ans.

**24.** Emploi conforme à la revendication 7, le sujet humain étant âgé de plus d'environ 60 ans.

**25.** Emploi conforme à la revendication 1, dans lequel le composé est représenté par la formule (II) suivante :

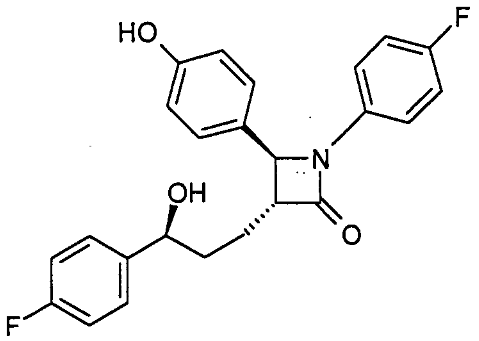

(II)

**26.** Emploi conforme à la revendication 1, dans lequel le composé est représenté par la formule (VIa) suivante :

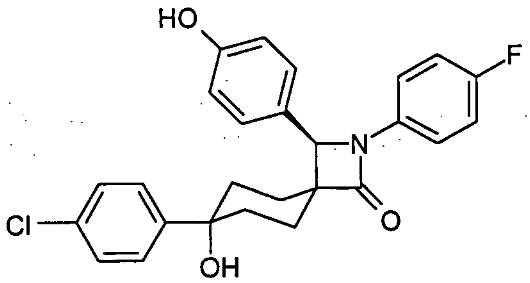

(VIa)

**27.** Emploi conforme à la revendication 1, dans lequel le composé est administré au sujet en une quantité de 0,1 à 1000 mg de composé par jour.

**28.** Emploi conforme à la revendication 1, dans lequel la composition comprend en outre au moins un inhibiteur de biosynthèse de cholestérol.

**29.** Emploi conforme à la revendication 28, dans lequel ledit inhibiteur de biosynthèse de cholestérol au nombre d'au moins un comprend au moins un inhibiteur de l'HMG-CoA réductase.

**30.** Emploi conforme à la revendication 29, dans lequel ledit inhibiteur de l'HMG-CoA réductase au nombre d'au moins un est choisi parmi les lovastatine, pravastatine, fluvastatine, simvastatine, atorvastatine, cérivastatine et rosuvastatine, ainsi que leurs mélanges.

**31.** Emploi conforme à la revendication 30, dans lequel ledit inhibiteur de l'HMG-CoA réductase au nombre d'au moins un est la simvastatine.

**32.** Emploi conforme à la revendication 1, dans lequel la composition comprend en outre au moins un dérivé d'acide fibrique.

**33.** Emploi conforme à la revendication 1, dans lequel la composition comprend en outre au moins un séquestrant pour acide biliaire.

**34.** Emploi conforme à la revendication 1, dans lequel la composition comprend en outre de l'acide nicotinique ou de l'un de ses dérivés.

**35.** Emploi conforme à la revendication 1, dans lequel la composition comprend en outre au moins un inhibiteur de l'acyl-CoA:cholestérol O-acyl-transférase.

**36.** Emploi conforme à la revendication 1, dans lequel la composition comprend en outre du probucol ou de l'un de ses dérivés.

**37.** Emploi conforme à la revendication 1, dans lequel la composition comprend en outre au moins un activateur des récepteurs de lipoprotéines basse densité.

**38.** Emploi conforme à la revendication 1, dans lequel la composition comprend en outre au moins un acide gras oméga-3.

**39.** Emploi conforme à la revendication 1, dans lequel la composition comprend en outre des fibres hydrosolubles naturelles d'au moins un type.

**40.** Emploi conforme à la revendication 1, dans lequel la composition comprend en outre au moins l'un des stérols végétaux, stanols végétaux et esters d'acide gras des stanols végétaux.

**41.** Emploi conforme à la revendication 1, dans lequel la composition comprend en outre au moins un antioxydant ou une vitamine.

**42.** Emploi conforme à la revendication 1, dans lequel la composition comprend en outre au moins un agent de traitement de la maladie d'Alzheimer différent du composé de formule I défini plus haut.

**43.** Emploi conforme à la revendication 1, dans lequel la composition comprend en outre au moins un agent de traitement de la maladie d'Alzheimer différent du composé de formule I défini plus haut, choisi dans l'ensemble formé par les inhibiteurs de cholinestérase, antagonistes de récepteurs muscariniques, antagonistes des récepteurs muscariniques M2, stimulants de libération d'acétylcholine, stimulants de capture de choline, antagonistes des récepteurs cholinergiques nicotiniques, vaccins anti-Aβ, inhibiteurs de γ-secrétase, inhibiteurs de β-secrétase, inhibiteurs d'agrégation de plaque amyloïde, oligonucléotides anti-sens de protéine précurseur de peptide amyloïde, inhibiteurs de recapture de monoamine, cellules souches humaines, agents de thérapie génique, agents nootropes, ligands de récepteurs d'AMPA, facteurs de croissance ou agonistes de récepteurs de facteurs de croissance, agents anti-inflammatoires, anti-oxydants / pièges à radicaux libres, stimulants de superoxyde dismutase, agents bloquant les canaux calciques, inhibiteurs d'apoptose, inhibiteurs de caspase, inhibiteurs de monoamine oxydase, oestrogènes, antagonistes de récepteurs NMDA, inhibiteurs de JNK (Jun N-terminal Kinase), agents chélatants cuivre/zinc, agonistes de récepteurs 5-HT1a, stimulants de NGF, agents neuroprotecteurs, antagonistes des récepteurs H3 d'histamine, inhibiteurs de calpaïne, inhibiteurs de poly-ADP-ribose polymérase, inhibiteurs de prolyl-endopeptidase, modulateurs des canaux calciques, antagonistes de facteurs de libération de corticotropine, inhibiteurs de protéines se liant à des facteurs de libération de corticotropine, modulateurs de GABA, antagonistes de récepteurs GABA-A, antagonistes de récepteurs GABA-B, ligands de neuroimmunophiline, ligands de récepteurs sigma, ligands de récepteurs de galanine, antagonistes de récepteurs adrénergiques alpha / imidazoline, agonistes de récepteurs du peptide intestinal vasoactif, agonistes inverses de benzodiazépines, agonistes de récepteurs de cannabinoïdes, agonistes de récepteurs de l'hormone de libération de thyréostimuline, inhibiteurs de protéine kinase C, antagonistes de récepteurs 5-HT3, antagonistes de récepteurs de prostaglandines, inhibiteurs de topoisomérase II, agonistes de récepteurs de stéroïdes, antagonistes de récepteurs de corticostéroïdes, modulateurs de l'oxyde nitrique, inhibiteurs de récepteurs RAGE, et agonistes des récepteurs de dopamine, ainsi que les combinaisons de tels agents.

**44.** Emploi d'une composition comprenant du composé représenté par la formule suivante :

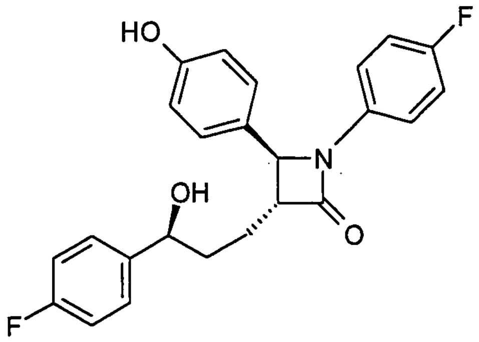

(II)

en vue de la fabrication d'un médicament destiné au traitement, à la prévention ou à l'amélioration des symptômes de la maladie d'Alzheimer chez un sujet.

**45.** Emploi d'une composition comprenant au moins un composé représenté par la formule (I) :

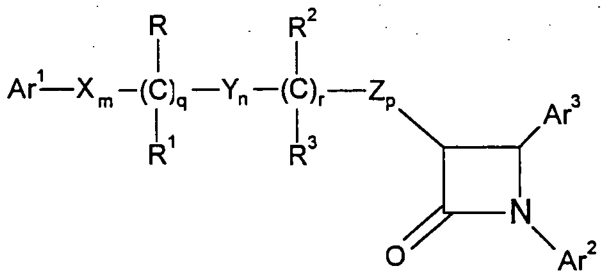

(I)

ou un sel pharmacologiquement admissible d'un tel composé, ou un solvat d'un tel composé ou sel, dans laquelle formule :

$Ar^1$ et $Ar^2$ représentent chacun, indépendamment, un groupe aryle ou un groupe aryle à substituant(s) $R^4$ ;
$Ar^3$ représente un groupe aryle ou un groupe aryle à substituant(s) $R^5$ ;
X, Y et Z représentent chacun, indépendamment, un chaînon $-CH_2-$, -CH(alkyle inférieur)- ou -C(alkyle inférieur)$_2$- ;
R et $R^2$ représentent chacun, indépendamment, un groupe de formule $-OR^6$, $-O(CO)R^6$, $-O(CO)OR^9$ ou $-O(CO)NR^6R^7$;
$R^1$ et $R^3$ représentent chacun, indépendamment, un atome d'hydrogène ou un groupe alkyle inférieur ou aryle ;
q vaut 0 ou 1 ;
r vaut 0 ou 1 ;
m, n et p valent chacun, indépendamment, 0, 1, 2, 3 ou 4 ;
sous réserve qu'au moins l'un des indices q et r vaille 1, et que la somme des indices m, n, p, q et r vaille 1, 2, 3, 4, 5 ou 6 ;
et sous réserve que, si p vaut 0 et r vaut 1, la somme des indices m, n et q vaille 1, 2, 3, 4 ou 5 ;
$R^4$ représente 1 à 5 substituants choisis indépendamment dans l'ensemble constitué par les groupes alkyle inférieur, les groupes de formules $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, -(alkylène inférieur)-$COOR^6$, $-CH=CH-COOR^6$, $-CF_3$, -CN et $NO_2$, et les atomes d'halogène ;
$R^5$ représente 1 à 5 substituants choisis indépendamment dans l'ensemble constitué par les groupes de formules $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, -(alkylène inférieur)-$COOR^6$ et $-CH=CH-COOR^6$ ;
$R^6$, $R^7$ et $R^8$ représentent chacun, indépendamment, un atome d'hydrogène ou un groupe alkyle inférieur, aryle, ou alkyle inférieur à substituant aryle ; et $R^9$ représente un groupe alkyle inférieur, aryle, ou alkyle inférieur à substituant aryle ;

en vue de la fabrication d'un médicament conçu pour réguler la production ou le taux d'au moins un peptide β-amyloïde chez un sujet.

**46.** Emploi d'une composition comprenant au moins un composé représenté par la formule (I) :

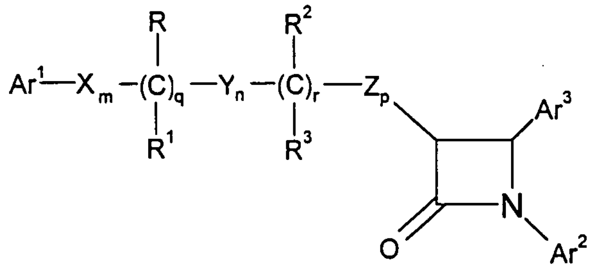

(I)

ou un sel pharmacologiquement admissible d'un tel composé, ou un solvat d'un tel composé ou sel, dans laquelle formule :

$Ar^1$ et $Ar^2$ représentent chacun, indépendamment, un groupe aryle ou un groupe aryle à substituant(s) $R^4$ ;
$Ar^3$ représente un groupe aryle ou un groupe aryle à substituant(s) $R^5$ ; X, Y et Z représentent chacun, indépendamment, un chaînon $-CH_2-$, -CH(alkyle inférieur)- ou -C(alkyle inférieur)$_2$- ;
R et $R^2$ représentent chacun, indépendamment, un groupe de formule $-OR^6$, $-O(CO)R^6$, $-O(CO)OR^9$ ou $-O(CO)NR^6R^7$ ;
$R^1$ et $R^3$ représentent chacun, indépendamment, un atome d'hydrogène ou un groupe alkyle inférieur ou aryle;
q vaut 0 ou 1 ;
r vaut 0 ou 1 ;
m, n et p valent chacun, indépendamment, 0, 1, 2, 3 ou 4 ;
sous réserve qu'au moins l'un des indices q et r vaille 1, et que la somme des indices m, n, p, q et r vaille 1, 2, 3, 4, 5 ou 6 ;
et sous réserve que, si p vaut 0 et r vaut 1, la somme des indices m, n et q vaille 1, 2, 3, 4 ou 5 ;
$R^4$ représente 1 à 5 substituants choisis indépendamment dans l'ensemble constitué par les groupes alkyle inférieur, les groupes de formules $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, -(alkylène inférieur)-$COOR^6$, $-CH=CH-COOR^6$, $-CF_3$, -CN et $NO_2$, et les atomes d'halogène ;
$R^5$ représente 1 à 5 substituants choisis indépendamment dans l'ensemble constitué par les groupes de formules $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, -(alkylène inférieur)-$COOR^6$ et $-CH=CH-COOR^6$ ;
$R^6$, $R^7$ et $R^8$ représentent chacun, indépendamment, un atome d'hydrogène ou un groupe alkyle inférieur, aryle, ou alkyle inférieur à substituant aryle ;
et $R^9$ représente un groupe alkyle inférieur, aryle, ou alkyle inférieur à substituant aryle ;

en vue de la fabrication d'un médicament conçu pour réguler la quantité de l'isoforme 4 de l'apolipoprotéine E dans la circulation sanguine et/ou le cerveau d'un sujet.

**47.** Emploi d'une composition comprenant au moins un composé représenté par la formule (III) :

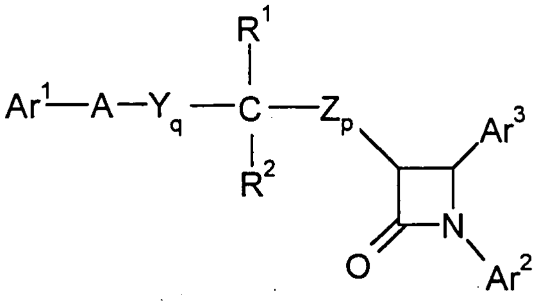

(III)

ou un sel pharmacologiquement admissible d'un tel composé, ou un solvat d'un tel composé ou sel, dans laquelle formule (III) :

$Ar^1$ représente un groupe aryle à substituant(s) $R^3$ ;
$Ar^2$ représente un groupe aryle à substituant(s) $R^4$ ;
$Ar^3$ représente un groupe aryle à substituant(s) $R^5$ ;
Y et Z représentent chacun, indépendamment, un chaînon $-CH_2-$, -CH(alkyle inférieur)- ou $-C(\text{alkyle inférieur})_2-$ ;
A représente un chaînon -O-, -S-, -S(O)- ou $-S(O)_2-$ ;
$R^1$ représente un groupe de formule $-OR^6$, $-O(CO)R^6$, $-O(CO)OR^9$ ou $-O(CO)NR^6R^7$ ;
$R^2$ représente un atome d'hydrogène ou un groupe alkyle inférieur ou aryle ;
ou $R^1$ et $R^2$ représentent ensemble un substituant oxo =O ;
q vaut 1, 2 ou 3 ;
p vaut 0, 1, 2, 3 ou 4 ;
$R^5$ représente 1 à 3 substituants choisis indépendamment dans l'ensemble constitué par les groupes de formules $-OR^6$, $-O(CO)R^6$, $-O(CO)OR^9$, $-O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-(alkyle inférieur), $-NR^6SO_2$-(aryle), $-CONR^6R^7$, $-COR^6$, $-SO_2NR^6R^7$, $-S(O)_{0-2}$-(alkyle), $-S(O)_{0-2}$-(aryle), $-O(CH_2)_{1-10}COOR^6$, $-O(CH_2)_{1-10}CONR^6R^7$, -(alkylène inférieur)-$COOR^6$, $-CH=CH-COOR^6$, les atomes d'halogène en position ortho ou méta, et les groupes alkyle inférieur en position ortho ou méta ;
$R^3$ et $R^4$ représentent chacun, indépendamment, 1 à 3 substituants choisis indépendamment parmi un atome d'hydrogène, les substituants mentionnés à propos de $R^5$, les atomes d'halogène en position para, les groupes alkyle inférieur en position para, les groupes aryle, et les groupes $-NO_2$ et $-CF_3$ ; $R^6$, $R^7$ et $R^8$ représentent chacun, indépendamment, un atome d'hydrogène ou un groupe alkyle inférieur, aryle, ou alkyle inférieur à substituant aryle ;
et $R^9$ représente un groupe alkyle inférieur, aryle, ou alkyle inférieur à substituant aryle ;

en vue de la fabrication d'un médicament destiné au traitement, à la prévention ou à l'amélioration des symptômes de la maladie d'Alzheimer chez un sujet.

**48.** Emploi d'une composition comprenant au moins un composé représenté par la formule (III) :

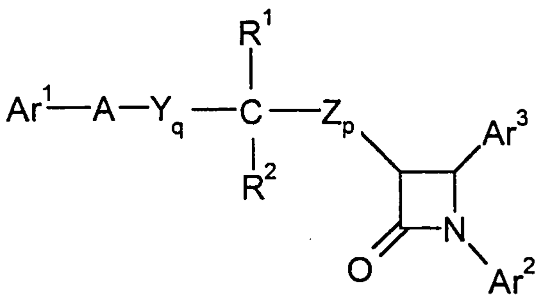

(III)

ou un sel pharmacologiquement admissible d'un tel composé, ou un solvat d'un tel composé ou sel, dans laquelle formule (III) :

$Ar^1$ représente un groupe aryle à substituant(s) $R^3$ ;
$Ar^2$ représente un groupe aryle à substituant(s) $R^4$ ;
$Ar^3$ représente un groupe aryle à substituant(s) $R^5$ ;
Y et Z représentent chacun, indépendamment, un chaînon $-CH_2-$, -CH(alkyle inférieur)- ou -C(alkyle inférieur)$_2$- ;
A représente un chaînon -O-, -S-, -S(O)- ou $-S(O)_2-$ ;
$R^1$ représente un groupe de formule $-OR^6$, $-O(CO)R^6$, $-O(CO)OR^9$ ou $-O(CO)NR^6R^7$ ;
$R^2$ représente un atome d'hydrogène ou un groupe alkyle inférieur ou aryle ; ou $R^1$ et $R^2$ représentent ensemble un substituant oxo =O ;
q vaut 1, 2 ou 3 ;
p vaut 0, 1, 2, 3 ou 4 ;
$R^5$ représente 1 à 3 substituants choisis indépendamment dans l'ensemble constitué par les groupes de formules $-OR^6$, $-O(CO)R^6$, $-O(CO)OR^9$, $-O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-(alkyle inférieur), $-NR^6SO_2$-(aryle), $-CONR^6R^7$, $-COR^6$, $-SO_2NR^6R^7$, $-S(O)_{0-2}$-(alkyle), $-S(O)_{0-2}$-(aryle), $-O(CH_2)_{1-10}COOR^6$, $-O(CH_2)_{1-10}CONR^6R^7$, -(alkylène inférieur)-$COOR^6$, $-CH=CH-COOR^6$, les atomes d'halogène en position ortho ou méta, et les groupes alkyle inférieur en position ortho ou méta ;
$R^3$ et $R^4$ représentent chacun, indépendamment, 1 à 3 substituants choisis indépendamment parmi un atome d'hydrogène, les substituants mentionnés à propos de $R^5$, les atomes d'halogène en position para, les groupes alkyle inférieur en position para, les groupes aryle, et les groupes $-NO_2$ et $-CF_3$ ;
$R^6$, $R^7$ et $R^8$ représentent chacun, indépendamment, un atome d'hydrogène ou un groupe alkyle inférieur, aryle, ou alkyle inférieur à substituant aryle;
et $R^9$ représente un groupe alkyle inférieur, aryle, ou alkyle inférieur à substituant aryle ;

en vue de la fabrication d'un médicament conçu pour réguler la production ou le taux d'au moins un peptide β-amyloïde chez un sujet.

**49.** Emploi d'une composition comprenant au moins un composé représenté par la formule (III) :

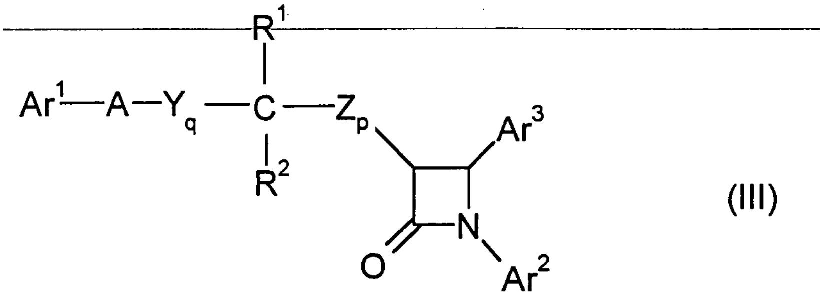

(III)

ou un sel pharmacologiquement admissible d'un tel composé, ou un solvat d'un tel composé ou sel, dans laquelle formule (III) :

$Ar^1$ représente un groupe aryle à substituant(s) $R^3$ ;
$Ar^2$ représente un groupe aryle à substituant(s) $R^4$ ;
$Ar^3$ représente un groupe aryle à substituant(s) $R^5$ ;
Y et Z représentent chacun, indépendamment, un chaînon $-CH_2-$, -CH(alkyle inférieur)- ou -C(alkyle inférieur)$_2$- ;
A représente un chaînon -O-, -S-, -S(O)- ou $-S(O)_2-$ ;
$R^1$ représente un groupe de formule $-OR^6$, $-O(CO)R^6$, $-O(CO)OR^9$ ou $-O(CO)NR^6R^7$ ;
$R^2$ représente un atome d'hydrogène ou un groupe alkyle inférieur ou aryle ; ou $R^1$ et $R^2$ représentent ensemble un substituant oxo =O ;
q vaut 1, 2 ou 3 ;
p vaut 0, 1, 2, 3 ou 4 ;
$R^5$ représente 1 à 3 substituants choisis indépendamment dans l'ensemble constitué par les groupes de formules $-OR^6$, $-O(CO)R^6$, $-O(CO)OR^9$, $-O(CH_2)_{1-5}OR^9$, $-O(CO)NR^6R^7$, $-NR^6R^7$, $-NR^6(CO)R^7$, $-NR^6(CO)OR^9$, $-NR^6(CO)NR^7R^8$, $-NR^6SO_2$-(alkyle inférieur), $-NR^6SO_2$-(aryle), $-CONR^6R^7$, $-COR^6$, $-SO_2NR^6R^7$, $-S(O)_{0-2}$-(alkyle), $-S(O)_{0-2}$-(aryle), $-O(CH_2)_{1-10}COOR^6$, $-O(CH_2)_{1-10}CONR^6R^7$, -(alkylène inférieur)-$COOR^6$, $-CH=CH-COOR^6$, les atomes d'halogène en position ortho ou méta, et les groupes alkyle inférieur en position ortho ou méta ;
$R^3$ et $R^4$ représentent chacun, indépendamment, 1 à 3 substituants choisis indépendamment parmi un atome

d'hydrogène, les substituants mentionnés à propos de $R^5$, les atomes d'halogène en position para, les groupes alkyle inférieur en position para, les groupes aryle, et les groupes $-NO_2$ et $-CF_3$ ; $R^6$, $R^7$ et $R^8$ représentent chacun, indépendamment, un atome d'hydrogène ou un groupe alkyle inférieur, aryle, ou alkyle inférieur à substituant aryle;

et $R^9$ représente un groupe alkyle inférieur, aryle, ou alkyle inférieur à substituant aryle ;

en vue de la fabrication d'un médicament conçu pour réguler la quantité de l'isoforme 4 de l'apolipoprotéine E dans la circulation sanguine et/ou le cerveau d'un sujet.

**50.** Emploi d'une composition comprenant au moins un composé représenté par la formule (IV) :

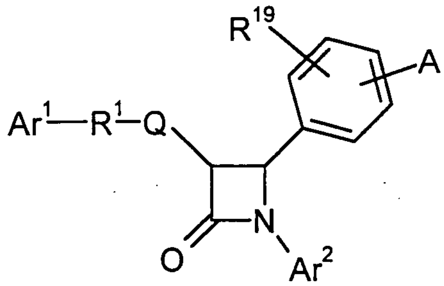

(IV)

ou un sel pharmacologiquement admissible d'un tel composé, ou un solvat d'un tel composé ou sel, dans laquelle formule (IV) :

A représente une entité choisie parmi un groupe hétérocycloalkyle à substituant(s) $R^2$, un groupe hétéroaryle à substituant(s) $R^2$, un groupe hétérocycloalkyle benzocondensé à substituant(s) $R^2$, et un groupe hétéroaryle benzocondensé à substituant(s) $R^2$ ;

$Ar^1$ représente un groupe aryle ou aryle à substituant(s) $R^3$ ;

$Ar^2$ représente un groupe aryle ou aryle à substituant(s) $R^4$ ;

Q représente une liaison ou un groupe formant, avec l'atome de carbone en position 3 du cycle azétidinone, un groupe spiro de formule

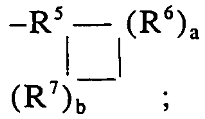

;

$R^1$ représente un groupe choisi parmi les suivants :

- $(CH_2)_q$- où q vaut de 2 à 6, et peut aussi valoir 0 ou 1 si Q représente un groupe spiro,
- $(CH_2)_e$-G-$(CH_2)_r$- où G représente un groupe phénylène ou un chaînon
- O-, -C(O)-, $-NR^8-$ ou $-S(O)_{0-2}-$, e vaut de 0 à 5 et r vaut de 0 à 5, sous réserve que la somme e+r vaille de 1 à 6, alcénylène en $C_{2-6}$,

et $-(CH_2)_f-V-(CH_2)_g-$ où V représente un groupe cycloalkylène en $C_{3-6}$, f vaut de 1 à 5 et g vaut de 0 à 5, sous réserve que la somme f+g vaille de 1 à 6 ;

$R^5$ représente un chaînon choisi parmi :

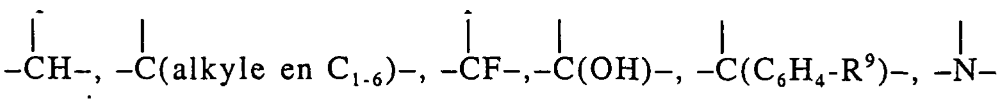

et

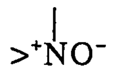 ;

$R^6$ et $R^7$ représentent chacun, indépendamment, un chaînon $-CH_2-$, -CH(alkyle en $C_{1-6}$)- ou -C(alkyle en $C_{1-6}$)$_2$-, ou un groupe -CH=CH- ou -C(alkyle en $C_{1-6}$)=CH- ;
ou bien l'entité représentée par $R^5$ constitue, conjointement avec une entité adjacente représentée par $R^6$ ou par $R^7$, un groupe -CH=CH- ou -C(alkyle en $C_{1-6}$)=CH- ;
a et b valent chacun, indépendamment, 0, 1, 2 ou 3, sous réserve qu'ils ne vaillent pas tous deux 0, que a vaille 1 si $R^6$ représente un groupe -CH=CH- ou -C(alkyle en $C_{1-6}$)=CH-, et que b vaille 1 si $R^7$ représente un groupe -CH=CH- ou -C(alkyle en $C_{1-6}$)=CH-, et étant entendu que, si a vaut 2 ou 3, les entités représentées par les symboles $R^6$ peuvent être identiques ou différentes, et que, si b vaut 2 ou 3, les entités représentées par les symboles $R^7$ peuvent être identiques ou différentes ;
étant entendu que, si Q représente une liaison, $R^1$ peut aussi représenter un groupe de formule

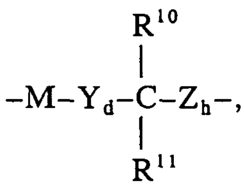, 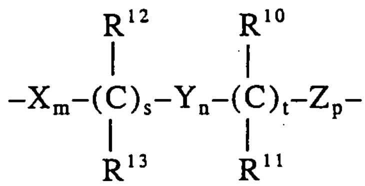 ou 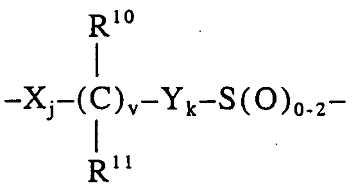

où M représente un chaînon -O-, -S-, -S(O)- ou $-S(O)_2-$ ;
X, Y et Z représentent chacun, indépendamment, un chaînon $-CH_2-$, -CH(alkyle inférieur)- ou -C(alkyle inférieur)$_2$- ;
$R^{10}$ et $R^{12}$ représentent chacun, indépendamment, un groupe de formule $-OR^{14}$, $-O(CO)R^{14}$, $-O(CO)OR^{16}$ ou $-O(CO)NR^{14}R^{15}$ ;
$R^{11}$ et $R^{13}$ représentent chacun, indépendamment, un atome d'hydrogène ou un groupe alkyle en $C_{1-6}$ ou aryle ;
ou $R^{10}$ et $R^{11}$ représentent ensemble un substituant oxo =O ;
ou $R^{12}$ et $R^{13}$ représentent ensemble un substituant oxo =O ;
d vaut 1, 2 ou 3 ;
h vaut 0, 1, 2, 3 ou 4 ;
s vaut 0 ou 1, t vaut 0 ou 1, et m, n et p valent chacun, indépendamment, de 0 à 4, sous réserve qu'au moins l'un de s et t vaille 1 et que la somme m+n+p+s+t vaille de 1 à 6, sous réserve que, si p vaut 0 et t vaut 1, la somme m+s+n vaille de 1 à 5, et sous réserve que, si p vaut 0 et s vaut 1, la somme m+t+n vaille de 1 à 5 ;
v vaut 0 ou 1 ;
j et k valent chacun, indépendamment, de 1 à 5, sous réserve que la somme j+k+v vaille de 1 à 5 ;
$R^2$ représente 1 à 3 substituants qui, s'ils sont placés sur des atomes de carbone de cycle, sont choisis parmi les atomes d'hydrogène et d'halogène et les groupes alkyle en $C_{1-10}$, alcényle en $C_{2-10}$, alcynyle en $C_{2-10}$, cycloalkyle en $C_{3-6}$, cycloalcényle en $C_{3-6}$, aryle à substituant(s) $R^{17}$, benzyle à substituant(s) $R^{17}$, aryloxy à substituant(s) $R^{17}$, benzyloxy à substituant(s) $R^{17}$, $-NR^{14}R^{15}$, -(alkylène en $C_{1-6}$)-$NR^{14}R^{15}$, -(alkylène en $C_{1-6}$)-C(O)-$NR^{14}R^{15}$, $-NHC(O)R^{16}$, -OH, alcoxy en $C_{1-6}$, $-OC(O)R^{16}$, $-COR^{14}$, hydroxyalkyle en $C_{1-6}$, (alcoxy en $C_{1-6}$)-alkyle en $C_{1-6}$, $-NO_2$, $-S(O)_{0-2}R^{16}$, $-SO_2NR^{14}R^{15}$ et -(alkylène en $C_{1-6}$)-$COOR^{14}$, étant entendu que, quand $R^2$ représente un substituant placé sur un groupe hétérocycloalkyle, il s'agit de l'un des substituants indiqués ci-dessus ou d'un substituant oxo =O ou

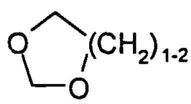 ,

et que, quand $R^2$ représente un substituant placé sur un atome d'azote qui peut en porter, il s'agit d'un atome d'hydrogène ou d'un groupe alkyle en $C_{1-6}$, aryle, alcoxy en $C_{1-6}$, aryloxy, (alkyle en $C_{1-6}$)carbonyle, arylcarbonyle, hydroxy, $-(CH_2)_{1-6}C(O)NR^{18}R^{18}$,

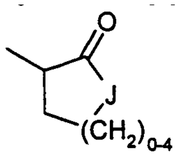

ou

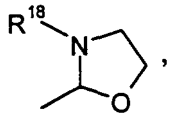

où J représente un chaînon -O-, -NH-, -$NR^{18}$- ou -$CH_2$- ;

$R^3$ et $R^4$ représentent chacun, indépendamment, 1 à 3 substituants choisis indépendamment dans l'ensemble constitué par les groupes alkyle en $C_{1-6}$, -$OR^{14}$, -$O(CO)R^{14}$, -$O(CO)OR^{16}$, -$O(CH_2)_{1-5}OR^{14}$, -$O(CO)NR^{14}R^{15}$, -$NR^{14}R^{15}$, -$NR^{14}(CO)R^{15}$, -$NR^{14}(CO)OR^{16}$, -$NR^{14}(CO)NR^{15}R^{19}$, -$NR^{14}SO_2R^{16}$, -$COOR^{14}$, -$CONR^{14}R^{15}$, -$COR^{14}$, -$SO_2NR^{14}R^{15}$, -$S(O)_{0-2}R^{16}$, -$O(CH_2)_{1-10}COOR^{14}$, -$O(CH_2)_{1-10}CONR^{14}R^{15}$, -(alkylène en $C_{1-6}$)-$COOR^{14}$, -CH=CH-$COOR^{14}$, -$CF_3$, -CN et -$NO_2$, et les atomes d'halogène ;

$R^8$ représente un atome d'hydrogène ou un groupe alkyle en $C_{1-6}$, aryl-(alkyle en $C_{1-6}$), -$C(O)R^{14}$ ou -$COOR^{14}$ ;

$R^9$ et $R^{17}$ représentent chacun, indépendamment, 1 à 3 substituants choisis indépendamment dans l'ensemble constitué par les atomes d'hydrogène, les groupes alkyle en $C_{1-6}$, alcoxy en $C_{1-6}$, -COOH, -$NO_2$, -$NR^{14}R^{15}$ et - OH, et les atomes d'halogène ;

$R^{14}$ et $R^{15}$ représentent chacun, indépendamment, un atome d'hydrogène ou un groupe alkyle en $C_{1-6}$, aryle ou aryl-(alkyle en $C_{1-6}$) ;

$R^{16}$ représente un groupe alkyle en $C_{1-6}$, aryle ou aryle à substituant(s) $R^{17}$ ;

$R^{18}$ représente un atome d'hydrogène ou un groupe alkyle en $C_{1-6}$ ;

et $R^{19}$ représente un atome d'hydrogène ou un groupe hydroxy ou alcoxy en $C_{1-6}$ ;

en vue de la fabrication d'un médicament destiné au traitement, à la prévention ou à l'amélioration des symptômes de la maladie d'Alzheimer chez un sujet.

**51.** Emploi d'une composition comprenant au moins un composé représenté par la formule (IV) :

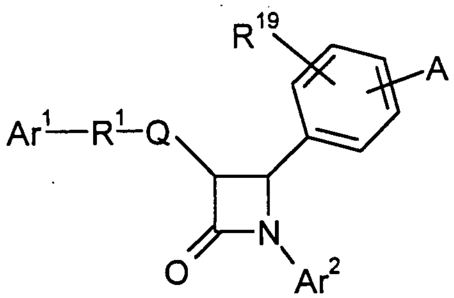

(IV)

ou un sel pharmacologiquement admissible d'un tel composé, ou un solvat d'un tel composé ou sel, dans laquelle formule (IV) :

A représente une entité choisie parmi un groupe hétérocycloalkyle à substituant(s) $R^2$, un groupe hétéroaryle à substituant(s) $R^2$, un groupe hétérocycloalkyle benzocondensé à substituant(s) $R^2$, et un groupe hétéroaryle benzocondensé à substituant(s) $R^2$ ;

$Ar^1$ représente un groupe aryle ou aryle à substituant(s) $R^3$ ;

$Ar^2$ représente un groupe aryle ou aryle à substituant(s) $R^4$ ;

Q représente une liaison ou un groupe formant, avec l'atome de carbone en position 3 du cycle azétidinone, un groupe spiro de formule

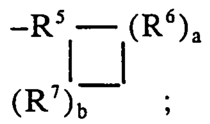

$R^1$ représente un groupe choisi parmi les suivants :

- $(CH_2)_q$- où q vaut de 2 à 6, et peut aussi valoir 0 ou 1 si Q représente un groupe spiro,
- $(CH_2)_e$-G-$(CH_2)_r$- où G représente un groupe phénylène ou un chaînon
- O-, -C(O)-, -$NR^8$- ou -$S(O)_{0-2}$-, e vaut de 0 à 5 et r vaut de 0 à 5, sous réserve que la somme e+r vaille de 1 à 6,

alcénylène en $C_{2-6}$,
et -$(CH_2)_f$-V-$(CH_2)_g$- où V représente un groupe cycloalkylène en $C_{3-6}$, f vaut de 1 à 5 et g vaut de 0 à 5, sous réserve que la somme f+g vaille de 1 à 6 ;
$R^5$ représente un chaînon choisi parmi :

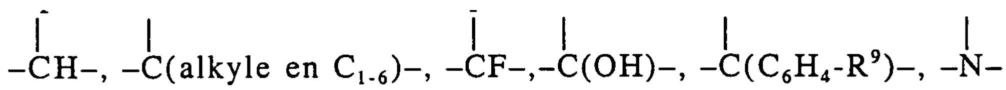

et

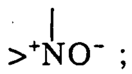

$R^6$ et $R^7$ représentent chacun, indépendamment, un chaînon -$CH_2$-, -CH(alkyle en $C_{1-6}$)- ou -C(alkyle en $C_{1-6})_2$-, ou un groupe -CH=CH- ou -C(alkyle en $C_{1-6}$)=CH- ;
ou bien l'entité représentée par $R^5$ constitue, conjointement avec une entité adjacente représentée par $R^6$ ou par $R^7$, un groupe -CH=CHou -C(alkyle en $C_{1-6}$)=CH- ;
a et b valent chacun, indépendamment, 0, 1, 2 ou 3, sous réserve qu'ils ne vaillent pas tous deux 0, que a vaille 1 si $R^6$ représente un groupe -CH=CHou- C(alkyle en $C_{1-6}$)=CH-, et que b vaille 1 si $R^7$ représente un groupe -CH=CH- ou -C(alkyle en $C_{1-6}$)=CH-, et étant entendu que, si a vaut 2 ou 3, les entités représentées par les symboles $R^6$ peuvent être identiques ou différentes, et que, si b vaut 2 ou 3, les entités représentées par les symboles $R^7$ peuvent être identiques ou différentes ;
étant entendu que, si Q représente une liaison, $R^1$ peut aussi représenter un groupe de formule

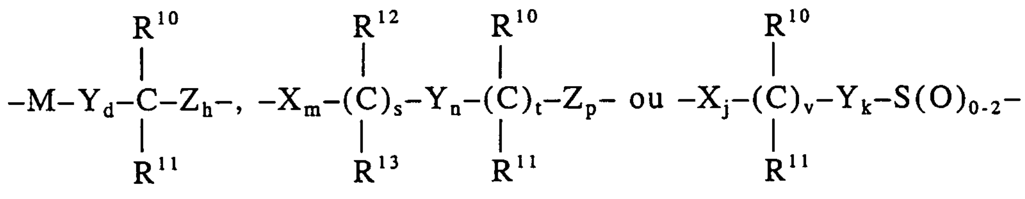

où M représente un chaînon -O-, -S-, -S(O)- ou -$S(O)_2$- ;
X, Y et Z représentent chacun, indépendamment, un chaînon -$CH_2$-, -CH(alkyle inférieur)- ou -C(alkyle inférieur$)_2$- ;
$R^{10}$ et $R^{12}$ représentent chacun, indépendamment, un groupe de formule -$OR^{14}$, -O(CO)$R^{14}$, -O(CO)$OR^{16}$ ou -O(CO)$NR^{14}R^{15}$ ;
$R^{11}$ et $R^{13}$ représentent chacun, indépendamment, un atome d'hydrogène ou un groupe alkyle en $C_{1-6}$ ou aryle ;
ou $R^{10}$ et $R^{11}$ représentent ensemble un substituant oxo =O ;
ou $R^{12}$ et $R^{13}$ représentent ensemble un substituant oxo =O ;
d vaut 1, 2 ou 3 ;

h vaut 0, 1, 2, 3 ou 4 ;
s vaut 0 ou 1, t vaut 0 ou 1, et m, n et p valent chacun, indépendamment, de 0 à 4, sous réserve qu'au moins l'un de s et t vaille 1 et que la somme m+n+p+s+t vaille de 1 à 6, sous réserve que, si p vaut 0 et t vaut 1, la somme m+s+n vaille de 1 à 5, et sous réserve que, si p vaut 0 et s vaut 1, la somme m+t+n vaille de 1 à 5 ;
v vaut 0 ou 1 ;
j et k valent chacun, indépendamment, de 1 à 5, sous réserve que la somme j+k+v vaille de 1 à 5 ;
$R^2$ représente 1 à 3 substituants qui, s'ils sont placés sur des atomes de carbone du cycle, sont choisis parmi les atomes d'hydrogène et d'halogène et les groupes alkyle en $C_{1-10}$, alcényle en $C_{2-10}$, alcynyle en $C_{2-10}$, cycloalkyle en $C_{3-6}$, cycloalcényle en $C_{3-6}$, aryle à substituant(s) $R^{17}$, benzyle à substituant(s) $R^{17}$, aryloxy à substituant(s) $R^{17}$, benzyloxy à substituant(s) $R^{17}$, $-NR^{14}R^{15}$, -(alkylène en $C_{1-6}$)-$NR^{14}R^{15}$, -(alkylène en $C_{1-6}$)-C(O)-$NR^{14}R^{15}$, $-NHC(O)R^{16}$, -OH, alcoxy en $C_{1-6}$, $-OC(O)R^{16}$, $-COR^{14}$, hydroxyalkyle en $C_{1-6}$, (alcoxy en $C_{1-6}$)-alkyle en $C_{1-6}$, $-NO_2$, $-S(O)_{0-2}R^{16}$, $-SO_2NR^{14}R^{15}$ et -(alkylène en $C_{1-6}$)-$COOR^{14}$, étant entendu que, quand $R^2$ représente un substituant placé sur un groupe hétérocycloalkyle, il s'agit de l'un des substituants indiqués ci-dessus ou d'un substituant oxo =O ou

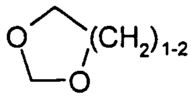 ,

et que, quand $R^2$ représente un substituant placé sur un atome d'azote qui peut en porter, il s'agit d'un atome d'hydrogène ou d'un groupe alkyle en $C_{1-6}$, aryle, alcoxy en $C_{1-6}$, aryloxy, (alkyle en $C_{1-6}$)carbonyle, arylcarbonyle, hydroxy, $-(CH_2)_{1-6}C(O)NR^{18}R^{18}$,

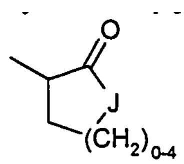

ou

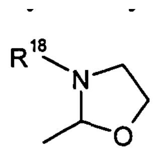 ,

où J représente un chaînon -O-, -NH-, $-NR^{18}$- ou $-CH_2$- ;
$R^3$ et $R^4$ représentent chacun, indépendamment, 1 à 3 substituants choisis indépendamment dans l'ensemble constitué par les groupes alkyle en $C_{1-6}$, $-OR^{14}$, $-O(CO)R^{14}$, $-O(CO)OR^{16}$, $-O(CH_2)_{1-5}OR^{14}$, $-O(CO)NR^{14}R^{15}$, $-NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $-S(O)_{0-2}R^{16}$, $-O(CH_2)_{1-10}COOR^{14}$, $-O(CH_2)_{1-10}CONR^{14}R^{15}$, -(alkylène en $C_{1-6}$)-$COOR^{14}$, $-CH=CH-COOR^{14}$, $-CF_3$, -CN et $-NO_2$, et les atomes d'halogène ;
$R^8$ représente un atome d'hydrogène ou un groupe alkyle en $C_{1-6}$, aryl-(alkyle en $C_{1-6}$), $-C(O)R^{14}$ ou $-COOR^{14}$ ;
$R^9$ et $R^{17}$ représentent chacun, indépendamment, 1 à 3 substituants choisis indépendamment dans l'ensemble constitué par les atomes d'hydrogène, les groupes alkyle en $C_{1-6}$, alcoxy en $C_{1-6}$, -COOH, $-NO_2$, $-NR^{14}R^{15}$ et - OH, et les atomes d'halogène ;
$R^{14}$ et $R^{15}$ représentent chacun, indépendamment, un atome d'hydrogène ou un groupe alkyle en $C_{1-6}$, aryle ou aryl-(alkyle en $C_{1-6}$) ;
$R^{16}$ représente un groupe alkyle en $C_{1-6}$, aryle ou aryle à substituant(s) $R^{17}$ ;
$R^{18}$ représente un atome d'hydrogène ou un groupe alkyle en $C_{1-6}$ ;
et $R^{19}$ représente un atome d'hydrogène ou un groupe hydroxy ou alcoxy en $C_{1-6}$ ;

en vue de la fabrication d'un médicament conçu pour réguler la production ou le taux d'au moins un peptide β-amyloïde chez un sujet.

**52.** Emploi d'une composition comprenant au moins un composé représenté par la formule (IV) :

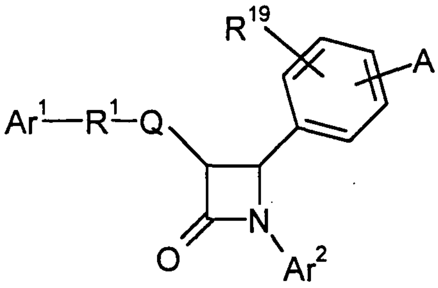

(IV)

ou un sel pharmacologiquement admissible d'un tel composé, ou un solvat d'un tel composé ou sel, dans laquelle formule (IV) :

A représente une entité choisie parmi un groupe hétérocycloalkyle à substituant(s) $R^2$, un groupe hétéroaryle à substituant(s) $R^2$, un groupe hétérocycloalkyle benzocondensé à substituant(s) $R^2$, et un groupe hétéroaryle benzocondensé à substituant(s) $R^2$ ;
$Ar^1$ représente un groupe aryle ou aryle à substituant(s) $R^3$ ;
$Ar^2$ représente un groupe aryle ou aryle à substituant(s) $R^4$;
Q représente une liaison ou un groupe formant, avec l'atome de carbone en position 3 du cycle azétidinone, un groupe spiro de formule

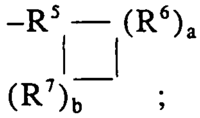

;

$R^1$ représente un groupe choisi parmi les suivants :

- $(CH_2)_q$- où q vaut de 2 à 6, et peut aussi valoir 0 ou 1 si Q représente un groupe spiro,
- $(CH_2)_e$-G-$(CH_2)_r$- où G représente un groupe phénylène ou un chaînon
- O-,-C(O)-, -$NR^8$-ou -$S(O)_{0-2}$-, e vaut de 0 à 5 et r vaut de 0 à 5, sous réserve que la somme e+r vaille de 1 à 6, alcénylène en $C_{2-6}$,

et -$(CH_2)_f$-V-$(CH_2)_g$- où V représente un groupe cycloalkylène en $C_{3-6}$, f vaut de 1 à 5 et g vaut de 0 à 5, sous réserve que la somme f+g vaille de 1 à 6 ;
$R^5$ représente un chaînon choisi parmi :

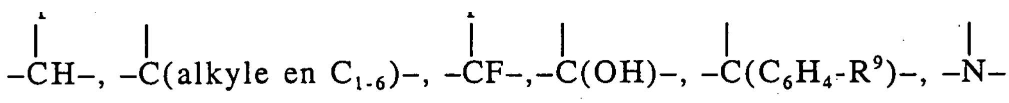

et

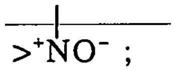

$R^6$ et $R^7$ représentent chacun, indépendamment, un chaînon -$CH_2$-, -CH(alkyle en $C_{1-6}$)- ou -C(alkyle en $C_{1-6})_2$-, ou un groupe -CH=CH- ou -C(alkyle en $C_{1-6}$)=CH- ;
ou bien l'entité représentée par $R^5$ constitue, conjointement avec une entité adjacente représentée par $R^6$ ou par $R^7$, un groupe -CH=CHou -C(alkyle en $C_{1-6}$)=CH- ;
a et b valent chacun, indépendamment, 0, 1, 2 ou 3, sous réserve qu'ils ne vaillent pas tous deux 0, que a vaille 1 si $R^6$ représente un groupe -CH=CHou -C(alkyle en $C_{1-6}$)=CH-, et que b vaille 1 si $R^7$ représente un groupe -CH=CH- ou -C(alkyle en $C_{1-6}$)=CH-, et étant entendu que, si a vaut 2 ou 3, les entités représentées par les

symboles $R^6$ peuvent être identiques ou différentes, et que, si b vaut 2 ou 3, les entités représentées par les symboles $R^7$ peuvent être identiques ou différentes ; étant entendu que, si Q représente une liaison, $R^1$ peut aussi représenter un groupe de formule

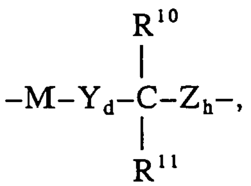 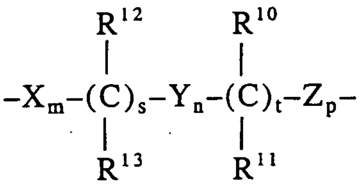 ou 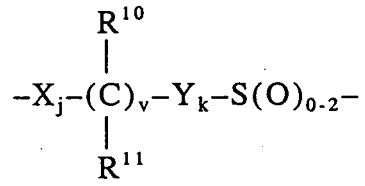

M représente un chaînon -O-, -S-, -S(O)- ou $-S(O)_2-$ ;
X, Y et Z représentent chacun, indépendamment, un chaînon $-CH_2-$, -CH(alkyle inférieur)- ou -C(alkyle inférieur)$_2$- ;
$R^{10}$ et $R^{12}$ représentent chacun, indépendamment, un groupe de formule $-OR^{14}$, $-O(CO)R^{14}$, $-O(CO)OR^{16}$ ou $-O(CO)NR^{14}R^{15}$ ;
$R^{11}$ et $R^{13}$ représentent chacun, indépendamment, un atome d'hydrogène ou un groupe alkyle en $C_{1-6}$ ou aryle ;
ou $R^{10}$ et $R^{11}$ représentent ensemble un substituant oxo =O ;
ou $R^{12}$ et $R^{13}$ représentent ensemble un substituant oxo =O ;
d vaut 1, 2 ou 3 ;
h vaut 0, 1, 2, 3 ou 4 ;
s vaut 0 ou 1, t vaut 0 ou 1, et m, n et p valent chacun, indépendamment, de 0 à 4, sous réserve qu'au moins l'un de s et t vaille 1 et que la somme m+n+p+s+t vaille de 1 à 6, sous réserve que, si p vaut 0 et t vaut 1, 1a somme m+s+n vaille de 1 à 5, et sous réserve que, si p vaut 0 et s vaut 1, la somme m+t+n vaille de 1 à 5 ;
v vaut 0 ou 1 ;
j et k valent chacun, indépendamment, de 1 à 5, sous réserve que la somme j+k+v vaille de 1 à 5 ;
$R^2$ représente 1 à 3 substituants qui, s'ils sont placés sur des atomes de carbone de cycle, sont choisis parmi les atomes d'hydrogène et d'halogène et les groupes alkyle en $C_{1-10}$, alcényle en $C_{2-10}$, alcynyle en $C_{2-10}$, cycloalkyle en $C_{3-6}$, cycloalcényle en $C_{3-6}$, aryle à substituant(s) $R^{17}$, benzyle à substituant(s) $R^{17}$, aryloxy à substituant(s) $R^{17}$, benzyloxy à substituant(s) $R^{17}$, $-NR^{14}R^{15}$, -(alkylène en $C_{1-6}$)$NR^{14}R^{15}$, -(alkylène en $C_{1-6}$)-C(O)-$NR^{14}R^{15}$, $-NHC(O)R^{16}$, -OH, alcoxy en $C_{1-6}$, $-OC(O)R^{16}$, $-COR^{14}$, hydroxyalkyle en $C_{1-6}$, (alcoxy en $C_{1-6}$)-alkyle en $C_{1-6}$, $-NO_2$, $-S(O)_{0-2}R^{16}$, $-SO_2NR^{14}R^{15}$ et -(alkylène en $C_{1-6}$)-$COOR^{14}$, étant entendu que, quand $R^2$ représente un substituant placé sur un groupe hétérocycloalkyle, il s'agit de l'un des substituants indiqués ci-dessus ou d'un substituant oxo =O ou

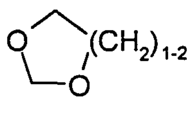 ,

et que, quand $R^2$ représente un substituant placé sur un atome d'azote qui peut en porter, il s'agit d'un atome d'hydrogène ou d'un groupe alkyle en $C_{1-6}$, aryle, alcoxy en $C_{1-6}$, aryloxy, (alkyle en $C_{1-6}$)carbonyle, arylcarbonyle, hydroxy, $-(CH_2)_{1-6}C(O)NR^{18}R^{18}$,

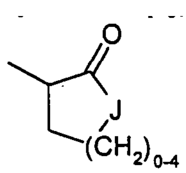

ou

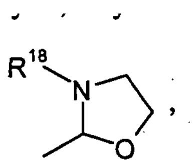

où J représente un chaînon -O-, -NH-, $-NR^{18}$ ou $-CH_2-$ ;
$R^3$ et $R^4$ représentent chacun, indépendamment, 1 à 3 substituants choisis indépendamment dans l'ensemble constitué par les groupes alkyle en $C_{1-6}$, $-OR^{14}$, $-O(CO)R^{14}$, $-O(CO)OR^{16}$, $-O(CH_2)_{1-5}OR^{14}$, $-O(CO)NR^{14}R^{15}$, $-NR^{14}R^{15}$, $-NR^{14}(CO)R^{15}$, $-NR^{14}(CO)OR^{16}$, $-NR^{14}(CO)NR^{15}R^{19}$, $-NR^{14}SO_2R^{16}$, $-COOR^{14}$, $-CONR^{14}R^{15}$, $-COR^{14}$, $-SO_2NR^{14}R^{15}$, $-S(O)_{0-2}R^{16}$, $-O(CH_2)_{1-10}COOR^{14}$,- $O(CH_2)_{1-10}CONR^{14}R^{15}$, -(alkylène en $C_{1-6}$)-$COOR^{14}$, $-CH=CH-COOR^{14}$, $-CF_3$, -CN et $-NO_2$, et les atomes d'halogène ;
$R^8$ représente un atome d'hydrogène ou un groupe alkyle en $C_{1-6}$, aryl-(alkyle en $C_{1-6}$), $-C(O)R^{14}$ ou $-COOR^{14}$;
$R^9$ et $R^{17}$ représentent chacun, indépendamment, 1 à 3 substituants choisis indépendamment dans l'ensemble constitué par les atomes d'hydrogène, les groupes alkyle en $C_{1-6}$, alcoxy en $C_{1-6}$, -COOH, $-NO_2$, $-NR^{14}R^{15}$ et - OH, et les atomes d'halogène ;
$R^{14}$ et $R^{15}$ représentent chacun, indépendamment, un atome d'hydrogène ou un groupe alkyle en $C_{1-6}$, aryle ou aryl-(alkyle en $C_{1-6}$) ;
$R^{16}$ représente un groupe alkyle en $C_{1-6}$, aryle ou aryle à substituant(s) $R^{17}$ ;
$R^{18}$ représente un atome d'hydrogène ou un groupe alkyle en $C_{1-6}$ ;
et $R^{19}$ représente un atome d'hydrogène ou un groupe hydroxy ou alcoxy en $C_{1-6}$ ;

en vue de la fabrication d'un médicament conçu pour réguler la quantité de l'isoforme 4 de l'apolipoprotéine E dans la circulation sanguine et/ou le cerveau d'un sujet.

**53.** Emploi d'une composition comprenant au moins un composé représenté par la formule (V) :

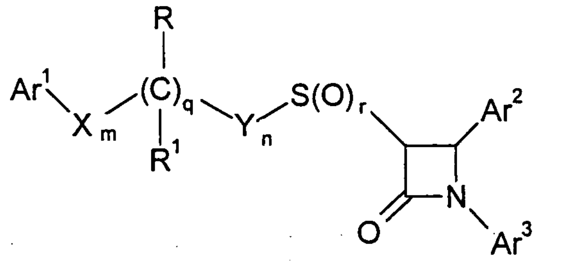

(V)

ou un sel pharmacologiquement admissible d'un tel composé, ou un solvat d'un tel composé ou sel, dans laquelle formule (V) :

$Ar^1$ représente un groupe aryle, aryle à substituant(s) $R^{10}$ ou hétéroaryle ;
$Ar^2$ représente un groupe aryle ou aryle à substituant(s) $R^4$ ;
$Ar^3$ représente un groupe aryle ou aryle à substituant(s) $R^5$ ;
X et Y représentent chacun, indépendamment, un chaînon $-CH_2-$, -CH(alkyle inférieur)- ou -C(alkyle inférieur)$_2$- ;
R représente un groupe de formule $-OR^6$ $-O(CO)R^6$, $-O(CO)OR^9$ ou-$O(CO)NR^6R^7$ ;
$R^1$ représente un atome d'hydrogène ou un groupe alkyle inférieur ou aryle ; ou R et $R^1$ représentent ensemble un substituant oxo =O ;
q vaut 0 ou 1 ;
r vaut 0, 1 ou 2 ;
m et n valent chacun, indépendamment, 0, 1, 2, 3, 4 ou 5, sous réserve que la somme m+n+q vaille 1, 2, 3, 4 ou 5 ;
$R^4$ représente 1 à 5 substituants choisis indépendamment parmi les groupes alkyle inférieur, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, -(alkylène inférieur)-$COOR^6$ et $-CH=CH-COOR^6$;
$R^5$ représente 1 à 5 substituants choisis indépendamment parmi les groupes $-OR^6$ $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, -(alkylène infé-

rieur)-$COOR^6$, $-CH=CH-COOR^6$, $-CF_3$, -CN et $-NO_2$, et les atomes d'halogène ;

$R^6$, $R^7$ et $R^8$ représentent chacun, indépendamment, un atome d'hydrogène ou un groupe alkyle inférieur, aryle ou aryl-(alkyle inférieur) ;

$R^9$ représente un groupe alkyle inférieur, aryle ou aryl-(alkyle inférieur) ; et $R^{10}$ représente 1 à 5 substituants choisis indépendamment parmi les groupes alkyle inférieur, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, $-CF_3$, -CN et $-NO_2$, et les atomes d'halogène ;

en vue de la fabrication d'un médicament destiné au traitement, à la prévention ou à l'amélioration des symptômes de la maladie d'Alzheimer chez un sujet.

**54.** Emploi d'une composition comprenant au moins un composé représenté par la formule (V) :

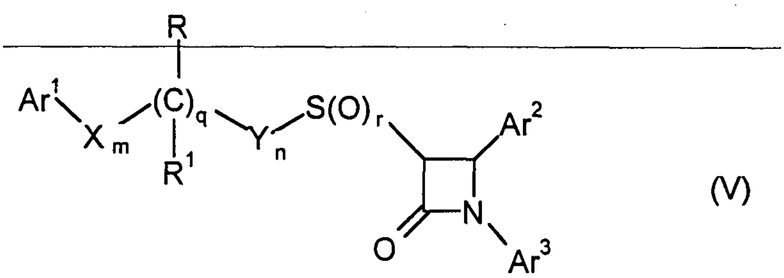

(V)

ou un sel pharmacologiquement admissible d'un tel composé, ou un solvat d'un tel composé ou sel, dans laquelle formule (V) :

$Ar^1$ représente un groupe aryle, aryle à substituant(s) $R^{10}$ ou hétéroaryle ; $Ar^2$ représente un groupe aryle ou aryle à substituant(s) $R^4$ ;

$Ar^3$ représente un groupe aryle ou aryle à substituant(s) $R^5$ ;

X et Y représentent chacun, indépendamment, un chaînon $-CH_2-$, -CH(alkyle inférieur)- ou -C(alkyle inférieur)$_2$- ;

R représente un groupe de formule $-OR^6$, $-O(CO)R^6$, $-O(CO)OR^9$ ou $-O(CO)NR^6R^7$ ;

$R^1$ représente un atome d'hydrogène ou un groupe alkyle inférieur ou aryle ; ou R et $R^1$ représentent ensemble un substituant oxo =O ;

q vaut 0 ou 1 ;

r vaut 0, 1 ou 2 ;

m et n valent chacun, indépendamment, 0, 1, 2, 3, 4 ou 5, sous réserve que la somme m+n+q vaille 1, 2, 3, 4 ou 5 ;

$R^4$ représente 1 à 5 substituants choisis indépendamment parmi les groupes alkyle inférieur, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, -(alkylène inférieur)-$COOR^6$ et $-CH=CH-COOR^6$ ;

$R^5$ représente 1 à 5 substituants choisis indépendamment parmi les groupes $-OR^6$, $-O(CO)R^6$, $-O(CO)OR^9$, $-O(CH_2)_{1-5}OR^6$, $-O(CO)NR^6R^7$ $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$,- $O(CH_2)_{1-10}CONR^6R^7$, -(alkylène inférieur)-$COOR^6$, $-CH=CH-COOR^6$, $-CF_3$, -CN et $-NO_2$, et les atomes d'halogène ;

$R^6$, $R^7$ et $R^8$ représentent chacun, indépendamment, un atome d'hydrogène ou un groupe alkyle inférieur, aryle ou aryl-(alkyle inférieur) ;

$R^9$ représente un groupe alkyle inférieur, aryle ou aryl-(alkyle inférieur) ; et $R^{10}$ représente 1 à 5 substituants choisis indépendamment parmi les groupes alkyle inférieur, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, $-CF_3$, -CN et $-NO_2$, et les atomes d'halogène ;

en vue de la fabrication d'un médicament conçu pour réguler la production ou le taux d'au moins un peptide β-amyloïde chez un sujet.

**55.** Emploi d'une composition comprenant au moins un composé représenté par la formule (V) :

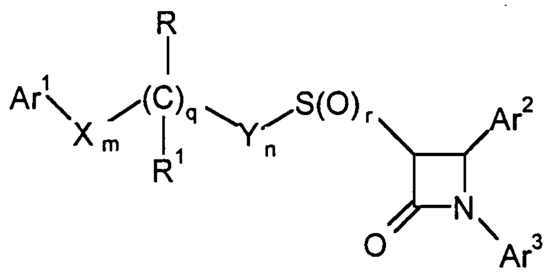

(V)

ou un sel pharmacologiquement admissible d'un tel composé, ou un solvat d'un tel composé ou sel, dans laquelle formule (V) :

$Ar^1$ représente un groupe aryle, aryle à substituant(s) $R^{10}$ ou hétéroaryle ; $Ar^2$ représente un groupe aryle ou aryle à substituant(s) $R^4$ ;
$Ar^3$ représente un groupe aryle ou aryle à substituant(s) $R^5$ ;
X et Y représentent chacun, indépendamment, un chaînon $-CH_2-$, -CH(alkyle inférieur)- ou $-C(\text{alkyle inférieur})_2-$ ;
R représente un groupe de formule $-OR^6$, $-O(CO)R^6$, $-O(CO)OR^9$ ou $-O(CO)NR^6R^7$ ;
$R^1$ représente un atome d'hydrogène ou un groupe alkyle inférieur ou aryle ;
ou R et $R^1$ représentent ensemble un substituant oxo =O ;
q vaut 0 ou 1 ;
r vaut 0, 1 ou 2 ;
m et n valent chacun, indépendamment, 0, 1, 2, 3, 4 ou 5, sous réserve que la somme m+n+q vaille 1, 2, 3, 4 ou 5 ;
$R^4$ représente 1 à 5 substituants choisis indépendamment parmi les groupes alkyle inférieur, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, -(alkylène inférieur)-$COOR^6$ et $-CH=CH-COOR^6$ ;
$R^5$ représente 1 à 5 substituants choisis indépendamment parmi les groupes $-OR^6$, $-O(CO)R^6$, $-O(CO)OR^9$, $-O(CH_2)_{1-5}OR^6$, ,$-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, -(alkylène inférieur)-$COOR^6$, $-CH=CH-COOR^{14}$, $-CF_3$, -CN et $-NO_2$, et les atomes d'halogène ;
$R^6$, $R^7$ et $R^8$ représentent chacun, indépendamment, un atome d'hydrogène ou un groupe alkyle inférieur, aryle ou aryl-(alkyle inférieur) ;
$R^9$ représente un groupe alkyle inférieur, aryle ou aryl-(alkyle inférieur) ; et $R^{10}$ représente 1 à 5 substituants choisis indépendamment parmi les groupes alkyle inférieur, $-OR^6$, $-O(CO)R^6$, $-O(CO)OR^9$, $-O(CH_2)_{1-5}OR^6$, $-O(CO)NR^6R^7$, $-NR^6R^7$, $-NR^6(CO)R^7$, $-NR^6(CO)OR^9$, $-NR^6(CO)NR^7R^8$, $-NR^6SO_2R^9$, $-COOR^6$, $-CONR^6R^7$, $-COR^6$, $-SO_2NR^6R^7$, $-S(O)_{0-2}R^9$, $-O(CH_2)_{1-10}COOR^6$, $-O(CH_2)_{1-10}CONR^6R^7$, $-CF_3$, -CN et $-NO_2$, et les atomes d'halogène ;

en vue de la fabrication d'un médicament conçu pour réguler la quantité de l'isoforme 4 de l'apolipoprotéine E dans la circulation sanguine et/ou le cerveau d'un sujet.

**56.** Emploi d'une composition comprenant au moins un composé représenté par la formule (VI) :

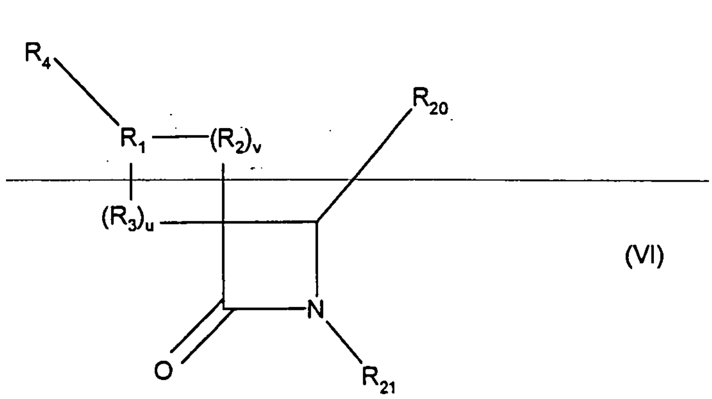

(VI)

ou un sel pharmacologiquement admissible d'un tel composé, ou un solvat d'un tel composé ou sel, dans laquelle formule :

$R_1$ représente >CH-, >C(alkyle inférieur)-, >CF-, >C(OH)-, >C($C_6H_5$)-, >C($C_6H_4$-$R^{15}$)-, >N- ou >$^+$N-O$^-$ ;
$R_2$ et $R_3$ représentent chacun, indépendamment, un chaînon -$CH_2$-, -CH(alkyle inférieur)- ou -C(alkyle inférieur)$_2$-, ou un groupe -CH=CH- ou -C(alkyle inférieur)=CH- ;
ou bien l'entité représentée par $R_1$ constitue, conjointement avec une entité adjacente représentée par $R_2$ ou par $R_3$, un groupe -CH=CH- ou -CH=C(alkyle inférieur)- ;
u et v valent chacun, indépendamment, 0, 1, 2 ou 3, sous réserve qu'ils ne vaillent pas tous deux 0, que v vaille 1 si $R_2$ représente un groupe -CH=CH- ou -C(alkyle inférieur)=CH-, et que u vaille 1 si $R_3$ représente un groupe -CH=CH- ou -C(alkyle inférieur)=CH-, et étant entendu que, si v vaut 2 ou 3, les entités représentées par les symboles $R_2$ peuvent être identiques ou différentes, et que, si u vaut 2 ou 3, les entités représentées par les symboles $R_3$ peuvent être identiques ou différentes ;
$R_4$ représente une entité choisie parmi
B-$(CH_2)_m$C(O)- où m vaut 0, 1, 2, 3, 4 ou 5,
B-$(CH_2)_q$- où q vaut 0, 1, 2, 3, 4, 5 ou 6,
B-$(CH_2)_e$-Z-$(CH_2)_r$- où Z représente -O-, -C(O)-, un groupe phénylène, -N($R_8$)- ou -S(O)$_{0-2}$-, e vaut 0, 1, 2, 3, 4 ou 5, et r vaut 0, 1, 2, 3, 4 ou 5, sous réserve que la somme e+r vaille 0, 1, 2, 3, 4, 5 ou 6,
B-(alcénylène en $C_{2-6}$)-,
B-(alcadiénylène en $C_{4-6}$)-,
B-$(CH_2)_t$-Z-(alcénylène en $C_{2-6}$)-, où Z a la signification indiquée ci-dessus, et t vaut 0, 1, 2 ou 3, sous réserve que la somme de t et du nombre d'atomes de carbone de la chaîne alcénylène vaille 2, 3, 4, 5 ou 6,
B-$(CH_2)_f$V-$(CH_2)_g$-, où V représente un groupe cycloalkylène en $C_{3-6}$,
f vaut 1, 2, 3, 4 ou 5, et g vaut 0, 1, 2, 3, 4 ou 5, sous réserve que la somme f+g vaille 1, 2, 3, 4, 5 ou 6,
B-$(CH_2)_t$-V-(alcénylène en $C_{2-6}$)- ou B-(alcénylène en $C_{2-6}$)-V-$(CH_2)_t$-, où V et t ont les significations indiquées ci-dessus, sous réserve que la somme de t et du nombre d'atomes de carbone de la chaîne alcénylène vaille 2, 3, 4, 5 ou 6,
B-$(CH_2)_a$-Z-$(CH_2)_b$-V-$(CH_2)_d$-, où Z et V ont les significations indiquées ci-dessus et a, b et d valent chacun, indépendamment, 0, 1, 2, 3, 4, 5 ou 6, sous réserve que la somme a+b+d vaille 0, 1, 2, 3, 4, 5 ou 6, ou T-$(CH_2)_s$-, où T représente un groupe cycloalkyle comportant 3 à 6 atomes de carbone et s vaut 0, 1, 2, 3, 4, 5 ou 6 ;
ou $R_1$ et $R_4$ représentent ensemble un groupe B-CH=C<;
B représente une entité choisie parmi les groupes indanyle, indényle, naphtyle, tétrahydronaphtyle, hétéroaryle, ou hétéroaryle à substituant(s) W, le groupe hétéroaryle étant choisi parmi les groupes pyrrolyle, pyridinyle, pyrimidinyle, pyrazinyle, triazinyle, imidazolyle, thiazolyle, pyrazolyle, thiényle, oxazolyle et furanyle, y compris, dans le cas des groupes hétéroaryles azotés, les N-oxydes de ces groupes, ou un groupe de formule

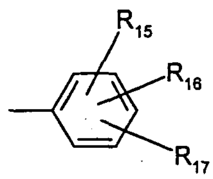 ;

W représente 1 à 3 substituants choisis indépendamment dans l'ensemble formé par les atomes d'halogène et les groupes alkyle inférieur, hydroxyalkyle inférieur, alcoxy inférieur, alcoxyalkyle, alcoxyalcoxy, alcoxycarbonylalcoxy, (alcoxy inférieur)-imino-alkyle inférieur, alcanedioyle inférieur, (alkyle inférieur)-alcanedioyle inférieur, allyloxy, -$CF_3$, -$OCF_3$, benzyle, $R_7$-benzyle, benzyloxy, $R_7$-benzyloxy, phénoxy, $R_7$-phénoxy, dioxolanyle, -$NO_2$, -N($R_8$)($R_9$), ($R_8$)($R_9$)N-(alkylène inférieur)-, ($R_8$)($R_9$)N-(alkylène-oxy inférieur), -OH, -CN, -$N_3$, -NHC(O)O$R_{10}$,- NHC(O)$R_{10}$, $R_{11}O_2$SNH-, ($R_{11}O_2S)_2$N-, -$SO_2NH_2$, S(O)$_{0-2}R_8$, tertiobutyldiméthyl-silyloxyméthyl, -C(O)$R_{12}$, -COO$R_{19}$, -CON($R_8$)($R_9$), -CH=CHC(O)$R_{12}$, -(alkylène inférieur)-C(O)$R_{12}$, $R_{10}$C(O)-(alkylène-oxy inférieur)-, ($R_8$)($R_9$)NC(O)-(alkylène-oxy inférieur)-, et

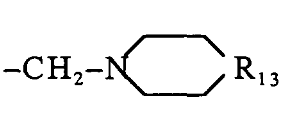

pour les substituants placés sur un atome de carbone, les substituants placés sur un atome d'azote d'un cycle

hétéroaryle, s'il y en a, étant choisis parmi les groupes alkyle inférieur, alcoxy inférieur, $-C(O)OR_{10}$, $-C(O)R_{10}$, -OH, $(R_8)(R_9)N$-(alkylène inférieur)-, $(R_8)(R_9)N$-(alkylène-oxy inférieur), $-SO_2NH_2$ et 2-(triméthylsilyl)-éthoxyméthyl ;

$R_7$ représente 1 à 3 substituants, indépendamment choisis dans l'ensemble formé par les groupes alkyle inférieur, alcoxy inférieur, -COOH, $-NO_2$, $-N(R_8)(R_9)$ et -OH et les atomes d'halogène ;

$R_8$ et $R_9$ représentent chacun, indépendamment, un atome d'hydrogène ou un groupe alkyle inférieur ;

$R_{10}$ représente un groupe alkyle inférieur, phényle, $R_7$-phényle, benzyle ou $R_7$-benzyle ;

$R_{11}$ représente un groupe -OH, alkyle inférieur, phényle, $R_7$-phényle, benzyle ou $R_7$-benzyle ;

$R_{12}$ représente un atome d'hydrogène ou un groupe -OH, alcoxy, phénoxy, benzyloxy,

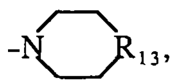

$-N(R_8)(R_9)$, alkyle inférieur, phényle ou $R_7$-phényle ;

$R_{13}$ représente -O-, $-CH_2-$, -NH-, -N(alkyle inférieur) ou $-NC(O)R_{19}$ ;

$R_{15}$, $R_{16}$ et $R_{17}$ représentent chacun, indépendamment, un atome d'hydrogène ou l'un des groupes mentionnés à propos de W, ou $R_{15}$ représente un atome d'hydrogène et les entités représentées par $R_{16}$ et $R_{17}$ constituent, conjointement avec les atomes de carbone adjacents auxquelles elles sont liées, un cycle dioxolanyle ;

$R_{19}$ représente un atome d'hydrogène ou un groupe alkyle inférieur, phényle ou phényle-(alkyle inférieur) ;

et $R_{20}$ et $R_{21}$ représentent chacun, indépendamment, une entité choisie parmi les groupes phényle, phényle à substituant(s) W, naphtyle, naphtyle à substituant(s) W, indanyle, indényle, tétrahydronaphtyle, benzodioxolyle, hétéroaryle, hétéroaryle à substituant(s) W, hétéroaryle benzocondensé, hétéroaryle benzocondensé à substituant(s) W, et cyclopropyle, le terme "hétéroaryle" ayant la définition indiquée plus haut ;

en vue de la fabrication d'un médicament destiné au traitement, à la prévention ou à l'amélioration des symptômes de la maladie d'Alzheimer chez un sujet.

**57.** Emploi d'une composition comprenant au moins un composé représenté par la formule (VI) :

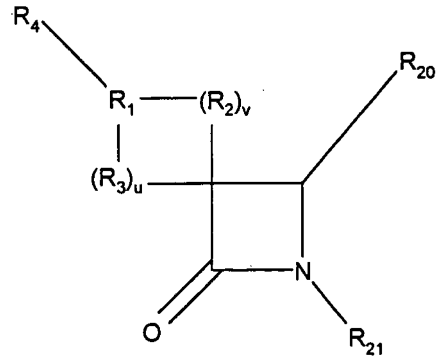

(VI)

ou un sel pharmacologiquement admissible d'un tel composé, ou un solvat d'un tel composé ou sel, dans laquelle formule :

$R_1$ représente >CH-, >C(alkyle inférieur)-, >CF-, >C(OH)-, $>C(C_6H_5)-$, $>C(C_6H_4-R^{15})-$, >N- ou $>^+N-O^-$ ;

$R_2$ et $R_3$ représentent chacun, indépendamment, un chaînon $-CH_2-$, -CH(alkyle inférieur)- ou -C(alkyle inférieur)$_2$-, ou un groupe -CH=CHou -C(alkyle inférieur)=CH- ;

ou bien l'entité représentée par $R_1$ constitue, conjointement avec une entité adjacente représentée par $R_2$ ou par $R_3$, un groupe -CH=CHou -CH=C(alkyle inférieur)- ;

u et v valent chacun, indépendamment, 0, 1, 2 ou 3, sous réserve qu'ils ne vaillent pas tous deux 0, que v vaille 1 si $R_2$ représente un groupe -CH=CHou -C(alkyle inférieur)=CH-, et que u vaille 1 si $R_3$ représente un groupe -CH=CH- ou -C(alkyle inférieur)=CH-, et étant entendu que, si v vaut 2 ou 3, les entités représentées par les symboles $R_2$ peuvent être identiques ou différentes, et que, si u vaut 2 ou 3, les entités représentées par les symboles $R_3$ peuvent être identiques ou différentes ;

$R_4$ représente une entité choisie parmi

B-$(CH_2)_m$C(O)- où m vaut 0, 1, 2, 3, 4 ou 5,
B-$(CH_2)_q$- où q vaut 0, 1, 2, 3, 4, 5 ou 6,
B-$(CH_2)_e$-Z-$(CH_2)_r$- où Z représente -O-, -C(O)-, un groupe phénylène, -N($R_8$)- ou -$S(O)_{0\text{-}2}$-, e vaut 0, 1, 2, 3, 4 ou 5, et r vaut 0, 1, 2, 3, 4 ou 5, sous réserve que la somme e+r vaille 0, 1, 2, 3, 4, 5 ou 6,
B-(alcénylène en $C_{2\text{-}6}$)-,
B-(alcadiénylène en $C_{4\text{-}6}$)-,
B-$(CH_2)_t$-Z-(alcénylène en $C_{2\text{-}6}$)-, où Z a la signification indiquée ci-dessus, et t vaut 0, 1, 2 ou 3, sous réserve que la somme de t et du nombre d'atomes de carbone de la chaîne alcénylène vaille 2, 3, 4, 5 ou 6,
B-$(CH_2)_f$-V-$(CH_2)_g$-, où V représente un groupe cycloalkylène en $C_{3\text{-}6}$,
f vaut 1, 2, 3, 4 ou 5, et g vaut 0, 1, 2, 3, 4 ou 5, sous réserve que la somme f+g vaille 1, 2, 3, 4, 5 ou 6,
B-$(CH_2)_t$-V-(alcénylène en $C_{2\text{-}6}$)- ou B-(alcénylène en $C_{2\text{-}6}$)-V-$(CH_2)_t$-, où V et t ont les significations indiquées ci-dessus, sous réserve que la somme de t et du nombre d'atomes de carbone de la chaîne alcénylène vaille 2, 3, 4, 5 ou 6,
B-$(CH_2)_a$-Z-$(CH_2)_b$-V-$(CH_2)_d$-, où Z et V ont les significations indiquées ci-dessus et a, b et d valent chacun, indépendamment, 0, 1, 2, 3, 4, 5 ou 6, sous réserve que la somme a+b+d vaille 0, 1, 2, 3, 4, 5 ou 6,
ou T-$(CH_2)_s$-, où T représente un groupe cycloalkyle comportant 3 à 6 atomes de carbone et s vaut 0, 1, 2, 3, 4, 5 ou 6 ;
ou $R_1$ et $R_4$ représentent ensemble un groupe B-CH=C<;
B représente une entité choisie parmi les groupes indanyle, indényle, naphtyle, tétrahydronaphtyle, hétéroaryle, ou hétéroaryle à substituant(s) W, le groupe hétéroaryle étant choisi parmi les groupes pyrrolyle, pyridinyle, pyrimidinyle, pyrazinyle, triazinyle, imidazolyle, thiazolyle, pyrazolyle, thiényle, oxazolyle et furanyle, y compris, dans le cas des groupes hétéroaryles azotés, les N-oxydes de ces groupes, ou un groupe de formule

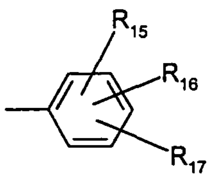
;

W représente 1 à 3 substituants choisis indépendamment dans l'ensemble formé par les atomes d'halogène et les groupes alkyle inférieur, hydroxyalkyle inférieur, alcoxy inférieur, alcoxyalkyle, alcoxyalcoxy, alcoxycarbonylalcoxy, (alcoxy inférieur)-imino-alkyle inférieur, alcanedioyle inférieur, (alkyle inférieur)-alcanedioyle inférieur, allyloxy, -$CF_3$, -$OCF_3$, benzyle, $R_7$-benzyle, benzyloxy, $R_7$-benzyloxy, phénoxy, $R_7$-phénoxy, dioxolanyle, -$NO_2$, -N($R_8$)($R_9$), ($R_8$)($R_9$)N-(alkylène inférieur)-, ($R_8$)($R_9$)N-(alkylène-oxy inférieur), -OH, -CN, -$N_3$, -NHC(O)O$R_{10}$, -NHC(O)$R_{10}$, $R_{11}O_2$SNH-, $(R_{11}O_2S)_2$N-, -$SO_2NH_2$, $S(O)_{0\text{-}2}R_8$, tertiobutyldiméthyl-silyloxyméthyl, -C(O)$R_{12}$, -COO$R_{19}$, -CON($R_8$)($R_9$), -CH=CHC(O)$R_{12}$, -(alkylène inférieur)-C(O)$R_{12}$, $R_{10}$C(O)-(alkylène-oxy inférieur)-, ($R_8$)($R_9$)NC(O)-(alkylène-oxy inférieur)-, et

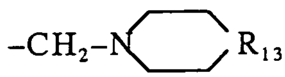

pour les substituants placés sur un atome de carbone, les substituants placés sur un atome d'azote d'un cycle hétéroaryle, s'il y en a, étant choisis parmi les groupes alkyle inférieur, alcoxy inférieur, -C(O)O$R_{10}$, -C(O)$R_{10}$, -OH, ($R_8$)($R_9$)N-(alkylène inférieur)-, ($R_8$)($R_9$)N-(alkylène-oxy inférieur), -$SO_2$NH, et 2-(triméthylsilyl)-éthoxyméthyl ;
$R_7$ représente 1 à 3 substituants, indépendamment choisis dans l'ensemble formé par les groupes alkyle inférieur, alcoxy inférieur, -COOH, -$NO_2$, -N($R_8$)($R_9$) et -OH et les atomes d'halogène ;
$R_8$ et $R_9$ représentent chacun, indépendamment, un atome d'hydrogène ou un groupe alkyle inférieur ;
$R_{10}$ représente un groupe alkyle inférieur, phényle, $R_7$-phényle, benzyle ou $R_7$-benzyle ;
$R_{11}$ représente un groupe -OH, alkyle inférieur, phényle, $R_7$-phényle, benzyle ou $R_7$-benzyle ;
$R_{12}$ représente un atome d'hydrogène ou un groupe -OH, alcoxy, phénoxy, benzyloxy,

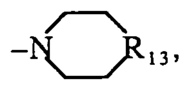

$-N(R_8)(R_9)$, alkyle inférieur, phényle ou $R_7$-phényle ;

$R_{13}$ représente -O-, $-CH_2-$, -NH-, -N(alkyle inférieur) ou $-NC(O)R_{19}$ ;

$R_{15}$, $R_{16}$ et $R_{17}$ représentent chacun, indépendamment, un atome d'hydrogène ou l'un des groupes mentionnés à propos de W, ou $R_{15}$ représente un atome d'hydrogène et les entités représentées par $R_{16}$ et $R_{17}$ constituent, conjointement avec les atomes de carbone adjacents auxquelles elles sont liées, un cycle dioxolanyle ;

$R_{19}$ représente un atome d'hydrogène ou un groupe alkyle inférieur, phényle ou phényle-(alkyle inférieur) ;

et $R_{20}$ et $R_{21}$ représentent chacun, indépendamment, une entité choisie parmi les groupes phényle, phényle à substituant(s) W, naphtyle, naphtyle à substituant(s) W, indanyle, indényle, tétrahydronaphtyle, benzodioxolyle, hétéroaryle, hétéroaryle à substituant(s) W, hétéroaryle benzocondensé, hétéroaryle benzocondensé à substituant(s) W, et cyclopropyle, le terme "hétéroaryle" ayant la définition indiquée plus haut ;

en vue de la fabrication d'un médicament conçu pour réguler la production ou le taux d'au moins un peptide β-amyloïde chez un sujet.

**58.** Emploi d'une composition comprenant au moins un composé représenté par la formule (VI) :

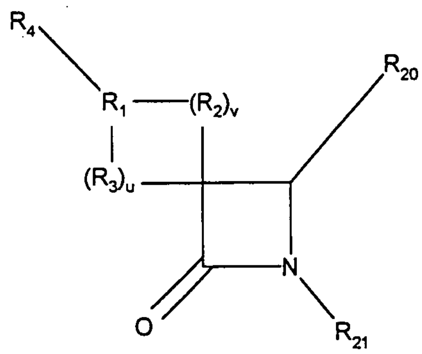

(VI)

ou un sel pharmacologiquement admissible d'un tel composé, ou un solvat d'un tel composé ou sel, dans laquelle formule :

$R_1$ représente >CH-, >C(alkyle inférieur)-, >CF-, >C(OH)-, $>C(C_6H_5)-$, $>C(C_6H_4-R^{15})-$, >N- ou $>^{+}N-O-$ ;

$R_2$ et $R_3$ représentent chacun, indépendamment, un chaînon $-CH_2-$, -CH(alkyle inférieur)- ou $-C(\text{alkyle inférieur})_2-$, ou un groupe -CH=CH- ou -C(alkyle inférieur)=CH- ;

ou bien l'entité représentée par $R_1$ constitue, conjointement avec une entité adjacente représentée par $R_2$ ou par $R_3$, un groupe -CH=CH-
ou -CH=C(alkyle inférieur)- ;

u et v valent chacun, indépendamment, 0, 1, 2 ou 3, sous réserve qu'ils ne vaillent pas tous deux 0, que v vaille 1 si $R_2$ représente un groupe -CH=CH- ou -C(alkyle inférieur)=CH-, et que u vaille 1 si $R_3$ représente un groupe -CH=CH- ou -C(alkyle inférieur)=CH-, et étant entendu que, si v vaut 2 ou 3, les entités représentées par les symboles $R_2$ peuvent être identiques ou différentes, et que, si u vaut 2 ou 3, les entités représentées par les symboles $R_3$ peuvent être identiques ou différentes ;

$R_4$ représente une entité choisie parmi
$B-(CH_2)_mC(O)-$ où m vaut 0, 1, 2, 3, 4 ou 5,
$B-(CH_2)_q-$ où q vaut 0, 1, 2, 3, 4, 5 ou 6,
$B-(CH_2)_e-Z-(CH_2)_r-$ où Z représente -O-, -C(O)-, un groupe phénylène, $-N(R_8)-$ ou $-S(O)_{0-2}-$, e vaut 0, 1, 2, 3, 4 ou 5, et r vaut 0, 1, 2, 3, 4 ou
5, sous réserve que la somme e+r vaille 0, 1, 2, 3, 4, 5 ou 6,
B-(alcénylène en $C_{2-6}$)-,
B-(alcadiénylène en $C_{4-6}$)-,
$B-(CH_2)_t-Z-$(alcénylène en $C_{2-6}$)-, où Z a la signification indiquée ci-dessus, et t vaut 0, 1, 2 ou 3, sous réserve que la somme de t et du nombre d'atomes de carbone de la chaîne alcénylène vaille 2, 3, 4, 5 ou 6,

$B-(CH_2)_f-V-(CH_2)_g-$, où V représente un groupe cycloalkylène en $C_{3-6}$,

f vaut 1, 2, 3, 4 ou 5, et g vaut 0, 1, 2, 3, 4 ou 5, sous réserve que la somme f+g vaille 1, 2, 3, 4, 5 ou 6,

$B-(CH_2)_t-V-$(alcénylène en $C_{2-6}$)- ou B-(alcénylène en $C_{2-6}$)-$V-(CH_2)_t-$,

où V et t ont les significations indiquées ci-dessus, sous réserve que la somme de t et du nombre d'atomes de carbone de la chaîne alcénylène vaille 2, 3, 4, 5 ou 6,

$B-(CH_2)_a-Z-(CH_2)_b-V-(CH_2)_d-$, où Z et V ont les significations indiquées ci-dessus et a, b et d valent chacun, indépendamment, 0, 1, 2, 3, 4, 5 ou 6, sous réserve que la somme a+b+d vaille 0, 1, 2, 3, 4, 5 ou 6, ou $T-(CH_2)_s-$, où T représente un groupe cycloalkyle comportant 3 à 6 atomes de carbone et s vaut 0, 1, 2, 3, 4, 5 ou 6 ;

ou $R_1$ et $R_4$ représentent ensemble un groupe B-CH=C< ;

B représente une entité choisie parmi les groupes indanyle, indényle, naphtyle, tétrahydronaphtyle, hétéroaryle, ou hétéroaryle à substituant(s) W, le groupe hétéroaryle étant choisi parmi les groupes pyrrolyle, pyridinyle, pyrimidinyle, pyrazinyle, triazinyle, imidazolyle, thiazolyle, pyrazolyle, thiényle, oxazolyle et furanyle, y compris, dans le cas des groupes hétéroaryles azotés, les N-oxydes de ces groupes, ou un groupe de formule

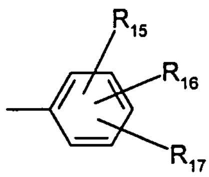 ;

W représente 1 à 3 substituants choisis indépendamment dans l'ensemble formé par les atomes d'halogène et les groupes alkyle inférieur, hydroxyalkyle inférieur, alcoxy inférieur, alcoxyalkyle, alcoxyalcoxy, alcoxycarbonylalcoxy, (alcoxy inférieur)-imino-alkyle inférieur, alcanedioyle inférieur, (alkyle inférieur)-alcanedioyle inférieur, allyloxy, $-CF_3$, $-OCF_3$, benzyle, $R_7$-benzyle, benzyloxy, $R_7$-benzyloxy, phénoxy, $R_7$-phénoxy, dioxolanyle, $-NO_2$, $-N(R_8)(R_9)$, $(R_8)(R_9)N$-(alkylène inférieur)-, $(R_8)(R_9)N$-(alkylène-oxy inférieur), -OH, -CN, $-N_3$, $-NHC(O)OR_{10}$, $-NHC(O)R_{10}$, $R_{11}O_2SNH-$, $(R_{11}O_2S)_2N-$, $-SO_2NH_2$, $S(O)_{0-2}R_8$, tertiobutyldiméthyl-silyloxyméthyl, $-C(O)R_{12}$, $-COOR_{19}$, $-CON(R_8)(R_9)$, $-CH=CHC(O)R_{12}$, -(alkylène inférieur)-$C(O)R_{12}$, $R_{10}C(O)$-(alkylène-oxy inférieur)-, $(R_8)(R_9)NC(O)$-(alkylène-oxy inférieur)-, et

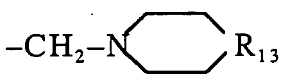

pour les substituants placés sur un atome de carbone, les substituants placés sur un atome d'azote d'un cycle hétéroaryle, s'il y en a, étant choisis parmi les groupes alkyle inférieur, alcoxy inférieur, $-C(O)OR_{10}$, $-C(O)R_{10}$, -OH, $(R_8)(R_9)N$-(alkylène inférieur)-, $(R_8)(R_9)N$-(alkylène-oxy inférieur), $-SO_2NH_2$ et 2-(triméthylsilyl)-éthoxyméthyl ;

$R_7$ représente 1 à 3 substituants, indépendamment choisis dans l'ensemble formé par les groupes alkyle inférieur, alcoxy inférieur, -COOH, $-NO_2$, $-N(R_8)(R_9)$ et -OH et les atomes d'halogène ;

$R_8$ et $R_9$ représentent chacun, indépendamment, un atome d'hydrogène ou un groupe alkyle inférieur ;

$R_{10}$ représente un groupe alkyle inférieur, phényle, $R_7$-phényle, benzyle ou $R_7$-benzyle ;

$R_{11}$ représente un groupe -OH, alkyle inférieur, phényle, $R_7$-phényle, benzyle ou $R_7$-benzyle ;

$R_{12}$ représente un atome d'hydrogène ou un groupe -OH, alcoxy, phénoxy, benzyloxy,

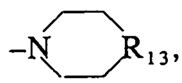,

$-N(R_8)(R_9)$, alkyle inférieur, phényle ou $R_7$-phényle ;

$R_{13}$ représente -O-, $-CH_2-$, -NH-, -N(alkyle inférieur) ou $-NC(O)R_{19}$ ;

$R_{15}$, $R_{16}$ et $R_{17}$ représentent chacun, indépendamment, un atome d'hydrogène ou l'un des groupes mentionnés à propos de W, ou $R_{15}$ représente un atome d'hydrogène et les entités représentées par $R_{16}$ et $R_{17}$ constituent, conjointement avec les atomes de carbone adjacents auxquelles elles sont liées, un cycle dioxolanyle ;

$R_{19}$ représente un atome d'hydrogène ou un groupe alkyle inférieur, phényle ou phényle-(alkyle inférieur) ;

et $R_{20}$ et $R_{21}$ représentent chacun, indépendamment, une entité choisie parmi les groupes phényle, phényle à substituant(s) W, naphtyle, naphtyle à substituant(s) W, indanyle, indényle, tétrahydronaphtyle, benzodioxolyle,

hétéroaryle, hétéroaryle à substituant(s) W, hétéroaryle benzocondensé, hétéroaryle benzocondensé à substituant(s) W, et cyclopropyle, le terme "hétéroaryle" ayant la définition indiquée plus haut ;

en vue de la fabrication d'un médicament conçu pour réguler la quantité de l'isoforme 4 de l'apolipoprotéine E dans la circulation sanguine et/ou le cerveau d'un sujet.

**59.** Emploi d'une composition comprenant au moins un composé représenté par la formule (VII) :

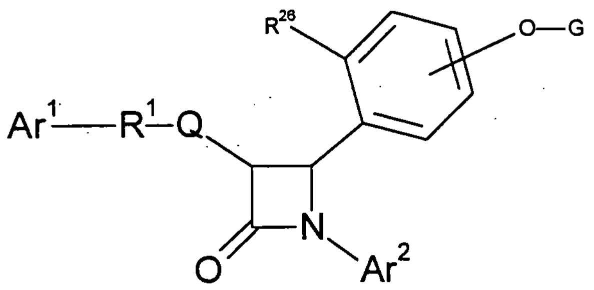

(VII)

ou un sel pharmacologiquement admissible d'un tel composé, ou un solvat d'un tel composé ou sel, dans laquelle formule (VII) :

$R^{26}$ représente un atome d'hydrogène ou un groupe $-OG^1$ ;
G et $G^1$ représentent chacun, indépendamment, une entité choisie dans l'ensemble formé par un atome d'hydrogène et les groupes de formules

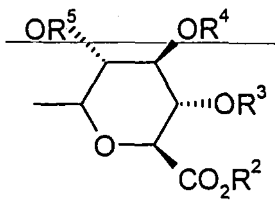

,

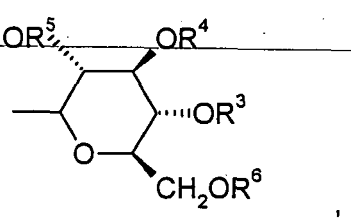

,

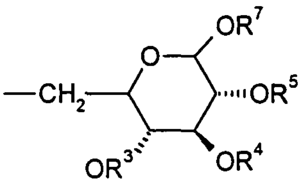

,

et

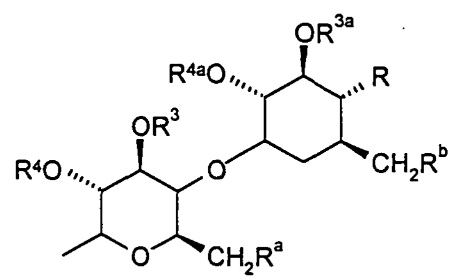

sous réserve que, si $R^{26}$ représente un atome d'hydrogène ou un groupe -OH, G ne représente pas un atome d'hydrogène ;
R, $R^a$ et $R^b$ représentent chacun, indépendamment, une entité choisie parmi les atomes d'hydrogène et d'halogène et les groupes -OH, $-NH_2$, azido, (alcoxy en $C_{1-6}$)-alcoxy en $C_{1-6}$ et $-W-R^{30}$ ;
W représente indépendamment une entité choisie parmi -NH-C(O)-, -O-C(O)-, $-O-C(O)-N(R^{31})-$, $-NH-C(O)-N(R^{31})-$ et $-O-C(S)-N(R^{31})-$ ; $R^2$ et $R^6$ représentent chacun, indépendamment, un atome d'hydrogène ou un groupe alkyle en $C_{1-6}$, aryle ou aryl-(alkyle en $C_{1-6}$) ;
$R^3$, $R^4$, $R^5$, $R^7$, $R^{3a}$ et $R^{4a}$ représentent chacun, indépendamment, un atome d'hydrogène ou un groupe alkyle en $C_{1-6}$, aryl-(alkyle en $C_{1-6}$), -C(O)-(alkyle en $C_{1-6}$) ou -C(O)-aryle ;
$R^{30}$ représente une entité choisie parmi les groupes T à substituant(s) $R^{32}$, (T à substituant(s) $R^{32}$)-(alkyle en $C_{1-6}$), alcényle en $C_{2-4}$ à substituant(s) $R^{32}$, alkyle en $C_{1-6}$ à substituant(s) $R^{32}$, cycloalkyle en $C_{3-7}$ à substituant(s) $R^{32}$, et (cycloalkyle en $C_{3-7}$ à substituant(s) $R^{32}$)-alkyle en $C_{1-6}$ ;
$R^{31}$ représente un atome d'hydrogène ou un groupe alkyle en $C_{1-4}$ ;

T représente une entité choisie parmi les groupes phényle, furyle, thiényle, pyrrolyle, oxazolyle, isoxazolyle, thiazolyle, isothiazolyle, benzothiazolyle, thiadiazolyle, pyrazolyle, imidazolyle et pyridyle ;
$R^{32}$ représente indépendamment 1 à 3 substituants choisis indépendamment parmi les substituants halogéno, alkyle en $C_{1-4}$, hydroxy, phénoxy, $-CF_3$,- $NO_2$, alcoxy en $C_{1-4}$, méthylènedioxy, oxo, alkylsulfanyle en $C_{1-4}$, alkylsulfinyle en $C_{1-4}$, alkylsulfonyle en $C_{1-4}$, $-N(CH_3)_2$, -C(O)-NH(alkyle en $C_{1-4}$), -C(O)-N(alkyle en $C_{1-4}$)$_2$, -C (O)-(alkyle en $C_{1-4}$), -C(O)-(alcoxy en $C_{1-4}$) et pyrrolidinyl-carbonyle ;
ou bien $R^{32}$ représente une liaison covalente qui forme, avec l'entité représentée par $R^{31}$ et l'atome d'azote auquel est lié celle-ci, un groupe pyrrolidinyle, pipéridinyle, N-méthyl-pipérazinyle, indolinyle ou morpholinyle, ou un groupe pyrrolidinyle, pipéridinyle, N-méthyl-pipérazinyle, indolinyle ou morpholinyle portant, dans chaque cas, un substituant (alcoxy en $C_{1-4}$)-carbonyle ;
$Ar^1$ représente un groupe aryle ou aryle à substituant(s) $R^{10}$ ;
$Ar^2$ représente un groupe aryle ou aryle à substituant(s) $R^{11}$ ;
Q représente une liaison ou un groupe formant, avec l'atome de carbone en position 3 du cycle azétidinone, un groupe spiro de formule

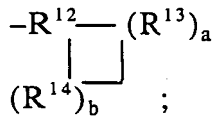

$R^1$ représente un groupe choisi parmi les suivants :

- $-(CH_2)_q-$ où q vaut de 2 à 6, et peut aussi valoir 0 ou 1 si Q représente un groupe spiro,
- $-(CH_2)_e-E-(CH_2)_r-$où E représente un groupe phénylène ou un chaînon
- -O-, -C(O)-, $-NR^{22}-$ ou -S $(O)_{0-2}$, e vaut de 0 à 5 et r vaut de 0 à 5, sous réserve que la somme e+r vaille de 1 à 6, alcénylène en $C_{2-6}$,

et $-(CH_2)_f-V-(CH_2)_g-$ où V représente un groupe cycloalkylène en $C_{3-6}$,
f vaut de 1 à 5 et g vaut de 0 à 5, sous réserve que la somme f+g vaille de 1 à 6,
$R^{12}$ représente un chaînon choisi parmi :

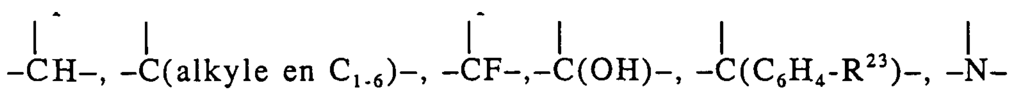

et

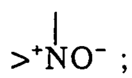

$R^{13}$ et $R^{14}$ représentent chacun, indépendamment, un chaînon $-CH_2-$, -CH(alkyle en $C_{1-6}$)- ou -C(alkyle en $C_{1-6}$)$_2$-, ou un groupe -CH=CHou -C(alkyle en $C_{1-6}$)=CH- ;
ou bien l'entité représentée par $R^{12}$ constitue, conjointement avec une entité adjacente représentée par $R^{13}$ ou par $R^{14}$, un groupe -CH=CHou -C(alkyle en $C_{1-6}$)=CH- ;
a et b valent chacun, indépendamment, 0, 1, 2 ou 3, sous réserve qu'ils ne vaillent pas tous deux 0, que a vaille 1 si $R^{13}$ représente un groupe -CH=CH- ou -C(alkyle en $C_{1-6}$)=CH-, et que b vaille 1 si $R^{14}$ représente un groupe -CH=CH- ou -C(alkyle en $C_{1-6}$)=CH-, et étant entendu que, si a vaut 2 ou 3, les entités représentées par les symboles $R^{13}$ peuvent être identiques ou différentes, et que, si b vaut 2 ou 3, les entités représentées par les symboles $R^{14}$ peuvent être identiques ou différentes ;
étant entendu que, si Q représente une liaison, $R^1$ peut aussi représenter un groupe de formule

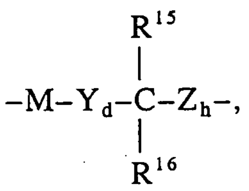 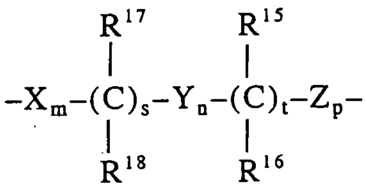 ou 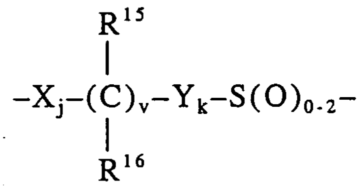

M représente un chaînon -O-, -S-, -S(O)- ou $-S(O)_2-$ ;
X, Y et Z représentent chacun, indépendamment, un chaînon $-CH_2-$, -CH(alkyle en $C_{1-6}$)- ou -C(alkyle en $C_{1-6})_2-$ ;
$R^{10}$ et $R^{11}$ représentent chacun, indépendamment, 1 à 3 substituants choisis indépendamment dans l'ensemble constitué par les groupes alkyle en $C_{1-6}$, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$,$-COR^{19}$, $-SO_2NR^{19}R^{20}$, $-S(O)_{0-2}R^{21}$, $-O(CH_2)_{1-10}COOR^{19}$, $-O(CH_2)_{1-10}CONR^{19}R^{20}$, -(alkylène en $C_{1-6}$)-$COOR^{19}$, $-CH=CH-COOR^{19}$, $-CF_3$, -CN et $-NO_2$, et les atomes d'halogène ;
$R^{15}$ et $R^{17}$ représentent chacun, indépendamment, un groupe de formule $-OR^{19}$, $-O(CO)R^{19}$, $-O(CO)OR^{21}$ ou $-O(CO)NR^{19}R^{20}$ ;
$R^{16}$ et $R^{18}$ représentent chacun, indépendamment, un atome d'hydrogène ou un groupe alkyle en $C_{1-6}$ ou aryle ;
ou $R^{15}$ et $R^{16}$ représentent ensemble un substituant oxo =O ;
ou $R^{17}$ et $R^{18}$ représentent ensemble un substituant oxo =O ;
d vaut 1, 2 ou 3 ;
h vaut 0, 1, 2, 3 ou 4 ;
s vaut 0 ou 1, t vaut 0 ou 1, et m, n et p valent chacun, indépendamment, de 0 à 4, sous réserve qu'au moins l'un de s et t vaille 1 et que la somme m+n+p+s+t vaille de 1 à 6, sous réserve que, si p vaut 0 et t vaut 1, la somme m+s+n vaille de 1 à 5, et sous réserve que, si p vaut 0 et s vaut 1, la somme m+t+n vaille de 1 à 5 ;
v vaut 0 ou 1 ;
j et k valent chacun, indépendamment, de 1 à 5, sous réserve que la somme j+k+v vaille de 1 à 5 ;
et si Q représente une liaison et $R^1$ représente un groupe de formule

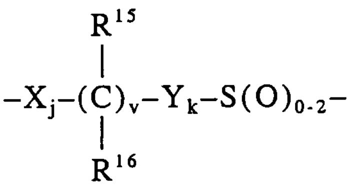 ,

$Ar^1$ peut aussi représenter un groupe pyridyle, isoxazolyle, furanyle, pyrrolyle, thiényle, imidazolyle, pyrazolyle, thiazolyle, pyrazinyle, pyrimidinyle ou pyridazinyle ;
$R^{19}$ et $R^{20}$ représentent chacun, indépendamment, un atome d'hydrogène ou un groupe alkyle en $C_{1-6}$, aryle ou aryl-(alkyle en $C_{1-6}$),
$R^{21}$ représente un groupe alkyle en $C_{1-6}$, aryle ou aryle à substituant(s) $R^{24}$ ;
$R^{22}$ représente un atome d'hydrogène ou un groupe alkyle en $C_{1-6}$, aryl-(alkyle en $C_{1-6}$), $-C(O)R^{19}$ ou $-COOR^{19}$ ;
$R^{23}$ et $R^{24}$ représentent chacun, indépendamment, 1 à 3 substituants choisis indépendamment dans l'ensemble constitué par les atomes d'hydrogène et d'halogène et les groupes alkyle en $C_{1-6}$, alcoxy en $C_{1-6}$, -COOH, $-NO_2$, $-NR^{19}R^{20}$ et - OH ;
et $R^{25}$ représente un atome d'hydrogène ou un groupe hydroxy ou alcoxy en $C_{1-6}$ ;

en vue de la fabrication d'un médicament destiné au traitement, à la prévention ou à l'amélioration des symptômes de la maladie d'Alzheimer chez un sujet.

**60.** Emploi d'une composition comprenant au moins un composé représenté par la formule (VII) :

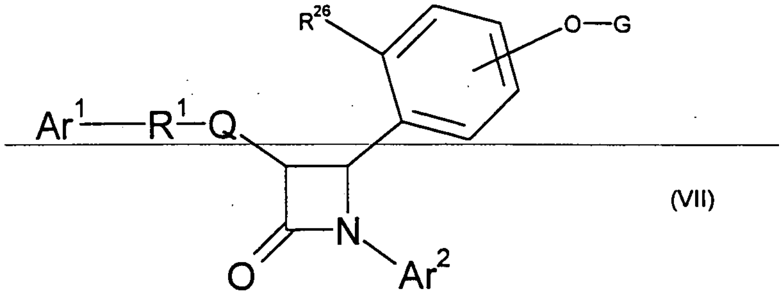

(VII)

ou un sel pharmacologiquement admissible d'un tel composé, ou un solvat d'un tel composé ou sel, dans laquelle formule (VII) :

$R^{26}$ représente un atome d'hydrogène ou un groupe $-OG^1$ ;
G et $G^1$ représentent chacun, indépendamment, une entité choisie dans l'ensemble formé par un atome d'hydrogène et les groupes de formules

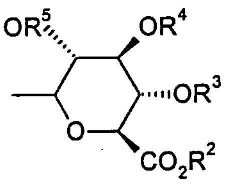 ,
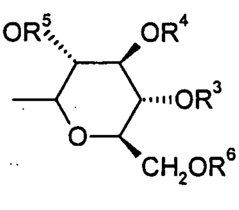 ,

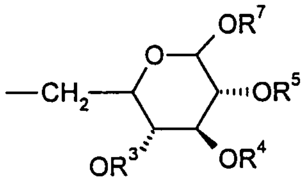 , et 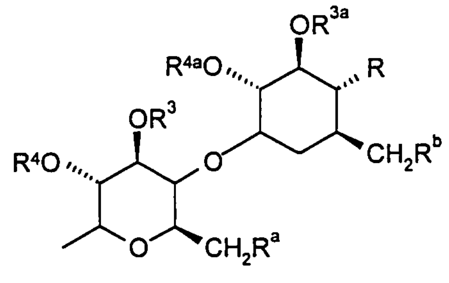

sous réserve que, si $R^{26}$ représente un atome d'hydrogène ou un groupe -OH, G ne représente pas un atome d'hydrogène ;
R, $R^a$ et $R^b$ représentent chacun, indépendamment, une entité choisie parmi les atomes d'hydrogène et d'halogène et les groupes -OH, $-NH_2$, azido, (alcoxy en $C_{1-6}$)-alcoxy en $C_{1-6}$ et $-W-R^{30}$;
W représente indépendamment une entité choisie parmi -NH-C(O)-, -O-C(O)-, $-O-C(O)-N(R^{31})-$, $-NH-C(O)-N(R^{31})-$ et $-O-C(S)-N(R^{31})-$; $R^2$ et $R^6$ représentent chacun, indépendamment, un atome d'hydrogène ou un groupe alkyle en $C_{1-6}$, aryle ou aryl-(alkyle en $C_{1-6}$) ;
$R^3$, $R^4$, $R^5$, $R^7$, $R^{3a}$ et $R^{4a}$ représentent chacun, indépendamment, un atome d'hydrogène ou un groupe alkyle en $C_{1-6}$, aryl-(alkyle en $C_{1-6}$), -C(O)-(alkyle en $C_{1-6}$) ou -C(O)-aryle ;
$R^{30}$ représente une entité choisie parmi les groupes T à substituant(s) $R^{32}$, (T à substituant(s) $R^{32}$)-(alkyle en $C_{1-6}$), alcényle en $C_{2-4}$ à substituant(s) $R^{32}$, alkyle en $C_{1-6}$ à substituant(s) $R^{32}$, cycloalkyle en $C_{3-7}$ à substituant (s) $R^{32}$, et (cycloalkyle en $C_{3-7}$ à substituant(s) $R^{32}$)-alkyle en $C_{1-6}$ ;
$R^{31}$ représente un atome d'hydrogène ou un groupe alkyle en $C_{1-4}$ ;
T représente une entité choisie parmi les groupes phényle, furyle, thiényle, pyrrolyle, oxazolyle, isoxazolyle, thiazolyle, isothiazolyle, benzothiazolyle, thiadiazolyle, pyrazolyle, imidazolyle et pyridyle ;
$R^{32}$ représente indépendamment 1 à 3 substituants choisis indépendamment parmi les substituants halogéno, alkyle en $C_{1-4}$, hydroxy, phénoxy, $-CF_3$, $-NO_2$, alcoxy en $C_{1-4}$, méthylènedioxy, oxo, alkylsulfanyle en $C_{1-4}$, alkylsulfinyle en $C_{1-4}$, alkylsulfonyle en $C_{1-4}$, $-N(CH_3)_2$, -C(O)-NH(alkyle en $C_{1-4}$), -C(O)-N(alkyle en $C_{1-4}$)$_2$, -C(O)-(alkyle en $C_{1-4}$), -C(O)-(alcoxy en $C_{1-4}$) et pyrrolidinyl-carbonyle ;

ou bien $R^{32}$ représente une liaison covalente qui forme, avec l'entité représentée par $R^{31}$ et l'atome d'azote auquel est lié celle-ci, un groupe pyrrolidinyle, pipéridinyle, N-méthyl-pipérazinyle, indolinyle ou morpholinyle, ou un groupe pyrrolidinyle, pipéridinyle, N-méthyl-pipérazinyle, indolinyle ou morpholinyle portant, dans chaque cas, un substituant (alcoxy en $C_{1-4}$)-carbonyle ;
$Ar^1$ représente un groupe aryle ou aryle à substituant(s) $R^{10}$ ;
$Ar^2$ représente un groupe aryle ou aryle à substituant(s) $R^{11}$ ;
Q représente une liaison ou un groupe formant, avec l'atome de carbone en position 3 du cycle azétidinone, un groupe spiro de formule

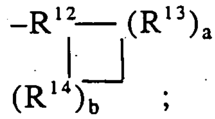

$R^1$ représente un groupe choisi parmi les suivants :

- $-(CH_2)_q-$ où q vaut de 2 à 6, et peut aussi valoir 0 ou 1 si Q représente un groupe spiro,
- $-(CH_2)_e-E-(CH_2)_r-$ où E représente un groupe phénylène ou un chaînon
- -O-, -C(O)-, $-NR^{22}-$ ou $-S(O)_{0-2}$, e vaut de 0 à 5 et r vaut de 0 à 5, sous réserve que la somme e+r vaille de 1 à 6,

alcénylène en $C_{2-6}$,
et $-(CH_2)_f-V-(CH_2)_g-$ où V représente un groupe cycloalkylène en $C_{3-6}$,
f vaut de 1 à 5 et g vaut de 0 à 5, sous réserve que la somme f+g vaille de 1 à 6,
$R^{12}$ représente un chaînon choisi parmi :

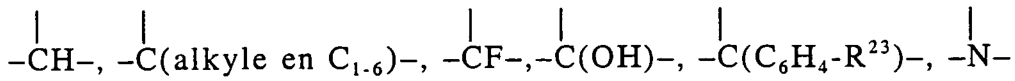

et

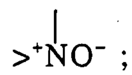

$R^{13}$ et $R^{14}$ représentent chacun, indépendamment, un chaînon $-CH_2-$, -CH(alkyle en $C_{1-6}$)- ou -C(alkyle en $C_{1-6})_2$-, ou un groupe -CH=CHou -C(alkyle en $C_{1-6}$)=CH- ;
ou bien l'entité représentée par $R^{12}$ constitue, conjointement avec une entité adjacente représentée par $R^{13}$ ou par $R^{14}$, un groupe -CH=CHou -C(alkyle en $C_{1-6}$)=CH- ;
a et b valent chacun, indépendamment, 0, 1, 2 ou 3, sous réserve qu'ils ne vaillent pas tous deux 0, que a vaille 1 si $R^{13}$ représente un groupe -CH=CH- ou -C(alkyle en $C_{1-6}$)=CH-, et que b vaille 1 si $R^{14}$ représente un groupe -CH=CH- ou -C(alkyle en $C_{1-6}$)=CH-, et étant entendu que, si a vaut 2 ou 3, les entités représentées par les symboles $R^{13}$ peuvent être identiques ou différentes, et que, si b vaut 2 ou 3, les entités représentées par les symboles $R^{14}$ peuvent être identiques ou différentes ;
étant entendu que, si Q représente une liaison, $R^1$ peut aussi représenter un groupe de formule

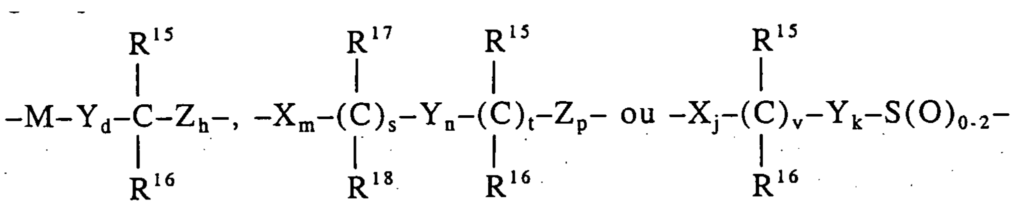

M représente un chaînon -O-, -S-, -S(O)- ou $-S(O)_2-$ ;
X, Y et Z représentent chacun, indépendamment, un chaînon $-CH_2-$, -CH(alkyle en $C_{1-6}$)- ou -C(alkyle en $C_{1-6})_2$- ;
$R^{10}$ et $R^{11}$ représentent chacun, indépendamment, 1 à 3 substituants choisis indépendamment dans l'ensemble

constitué par les groupes alkyle en $C_{1-6}$, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $-S(O)_{0-2}R^{21}$, $-O(CH_2)_{1-10}COOR^{19}$,- $O(CH_2)_{1-10}CONR^{19}R^{20}$, -(alkylène en $C_{1-6}$)-$COOR^{19}$, $-CH=CH-COOR^{19}$, $-CF_3$, -CN et $-NO_2$, et les atomes d'halogène ;

$R^{15}$ et $R^{17}$ représentent chacun, indépendamment, un groupe de formule $-OR^{19}$, $-O(CO)R^{19}$, $-O(CO)OR^{21}$ ou $-O(CO)NR^{19}R^{20}$ ;

$R^{16}$ et $R^{18}$ représentent chacun, indépendamment, un atome d'hydrogène ou un groupe alkyle en $C_{1-6}$ ou aryle ;

ou $R^{15}$ et $R^{16}$ représentent ensemble un substituant oxo =O ;

ou $R^{17}$ et $R^{18}$ représentent ensemble un substituant oxo =O ;

d vaut 1, 2 ou 3 ;

h vaut 0, 1, 2, 3 ou 4 ;

s vaut 0 ou 1, t vaut 0 ou 1, et m, n et p valent chacun, indépendamment, de 0 à 4, sous réserve qu'au moins l'un de s et t vaille 1 et que la somme m+n+p+s+t vaille de 1 à 6, sous réserve que, si p vaut 0 et t vaut 1, la somme m+s+n vaille de 1 à 5, et sous réserve que, si p vaut 0 et s vaut 1, la somme m+t+n vaille de 1 à 5 ;

v vaut 0 ou 1 ;

j et k valent chacun, indépendamment, de 1 à 5, sous réserve que la somme j+k+v vaille de 1 à 5 ;

et si Q représente une liaison et $R^1$ représente un groupe de formule

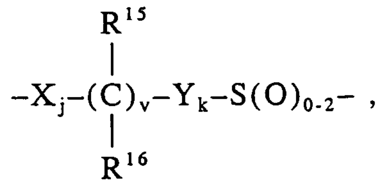

Ar$^1$ peut aussi représenter un groupe pyridyle, isoxazolyle, furanyle, pyrrolyle, thiényle, imidazolyle, pyrazolyle, thiazolyle, pyrazinyle, pyrimidinyle ou pyridazinyle ;

$R^{19}$ et $R^{20}$ représentent chacun, indépendamment, un atome d'hydrogène ou un groupe alkyle en $C_{1-6}$, aryle ou aryl-(alkyle en $C_{1-6}$),

$R^{21}$ représente un groupe alkyle en $C_{1-6}$, aryle ou aryle à substituant(s) $R^{24}$ ; $R^{22}$ représente un atome d'hydrogène ou un groupe alkyle en $C_{1-6}$, aryl-(alkyle en $C_{1-6}$), $-C(O)R^{19}$ ou $-COOR^{19}$ ;

$R^{23}$ et $R^{24}$ représentent chacun, indépendamment, 1 à 3 substituants choisis indépendamment dans l'ensemble constitué par les atomes d'hydrogène et d'halogène et les groupes alkyle en $C_{1-6}$, alcoxy en $C_{1-6}$, -COOH, $-NO_2$, $-NR^{19}R^{20}$ et - OH ;

et $R^{25}$ représente un atome d'hydrogène ou un groupe hydroxy ou alcoxy en $C_{1-6}$;

en vue de la fabrication d'un médicament conçu pour réguler la production ou le taux d'au moins un peptide β-amyloïde chez un sujet.

**61.** Emploi d'une composition comprenant au moins un composé représenté par la formule (VII) :

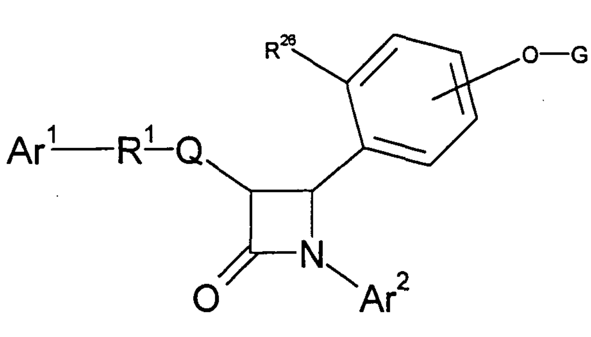

(VII)

ou un sel pharmacologiquement admissible d'un tel composé, ou un solvat d'un tel composé ou sel, dans laquelle formule (VII) :

$R^{26}$ représente un atome d'hydrogène ou un groupe $-OG^1$ ;

G et $G^1$ représentent chacun, indépendamment, une entité choisie dans l'ensemble formé par un atome d'hydrogène et les groupes de formules

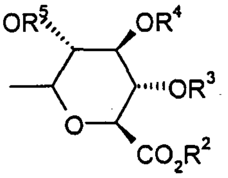
,
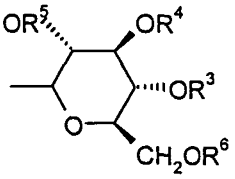
,

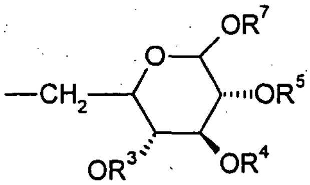
,
et
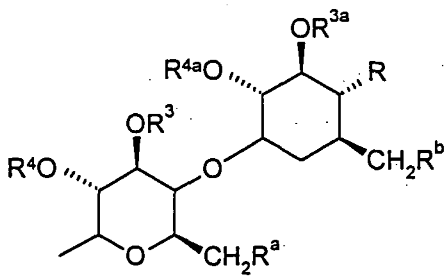

sous réserve que, si $R^{26}$ représente un atome d'hydrogène ou un groupe -OH, G ne représente pas un atome d'hydrogène ;
R, $R^a$ et $R^b$ représentent chacun, indépendamment, une entité choisie parmi les atomes d'hydrogène et d'halogène et les groupes -OH, $-NH_2$, azido, (alcoxy en $C_{1-6}$)-alcoxy en $C_{1-6}$ et $-W-R^{30}$ ;
W représente indépendamment une entité choisie parmi -NH-C(O)-, -O-C(O)-, $-O-C(O)-N(R^{31})-$, $-NH-C(O)-N(R^{31})-$ et $-O-C(S)-N(R^{31})-$ ; $R^2$ et $R^6$ représentent chacun, indépendamment, un atome d'hydrogène ou un groupe alkyle en $C_{1-6}$, aryle ou aryl-(alkyle en $C_{1-6}$) ;
$R^3$, $R^4$, $R^5$, $R^7$, $R^{3a}$ et $R^{4a}$ représentent chacun, indépendamment, un atome d'hydrogène ou un groupe alkyle en $C_{1-6}$, aryl-(alkyle en $C_{1-6}$), -C(O)-(alkyle en $C_{1-6}$) ou -C(O)-aryle ;
$R^{30}$ représente une entité choisie parmi les groupes T à substituant(s) $R^{32}$, (T à substituant(s) $R^{32}$)-(alkyle en $C_{1-6}$), alcényle en $C_{2-4}$ à substituant(s) $R^{32}$, alkyle en $C_{1-6}$ à substituant(s) $R^{32}$, cycloalkyle en $C_{3-7}$ à substituant (s) $R^{32}$, et (cycloalkyle en $C_{3-7}$ à substituant(s) $R^{32}$)-alkyle en $C_{1-6}$ ;
$R^{31}$ représente un atome d'hydrogène ou un groupe alkyle en $C_{1-4}$ ;
T représente une entité choisie parmi les groupes phényle, furyle, thiényle, pyrrolyle, oxazolyle, isoxazolyle, thiazolyle, isothiazolyle, benzothiazolyle, thiadiazolyle, pyrazolyle, imidazolyle et pyridyle ;
$R^{32}$ représente indépendamment 1 à 3 substituants choisis indépendamment parmi les substituants halogéno, alkyle en $C_{1-4}$, hydroxy, phénoxy, $-CF_3$, $-NO_2$, alcoxy en $C_{1-4}$, méthylènedioxy, oxo, alkylsulfanyle en $C_{1-4}$, alkylsulfinyle en $C_{1-4}$, alkylsulfonyle en $C_{1-4}$, $-N(CH_3)_2$, -C(O)-NH(alkyle en $C_{1-4}$), -C(O)-N(alkyle en $C_{1-4}$)$_2$, -C (O)-(alkyle en $C_{1-4}$), -C(O)-(alcoxy en $C_{1-4}$) et pyrrolidinyl-carbonyle ;
ou bien $R^{32}$ représente une liaison covalente qui forme, avec l'entité représentée par $R^{31}$ et l'atome d'azote auquel est lié celle-ci, un groupe pyrrolidinyle, pipéridinyle, N-méthyl-pipérazinyle, indolinyle ou morpholinyle, ou un groupe pyrrolidinyle, pipéridinyle, N-méthyl-pipérazinyle, indolinyle ou morpholinyle portant, dans chaque cas, un substituant (alcoxy en $C_{1-4}$)-carbonyle ;
$Ar^1$ représente un groupe aryle ou aryle à substituant(s) $R^{10}$ ;
$Ar^2$ représente un groupe aryle ou aryle à substituant(s) $R^{11}$ ;
Q représente une liaison ou un groupe formant, avec l'atome de carbone en position 3 du cycle azétidinone, un groupe spiro de formule

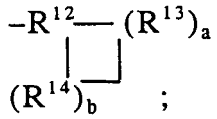
;

$R^1$ représente un groupe choisi parmi les suivants :

- $(CH_2)_q$- où q vaut de 2 à 6, et peut aussi valoir 0 ou 1 si Q représente un groupe spiro,
- $(CH_2)_e$-E-$(CH_2)_r$- où E représente un groupe phénylène ou un chaînon
- -O-, -C(O)-, -$NR^{22}$- ou -$S(O)_{0-2}$, e vaut de 0 à 5 et r vaut de 0 à 5, sous réserve que la somme e+r vaille de 1 à 6,

alcénylène en $C_{2-6}$,
et -$(CH_2)_f$-V-$(CH_2)_g$- où V représente un groupe cycloalkylène en $C_{3-6}$,
f vaut de 1 à 5 et g vaut de 0 à 5, sous réserve que la somme f+g vaille de 1 à 6,
$R^{12}$ représente un chaînon choisi parmi :

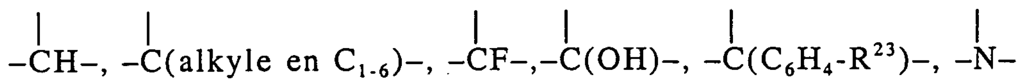

et

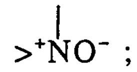

$R^{13}$ et $R^{14}$ représentent chacun, indépendamment, un chaînon -$CH_2$-, -CH(alkyle en $C_{1-6}$)- ou -C(alkyle en $C_{1-6})_2$-, ou un groupe -CH=CHou -C(alkyle en $C_{1-6}$)=CH- ;
ou bien l'entité représentée par $R^{12}$ constitue, conjointement avec une entité adjacente représentée par $R^{13}$ ou par $R^{14}$, un groupe -CH=CHou -C(alkyle en $C_{1-6}$)=CH- ;
a et b valent chacun, indépendamment, 0, 1, 2 ou 3, sous réserve qu'ils ne vaillent pas tous deux 0, que a vaille 1 si $R^{13}$ représente un groupe -CH=CH- ou -C(alkyle en $C_{1-6}$)=CH-, et que b vaille 1 si $R^{14}$ représente un groupe -CH=CH- ou -C(alkyle en $C_{1-6}$)=CH-, et étant entendu que, si a vaut 2 ou 3, les entités représentées par les symboles $R^{13}$ peuvent être identiques ou différentes, et que, si b vaut 2 ou 3, les entités représentées par les symboles $R^{14}$ peuvent être identiques ou différentes ;
étant entendu que, si Q représente une liaison, $R^1$ peut aussi représenter un groupe de formule

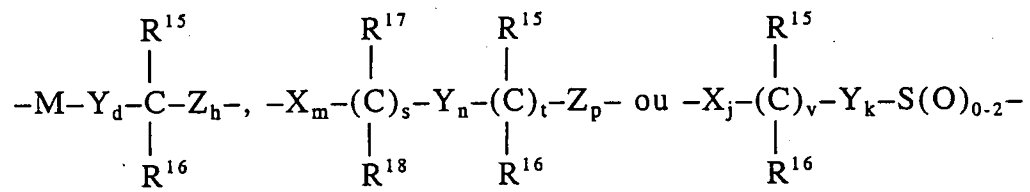

M représente un chaînon -O-, -S-, -S(O)- ou -$S(O)_2$- ;
X, Y et Z représentent chacun, indépendamment, un chaînon -$CH_2$-, -CH(alkyle en $C_{1-6}$)- ou -C(alkyle en $C_{1-6})_2$- ;
$R^{10}$ et $R^{11}$ représentent chacun, indépendamment, 1 à 3 substituants choisis indépendamment dans l'ensemble constitué par les groupes alkyle en $C_{1-6}$, -$OR^{19}$, -O(CO)$R^{19}$, -O(CO)$OR^{21}$, -O$(CH_2)_{1-5}OR^{19}$, -O(CO)$NR^{19}R^{20}$, -$NR^{19}R^{20}$, -$NR^{19}$(CO)$R^{20}$, -$NR^{19}$(CO)$OR^{21}$, -$NR^{19}$(CO)$NR^{20}R^{25}$, -$NR^{19}SO_2R^{21}$, -$COOR^{19}$, -$CONR^{19}R^{20}$, -$COR^{19}$, -$SO_2NR^{19}R^{20}$, -$S(O)_{0-2}R^{21}$, -O$(CH_2)_{1-10}COOR^{19}$, -O$(CH_2)_{1-10}$CO-$NR^{19}R^{20}$, -(alkylène en $C_{1-6}$)-$COOR^{19}$, -CH=CH-$COOR^{19}$, -$CF_3$, -CN et -$NO_2$, et les atomes d'halogène ;
$R^{15}$ et $R^{17}$ représentent chacun, indépendamment, un groupe de formule -$OR^{19}$, -O(CO)$R^{19}$, -O(CO)$OR^{21}$ ou -O(CO)$NR^{19}R^{20}$ ;
$R^{16}$ et $R^{18}$ représentent chacun, indépendamment, un atome d'hydrogène ou un groupe alkyle en $C_{1-6}$ ou aryle ;
ou $R^{15}$ et $R^{16}$ représentent ensemble un substituant oxo =O ;
ou $R^{17}$ et $R^{18}$ représentent ensemble un substituant oxo =O ;
d vaut 1, 2 ou 3 ;
h vaut 0, 1, 2, 3 ou 4 ;
s vaut 0 ou 1, t vaut 0 ou 1, et m, n et p valent chacun, indépendamment, de 0 à 4, sous réserve qu'au moins l'un de s et t vaille 1 et que la somme m+n+p+s+t vaille de 1 à 6, sous réserve que, si p vaut 0 et t vaut 1, la somme m+s+n vaille de 1 à 5, et sous réserve que, si p vaut 0 et s vaut 1, la somme m+t+n vaille de 1 à 5 ;
v vaut 0 ou 1 ;

j et k valent chacun, indépendamment, de 1 à 5, sous réserve que la somme j+k+v vaille de 1 à 5 ;
et si Q représente une liaison et $R^1$ représente un groupe de formule

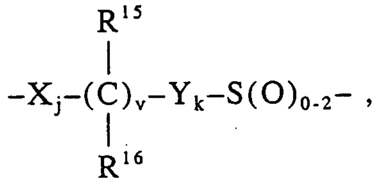

Ar[1] peut aussi représenter un groupe pyridyle, isoxazolyle, furanyle, pyrrolyle, thiényle, imidazolyle, pyrazolyle, thiazolyle, pyrazinyle, pyrimidinyle ou pyridazinyle ;
$R^{19}$ et $R^{20}$ représentent chacun, indépendamment, un atome d'hydrogène ou un groupe alkyle en $C_{1-6}$, aryle ou aryl-(alkyle en $C_{1-6}$),
$R^{21}$ représente un groupe alkyle en $C_{1-6}$, aryle ou aryle à substituant(s) $R^{24}$ ;
$R^{22}$ représente un atome d'hydrogène ou un groupe alkyle en $C_{1-6}$, aryl-(alkyle en $C_{1-6}$), -C(O)$R^{19}$ ou -COO$R^{19}$ ;
$R^{23}$ et $R^{24}$ représentent chacun, indépendamment, 1 à 3 substituants choisis indépendamment dans l'ensemble constitué par les atomes d'hydrogène et d'halogène et les groupes alkyle en $C_{1-6}$, alcoxy en $C_{1-6}$, -COOH, $-NO_2$, $-NR^{19}R^{20}$ et - OH ;
et $R^{25}$ représente un atome d'hydrogène ou un groupe hydroxy ou alcoxy en $C_{1-6}$ ;

en vue de la fabrication d'un médicament conçu pour réguler la quantité de l'isoforme 4 de l'apolipoprotéine E dans la circulation sanguine et/ou le cerveau d'un sujet.

**62.** Emploi d'une composition comprenant au moins un composé représenté par la formule (VIII) :

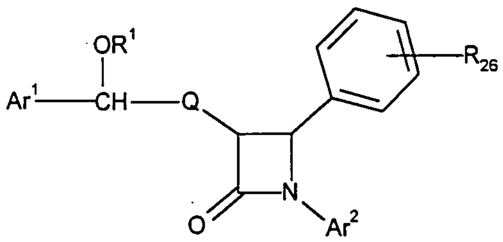

(VIII)

ou un sel pharmacologiquement admissible d'un tel composé, ou un solvat d'un tel composé ou sel,
dans laquelle formule :

$R^{26}$ représente une entité choisie parmi

a) un groupe -OH,
b) un groupe -OCH3,
c) un atome de fluor,
d) et un atome de chlore ;

$R^1$ représente une entité choisie dans l'ensemble formé par un atome d'hydrogène, les groupes de formules

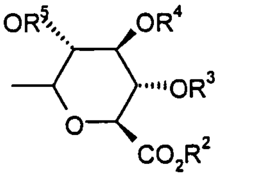

,

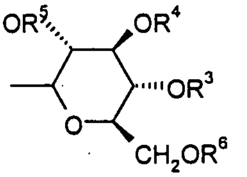

,

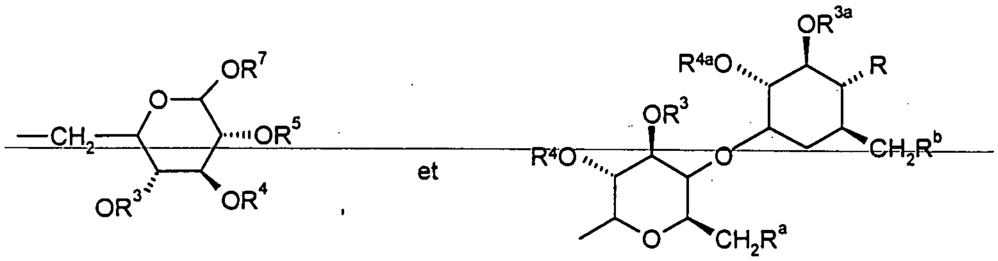

le groupe sulfo $-SO_3H$, et les restes d'acides aminés, naturels ou non-naturels ;

R, $R^a$ et $R^b$ représentent chacun, indépendamment, une entité choisie parmi les atomes d'hydrogène et d'halogène et les groupes -OH, $-NH_2$, azido, (alcoxy en $C_{1-6}$)-alcoxy en $C_{1-6}$ et $-W-R^{30}$ ;

W représente indépendamment une entité choisie parmi -NH-C(O)-, -O-C(O)-, $-O-C(O)-N(R^{31})-$, $-NH-C(O)-N(R^{31})-$ et $-O-C(S)-N(R^{31})-$ ;

$R^2$ et $R^6$ représentent chacun, indépendamment, un atome d'hydrogène ou un groupe alkyle en $C_{1-6}$, aryle ou aryl-(alkyle en $C_{1-6}$) ;

$R^3$, $R^4$, $R^5$, $R^7$, $R^{3a}$ et $R^{4a}$ représentent chacun, indépendamment, un atome d'hydrogène ou un groupe alkyle en $C_{1-6}$, aryl-(alkyle en $C_{1-6}$), -C(O)-(alkyle en $C_{1-6}$) ou -C(O)-aryle ;

$R^{30}$ représente indépendamment une entité choisie parmi les groupes T à substituant(s) $R^{32}$, (T à substituant(s) $R^{32}$)-(alkyle en $C_{1-6}$), alcényle en $C_{2-4}$ à substituant(s) $R^{32}$, alkyle en $C_{1-6}$ à substituant(s) $R^{32}$, cycloalkyle en $C_{3-7}$ à substituant(s) $R^{32}$, et (cycloalkyle en $C_{3-7}$ à substituant(s) $R^{32}$)-alkyle en $C_{1-6}$ ;

$R^{31}$ représente indépendamment un atome d'hydrogène ou un groupe alkyle en $C_{1-4}$ ;

T représente une entité choisie parmi les groupes phényle, furyle, thiényle, pyrrolyle, oxazolyle, isoxazolyle, thiazolyle, isothiazolyle, benzothiazolyle, thiadiazolyle, pyrazolyle, imidazolyle et pyridyle ;

$R^{32}$ représente indépendamment 1 à 3 substituants choisis indépendamment parmi les atomes d'hydrogène et d'halogène et les substituants alkyle en $C_{1-4}$, hydroxy, phénoxy, $-CF_3$, $-NO_2$, alcoxy en $C_{1-4}$, méthylènedioxy, oxo, alkylsulfanyle en $C_{1-4}$, alkylsulfinyle en $C_{1-4}$, alkylsulfonyle en $C_{1-4}$, $-N(CH_3)_2$, -C(O)-NH(alkyle en $C_{1-4}$), -C(O)-N(alkyle en $C_{1-4})_2$, -C(O)-(alkyle en $C_{1-4}$), -C(O)-(alcoxy en $C_{1-4}$) et pyrrolidinyl-carbonyle ;

ou bien $R^{32}$ représente une liaison covalente qui forme, avec l'entité représentée par $R^{31}$ et l'atome d'azote auquel est lié celle-ci, un groupe pyrrolidinyle, pipéridinyle, N-méthyl-pipérazinyle, indolinyle ou morpholinyle, ou un groupe pyrrolidinyle, pipéridinyle, N-méthyl-pipérazinyle, indolinyle ou morpholinyle portant, dans chaque cas, un substituant (alcoxy en $C_{1-4}$)-carbonyle;

$Ar^1$ représente un groupe aryle ou aryle à substituant(s) $R^{10}$ ;

$Ar^2$ représente un groupe aryle ou aryle à substituant(s) $R^{11}$ ;

Q représente un groupe $-(CH_2)q-$ où q vaut de 2 à 6, ou un groupe formant, avec l'atome de carbone en position 3 du cycle azétidinone, un groupe spiro de formule

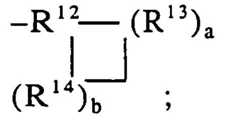 ;

$R^{12}$ représente un chaînon choisi parmi :

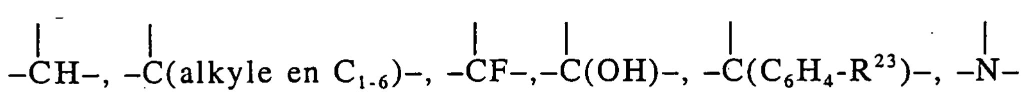

et

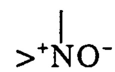 ;

$R^{13}$ et $R^{14}$ représentent chacun, indépendamment, un chaînon $-CH_2-$, -CH(alkyle en $C_{1-6}$)- ou -C(alkyle en

$C_{1-6})_2$-, ou un groupe -CH=CH- ou -C(alkyle en $C_{1-6}$)=CH- ;
ou bien l'entité représentée par $R^{12}$ constitue, conjointement avec une entité adjacente représentée par $R^{13}$ ou par $R^{14}$, un groupe -CH=CH- ou -C(alkyle en $C_{1-6}$)=CH- ;
a et b valent chacun, indépendamment, 0, 1, 2 ou 3, sous réserve qu'ils ne vaillent pas tous deux 0, que a vaille 1 si $R^{13}$ représente un groupe -CH=CH- ou -C(alkyle en $C_{1-6}$)=CH-, et que b vaille 1 si $R^{14}$ représente un groupe -CH=CH- ou -C(alkyle en $C_{1-6}$)=CH-, et étant entendu que, si a vaut 2 ou 3, les entités représentées par les symboles $R^{13}$ peuvent être identiques ou différentes, et que, si b vaut 2 ou 3, les entités représentées par les symboles $R^{14}$ peuvent être identiques ou différentes ;
$R^{10}$ et $R^{11}$ représentent chacun, indépendamment, 1 à 3 substituants choisis indépendamment dans l'ensemble constitué par les groupes alkyle en $C_{1-6}$, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$,- $NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $-S(O)_{0-2}R^{21}$, $-O(CH_2)_{1-10}COOR^{19}$, $-O(CH_2)_{1-10}CONR^{19}R^{20}$, -(alkylène en $C_{1-6}$)-$COOR^{19}$, -CH=CH-$COOR^{19}$, $-CF_3$, -CN et $-NO_2$, et les atomes d'halogène ;
$R^{19}$ et $R^{20}$ représentent chacun, indépendamment, un atome d'hydrogène ou un groupe alkyle en $C_{1-6}$, aryle ou aryl-(alkyle en $C_{1-6}$),
$R^{21}$ représente un groupe alkyle en $C_{1-6}$, aryle ou aryle à substituant(s) $R^{24}$; $R^{22}$ représente un atome d'hydrogène ou un groupe alkyle en $C_{1-6}$, aryl-(alkyle en $C_{1-6}$), $-C(O)R^{19}$ ou $-COOR^{19}$ ;
$R^{23}$ et $R^{24}$, représentent chacun, indépendamment, 1 à 3 substituants choisis indépendamment dans l'ensemble constitué par les atomes d'hydrogène et d'halogène et les groupes alkyle en $C_{1-6}$, alcoxy en $C_{1-6}$, -COOH, $-NO_2$, $-NR^{19}R^{20}$ et - OH ;
et $R^{25}$ représente un atome d'hydrogène ou un groupe hydroxy ou alcoxy en $C_{1-6}$ ;

en vue de la fabrication d'un médicament destiné au traitement, à la prévention ou à l'amélioration des symptômes de la maladie d'Alzheimer chez un sujet.

**63.** Emploi d'une composition comprenant au moins un composé représenté par la formule (VIII) :

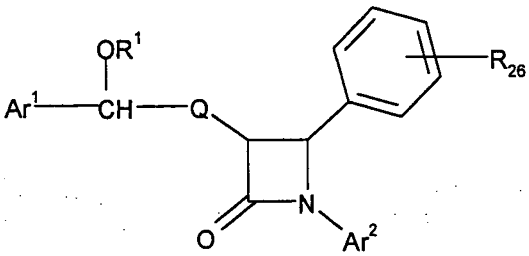

(VIII)

ou un sel pharmacologiquement admissible d'un tel composé, ou un solvat d'un tel composé ou sel, dans laquelle formule :

$R^{26}$ représente une entité choisie parmi

a) un groupe -OH,
b) un groupe -OCH3,
c) un atome de fluor,
d) et un atome de chlore ;

$R^1$ représente une entité choisie dans l'ensemble formé par un atome d'hydrogène, les groupes de formules

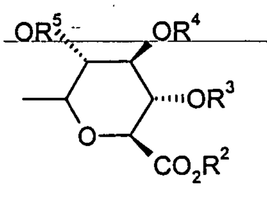

,

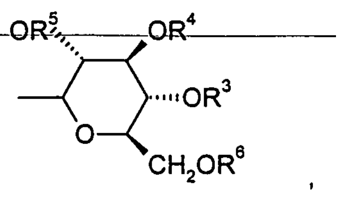

,

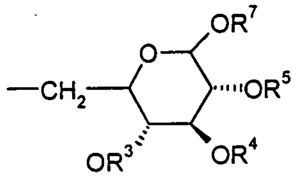

,

et

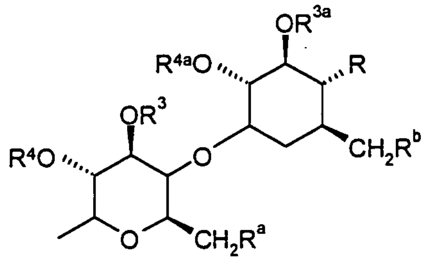

le groupe sulfo $-SO_3H$, et les restes d'acides aminés, naturels ou non-naturels ;
R, $R^a$ et $R^b$ représentent chacun, indépendamment, une entité choisie parmi les atomes d'hydrogène et d'halogène et les groupes -OH, $-NH_2$, azido, (alcoxy en $C_{1-6}$)-alcoxy en $C_{1-6}$ et $-W-R^{30}$ ;
W représente indépendamment une entité choisie parmi -NH-C(O)-, -O-C(O)-, $-O-C(O)-N(R^{31})-$, $-NH-C(O)-N(R^{31})-$ et $-O-C(S)-N(R^{31})-$ ; $R^2$ et $R^6$ représentent chacun, indépendamment, un atome d'hydrogène ou un groupe alkyle en $C_{1-6}$, aryle ou aryl-(alkyle en $C_{1-6}$) ;
$R^3$, $R^4$, $R^5$, $R^7$, $R^{3a}$ et $R^{4a}$ représentent chacun, indépendamment, un atome d'hydrogène ou un groupe alkyle en $C_{1-6}$, aryl-(alkyle en $C_{1-6}$), -C(O)-(alkyle en $C_{1-6}$) ou -C(O)-aryle ;
$R^{30}$ représente indépendamment une entité choisie parmi les groupes T à substituant(s) $R^{32}$, (T à substituant(s) $R^{32}$)-(alkyle en $C_{1-6}$), alcényle en $C_{2-4}$ à substituant(s) $R^{32}$, alkyle en $C_{1-6}$ à substituant(s) $R^{32}$, cycloalkyle en $C_{3-7}$ à substituant(s) $R^{32}$, et (cycloalkyle en $C_{3-7}$ à substituant(s) $R^{32}$)-alkyle en $C_{1-6}$ ;
$R^{31}$ représente indépendamment un atome d'hydrogène ou un groupe alkyle en $C_{1-4}$ ;
T représente une entité choisie parmi les groupes phényle, furyle, thiényle, pyrrolyle, oxazolyle, isoxazolyle, thiazolyle, isothiazolyle, benzothiazolyle, thiadiazolyle, pyrazolyle, imidazolyle et pyridyle ;
$R^{32}$ représente indépendamment 1 à 3 substituants choisis indépendamment parmi les atomes d'hydrogène et d'halogène et les substituants alkyle en $C_{1-4}$, hydroxy, phénoxy, $-CF_3$, $-NO_2$, alcoxy en $C_{1-4}$, méthylènedioxy, oxo, alkylsulfanyle en $C_{1-4}$, alkylsulfinyle en $C_{1-4}$, alkylsulfonyle en $C_{1-4}$, $-N(CH_3)_2$, -C(O)-NH(alkyle en $C_{1-4}$), -C(O)-N(alkyle en $C_{1-4}$)$_2$, -C(O)-(alkyle en $C_{1-4}$), -C(O)-(alcoxy en $C_{1-4}$) et pyrrolidinyl-carbonyle ;
ou bien $R^{32}$ représente une liaison covalente qui forme, avec l'entité représentée par $R^{31}$ et l'atome d'azote auquel est lié celle-ci, un groupe pyrrolidinyle, pipéridinyle, N-méthyl-pipérazinyle, indolinyle ou morpholinyle, ou un groupe pyrrolidinyle, pipéridinyle, N-méthyl-pipérazinyle, indolinyle ou morpholinyle portant, dans chaque cas, un substituant (alcoxy en $C_{1-4}$)-carbonyle ;
$Ar^1$ représente un groupe aryle ou aryle à substituant(s) $R^{10}$ ;
$Ar^2$ représente un groupe aryle ou aryle à substituant(s) $R^{11}$ ;
Q représente un groupe $-(CH_2)_q-$ où q vaut de 2 à 6, ou un groupe formant, avec l'atome de carbone en position 3 du cycle azétidinone, un groupe spiro de formule

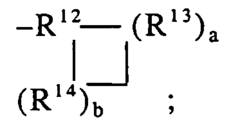 ;

$R^{12}$ représente un chaînon choisi parmi :

$$-CH-,\ -C(\text{alkyle en } C_{1-6})-,\ -CF-,\ -C(OH)-,\ -C(C_6H_4-R^{23})-,\ -N-$$

et

$$>^{+}NO^{-}\ ;$$

$R^{13}$ et $R^{14}$ représentent chacun, indépendamment, un chaînon $-CH_2-$, -CH(alkyle en $C_{1-6}$)- ou -C(alkyle en $C_{1-6}$)$_2$-, ou un groupe -CH=CH- ou -C(alkyle en $C_{1-6}$)=CH- ;
ou bien l'entité représentée par $R^{12}$ constitue, conjointement avec une entité adjacente représentée par $R^{13}$ou

par $R^{14}$, un groupe -CH=CH- ou -C(alkyle en $C_{1-6}$)=CH- ;

a et b valent chacun, indépendamment, 0, 1, 2 ou 3, sous réserve qu'ils ne vaillent pas tous deux 0, que a vaille 1 si $R^{13}$ représente un groupe -CH=CH- ou -C(alkyle en $C_{1-6}$)=CH-, et que b vaille 1 si $R^{14}$ représente un groupe -CH=CH- ou -C(alkyle en $C_{1-6}$)=CH-, et étant entendu que, si a vaut 2 ou 3, les entités représentées par les symboles $R^{13}$ peuvent être identiques ou différentes, et que, si b vaut 2 ou 3, les entités représentées par les symboles $R^{14}$ peuvent être identiques ou différentes ;

$R^{10}$ et $R^{11}$ représentent chacun, indépendamment, 1 à 3 substituants choisis indépendamment dans l'ensemble constitué par les groupes alkyle en $C_{1-6}$, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $-S(O)_{0-2}R^{21}$, $-O(CH_2)_{1-10}COOR^{19}$, $-O(CH_2)_{1-10}CO-NR^{19}R^{20}$, -(alkylène en $C_{1-6}$)-$COOR^{19}$, $-CH=CH-COOR^{19}$, $-CF_3$, -CN et $-NO_2$, et les atomes d'halogène ;

$R^{19}$ et $R^{20}$ représentent chacun, indépendamment, un atome d'hydrogène ou un groupe alkyle en $C_{1-6}$, aryle ou aryl-(alkyle en $C_{1-6}$),

$R^{21}$ représente un groupe alkyle en $C_{1-6}$, aryle ou aryle à substituant(s) $R^{24}$ ; $R^{22}$ représente un atome d'hydrogène ou un groupe alkyle en $C_{1-6}$, aryl-(alkyle en $C_{1-6}$), $-C(O)R^{19}$ ou $-COOR^{19}$ ;

$R^{23}$ et $R^{24}$ représentent chacun, indépendamment, 1 à 3 substituants choisis indépendamment dans l'ensemble constitué par les atomes d'hydrogène et d'halogène et les groupes alkyle en $C_{1-6}$, alcoxy en $C_{1-6}$, -COOH, $-NO_2$, $-NR^{19}R^{20}$ et - OH ;

et $R^{25}$ représente un atome d'hydrogène ou un groupe hydroxy ou alcoxy en $C_{1-6}$ ;

en vue de la fabrication d'un médicament conçu pour réguler la production ou le taux d'au moins un peptide β-amyloïde chez un sujet.

**64.** Emploi d'une composition comprenant au moins un composé représenté par la formule (VIII) :

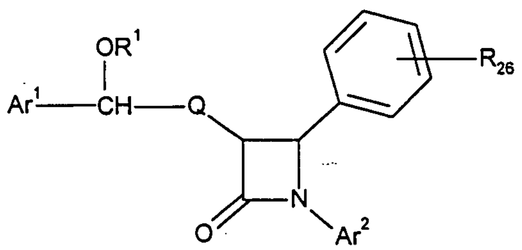

(VIII)

ou un sel pharmacologiquement admissible d'un tel composé, ou un solvat d'un tel composé ou sel, dans laquelle formule :

$R^{26}$ représente une entité choisie parmi

a) un groupe -OH,
b) un groupe -OCH3,
c) un atome de fluor,
d) et un atome de chlore ;

$R^1$ représente une entité choisie dans l'ensemble formé par un atome d'hydrogène, les groupes de formules

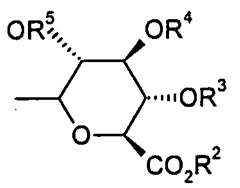 , 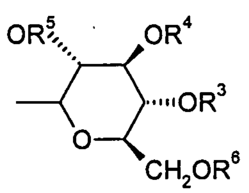 ,

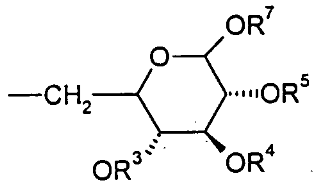 , et 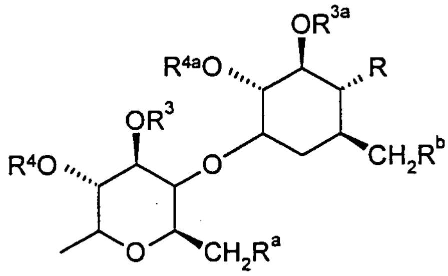

le groupe sulfo $-SO_3H$, et les restes d'acides aminés, naturels ou non-naturels ;
R, $R^a$ et $R^b$ représentent chacun, indépendamment, une entité choisie parmi les atomes d'hydrogène et d'halogène et les groupes -OH, $-NH_2$, azido, (alcoxy en $C_{1-6}$)-alcoxy en $C_{1-6}$ et $-W-R^{30}$ ;
W représente indépendamment une entité choisie parmi -NH-C(O)-, -O-C(O)-, $-O-C(O)-N(R^{31})-$, $-NH-C(O)-N(R^{31})-$ et $-O-C(S)-N(R^{31})-$ ;
$R^2$ et $R^6$ représentent chacun, indépendamment, un atome d'hydrogène ou un groupe alkyle en $C_{1-6}$, aryle ou aryl-(alkyle en $C_{1-6}$) ;
$R^3$, $R^4$, $R^5$, $R^7$, $R^{3a}$ et $R^{4a}$ représentent chacun, indépendamment, un atome d'hydrogène ou un groupe alkyle en $C_{1-6}$, aryl-(alkyle en $C_{1-6}$), -C(O)-(alkyle en $C_{1-6}$) ou -C(O)-aryle ;
$R^{30}$ représente indépendamment une entité choisie parmi les groupes T à substituant(s) $R^{32}$, (T à substituant(s) $R^{32}$)-(alkyle en $C_{1-6}$), alcényle en $C_{2-4}$ à substituant(s) $R^{32}$, alkyle en $C_{1-6}$ à substituant(s) $R^{32}$, cycloalkyle en $C_{3-7}$ à substituant(s) $R^{32}$, et (cycloalkyle en $C_{3-7}$ à substituant(s) $R^{32}$)-alkyle en $C_{1-6}$ ;
$R^{31}$ représente indépendamment un atome d'hydrogène ou un groupe alkyle en $C_{1-4}$ ;
T représente une entité choisie parmi les groupes phényle, furyle, thiényle, pyrrolyle, oxazolyle, isoxazolyle, thiazolyle, isothiazolyle, benzothiazolyle, thiadiazolyle, pyrazolyle, imidazolyle et pyridyle ;
$R^{32}$ représente indépendamment 1 à 3 substituants choisis indépendamment parmi les atomes d'hydrogène et d'halogène et les substituants alkyle en $C_{1-4}$, hydroxy, phénoxy, $-CF_3$, $-NO_2$, alcoxy en $C_{1-4}$, méthylènedioxy, oxo, alkylsulfanyle en $C_{1-4}$, alkylsulfinyle en $C_{1-4}$, alkylsulfonyle en $C_{1-4}$, $-N(CH_3)_2$, -C(O)-NH(alkyle en $C_{1-4}$), -C(O)-N(alkyle en $C_{1-4}$)$_2$, -C(O)-(alkyle en $C_{1-4}$), -C(O)-(alcoxy en $C_{1-4}$) et pyrrolidinyl-carbonyle ;
ou bien $R^{32}$ représente une liaison covalente qui forme, avec l'entité représentée par $R^{31}$ et l'atome d'azote auquel est lié celle-ci, un groupe pyrrolidinyle, pipéridinyle, N-méthyl-pipérazinyle, indolinyle ou morpholinyle, ou un groupe pyrrolidinyle, pipéridinyle, N-méthyl-pipérazinyle, indolinyle ou morpholinyle portant, dans chaque cas, un substituant (alcoxy en $C_{1-4}$)-carbonyle ;
$Ar^1$ représente un groupe aryle ou aryle à substituant(s) $R^{10}$ ;
$Ar^2$ représente un groupe aryle ou aryle à substituant(s) $R^{11}$ ;
Q représente un groupe $-(CH_2)_q-$ où q vaut de 2 à 6, ou un groupe formant, avec l'atome de carbone en position 3 du cycle azétidinone, un groupe spiro de formule

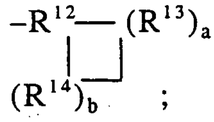 ;

$R^{12}$ représente un chaînon choisi parmi :

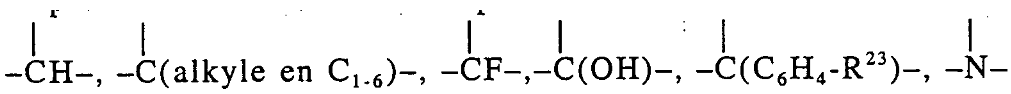

et

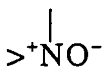 ;

$R^{13}$ et $R^{14}$ représentent chacun, indépendamment, un chaînon $-CH_2-$, -CH(alkyle en $C_{1-6}$)- ou -C(alkyle en

$C_{1-6})_2$-, ou un groupe -CH=CHou -C(alkyle en $C_{1-6}$)=CH- ;

ou bien l'entité représentée par $R^{12}$ constitue, conjointement avec une entité adjacente représentée par $R^{13}$ ou par $R^{14}$, un groupe -CH=CH- ou -C(alkyle en $C_{1-6}$)=CH- ;

a et b valent chacun, indépendamment, 0, 1, 2 ou 3, sous réserve qu'ils ne vaillent pas tous deux 0, que a vaille 1 si $R^{13}$ représente un groupe -CH=CH- ou -C(alkyle en $C_{1-6}$)=CH-, et que b vaille 1 si $R^{14}$ représente un groupe -CH=CH- ou -C(alkyle en $C_{1-6}$)=CH-, et étant entendu que, si a vaut 2 ou 3, les entités représentées par les symboles $R^{13}$ peuvent être identiques ou différentes, et que, si b vaut 2 ou 3, les entités représentées par les symboles $R^{14}$ peuvent être identiques ou différentes ;

$R^{10}$ et $R^{11}$ représentent chacun, indépendamment, 1 à 3 substituants choisis indépendamment dans l'ensemble constitué par les groupes alkyle en $C_{1-6}$, $-OR^{19}$, $-O(CO)R^{19}$, $-O(CO)OR^{21}$, $-O(CH_2)_{1-5}OR^{19}$, $-O(CO)NR^{19}R^{20}$, $-NR^{19}R^{20}$, $-NR^{19}(CO)R^{20}$, $-NR^{19}(CO)OR^{21}$, $-NR^{19}(CO)NR^{20}R^{25}$, $-NR^{19}SO_2R^{21}$, $-COOR^{19}$, $-CONR^{19}R^{20}$, $-COR^{19}$, $-SO_2NR^{19}R^{20}$, $-S(O)_{0-2}R^{21}$, $-O(CH_2)_{1-10}COOR^{19}$, $-O(CH_2)_{1-10}CO-NR^{19}R^{20}$, -(alkylène en $C_{1-6}$)-$COOR^{19}$, $-CH=CH-COOR^{19}$, $-CF_3$, -CN et $-NO_2$, et les atomes d'halogène ;

$R^{19}$ et $R^{20}$ représentent chacun, indépendamment, un atome d'hydrogène ou un groupe alkyle en $C_{1-6}$, aryle ou aryl-(alkyle en $C_{1-6}$),

$R^{21}$ représente un groupe alkyle en $C_{1-6}$, aryle ou aryle à substituant(s) $R^{24}$ ;

$R^{22}$ représente un atome d'hydrogène ou un groupe alkyle en $C_{1-6}$, aryl-(alkyle en $C_{1-6}$), $-C(O)R^{19}$ ou $-COOR^{19}$ ;

$R^{23}$ et $R^{24}$ représentent chacun, indépendamment, 1 à 3 substituants choisis indépendamment dans l'ensemble constitué par les atomes d'hydrogène et d'halogène et les groupes alkyle en $C_{1-6}$, alcoxy en $C_{1-6}$, -COOH, $-NO_2$, $-NR^{19}R^{20}$ et - OH ;

et $R^{25}$ représente un atome d'hydrogène ou un groupe hydroxy ou alcoxy en $C_{1-6}$ ;

en vue de la fabrication d'un médicament conçu pour réguler la quantité de l'isoforme 4 de l'apolipoprotéine E dans la circulation sanguine et/ou le cerveau d'un sujet.